# EUROPEAN PATENT APPLICATION

(11) **EP 3 251 674 A2**
(43) Date of publication of application: **06.12.2017**
(21) Application number: 17162146.9
(22) Date of filing: 18.11.2013
(51) Int. Cl.: A61K 31/505, C07D 401/04, C07D 487/04

(54) **ANTIVIRAL AZASUGAR-CONTAINING NUCLEOSIDES**

(30) Priority: 16.11.2012 US 201261727468 P
(62) Divisional of application: 13855441.5
(71) Applicant: BioCryst Pharmaceuticals, Inc., Durham, NC 27703 (US)
(72) Inventor: BABU, Yarlagadda, S., Birmingham, AL 35244 (US); KOTIAN, Pravin, L, Birmingham, AL 35226 (US); BANTIA, Shanta, Birmingham, AL 35242 (US); WU, Minwan, Vestavia Hills, AL 35226 (US); KUMAR, Satish, V., Birmingham, AL 35244 (US)
(74) Representative: HGF Limited

(57) **Abstract**

Disclosed are compounds comprising an azasugar attached to a heterocyclic base, including pharmaceutically acceptable salts thereof, suitable for use in inhibiting viral RNA polymerase activity or viral replication, and treating viral infections. The compounds are characterized, in part, by favorable pharmacokinetics for the active pharmaceutical ingredient, particularly in conjunction with enteral administration, including, in particular, oral administration. Also disclosed are pharmaceutical compositions comprising one or more compounds mentioned above, or pharmaceutically acceptable salts thereof, as well as methods for preparing same. Also provided are methods for inhibiting viral RNA polymerase activity, viral replication, and treating viral infections.

## Description

### RELATED APPLICATIONS

This application claims the benefit of priority to United States Provisional Patent Application serial number 61/727,468, filed November 16, 2012.

### BACKGROUND OF THE INVENTION

Viruses are responsible for many infectious diseases in animals, including mammals and humans in particular. Unlike infections with bacteria, relatively few agents are effective for the prevention and treatment of viral infections. The biology of viral diseases is now well understood, including viral genome transcription, translation, and replication. In RNA-containing viruses an important enzyme is RNA-dependent RNA polymerase, which is responsible for viral genome replication. RNA-dependent RNA polymerase is an essential protein encoded in the genomes of all RNA-containing viruses with no DNA stage that have negative-sense RNA. The enzyme catalyzes synthesis of the RNA strand complementary to a given RNA template. Because replication of the virus depends on RNA polymerase, this enzyme is a promising target in the development of new anti-viral compounds.

### SUMMARY OF THE INVENTION

The invention provides compounds represented by the formula **R^{s}-B**, wherein R^{s} is an azasugar, and **B** is a heterocyclic base, including pharmaceutically acceptable salts thereof. The compounds are suitable for use in inhibiting viral RNA polymerase activity or viral replication, and treating viral infections. The compounds are characterized, in part, by favorable pharmacokinetics for the active pharmaceutical ingredient, particularly in conjunction with enteral administration, including, in particular, oral administration. The invention also provides pharmaceutical compositions comprising one or more compounds represented by formula **R^{s}-B**, or pharmaceutically acceptable salts thereof, as well as methods for preparing same. Also provided are methods for inhibiting viral RNA polymerase activity, viral replication, and treating viral infections.

### DETAILED DESCRIPTION OF THE INVENTION

One aspect of the invention relates to a compound represented by **R^{s}-B**, or a pharmaceutically acceptable salt thereof, wherein:
**R^{s}-B** is selected from the group consisting of: 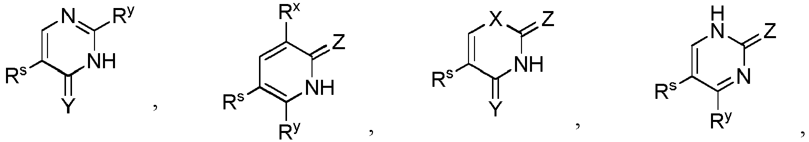 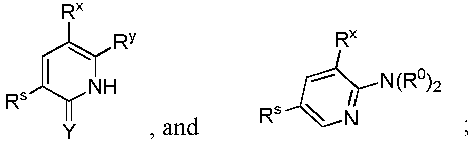
X is selected from the group consisting of O, S, and NR¹⁰;
   R¹⁰, independently for each occurrence, is selected from the group consisting of H, C₁-C₆alkyl, aryl, heteroaryl, aralkyl, and heteroaralkyl;
R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
R⁰, independently for each occurrence, is H or C₁-C₆alkyl;
R^{y} is selected from the group consisting of H, C₁-C₆alkyl, OR⁰, C₁-C₆alkenyl, C₁-C₆alkynyl, CN, N(R⁰)₂, and halide;
Y is selected from the group consisting of O and S;
Z is selected from the group consisting of O and S;
R^{s} is represented by the formula: 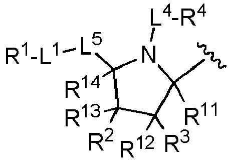
   L¹ and L⁴, each independently, are a bond or -C(R⁰)₂-O-;
   L⁵ is a bond, O, -C(R⁰)₂-O-, -C(R⁰)₂-S-, or -C(R⁰)₂-NH-;
   R¹ is selected from the group consisting of H, C₁-C₆alkyl, aminoacyl, aminothionyl, C₁-C₆acyl, R¹⁰OC(O)-, phosphoryl, and aminophosphoryl;
   R² and R³ are each independently H, halide, azide, C₁-C₆alkyl, C₁-C₆alkenyl, C₁-C₆alkynyl, CN, OR⁰, R¹⁰OC(O)-, or SR⁰;
      or R¹ and R², taken together, or R² and R³, taken together, may be selected from the group consisting of -OC(O)O-, -OC(S)O-, phosphoryl, and C₁-C₆alkylphosphoryl;
      or R² and R³, taken together, may form a bond between the carbon atoms to which they are attached;
   R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
   R¹¹ is selected from the group consisting of H, C₁-C₆alkyl, C₁-C₆alkenyl, C₁-C₆alkynyl, OR⁰, and CN;
   R¹² and R¹³ are each independently selected from the group consisting of H, C₁-C₆alkyl, OR⁰, C₁-C₆alkenyl, C₁-C₆alkynyl, CN, azide, and halide; and
   R¹⁴ is selected from the group consisting of H, C₁-C₆alkyl, OR⁰, C₁-C₆alkynyl, CN, azide, and halide.

One aspect of the invention relates to a compound represented by **R**^{s}**-B**, or a pharmaceutically acceptable salt thereof, wherein:
**R^{s}-B** is selected from the group consisting of: 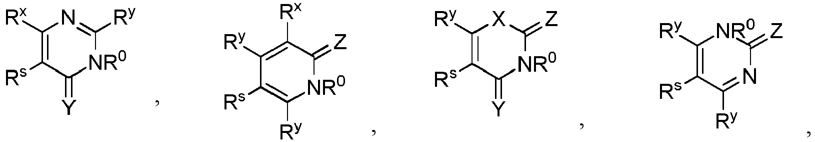 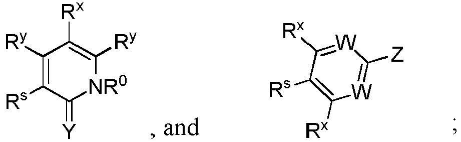
   W, independently for each occurrence, is CR^{x} or N;
   X is selected from the group consisting of O, S, and NR¹⁰;
      R¹⁰, independently for each occurrence, is selected from the group consisting of H, C₁-C₆alkyl, aryl, heteroaryl, aralkyl, and heteroaralkyl;
   R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
   R⁰, independently for each occurrence, is H or C₁-C₆alkyl;
   R^{y} is selected from the group consisting of H, C₁-C₆alkyl, OR⁰, C₁-C₆alkenyl, C₁-C₆alkynyl, CN, N(R⁰)₂, and halide;
   Y is selected from the group consisting of O and S;
   Z is selected from the group consisting of O and S;
   R^{s} is represented by the formula: 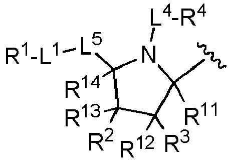
      L¹ and L⁴, each independently, are a bond or -C(R⁰)₂-O-;
      L⁵ is a bond, O, -C(R⁰)₂-O-, -C(R⁰)₂-S-, or -C(R⁰)₂-NH-;
      R¹ is selected from the group consisting of H, C₁-C₆alkyl, aminoacyl, aminothionyl, C₁-C₆acyl, R¹⁰OC(O)-, phosphoryl, and aminophosphoryl;
      R² and R³ are each independently H, halide, azide, C₁-C₆alkyl, C₁-C₆alkenyl, C₁-C₆alkynyl, CN, OR⁰, R¹⁰OC(O)-, or SR⁰;
         or R¹ and R², taken together, or R² and R³, taken together, may be selected from the group consisting of -OC(O)O-, -OC(S)O-, phosphoryl, and C₁-C₆alkylphosphoryl;
         or R² and R³, taken together, may form a bond between the carbon atoms to which they are attached;
      R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
      R¹¹ is selected from the group consisting of H, C₁-C₆alkyl, C₁-C₆alkenyl, C₁-C₆alkynyl, OR⁰, and CN;
      R¹² and R¹³ are each independently selected from the group consisting of H, C₁-C₆alkyl, C₁-C₆alkenyl, C₁-C₆alkynyl, OR⁰, CN, azide, and halide; and
      R¹⁴ is selected from the group consisting of H, C₁-C₆alkyl, OR⁰, C₁-C₆alkynyl, CN, azide, and halide.

One aspect of the invention relates to a compound represented by **R^{s}-B**, or a pharmaceutically acceptable salt thereof, wherein:
**R^{s}-B** is selected from the group consisting of: 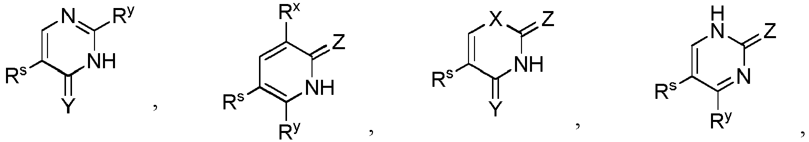 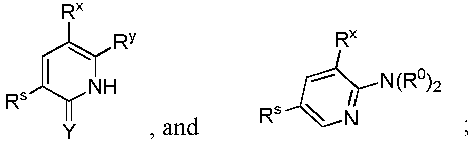
   X is selected from the group consisting of O, S, and NR¹⁰;
   R¹⁰, independently for each occurrence, is selected from the group consisting of H, C₁-C₆alkyl, aryl, heteroaryl, aralkyl, and heteroaralkyl;
   R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
   R⁰, independently for each occurrence, is H or C₁-C₆alkyl;
   R^{y} is selected from the group consisting of H, C₁-C₆alkyl, OR⁰, C₁-C₆alkenyl, C₁-C₆alkynyl, CN, N(R⁰)₂, and halide;
   Y is selected from the group consisting of O and S;
   Z is selected from the group consisting of O and S;
   R^{s} is selected from the group consisting of: 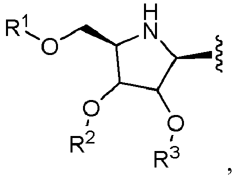 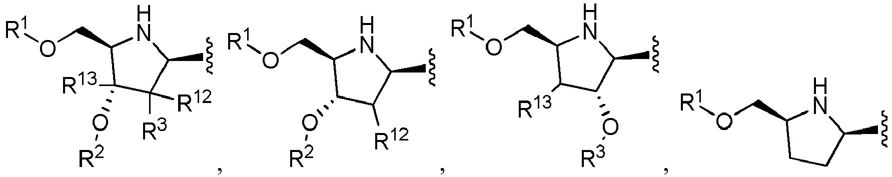 and 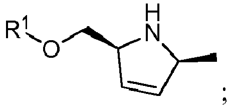
      R¹ is selected from the group consisting of H, C₁-C₆alkyl, aminoacyl, aminothionyl, C₁-C₆acyl, R¹⁰OC(O)-, phosphoryl, and aminophosphoryl;
      R² and R³ are each independently H, C₁-C₆alkyl, C₁-C₆alkenyl, or C₁-C₆alkynyl; and
      R¹² and R¹³ are each independently selected from the group consisting of H, C₁-C₆alkyl, C₁-C₆alkenyl, C₁-C₆alkynyl, OR⁰, CN, azide, and halide.

One embodiment of the invention relates to any one of the compounds described above, wherein R^{s} is selected from the group consisting of:

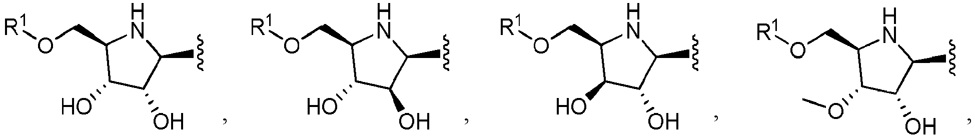

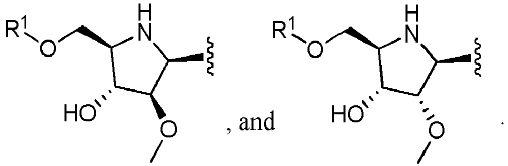

Another embodiment of the invention relates to any one of the compounds described above, wherein R^{s} is selected from the group consisting of:

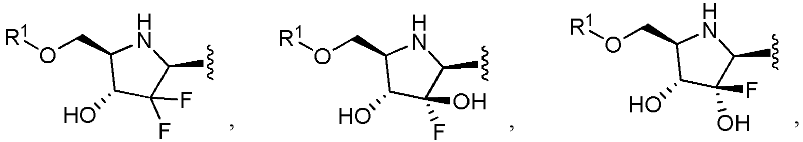

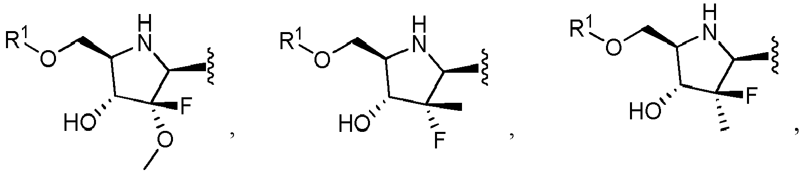

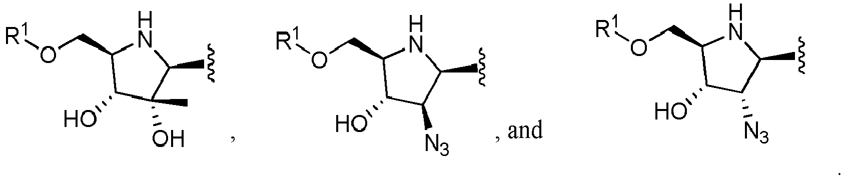

Yet another embodiment of the invention relates to any one of the compounds described above, wherein R^{s} is selected from the group consisting of:

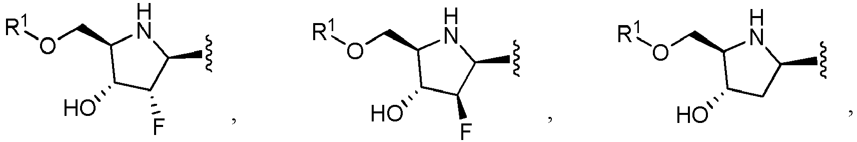

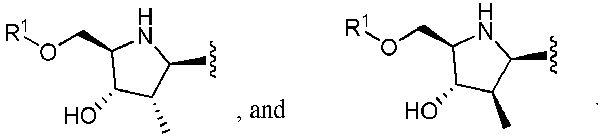

Still yet another embodiment of the invention relates to any one of the compounds described above, wherein R^{s} is selected from the group consisting of:

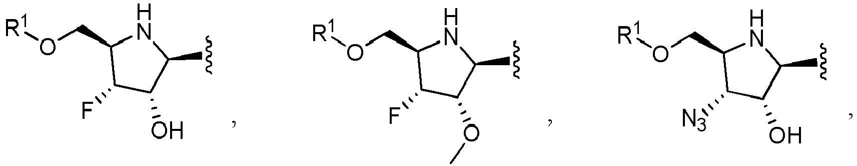

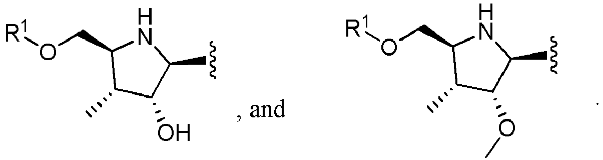

Another embodiment of the invention relates to any one of the compounds described above, wherein R^{s} is selected from the group consisting of:

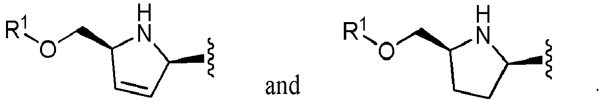

Another aspect of the invention relates to any one of the compounds described above, wherein R¹ is H, methyl, or ethyl.

Another aspect of the invention relates to any one of the compounds described above, wherein R¹ is phosphate, pyrophosphate, phosphoramidite, phosphite, or phosphonate.

Another embodiment of the invention relates to any one of the compounds as decribed above, wherein R¹ is phosphoryl or aminophosphoryl.

Yet another embodiment of the invention relates to any one of the compounds described above, wherein R¹ is aminophosphoryl;
aminophosphoryl is -P(=O)(OR⁵⁰)NR⁵¹R⁵²;
R⁵⁰ is selected from the group consisting of H, (C₁-C₆)alkyl, aryl, arylalkyl, heteroaryl, heteroaralkyl, and -(CH₂)ₘSC(=O)C(CH₃)₂CH₂OH;
m is 1 or 2;
R⁵¹ is H or C₁-C₆alkyl;
R⁵² is selected from the group consisting of H, C₁-C₆alkyl, aryl, aralkyl, heteroaryl, heteroaralkyl, and -CR⁶⁰R⁶¹C(=O)OR⁶²;
R⁶⁰ and R⁶¹ each independently are H or C₁-C₆alkyl; and
R⁶² is selected from the group consisting of H, C₁-C₆alkyl, aryl, aralkyl, heteroaryl, and heteroaralkyl.

One embodiment of the invention relates to any one of the compounds described above, wherein **R^{s}-B** is selected from the group consisting of: 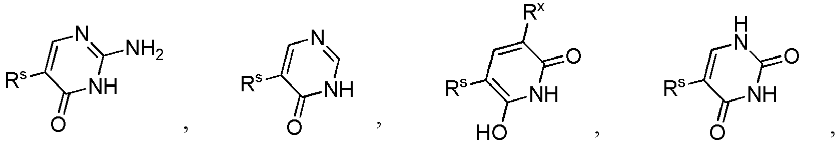 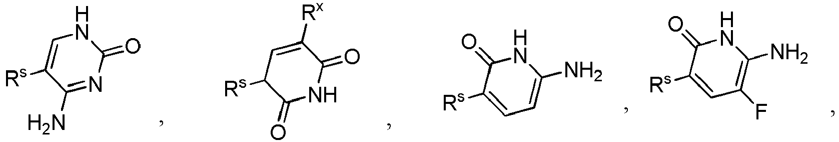 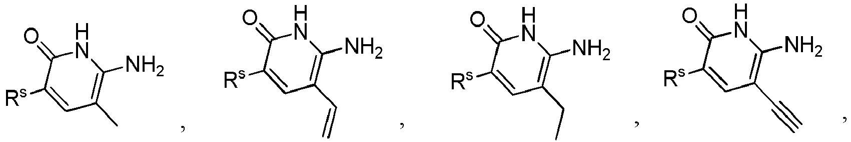 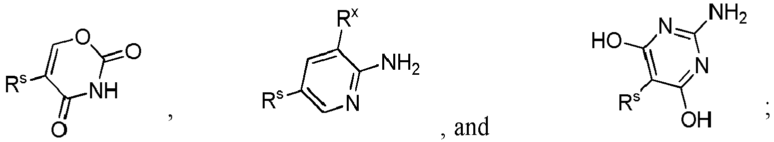 and
R^{x} is selected from the group consisting of H, C₁-C₆alkyl, OR⁰, C₁-C₆alkenyl, C₁-C₆alkynyl, CN, NH₂, aminoalkyl, NO₂, azide, halide, aryl, and heteroaryl.

Another embodiment of the invention relates to any one of the compounds described above, wherein R^{x} is F, Cl, Br, or I.

Yet another embodiment of the invention relates to any one of the compounds described above, wherein R^{x} is H, methyl, ethyl, or propyl.

Still yet another embodiment of the invention relates to any one of the compounds described above, wherein R^{x} is ethenyl or ethynyl.

Another embodiment of the invention relates to any one of the compounds described above, wherein R^{x} is CN, NH₂, or aminoalkyl.

One aspect of the invention relates to a compound represented by **R**^{s}**-B**, or a pharmaceutically acceptable salt thereof, wherein:
**R^{s}-B** is selected from the group consisting of: 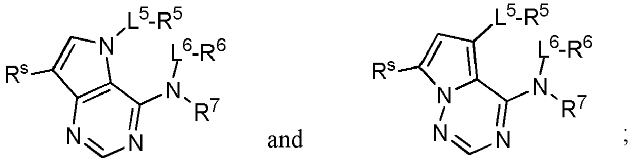
   R^{s} is represented by the formula: 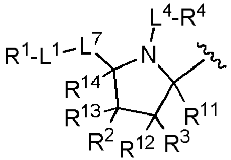
      L¹ is a bond;
      L⁴, L⁵, and L⁶, each independently, are a bond or -C(R⁰)₂-O-;
      L⁷ is O, -CH₂-, -CH(C₁-C₆alkyl)-, -C(R⁰)₂-S-, or -C(R⁰)₂-NH-;
         R⁰, independently for each occurrence, is H or C₁-C₆alkyl;
      R¹ is selected from the group consisting of H, C₁-C₆alkyl, aminoacyl, aminothionyl, C₁-C₆acyl, R¹⁰OC(O)-, phosphoryl, and aminophosphoryl;
      R² and R³ are each independently H, halide, C₁-C₆alkyl, C₁-C₆alkenyl, C₁-C₆alkynyl, CN, OR⁰, R¹⁰OC(O)-, or SR⁰;
         or R¹ and R², taken together, or R² and R³, taken together, may be selected from the group consisting of -OC(O)O-, -OC(S)O-, phosphoryl, and C₁-C₆alkylphosphoryl;
         or R² and R³, taken together, may form a bond between the carbon atoms to which they are attached;
      R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
         R¹⁰, independently for each occurrence, is selected from the group consisting of H, C₁-C₆alkyl, aryl, heteroaryl, aralkyl, and heteroaralkyl;
   R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
   R⁷ is H; or R⁶, R⁷, and the nitrogen to which they are bonded, taken together, represent -N=CR²⁰R²¹;
      R²⁰ and R²¹, each independently, are selected from the group consisting of H, C₁-C₆alkyl, amino, aryl, heteroaryl, aralkyl, and heteroaralkyl;
      R¹¹ is selected from the group consisting of H, C₁-C₆alkyl, C₁-C₆alkenyl, C₁-C₆alkynyl, OR⁰, CN, and halide;
      R¹² and R¹³ are each independently selected from the group consisting of H, C₁-C₆alkyl, C₁-C₆alkenyl, C₁-C₆alkynyl, OR⁰, CN, azide, and halide; and
      R¹⁴ is selected from the group consisting of H, C₁-C₆alkyl, OR⁰, C₁-C₆alkynyl, CN, azide, and halide.

One aspect of the invention relates to a compound represented by **R^{s}-B**, or a pharmaceutically acceptable salt thereof, wherein:
**R^{s}-B** is selected from the group consisting of: 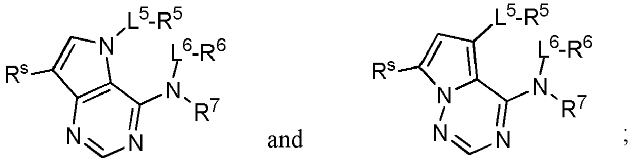
   R^{s} is represented by the formula: 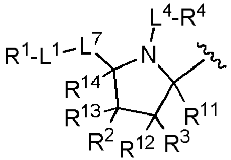
      L¹, L⁴, L⁵, and L⁶, each independently, are a bond or -C(R⁰)₂-O-;
      L⁷ is a bond, O, -C(R⁰)₂-O-, -C(R⁰)₂-S-, or -C(R⁰)₂-NH-;
         R⁰, independently for each occurrence, is H or C₁-C₆alkyl;
      R¹ is selected from the group consisting of H, C₁-C₆alkyl, aminoacyl, aminothionyl, C₁-C₆acyl, R¹⁰OC(O)-, phosphoryl, and aminophosphoryl;
      R² and R³ are each independently H, halide, C₁-C₆alkyl, C₁-C₆alkenyl, C₁-C₆alkynyl, CN, OR⁰, R¹⁰OC(O)-, or SR⁰;
         or R¹ and R², taken together, or R² and R³, taken together, may be selected from the group consisting of -OC(O)O-, -OC(S)O-, phosphoryl, and C₁-C₆alkylphosphoryl;
         or R² and R³, taken together, may form a bond between the carbon atoms to which they are attached;
      R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
         R¹⁰, independently for each occurrence, is selected from the group consisting of H, C₁-C₆alkyl, aryl, heteroaryl, aralkyl, and heteroaralkyl;
   R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
   R⁷ is H; or R⁶, R⁷, and the nitrogen to which they are bonded, taken together, represent -N=CR²⁰R²¹;
      R²⁰ and R²¹, each independently, are selected from the group consisting of H, C₁-C₆alkyl, amino, aryl, heteroaryl, aralkyl, and heteroaralkyl;
      R¹¹ is selected from the group consisting of C₁-C₆alkyl, C₁-C₆alkenyl, C₁-C₆alkynyl, OR⁰, and CN;
      R¹² and R¹³ are each independently selected from the group consisting of H, C₁-C₆alkyl, C₁-C₆alkenyl, C₁-C₆alkynyl, OR⁰, CN, azide, and halide; and
      R¹⁴ is selected from the group consisting of H, C₁-C₆alkyl, OR⁰, C₁-C₆alkynyl, CN, azide, and halide.

One aspect of the invention relates to a compound represented by **R^{s}-B**, or a pharmaceutically acceptable salt thereof, wherein:
**R^{s}-B** is selected from the group consisting of: 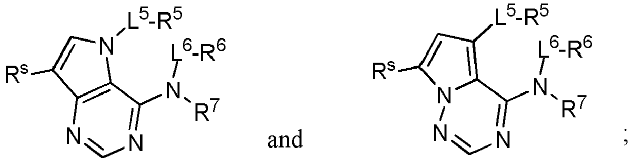
   R^{s} is represented by the formula: 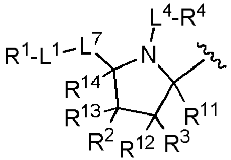
      L¹, L⁴, L⁵, and L⁶, each independently, are a bond or -C(R⁰)₂-O-;
      L⁷ is a bond, O, -C(R⁰)₂-O-, -C(R⁰)₂-S-, or -C(R⁰)₂-NH-;
         R⁰, independently for each occurrence, is H or C₁-C₆alkyl;
      R¹ is selected from the group consisting of H, C₁-C₆alkyl, aminoacyl, aminothionyl, C₁-C₆acyl, R¹⁰OC(O)-, phosphoryl, and aminophosphoryl;
      R² is H, halide, C₁-C₆alkyl, C₁-C₆alkenyl, C₁-C₆alkynyl, CN, OR⁰, R¹⁰OC(O)-, or SR⁰;
      R³ is H, halide, C₁-C₆alkyl, C₁-C₆alkenyl, C₁-C₆alkynyl, CN, R¹⁰OC(O)-, or SR⁰;
         or R¹ and R², taken together, or R² and R³, taken together, may be selected from the group consisting of -OC(O)O-, -OC(S)O-, phosphoryl, and C₁-C₆alkylphosphoryl;
         or R² and R³, taken together, may form a bond between the carbon atoms to which they are attached;
      R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
         R¹⁰, independently for each occurrence, is selected from the group consisting of H, C₁-C₆alkyl, aryl, heteroaryl, aralkyl, and heteroaralkyl;
   R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
   R⁷ is H; or R⁶, R⁷, and the nitrogen to which they are bonded, taken together, represent -N=CR²⁰R²¹;
      R²⁰ and R²¹, each independently, are selected from the group consisting of H, C₁-C₆alkyl, amino, aryl, heteroaryl, aralkyl, and heteroaralkyl;
      R¹¹ is selected from the group consisting of H, C₁-C₆alkyl, C₁-C₆alkenyl, C₁-C₆alkynyl, OR⁰, and CN;
      R¹² is selected from the group consisting of H, C₁-C₆alkyl, C₁-C₆alkenyl, C₁-C₆alkynyl, CN, azide, and halide;
      R¹³ is selected from the group consisting of H, C₁-C₆alkyl, C₁-C₆alkenyl, C₁-C₆alkynyl, OR⁰, CN, azide, and halide; and
      R¹⁴ is selected from the group consisting of H, C₁-C₆alkyl, OR⁰, C₁-C₆alkynyl, CN, azide, and halide.

One aspect of the invention relates to a compound represented by **R^{s}-B**, or a pharmaceutically acceptable salt thereof, wherein:
**R^{s}-B** is selected from the group consisting of: 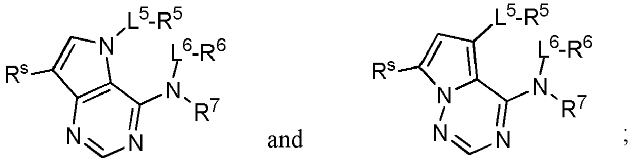
   R^{s} is represented by the formula: 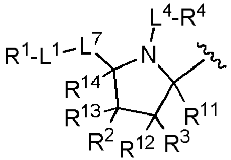
      L¹, L⁴, L⁵, and L⁶, each independently, are a bond or -C(R⁰)₂-O-;
      L⁷ is a bond, O, -C(R⁰)₂-O-, -C(R⁰)₂-S-, or -C(R⁰)₂-NH-;
         R⁰, independently for each occurrence, is H or C₁-C₆alkyl;
      R¹ is selected from the group consisting of H, C₁-C₆alkyl, aminoacyl, aminothionyl, C₁-C₆acyl, R¹⁰OC(O)-, phosphoryl, and aminophosphoryl;
      R² is H, halide, C₁-C₆alkyl, C₁-C₆alkenyl, C₁-C₆alkynyl, CN, R¹⁰OC(O)-, or SR⁰;
      R³ is H, halide, C₁-C₆alkyl, C₁-C₆alkenyl, C₁-C₆alkynyl, CN, OR⁰, R¹⁰OC(O)-, or SR⁰;
         or R¹ and R², taken together, or R² and R³, taken together, may be selected from the group consisting of -OC(O)O-, -OC(S)O-, phosphoryl, and C₁-C₆alkylphosphoryl;
         or R² and R³, taken together, may form a bond between the carbon atoms to which they are attached;
      R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
         R¹⁰, independently for each occurrence, is selected from the group consisting of H, C₁-C₆alkyl, aryl, heteroaryl, aralkyl, and heteroaralkyl;
   R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or R⁵ may be absent;
   R⁷ is H; or R⁶, R⁷, and the nitrogen to which they are bonded, taken together, represent -N=CR²⁰R²¹;
      R²⁰ and R²¹, each independently, are selected from the group consisting of H, C₁-C₆alkyl, amino, aryl, heteroaryl, aralkyl, and heteroaralkyl;
      R¹¹ is selected from the group consisting of H, C₁-C₆alkyl, C₁-C₆alkenyl, C₁-C₆alkynyl, OR⁰, and CN;
      R¹² is selected from the group consisting of C₁-C₆alkyl, OR⁰, alkynyl, CN, azide, and halide;
      R¹³ is selected from the group consisting of H, C₁-C₆alkyl, C₁-C₆alkenyl, C₁-C₆alkynyl, CN, azide, and halide; and
      R¹⁴ is selected from the group consisting of H, C₁-C₆alkyl, OR⁰, C₁-C₆alkynyl, CN, azide, and halide.

One aspect of the invention relates to a compound represented by **R^{s}-B**, or a pharmaceutically acceptable salt thereof, wherein:
**R^{s}-B** is selected from the group consisting of: 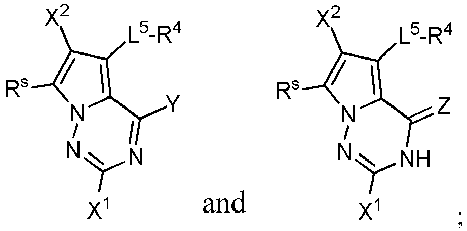
   R^{s} is selected from the group consisting of: 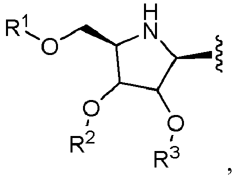 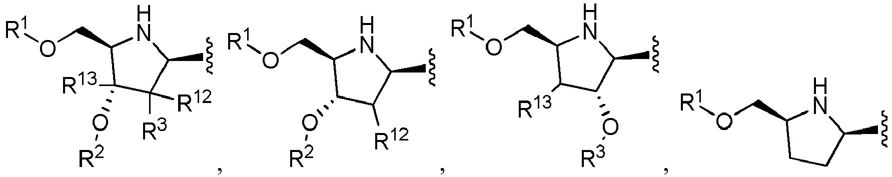 and 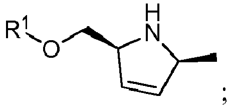
      R¹ is selected from the group consisting of H, C₁-C₆alkyl, aminoacyl, aminothionyl, C₁-C₆acyl, R¹⁰OC(O)-, phosphoryl, and aminophosphoryl;
      R² and R³ are each independently H, C₁-C₆alkyl, C₁-C₆alkenyl, or C₁-C₆alkynyl;
         R¹⁰, independently for each occurrence, is selected from the group consisting of H, C₁-C₆alkyl, aryl, heteroaryl, aralkyl, and heteroaralkyl;
      R¹² and R¹³ are each independently selected from the group consisting of H, C₁-C₆alkyl, C₁-C₆alkenyl, C₁-C₆alkynyl, OR⁰, CN, azide, and halide;
   L⁵ is a bond or -C(R⁰)₂-O-;
      R⁰, independently for each occurrence, is H or C₁-C₆alkyl;
   R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
   X¹ is selected from the group consisting of H, N(R⁰)₂, SH, thioalkyl, OR⁰, alkenyl, alkynyl, CN, and halide;
   X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
   Y is selected from the group consisting of H, OR⁰, N(R⁵)(R⁶), SH, thioalkyl, O-aryl, O-heteroaryl, and halide;
   Z is selected from the group consisting of O and S;
      R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
      R⁵, R⁶, and the nitrogen to which they are bonded, taken together,
      represent -N=CR²⁰R²¹; and
      R²⁰ and R²¹, each independently, are selected from the group consisting of H, C₁-C₆alkyl, amino, aryl, heteroaryl, aralkyl, and heteroaralkyl.

One aspect of the invention relates to a compound represented by **R^{s}-B**, or a pharmaceutically acceptable salt thereof, wherein:
**R^{s}-B** is selected from the group consisting of: 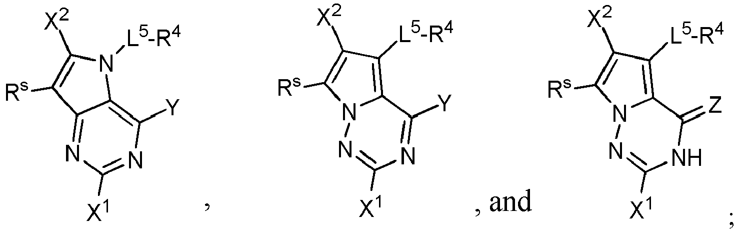
   R^{s} is selected from the group consisting of: 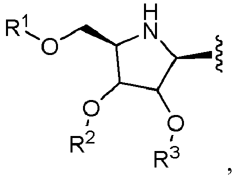 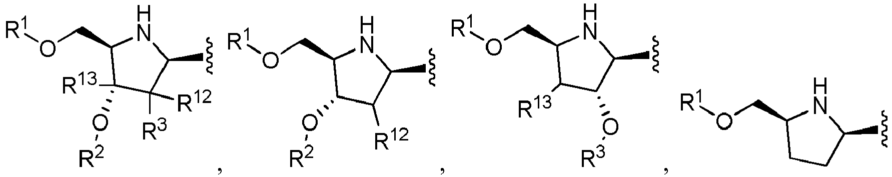 and 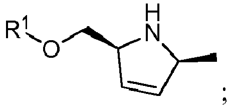
      R¹ is selected from the group consisting of H, C₁-C₆alkyl, aminoacyl, aminothionyl, C₁-C₆acyl, R¹⁰OC(O)-, phosphoryl, and aminophosphoryl;
      R² and R³ are each independently H, C₁-C₆alkyl, C₁-C₆alkenyl, or C₁-C₆alkynyl;
         R¹⁰, independently for each occurrence, is selected from the group consisting of H, C₁-C₆alkyl, aryl, heteroaryl, aralkyl, and heteroaralkyl;
      R¹² and R¹³ are each independently selected from the group consisting of H, C₁-C₆alkyl, C₁-C₆alkenyl, C₁-C₆alkynyl, OR⁰, CN, azide, and halide;
   L⁵ is a bond or -C(R⁰)₂-O-;
      R⁰, independently for each occurrence, is H or C₁-C₆alkyl;
   R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
   X¹ is selected from the group consisting of H, N(R⁰)₂, SH, thioalkyl, OR⁰, alkenyl, alkynyl, CN, and halide;
   X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
   Y is selected from the group consisting of H, OR⁰, N(R⁵)(R⁶), SH, thioalkyl, O-aryl, O-heteroaryl, and halide;
   Z is selected from the group consisting of O and S; and
      R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl, provided that both R⁵ and R⁶ are not H.

One aspect of the invention relates to a compound represented by **R^{s}-B**, or a pharmaceutically acceptable salt thereof, wherein:
**R^{s}-B** is selected from the group consisting of: 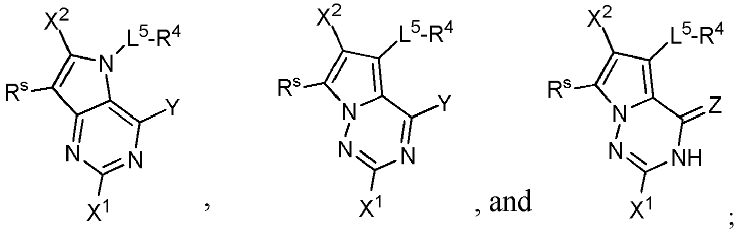
   R^{s} is selected from the group consisting of: 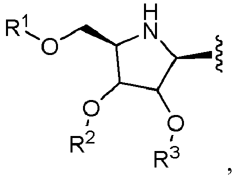 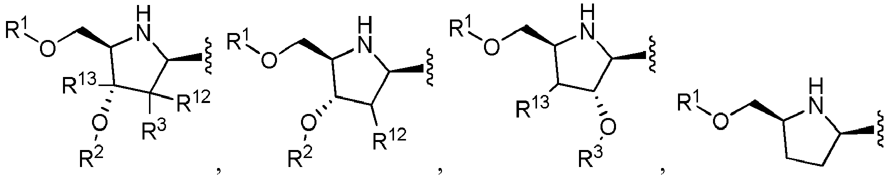 and 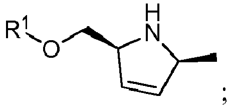
      R¹ is selected from the group consisting of H, C₁-C₆alkyl, aminoacyl, aminothionyl, C₁-C₆acyl, R¹⁰OC(O)-, phosphoryl, and aminophosphoryl;
         R¹⁰, independently for each occurrence, is selected from the group consisting of H, C₁-C₆alkyl, aryl, heteroaryl, aralkyl, and heteroaralkyl;
      R² and R³ are each independently H, C₁-C₆alkyl, C₁-C₆alkenyl, or C₁-C₆alkynyl;
      R¹² and R¹³ are each independently selected from the group consisting of H, C₁-C₆alkyl, C₁-C₆alkenyl, C₁-C₆alkynyl, OR⁰, CN, azide, and halide;
         R⁰, independently for each occurrence, is H or C₁-C₆alkyl;
   L⁵ is a bond or -C(R⁰)₂-O-;
   R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
   X¹ is selected from the group consisting of H, N(R⁰)₂, SH, thioalkyl, OR⁰, alkenyl, alkynyl, CN, and halide;
   X² is selected from the group consisting of N(R⁰)₂, SH, thioalkyl, OR⁰, O-aryl, O-heteroaryl, alkenyl, alkynyl, C₁-C₆acyl, carboxyl, CN, azide, and halide;
   Y is selected from the group consisting of H, OR⁰, N(R⁵)(R⁶), SH, thioalkyl, O-aryl, O-heteroaryl, and halide;
   Z is selected from the group consisting of O and S;
      R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
      R⁵, R⁶, and the nitrogen to which they are bonded, taken together,
      represent -N=CR²⁰R²¹; and
      R²⁰ and R²¹, each independently, are selected from the group consisting of H, C₁-C₆alkyl, amino, aryl, heteroaryl, aralkyl, and heteroaralkyl.

One aspect of the invention relates to a compound represented by **R^{s}-B**, or a pharmaceutically acceptable salt thereof, wherein:
**R^{s}-B** is selected from the group consisting of: 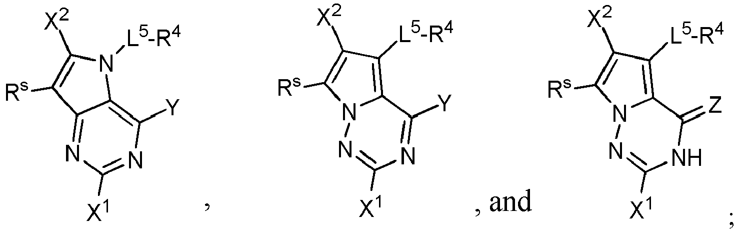
   R^{s} is selected from the group consisting of: 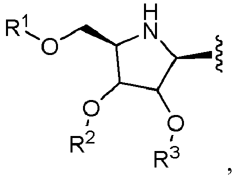 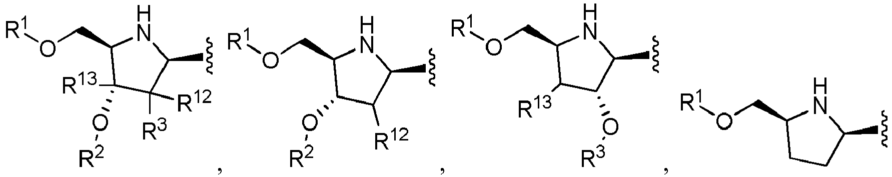 and 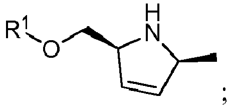
      R¹ is selected from the group consisting of H, C₁-C₆alkyl, aminoacyl, aminothionyl, C₁-C₆acyl, R¹⁰OC(O)-, phosphoryl, and aminophosphoryl;
      R² and R³ are each independently H, C₁-C₆alkyl, C₁-C₆alkenyl, or C₁-C₆alkynyl;
         R¹⁰, independently for each occurrence, is selected from the group consisting of H, C₁-C₆alkyl, aryl, heteroaryl, aralkyl, and heteroaralkyl;
      R¹² and R¹³ are each independently selected from the group consisting of H, C₁-C₆alkyl, C₁-C₆alkenyl, C₁-C₆alkynyl, OR⁰, CN, azide, and halide;
         R⁰, independently for each occurrence, is H or C₁-C₆alkyl;
   L⁵ is a bond or -C(R⁰)₂-O-;
   R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
   X¹ is selected from the group consisting of N(R⁰)₂, SH, thioalkyl, OR⁰, alkenyl, alkynyl, CN, and halide;
   X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
   Y is selected from the group consisting of H, OR⁰, N(R⁵)(R⁶), SH, thioalkyl, O-aryl, O-heteroaryl, and halide;
   Z is selected from the group consisting of O and S;
      R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
      R⁵, R⁶, and the nitrogen to which they are bonded, taken together,
      represent -N=CR²⁰R²¹; and
      R²⁰ and R²¹, each independently, are selected from the group consisting of H, C₁-C₆alkyl, amino, aryl, heteroaryl, aralkyl, and heteroaralkyl.

One embodiment of the invention relates to a compound as described above, wherein R^{s} is selected from the group consisting of:

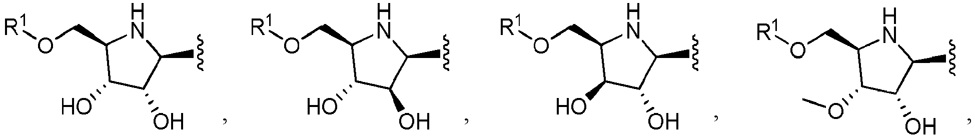

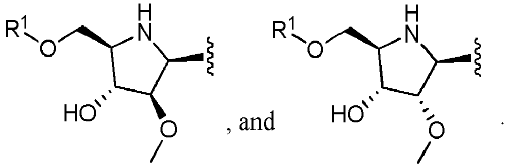

Another embodiment of the invention relates to a compound as described above, wherein R^{s} is selected from the group consisting of:

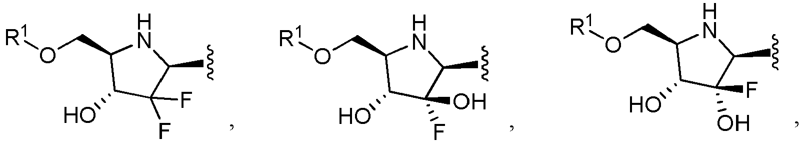

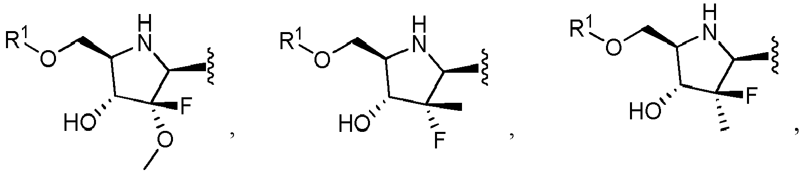

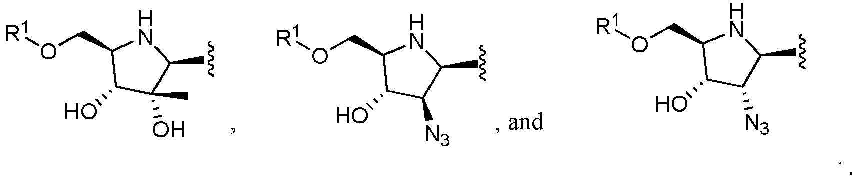

Yet another embodiment of the invention relates to a compound as described above, wherein R^{s} is selected from the group consisting of:

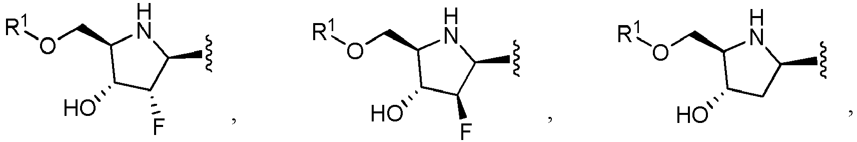

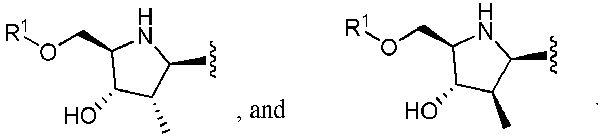

Still yet another embodiment of the invention relates to a compound as described above, wherein R^{s} is selected from the group consisting of:

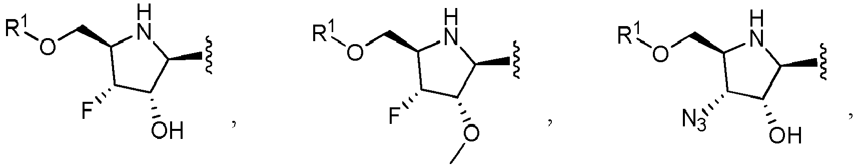

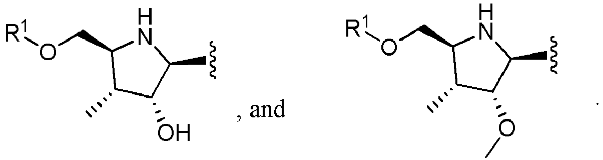

Another embodiment of the invention relates to a compound as described above, wherein R^{s} is selected from the group consisting of:

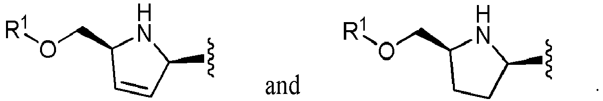

One embodiment of the invention relates to a compound as described above, wherein L⁵-R⁵ is H.

One embodiment of the invention relates to a compound as described above, wherein L⁴-R⁴ is H.

One embodiment of the invention relates to a compound as described above, wherein L⁷ is -CH(C₁-C₆alkyl)-.

One embodiment of the invention relates to a compound as described above, wherein R¹¹, R¹², R³, and R¹⁴ are H.

One embodiment of the invention relates to a compound as described above, wherein R¹², R¹³, and R¹⁴ are H.

One embodiment of the invention relates to a compound as described above, wherein R¹ is selected from the group consisting of phosphate, pyrophosphate, phosphoramidite, phosphite, and phosphonate.

Another embodiment of the invention relates to a compound as described above, wherein R¹ is phosphoryl or aminophosphoryl.

Yet another embodiment of the invention relates to a compound as described above, wherein R¹ is aminophosphoryl;
aminophosphoryl is -P(=O)(OR⁵⁰)NR⁵¹R⁵²;
R⁵⁰ is selected from the group consisting of H, (C₁-C₆)alkyl, aryl, arylalkyl, heteroaryl, heteroaralkyl, and -(CH₂)ₘSC(=O)C(CH₃)₂CH₂OH;
m is 1 or 2;
R⁵¹ is H or C₁-C₆alkyl;
R⁵² is selected from the group consisting of H, C₁-C₆alkyl, aryl, aralkyl, heteroaryl, heteroaralkyl, and -CR⁶⁰R⁶¹C(=O)OR⁶²;
R⁶⁰ and R⁶¹ each independently are H or C₁-C₆alkyl; and
R⁶² is selected from the group consisting of H, C₁-C₆alkyl, aryl, aralkyl, heteroaryl, and heteroaralkyl.

One embodiment of the invention relates to a compound as described above, wherein R⁵⁰ is H.

Another embodiment of the invention relates to a compound as described above, wherein R⁵⁰ is aryl.

Yet another embodiment of the invention relates to a compound as described above, wherein R⁵⁰ is -(CH₂)ₘSC(=O)C(CH₃)₂CH₂OH.

One embodiment of the invention relates to a compound as described above, wherein mis2.

One embodiment of the invention relates to a compound as described above, wherein R⁵¹ is H.

One embodiment of the invention relates to a compound as described above, wherein R⁵² is aralkyl.

Another embodiment of the invention relates to a compound as described above, wherein R⁵² is -CR⁶⁰R⁶¹C(=O)OR⁶².

One embodiment of the invention relates to a compound as described above, wherein R⁶⁰ is H; R⁶¹ is (C₁-C₆)alkyl; and R⁶² is (C₁-C₆)alkyl.

One embodiment of the invention relates to a compound as described above, wherein R¹ is selected from the group consisting of: 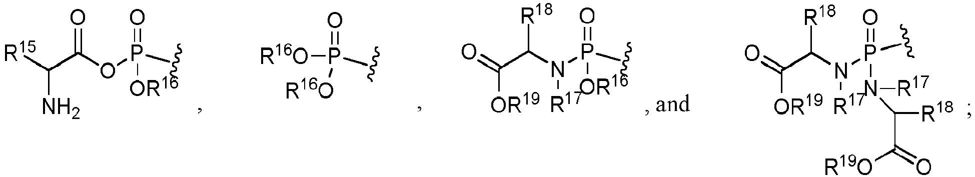 wherein:
R¹⁵ is selected from the group consisting of H, C₁-C₆alkyl, C₁-C₆haloalkyl, aminoalkyl,
thioalkyl, C₃-C₈cycloalkyl, C₂-C₇heterocyclyl, aryl, heteroaryl, aralkyl, heteroaralkyl, and naturally-occurring amino acid side chains;
R¹⁶ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl; or R¹⁶ taken together with the oxygen to which it is bonded may be a naturally-occurring amino acid;
R¹⁷ is, independently for each occurrence, selected from the group consisting of H,(
C₁-C₆alkyl, C₁-C₆haloalkyl, C₃-C₈cycloalkyl, C₂-C₇heterocyclyl, aryl, heteroaryl, aralkyl, heteroaralkyl, and acyl;
R¹⁸ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, heteroaralkyl, and naturally-occurring amino acid side chains; or R¹⁷ and R¹⁸, taken together, may form a 4-, 5-, or 6-membered ring; and
R¹⁹ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl.

Another aspect of the invention is a compound selected from the group consisting of: 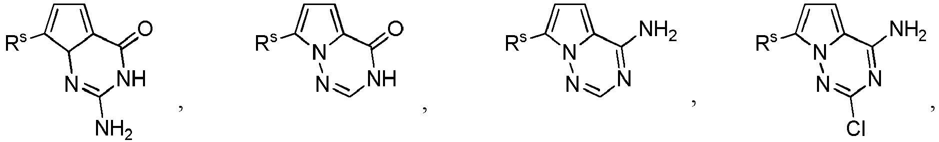 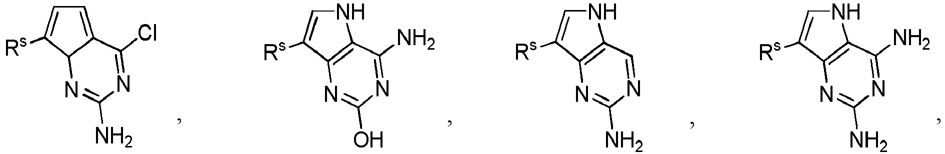 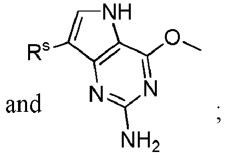 wherein R^{s} is selected from the group consisting of: 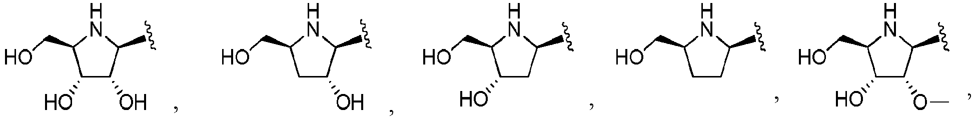 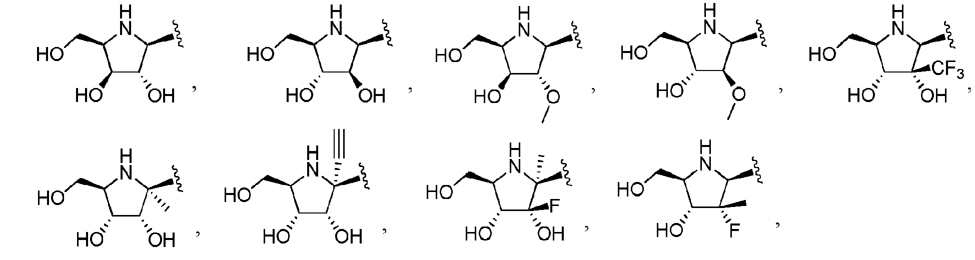 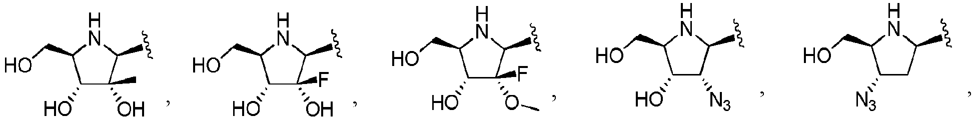 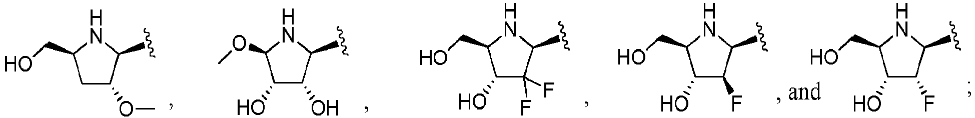 or a pharmaceutically acceptable salt thereof.

Another aspect of the invention is a compound, or a pharmaceutically acceptable salt thereof, selected from the group consisting of: 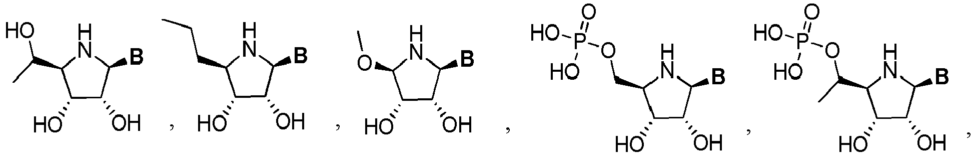 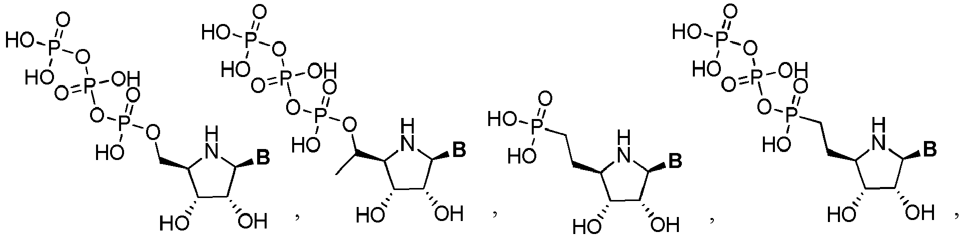 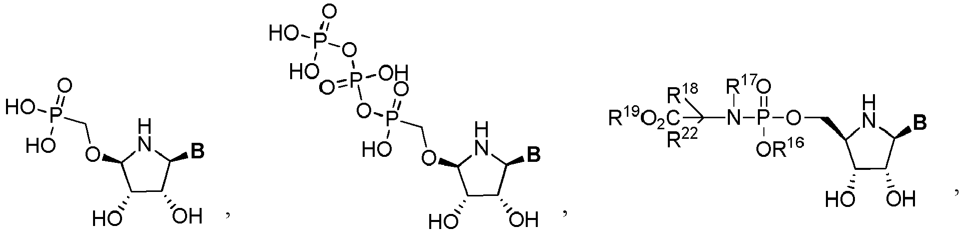 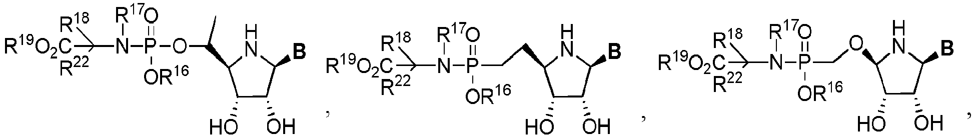 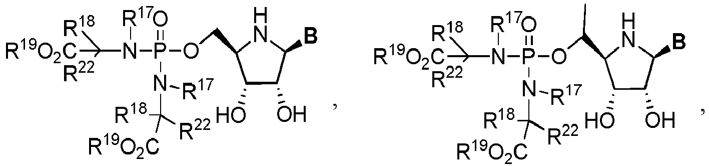 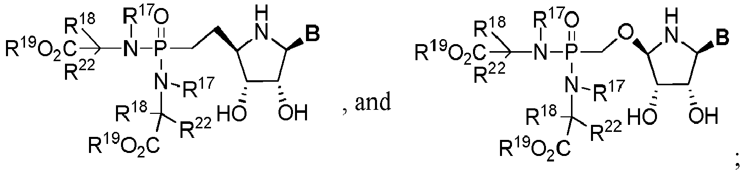 wherein:
R¹⁶ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl; or R¹⁶ taken together with the oxygen to which it is bonded may be a naturally-occurring amino acid;
R¹⁸ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, heteroaralkyl, and naturally-occurring amino acid side chains;
R¹⁹ is, independently for each occurrence, selected from the group consisting of H,
C₁-
   C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl;
R²² is, independently for each occurrence, selected from the group consisting of H,
C₁-C₆alkyl, C₁-C₆haloalkyl, C₃-C₈cycloalkyl, C₂-C₇heterocyclyl, aryl, heteroaryl, aralkyl, and heteroaralkyl; or R¹⁷ and R²², taken together, may form a 4-, 5-, or 6-membered ring; and
**B** is selected from the group consisting of:

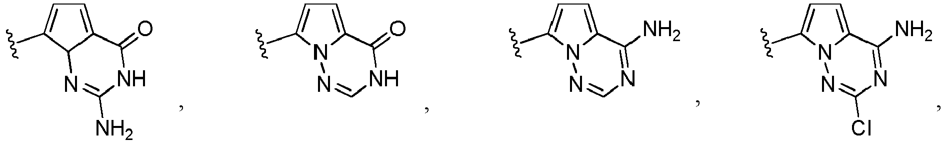

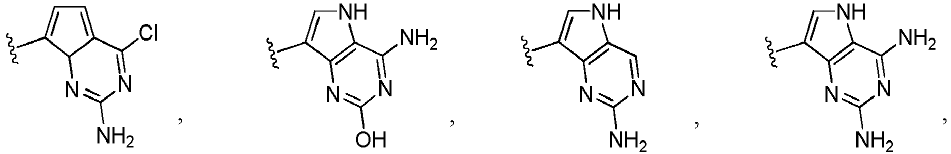

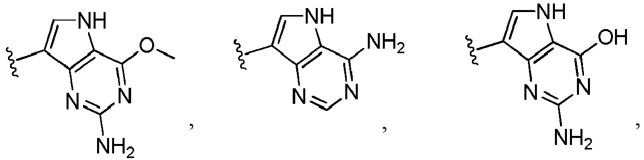

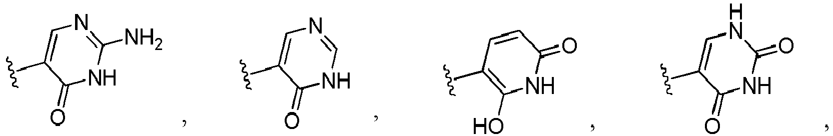

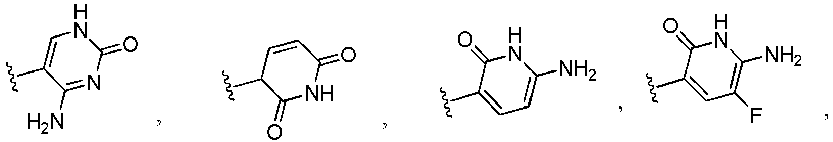

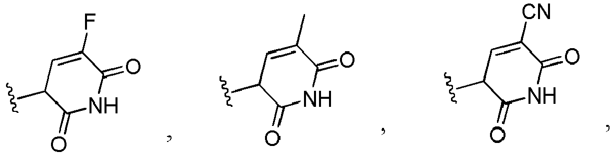

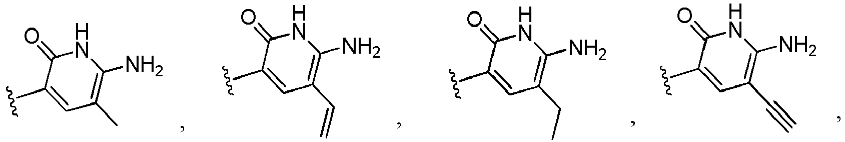

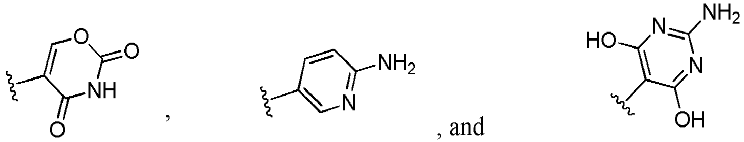

Another aspect of the invention is a compound selected from the group consisting of: 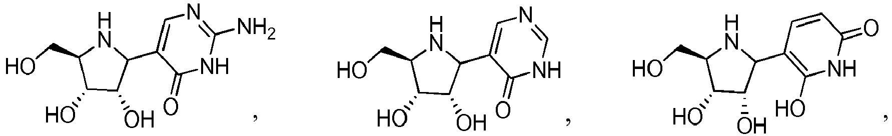 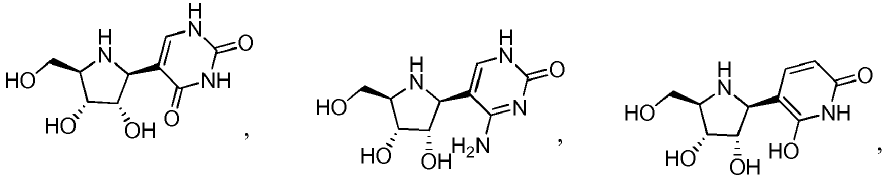 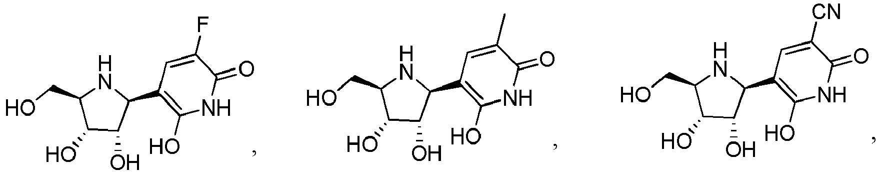 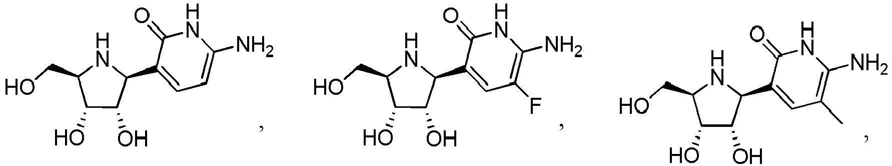 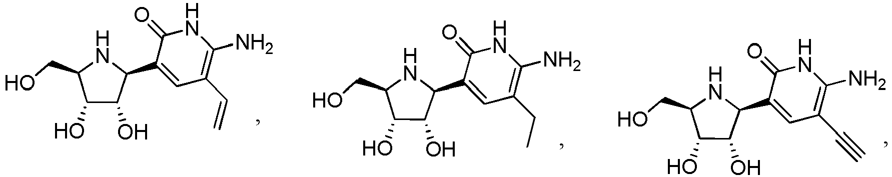 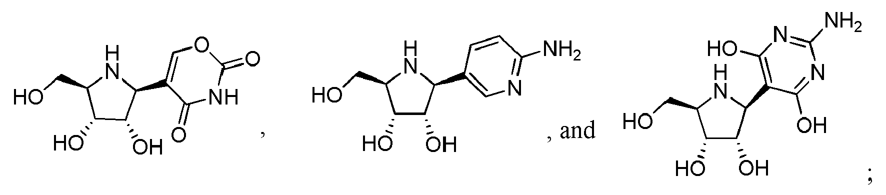 and pharmaceutically acceptable salts thereof.

Another aspects of the invention is a compound selected from the group consisting of: 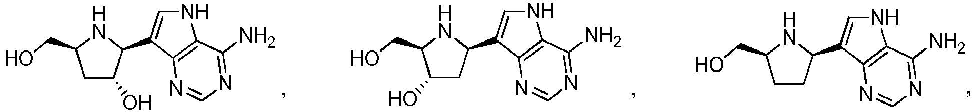 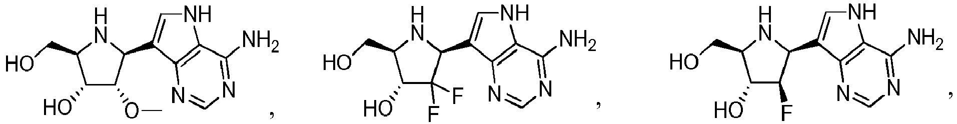 and 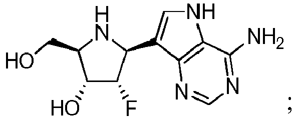 and pharmaceutically acceptable salts thereof.

An aspect of the invention is a pharmaceutical composition comprising a compound of the invention, or a pharmaceutically acceptable salt thereof, and a pharmaceutically acceptable carrier.

An aspect of the invention is a method of preparing a pharmaceutical composition. The method includes the step of combining a compound of the invention, or a pharmaceutically acceptable salt thereof, with a pharmaceutically acceptable carrier.

Compounds of the invention are useful for inhibiting nucleic acid polymerase activity of certain viruses. Compounds of the invention are also useful for inhibiting viral replication or treating viral infections.

Animal RNA viruses are classified into three distinct groups based on their genome and mode of replication (and the numerical groups based on the older Baltimore classification):
Double-stranded (ds) RNA viruses (Baltimore classification Group III) contain from one to a dozen different RNA molecules, each of which codes for one or more viral proteins. Examples of dsRNA viruses include reoviridae.

Positive-sense single-stranded (ss) RNA viruses (Baltimore classification Group IV) have their genome directly utilized as if it were mRNA, producing a single protein which is modified by host and viral proteins to form the various proteins needed for replication. One of these includes RNA-dependent RNA polymerase, which copies the viral RNA to form a double-stranded replicative form, which in turn directs the formation of new virions. Examples of positive-sense ssRNA viruses include togaviridae, flaviviridae, calciviridae, coronaviridae, picornaviridae, and togaviridae.

Negative-sense ssRNA viruses (Baltimore classification Group V) must have their genome copied by an RNA polymerase to form positive-sense RNA. This means that the virus must bring along with it the RNA-dependent RNA polymerase enzyme. The positive-sense RNA molecule then acts as viral mRNA, which is translated into proteins by the host ribosomes. The resultant protein goes on to direct the synthesis of new virions, such as capsid proteins and RNA replicase, which is used to produce new negative-sense RNA molecules. Negative-sense ssRNA viruses include bornaviridae, filoviridae, orthomyxoviridae, paramyxoviridae, rhabdoviridae, arenaviridae, and bunyaviridae.

Retroviruses (Baltimore classification Group VI) have a single-stranded RNA genome but are generally not considered RNA viruses because they use DNA intermediates to replicate. Reverse transcriptase, a viral enzyme that comes from the virus itself after it is uncoated, converts the viral RNA into a complementary strand of DNA, which is copied to produce a double stranded molecule of viral DNA. After this DNA is integrated, expression of the encoded genes may lead the formation of new virions. Retroviruses include without limitation HIV-1 and HIV-2.

An aspect of the invention is a method of inhibiting viral nucleic acid polymerase activity of a virus. The method includes the step of contacting a viral nucleic acid polymerase of the virus with an effective amount of a compound of the invention, or a pharmaceutically acceptable salt thereof.

In one embodiment, the viral nucleic acid polymerase is a DNA polymerase.

In one embodiment the viral nucleic acid polymerase is an RNA polymerase.

In one embodiment, the virus is selected from the group consisting of RNA viruses.

In one embodiment, the virus is selected from the group consisting of orthomyxoviridae, paramyxoviridae, arenaviridae, bunyaviridae, flaviviridae, filoviridae, togaviridae, picornaviridae, and coronaviridae.

In one embodiment, the virus is selected from the group consisting of adenovirus, rhinovirus, hepatitis A virus, hepatitis C virus, polio virus, measles virus, Ebola virus, Coxsackie virus, West Nile virus, smallpox virus, yellow fever virus, Dengue Fever virus, influenza A virus, influenza B virus, lassa virus, lymphocytic choriomeningitis virus, Junin virus, machuppo virus, guanarito virus, hantavirus, Rift Valley Fever virus, La Crosse virus, California encephalitis virus, Crimean-Congo virus, Marburg virus, Japanese encephalitis virus, Kyasanur Forest virus, Venezuelan equine encephalitis virus, Eastern equine encephalitis virus, Western equine encephalitis virus, severe acute respiratory syndrome (SARS) virus, parainfluenza virus, respiratory syncytial virus, Punta Toro virus, Tacaribe virus, and Pichinde virus.

In one embodiment, the virus is selected from the group consisting of adenovirus, Dengue Fever virus, Ebola virus, Marburg virus, influenza A virus, influenza B virus, Junin virus, measles virus, parainfluenza virus, Pichinde virus, Punta Toro virus, respiratory syncytial virus, rhinovirus, Rift Valley Fever virus, SARS virus, Tacaribe virus, Venezuelan equine encephalitis virus, West Nile virus, and yellow fever virus.

In one embodiment, the virus is selected from the group consisting of Ebola virus, yellow fever virus, Marburg virus, influenza A virus, and influenza B virus.

An aspect of the invention is a method of inhibiting replication of a virus. The method includes the step of contacting a virus with an effective amount of a compound of the invention, or a pharmaceutically acceptable salt thereof, thereby inhibiting replication of the virus.

In one embodiment, the virus is selected from the group consisting of RNA viruses.

In one embodiment, the virus is selected from the group consisting of orthomyxoviridae, paramyxoviridae, arenaviridae, bunyaviridae, flaviviridae, filoviridae, togaviridae, picornaviridae, and coronaviridae.

In one embodiment, the virus is selected from the group consisting of adenovirus, rhinovirus, hepatitis A virus, hepatitis C virus, polio virus, measles virus, Ebola virus, Coxsackie virus, West Nile virus, smallpox virus, yellow fever virus, Dengue Fever virus, influenza A virus, influenza B virus, lassa virus, lymphocytic choriomeningitis virus, Junin virus, machuppo virus, guanarito virus, hantavirus, Rift Valley Fever virus, La Crosse virus, California encephalitis virus, Crimean-Congo virus, Marburg virus, Japanese encephalitis virus, Kyasanur Forest virus, Venezuelan equine encephalitis virus, Eastern equine encephalitis virus, Western equine encephalitis virus, severe acute respiratory syndrome (SARS) virus, parainfluenza virus, respiratory syncytial virus, Punta Toro virus, Tacaribe virus, and Pichinde virus.

In one embodiment, the virus is selected from the group consisting of adenovirus, Dengue Fever virus, Ebola virus, Marburg virus, influenza A virus, influenza B virus, Junin virus, measles virus, parainfluenza virus, Pichinde virus, Punta Toro virus, respiratory syncytial virus, rhinovirus, Rift Valley Fever virus, SARS virus, Tacaribe virus, Venezuelan equine encephalitis virus, West Nile virus, and yellow fever virus.

In one embodiment, the virus is selected from the group consisting of Ebola virus, yellow fever virus, Marburg virus, influenza A virus, and influenza B virus.

An aspect of the invention is a method of treating a viral infection in a subject. The method includes the step of administering to a subject in need thereof an effective amount of a compound of the invention, or a pharmaceutically acceptable salt thereof.

In one embodiment, the virus is selected from the group consisting of RNA viruses.

In one embodiment, the virus is selected from the group consisting of orthomyxoviridae, paramyxoviridae, arenaviridae, bunyaviridae, flaviviridae, filoviridae, togaviridae, picornaviridae, and coronaviridae.

In one embodiment, the virus is selected from the group consisting of adenovirus, rhinovirus, hepatitis A virus, hepatitis C virus, polio virus, measles virus, Ebola virus, Coxsackie virus, West Nile virus, smallpox virus, yellow fever virus, Dengue Fever virus, influenza A virus, influenza B virus, lassa virus, lymphocytic choriomeningitis virus, Junin virus, machuppo virus, guanarito virus, hantavirus, Rift Valley Fever virus, La Crosse virus, California encephalitis virus, Crimean-Congo virus, Marburg virus, Japanese encephalitis virus, Kyasanur Forest virus, Venezuelan equine encephalitis virus, Eastern equine encephalitis virus, Western equine encephalitis virus, severe acute respiratory syndrome (SARS) virus, parainfluenza virus, respiratory syncytial virus, Punta Toro virus, Tacaribe virus, and Pichinde virus.

In one embodiment, the virus is selected from the group consisting of adenovirus, Dengue Fever virus, Ebola virus, Marburg virus, influenza A virus, influenza B virus, Junin virus, measles virus, parainfluenza virus, Pichinde virus, Punta Toro virus, respiratory syncytial virus, rhinovirus, Rift Valley Fever virus, SARS virus, Tacaribe virus, Venezuelan equine encephalitis virus, West Nile virus, and yellow fever virus.

In one embodiment, the virus is selected from the group consisting of Ebola virus, yellow fever virus, Marburg virus, influenza A virus, and influenza B virus.

### Definitions

The term "alkyl" as used herein is a term of art and refers to saturated aliphatic groups, including straight-chain alkyl groups, branched-chain alkyl groups, cycloalkyl (alicyclic) groups, alkyl substituted cycloalkyl groups, and cycloalkyl substituted alkyl groups. In certain embodiments, a straight chain or branched chain alkyl has about 30 or fewer carbon atoms in its backbone (e.g., C₁-C₃₀ for straight chain, C₃-C₃₀ for branched chain), and alternatively, about 20 or fewer. Likewise, cycloalkyls have from about 3 to about 10 carbon atoms in their ring structure, and alternatively about 5, 6 or 7 carbons in the ring structure.

The term "amino" is a term of art and as used herein refers to both unsubstituted and substituted amines, e.g., a moiety that may be represented by the general formulas: 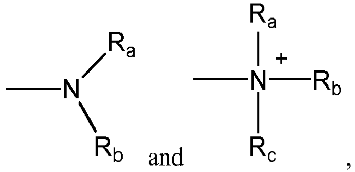 wherein Rₐ, R_{b}, and R_{c} each independently represent a hydrogen, an alkyl, an alkenyl, -(CH₂)ₓ-R_{d}, or Rₐ and R_{b}, taken together with the N atom to which they are attached complete a heterocycle having from 4 to 8 atoms in the ring structure; R_{d} represents an aryl, a cycloalkyl, a cycloalkenyl, a heterocyclyl or a polycyclyl; and x is zero or an integer in the range of 1 to 8. In certain embodiments, only one of Rₐ or R_{b} may be a carbonyl, e.g., Rₐ, R_{b}, and the nitrogen together do not form an imide. In other embodiments, Rₐ and R_{b} (and optionally R_{c}) each independently represent a hydrogen, an alkyl, an alkenyl, or -(CH₂)ₓ-R_{d}.

The term "acyl" is a term of art and as used herein refers to any group or radical of the form RCO- where R is any organic group, e.g., alkyl, aryl, heteroaryl, aralkyl, and heteroaralkyl. Representative acyl groups include acetyl, benzoyl, and malonyl.

The term "aminoalkyl" as used herein refers to an alkyl group substituted with one or more one amino groups.

The term "aminoacyl" is a term of art and as used herein refers to an acyl group substituted with one or more amino groups.

The term "aminothionyl" as used herein refers to an analog of an aminoacyl in which the O of RC(O)- has been replaced by sulfur, hence is of the form RC(S)-.

The term "phosphoryl" is a term of art and as used herein may in general be represented by the formula: 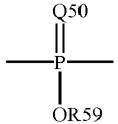 wherein Q50 represents S or O, and R59 represents hydrogen, a lower alkyl or an aryl; for example, -P(O)(OMe)- or -P(O)(OH)₂. When used to substitute, e.g., an alkyl, the phosphoryl group of the phosphorylalkyl may be represented by the general formulas: 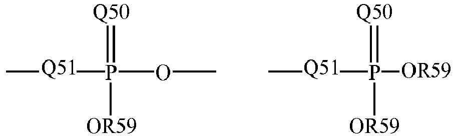 wherein Q50 and R59, each independently, are defined above, and Q51 represents O, S or N; for example, -O-P(O)(OH)OMe or -NH-P(O)(OH)₂. When Q50 is S, the phosphoryl moiety is a "phosphorothioate."

The term "aminophosphoryl" as used herein refers to a phosphoryl group substituted with at least one amino group, as defined herein; for example, -P(O)(OH)NMe₂.

The term "carbonyl" as used herein refers to -C(O)-.

The term "thiocarbonyl" as used herein refers to -C(S)-.

The term "alkylphosphoryl" as used herein refers to a phosphoryl group substituted with at least one alkyl group, as defined herein; for example, -P(O)(OH)Me.

The term "alkylthio" as used herein refers to alkyl-S-.

The term "aryl" is a term of art and as used herein refers to includes monocyclic, bicyclic and polycyclic aromatic hydrocarbon groups, for example, benzene, naphthalene, anthracene, and pyrene. The aromatic ring may be substituted at one or more ring positions with one or more substituents, such as halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, fluoroalkyl (such as trifluromethyl), cyano, or the like. The term "aryl" also includes polycyclic ring systems having two or more cyclic rings in which two or more carbons are common to two adjoining rings (the rings are "fused rings") wherein at least one of the rings is an aromatic hydrocarbon, e.g., the other cyclic rings may be cycloalkyls, cycloalkenyls, cycloalkynyls, aryls, heteroaryls, and/or heterocyclyls.

The term "heteroatom" is art-recognized, and includes an atom of any element other than carbon or hydrogen. Illustrative heteroatoms include boron, nitrogen, oxygen, phosphorus, sulfur and selenium, and alternatively oxygen, nitrogen or sulfur.

The term "heteroaryl" is a term of art and as used herein refers to a monocyclic, bicyclic and polycyclic aromatic group having one or more heteroatoms in the ring structure, for example, pyrrole, furan, thiophene, imidazole, oxazole, thiazole, triazole, pyrazole, pyridine, pyrazine, pyridazine and pyrimidine, and the like. The "heteroaryl" may be substituted at one or more ring positions with one or more substituents such as halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, fluoroalkyl (such as trifluromethyl), cyano, or the like. The term "heteroaryl" also includes polycyclic ring systems having two or more cyclic rings in which two or more carbons are common to two adjoining rings (the rings are "fused rings") wherein at least one of the rings is an aromatic group having one or more heteroatoms in the ring structure, e.g., the other cyclic rings may be cycloalkyls, cycloalkenyls, cycloalkynyls, aryls, heteroaryls, and/or heterocyclyls.

The term "aralkyl" is a term of art and as used herein refers to an alkyl group substituted with an aryl group.

The term "heteroaralkyl" is a term of art and as used herein refers to an alkyl group substituted with a heteroaryl group.

Certain compounds contained in compositions of the present invention may exist in particular geometric or stereoisomeric forms. In addition, compounds of the present invention may also be optically active. The present invention contemplates all such compounds, including *cis-* and *trans*-isomers, *(R)-* and (S)-enantiomers, diastereoisomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, fragmentation, decomposition, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

For purposes of the invention, the chemical elements are identified in accordance with the Periodic Table of the Elements, CAS version, Handbook of Chemistry and Physics, 67th Ed., 1986-87, inside cover.

Other chemistry terms herein are used according to conventional usage in the art, as exemplified by The McGraw-Hill Dictionary of Chemical Terms (ed. Parker, S., 1985), McGraw-Hill, San Francisco, incorporated herein by reference). Unless otherwise defined, all technical and scientific terms used herein have the same meaning as commonly understood by one of ordinary skill in the art to which this invention pertains.

The term "protecting group" as used herein temporary substituents which protect a potentially reactive functional group from undesired chemical transformations. Examples of such protecting groups include esters of carboxylic acids and boronic acids, ethers of alcohols, and acetals and ketals of aldehydes and ketones. For instance, the phrase "N-terminal protecting group" or "amino-protecting group" as used herein refers to various amino-protecting groups which can be employed to protect the N-terminus of an amino acid or peptide against undesirable reactions during synthetic procedures. Examples of suitable groups include acyl protecting groups such as, to illustrate, formyl, dansyl, acetyl, benzoyl, trifluoroacetyl, succinyl, and methoxysuccinyl; aromatic urethane protecting groups as, for example, benzyloxycarbonyl (Cbz); and aliphatic urethane protecting groups such as t-butoxycarbonyl (Boc) or 9-Fluorenylmethoxycarbonyl (Fmoc).

The term "amino-protecting group" or "*N*-terminal protecting group" refers to those groups intended to protect the α-*N-*terminal of an amino acid or peptide or to otherwise protect the amino group of an amino acid or peptide against undesirable reactions during synthetic procedures. Commonly used *N*-protecting groups are disclosed in Greene, Projective Groups In Organic Synthesis, (John Wiley & Sons, New York (1981)), which is hereby incorporated by reference. Additionally, protecting groups can be used as pro-drugs which are readily cleaved in vivo, for example, by enzymatic hydrolysis, to release the biologically active parent. α-*N*-protecting groups comprise lower alkanoyl groups such as formyl, acetyl ("Ac"), propionyl, pivaloyl, t-butylacetyl and the like; other acyl groups include 2-chloroacetyl, 2-bromoacetyl, trifluoroacetyl, trichloroacetyl, phthalyl, o-nitrophenoxyacetyl, -chlorobutyryl, benzoyl, 4-chlorobenzoyl, 4-bromobenzoyl, 4-nitrobenzoyl and the like; sulfonyl groups such as benzenesulfonyl, p-toluenesulfonyl and the like; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-ethoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyoxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl and the like; arylalkyl groups such as benzyl, triphenylmethyl, benzyloxymethyl, 9-fluorenylmethyloxycarbonyl (Fmoc) and the like and silyl groups such as trimethylsilyl and the like. Still other examples include theyl, succinyl, methoxysuccinyl, subery, adipyl, azelayl, dansyl, benzyloxycarbonyl, methoxyazelaly, methoxyadipyl, methoxysuberyl, and 2,4-dinitrophenyl.

The term "carboxy protecting group" or "C-terminal protecting group" refers to a carboxylic acid protecting ester or amide group employed to block or protect the carboxylic acid functionality while the reactions involving other functional sites of the compound are performed. Carboxy protecting groups are disclosed in Greene, Projective Groups in Organic Synthesis pp. 152-186 (1981), which is hereby incorporated by reference. Additionally, a carboxy protecting group can be used as a pro-drug whereby the carboxy protecting group can be readily cleaved in vivo, for example by enzymatic hydrolysis, to release the biologically active parent. Such carboxy protecting groups are well known to those skilled in the art, having been extensively used in the protection of carboxyl groups in the penicillin and cephalosporin fields as described in U.S. Pat. Nos. 3,840,556 and 3,719,667, the disclosures of which are hereby incorporated herein by reference. Representative carboxy protecting groups are C₁ -C₈ loweralkyl (e.g., methyl, ethyl or t-butyl and the like); arylalkyl such as phenethyl or benzyl and substituted derivatives thereof such as alkoxybenzyl or nitrobenzyl groups and the like; arylalkenyl such as phenylethenyl and the like; aryl and substituted derivatives thereofsuch as 5-indanyl and the like; dialkylaminoalkyl such as dimethylaminoethyl and the like); alkanoyloxyalkyl groups such as acetoxymethyl, butyryloxymethyl, valeryloxymethyl, isobutyryloxymethyl, isovaleryloxymethyl, 1-(propionyloxy)-1-ethyl, 1-(pivaloyloxyl)-1-ethyl, 1-methyl-1-(propionyloxy)-1-ethyl, pivaloyloxymethyl, propionyloxymethyl and the like; cycloalkanoyloxyalkyl groups such as cyclopropylcarbonyloxymethyl, cyclobutylcarbonyloxymethyl, cyclopentylcarbonyloxymethyl, cyclohexylcarbonyloxymethyl and the like; aroyloxyalkyl such as benzoyloxymethyl, benzoyloxyethyl and the like; arylalkylcarbonyloxyalkyl such as benzylcarbonyloxymethyl, 2-benzylcarbonyloxyethyl and the like; alkoxycarbonylalkyl or cycloalkyloxycarbonylalkyl such as methoxycarbonylmethyl, cyclohexyloxycarbonylmethyl, 1-methoxycarbonyl-1-ethyl and the like; alkoxycarbonyloxyalkyl or cycloalkyloxycarbonyloxyalkyl such as methoxycarbonyloxymethyl, t-butyloxycarbonyloxymethyl, 1-ethoxycarbonyloxy-1-ethyl, 1-cyclohexyloxycarbonyloxy-1-ethyl and the like; aryloxycarbonyloxyalkyl such as 2-(phenoxycarbonyloxy)ethyl, 2-(5-indanyloxycarbonyloxy)ethyl and the like; alkoxyalkylcarbonyloxyalkyl such as 2-(1-methoxy-2-methylpropan-2-oyloxy)ethyl and like; arylalkyloxycarbonyloxyalkyl such as 2-(benzyloxycarbonyloxy)ethyl and the like; arylalkenyloxycarbonyloxyalkyl such as 2-(3-phenylpropen-2-yloxycarbonyloxy)ethyl and the like; alkoxycarbonylaminoalkyl such as t-butyloxycarbonylaminomethyl and the like; alkylaminocarbonylaminoalkyl such as methylaminocarbonylaminomethyl and the like; alkanoylaminoalkyl such as acetylaminomethyl and the like; heterocycliccarbonyloxyalkyl such as 4-methylpiperazinylcarbonyloxymethyl and the like; dialkylaminocarbonylalkyl such as dimethylaminocarbonylmethyl, diethylaminocarbonylmethyl and the like; (5-(loweralkyl)-2-oxo-1,3-dioxolen-4-yl)alkyl such as (5-t-butyl-2-oxo-1,3-dioxolen-4-yl)methyl and the like; and (5-phenyl-2-oxo-1,3-dioxolen-4-yl)alkyl such as (5-phenyl-2-oxo-1,3-dioxolen-4-yl)methyl and the like. Representative amide carboxy protecting groups are aminocarbonyl and lower alkylaminocarbonyl groups. For example, aspartic acid may be protected at the α-C-terminal by an acid labile group (e.g., t-butyl) and protected at the β-*C*-terminal by a hydrogenation labile group (e.g., benzyl) then deprotected selectively during synthesis. As mentioned above, the protected carboxy group may also be a loweralkyl, cycloalkyl or arylalkyl ester, for example, methyl ester, ethyl ester, propyl ester, isopropyl ester, butyl ester, sec-butyl ester, isobutyl ester, amyl ester, isoamyl ester, octyl ester, cyclohexyl ester, phenylethyl ester and the like or an alkanoyloxyalkyl, cycloalkanoyloxyalkyl, aroyloxyalkyl or an arylalkylcarbonyloxyalkyl ester.

The term "amino acid" as used herein is a term of art and refers to alpha- and beta-aminocarboxylic acids, including so-called naturally occurring alpha-amino acids and non-naturally occurring amino acids. Naturally occurring alpha-amino acids specifically include alanine (Ala), arginine (Arg), asparagine (Asn), aspartic acid (Asp), cysteine (Cys), glutamic acid (Glu), glutamine (Gln), glycine (Gly), histidine (His), isoleucine (Ile), leucine (Leu), lysine (Lys), methionine (Met), ornithine (Orn), phenylalanine (Phe), proline (Pro), selenocysteine, serine (Ser), taurine, threonine (Thr), tryptophan (Trp), tyrosine (Tyr), and valine (Val). Polar naturally occurring alpha-amino acids include arginine, asparagine, aspartic acid, cysteine, glutamic acid, glutamine, histidine, lysine, ornithine, serine, threonine, and tyrosine. Nonpolar naturally occurring alpha-amino acids include alanine, glycine, isoleucine, leucine, methionine, phenylalanine, proline, tryptophan, and valine.

Non-naturally occurring amino acids include, but are not limited to, D-amino acids (i.e., an amino acid of an opposite chirality to the naturally occurring form), *N*-α-methyl amino acids, *C*-α-methyl amino acids, β-methyl amino acids, β-alanine (β-Ala), norvaline (Nva), norleucine (Nle), 4-aminobutyric acid (γ-Abu), 2-aminoisobutyric acid (Aib), 6-aminohexanoic acid (ε-Ahx), ornithine (orn), hydroxyproline (Hyp), sarcosine, citrulline, cysteic acid, cyclohexylalanine, α-amino isobutyric acid, t-butylglycine, t-butylalanine, 3-aminopropionic acid, 2,3-diaminopropionic acid (2,3-diaP), D- or L-phenylglycine, D- or L-2-naphthylalanine (2-Nal), 1,2,3,4-tetrahydroisoquinoline-3-carboxylic acid (Tic), D- or L-2-thienylalanine (Thi), D- or L-3-thienylalanine, D- or L-1-, 2-, 3- or 4-pyrenylalanine, D- or L-(2-pyridinyl)-alanine, D- or L-(3-pyridinyl)-alanine, D- or L-(2-pyrazinyl)-alanine, D- or L-(4-isopropyl)-phenylglycine, D-(trifluoromethyl)-phenylglycine, D-(trifluoromethyl)-phenylalanine, D-p-fluorophenylalanine, D- or L-p-biphenylalanine, D- or L-p-methoxybiphenylalanine, methionine sulphoxide (MSO) and homoarginine (Har). Other examples include D- or L-2-indole(alkyl)alanines and D- or L-alkylalanines, wherein alkyl is substituted or unsubstituted methyl, ethyl, propyl, hexyl, butyl, pentyl, isopropyl, iso-butyl, or iso-pentyl, and phosphono- or sulfated (e.g., -SO₃H) non-carboxylate amino acids.

Other examples of non-naturally occurring amino acids include 3-(2-chlorophenyl)-alanine, 3-chloro-phenylalanine, 4-chloro-phenylalanine, 2-fluoro-phenylalanine, 3-fluoro-phenylalanine, 4-fluoro-phenylalanine, 2-bromo-phenylalanine, 3-bromo-phenylalanine, 4-bromo-phenylalanine, homophenylalanine, 2-methyl-phenylalanine, 3-methyl-phenylalanine, 4-methyl-phenylalanine, 2,4-dimethyl-phenylalanine, 2-nitro-phenylalanine, 3-nitro-phenylalanine, 4-nitro-phenylalanine, 2,4-dinitro-phenylalanine, 1,2,3,4-Tetrahydroisoquinoline-3-carboxylic acid, 1,2,3,4-tetrahydronorharman-3-carboxylic acid, 1-naphthylalanine, 2-naphthylalanine, pentafluorophenylalanine, 2,4-dichloro-phenylalanine, 3,4-dichloro-phenylalanine, 3,4-difluoro-phenylalanine, 3,5-difluoro-phenylalanine, 2,4,5-trifluoro-phenylalanine, 2-trifluoromethyl-phenylalanine, 3-trifluoromethyl-phenylalanine, 4-trifluoromethyl-phenylalanine, 2-cyano-phenyalanine, 3-cyano-phenyalanine, 4-cyano-phenyalanine, 2-iodo-phenyalanine, 3-iodo-phenyalanine, 4-iodo-phenyalanine, 4-methoxyphenylalanine, 2-aminomethyl-phenylalanine, 3-aminomethyl-phenylalanine, 4-aminomethyl-phenylalanine, 2-carbamoyl-phenylalanine, 3-carbamoyl-phenylalanine, 4-carbamoyl-phenylalanine, m-tyrosine, 4-amino-phenylalanine, styrylalanine, 2-amino-5-phenyl-pentanoic acid, 9-anthrylalanine, 4-tert-butyl-phenylalanine, 3,3-diphenylalanine, 4,4'-diphenylalanine, benzoylphenylalanine, α-methyl-phenylalanine, α-methyl-4-fluoro-phenylalanine, 4-thiazolylalanine, 3-benzothienylalanine, 2-thienylalanine, 2-(5-bromothienyl)-alanine, 3-thienylalanine, 2-furylalanine, 2-pyridylalanine, 3-pyridylalanine, 4-pyridylalanine, 2,3-diaminopropionic acid, 2,4-diaminobutyric acid, allylglycine, 2-amino-4-bromo-4-pentenoic acid, propargylglycine, 4-aminocyclopent-2-enecarboxylic acid, 3-aminocyclopentanecarboxylic acid, 7-amino-heptanoic acid, dipropylglycine, pipecolic acid, azetidine-3-carboxylic acid, cyclopropylglycine, cyclopropylalanine, 2-methoxy-phenylglycine, 2-thienylglycine, 3-thienylglycine, α-benzyl-proline, α-(2-fluoro-benzyl)-proline, α-(3-fluoro-benzyl)-proline, α-(4-fluoro-benzyl)-proline, α-(2-chloro-benzyl)-proline, α-(3-chloro-benzyl)-proline, α-(4-chloro-benzyl)-proline, α-(2-bromo-benzyl)-proline, α-(3-bromo-benzyl)-proline, α-(4-bromo-benzyl)-proline, α-phenethyl-proline, α-(2-methyl-benzyl)-proline, α-(3-methyl-benzyl)-proline, α-(4-methyl-benzyl)-proline, α-(2-nitro-benzyl)-proline, α-(3-nitro-benzyl)-proline, α-(4-nitro-benzyl)-proline, α-(1-naphthalenylmethyl)-proline, α-(2-naphthalenylmethyl)-proline, α-(2,4-dichloro-benzyl)-proline, α-(3,4-dichloro-benzyl)-proline, α-(3,4-difluoro-benzyl)-proline, α-(2-trifluoromethyl-benzyl)-proline, α-(3-trifluoromethyl-benzyl)-proline, α-(4-trifluoromethyl-benzyl)-proline, α-(2-cyano-benzyl)-proline, α-(3-cyano-benzyl)-proline, α-(4-cyano-benzyl)-proline, α-(2-iodo-benzyl)-proline, α-(3-iodo-benzyl)-proline, α-(4-iodo-benzyl)-proline, α-(3-phenyl-allyl)-proline, α-(3-phenyl-propyl)-proline, α-(4-tert-butyl-benzyl)-proline, α-benzhydryl-proline, α-(4-biphenylmethyl)-proline, α-(4-thiazolylmethyl)-proline, α-(3-benzo[b]thiophenylmethyl)-proline, α-(2-thiophenylmethyl)-proline, α-(5-bromo-2-thiophenylmethyl)-proline, α-(3-thiophenylmethyl)-proline, α-(2-furanylmethyl)-proline, α-(2-pyridinylmethyl)-proline, α-(3-pyridinylmethyl)-proline, α-(4-pyridinylmethyl)-proline, α-allyl-proline, α-propynyl-proline, γ-benzyl-proline, γ-(2-fluoro-benzyl)-proline, γ-(3-fluoro-benzyl)-proline, γ-(4-fluoro-benzyl)-proline, γ-(2-chloro-benzyl)-proline, γ-(3-chloro-benzyl)-proline, γ-(4-chloro-benzyl)-proline, γ-(2-bromo-benzyl)-proline, γ-(3-bromo-benzyl)-proline, γ-(4-bromo-benzyl)-proline, γ-(2-methyl-benzyl)-proline, γ-(3-methyl-benzyl)-proline, γ-(4-methyl-benzyl)-proline, γ-(2-nitro-benzyl)-proline, γ-(3-nitro-benzyl)-proline, γ-(4-nitro-benzyl)-proline, γ-(1-naphthalenylmethyl)-proline, γ-(2-naphthalenylmethyl)-proline, γ-(2,4-dichloro-benzyl)-proline, γ-(3,4-dichloro-benzyl)-proline, γ-(3,4-difluoro-benzyl)-proline, γ-(2-trifluoromethyl-benzyl)-proline, γ-(3-trifluoromethyl-benzyl)-proline, γ-(4-trifluoromethyl-benzyl)-proline, γ-(2-cyano-benzyl)-proline, γ-(3-cyano-benzyl)-proline, γ-(4-cyano-benzyl)-proline, γ-(2-iodo-benzyl)-proline, γ-(3-iodo-benzyl)-proline, γ-(4-iodo-benzyl)-proline, γ-(3-phenyl-allyl-benzyl)-proline, γ-(3-phenyl-propyl-benzyl)-proline, γ-(4-tert-butyl-benzyl)-proline, γ-benzhydryl-proline, γ-(4-biphenylmethyl)-proline, γ-(4-thiazolylmethyl)-proline, γ-(3-benzothioienylmethyl)-proline, γ-(2-thienylmethyl)-proline, γ-(3-thienylmethyl)-proline, γ-(2-furanylmethyl)-proline, γ-(2-pyridinylmethyl)-proline, γ-(3-pyridinylmethyl)-proline, γ-(4-pyridinylmethyl)-proline, γ-allyl-proline, γ-propynyl-proline, trans-4-phenyl-pyrrolidine-3-carboxylic acid, trans-4-(2-fluoro-phenyl)-pyrrolidine-3-carboxylic acid, trans-4-(3-fluoro-phenyl)-pyrrolidine-3-carboxylic acid, trans-4-(4-fluoro-phenyl)-pyrrolidine-3-carboxylic acid, trans-4-(2-chlorophenyl)-pyrrolidine-3-carboxylic acid, trans-4-(3-chloro-phenyl)-pyrrolidine-3-carboxylic acid, trans-4-(4-chloro-phenyl)-pyrrolidine-3-carboxylic acid, trans-4-(2-bromo-phenyl)-pyrrolidine-3-carboxylic acid, trans-4-(3-bromo-phenyl)-pyrrolidine-3-carboxylic acid, trans-4-(4-bromo-phenyl)-pyrrolidine-3-carboxylic acid, trans-4-(2-methyl-phenyl)-pyrrolidine-3-carboxylic acid, trans-4-(3-methyl-phenyl)-pyrrolidine-3-carboxylic acid, trans-4-(4-methylphenyl)-pyrrolidine-3-carboxylic acid, trans-4-(2-nitro-phenyl)-pyrrolidine-3-carboxylic acid, trans-4-(3-nitro-phenyl)-pyrrolidine-3-carboxylic acid, trans-4-(4-nitro-phenyl)-pyrrolidine-3-carboxylic acid, trans-4-(1-naphthyl)-pyrrolidine-3-carboxylic acid, trans-4-(2-naphthyl)-pyrrolidine-3-carboxylic acid, trans-4-(2,5-dichloro-phenyl)-pyrrolidine-3-carboxylic acid, trans-4-(2,3-dichloro-phenyl)-pyrrolidine-3-carboxylic acid, trans-4-(2-trifluoromethylphenyl)-pyrrolidine-3-carboxylic acid, trans-4-(3-trifluoromethyl-phenyl)-pyrrolidine-3-carboxylic acid, trans-4-(4-trifluoromethyl-phenyl)-pyrrolidine-3-carboxylic acid, trans-4-(2-cyano-phenyl)-pyrrolidine-3-carboxylic acid, trans-4-(3-cyano-phenyl)-pyrrolidine-3-carboxylic acid, trans-4-(4-cyano-phenyl)-pyrrolidine-3-carboxylic acid, trans-4-(2-methoxyphenyl)-pyrrolidine-3-carboxylic acid, trans-4-(3-methoxy-phenyl)-pyrrolidine-3-carboxylic acid, trans-4-(4-methoxy-phenyl)-pyrrolidine-3-carboxylic acid, trans-4-(2-hydroxy-phenyl)-pyrrolidine-3-carboxylic acid, trans-4-(3-hydroxy-phenyl)-pyrrolidine-3-carboxylic acid, trans-4-(4-hydroxy-phenyl)-pyrrolidine-3-carboxylic acid, trans-4-(2,3-dimethoxy-phenyl)-pyrrolidine-3-carboxylic acid, trans-4-(3,4-dimethoxy-phenyl)-pyrrolidine-3-carboxylic acid, trans-4-(3,5-dimethoxy-phenyl)-pyrrolidine-3-carboxylic acid, trans-4-(2-pyridinyl)-pyrrolidine-3-carboxylic acid, trans-4-(3-pyridinyl)-pyrrolidine-3-carboxylic acid, trans-4-(6-methoxy-3-pyridinyl)-pyrrolidine-3-carboxylic acid, trans-4-(4-pyridinyl)-pyrrolidine-3-carboxylic acid, trans-4-(2-thienyl)-pyrrolidine-3-carboxylic acid, trans-4-(3-thienyl)-pyrrolidine-3-carboxylic acid, trans-4-(2-furanyl)-pyrrolidine-3-carboxylic acid, trans-4-isopropyl-pyrrolidine-3-carboxylic acid, 4-phosphonomethyl-phenylalanine, benzyl-phosphothreonine, (1'-amino-2-phenyl-ethyl)oxirane, (1'-amino-2-cyclohexyl-ethyl)oxirane, (1'-amino-2-[3-bromo-phenyl]ethyl)oxirane, (1'-amino-2-[4-(benzyloxy)phenyl]ethyl)oxirane, (1'-amino-2-[3,5-difluoro-phenyl]ethyl)oxirane, (1'-amino-2-[4-carbamoyl-phenyl]ethyl)oxirane, (1'-amino-2-[benzyloxy-ethyl])oxirane, (1'-amino-2-[4-nitro-phenyl]ethyl)oxirane, (1'-amino-3-phenyl-propyl)oxirane, (1'-amino-3-phenylpropyl)oxirane, and/or salts and/or protecting group variants thereof.

Beta-amino acids include, without limitation, beta-alanine (3-aminopropanoic acid).

The term "pharmaceutically acceptable salt" as used herein includes salts derived from inorganic or organic acids including, for example, hydrochloric, hydrobromic, sulfuric, nitric, perchloric, phosphoric, formic, acetic, lactic, maleic, fumaric, succinic, tartaric, glycolic, salicylic, citric, methanesulfonic, benzenesulfonic, benzoic, malonic, trifluoroacetic, trichloroacetic, naphthalene-2-sulfonic, and other acids. Pharmaceutically acceptable salt forms can include forms wherein the ratio of molecules comprising the salt is not 1:1. For example, the salt may comprise more than one inorganic or organic acid molecule per molecule of base, such as two hydrochloric acid molecules per molecule of compound of Formula I. As another example, the salt may comprise less than one inorganic or organic acid molecule per molecule of base, such as two molecules of compound of Formula I per molecule of tartaric acid.

The terms "carrier" and "pharmaceutically acceptable carrier" as used herein refer to a diluent, adjuvant, excipient, or vehicle with which a compound is administered or formulated for administration. Non-limiting examples of such pharmaceutically acceptable carriers include liquids, such as water, saline, and oils; and solids, such as gum acacia, gelatin, starch paste, talc, keratin, colloidal silica, urea, and the like. In addition, auxiliary, stabilizing, thickening, lubricating, flavoring, and coloring agents may be used. Other examples of suitable pharmaceutical carriers are described in *Remington's Pharmaceutical Sciences* by E.W. Martin, herein incorporated by reference in its entirety.

The term "treat" as used herein means prevent, halt or slow the progression of, or eliminate a disease or condition in a subject. In one embodiment "treat" means halt or slow the progression of, or eliminate a disease or condition in a subject. In one embodiment, "treat" means reduce at least one objective manifestation of a disease or condition.

The term "effective amount" as used herein refers to an amount that is sufficient to bring about a desired biological effect.

The term "inhibit" as used herein means decrease by an objectively measurable amount or extent. In various embodiments "inhibit" means decrease by at least 5, 10, 20, 30, 40, 50, 60, 70, 80, 90, or 95 percent compared to relevant control. In one embodiment "inhibit" means decrease 100 percent, i.e, halt or eliminate.

The term "subject" as used herein refers to a mammal. In various embodiments, a subject is a mouse, rat, rabbit, cat, dog, pig, sheep, horse, cow, or non-human primate. In one embodiment a subject is a human.

### General Methods of Preparation of Compounds of the Invention

Heterocycles and heteroaryls can be prepared from known methods as reported in the literature (**a**. Ring system handbook, published by American Chemical Society edition 1993 and subsequent supplements. **b.** The Chemistry of Heterocyclic Compounds; Weissberger, A., Ed.; Wiley: New York, 1962. **c.** Nesynov, E. P.; Grekov, A. P. The chemistry of 1,3,4-oxadiazole derivatives. Russ. Chem. Rev. 1964, 33, 508-515. **d.** Advances in Heterocyclic Chemistry; Katritzky, A. R., Boulton, A. J., Eds.; Academic Press: New York, 1966. **e.** In Comprehensive Heterocyclic Chemistry; Potts, K. T., Ed.; Pergamon Press: Oxford, 1984. **f.** Eloy, F. A review of the chemistry of 1,2,4-oxadiazoles. Fortschr. Chem. Forsch. 1965, 4, pp 807-876. **g.** Adv. Heterocycl. Chem. 1976**. h.** Comprehensive Heterocyclic Chemistry; Potts, K. T., Ed.; Pergamon Press: Oxford, 1984. i. Chem. Rev. 1961 61, 87-127. **j**. 1,2,4-Triazoles; John Wiley & Sons: New York, 1981; Vol 37). Functional groups during the synthesis may need to be protected and subsequently deprotected. Examples of suitable protecting groups can be found in Projective Groups in Organic Synthesis, fourth edition, edited by Greene and Wuts.

Representative processes which can be used to prepare compounds of the invention and intermediates useful for preparing same are shown in the following Schemes.

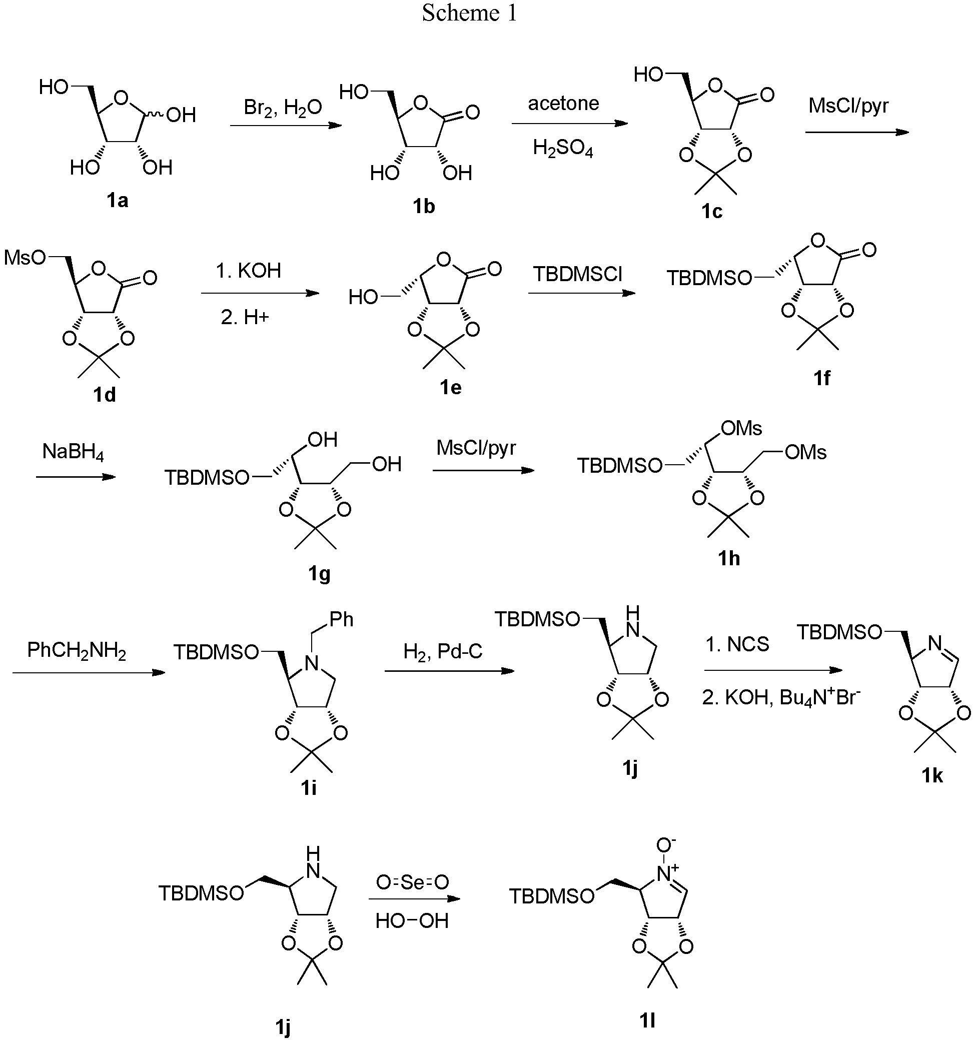

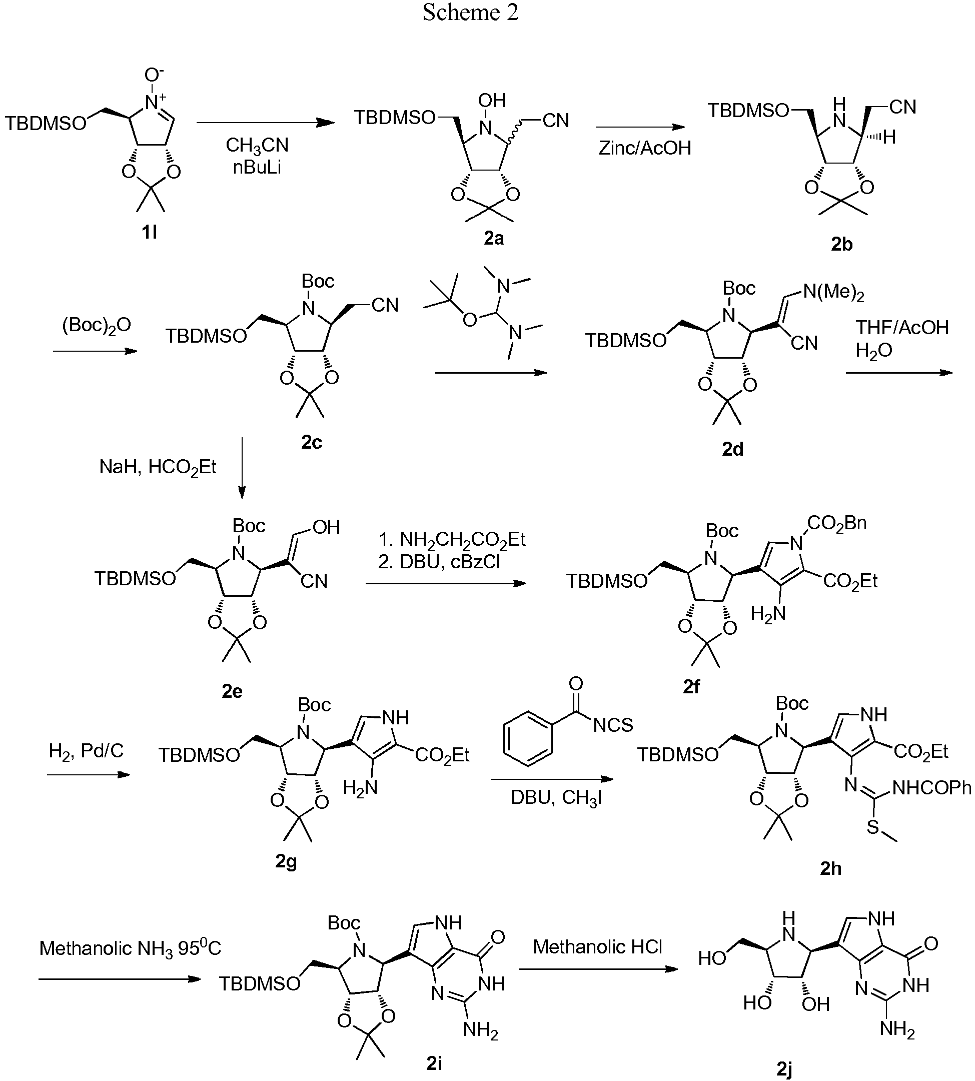

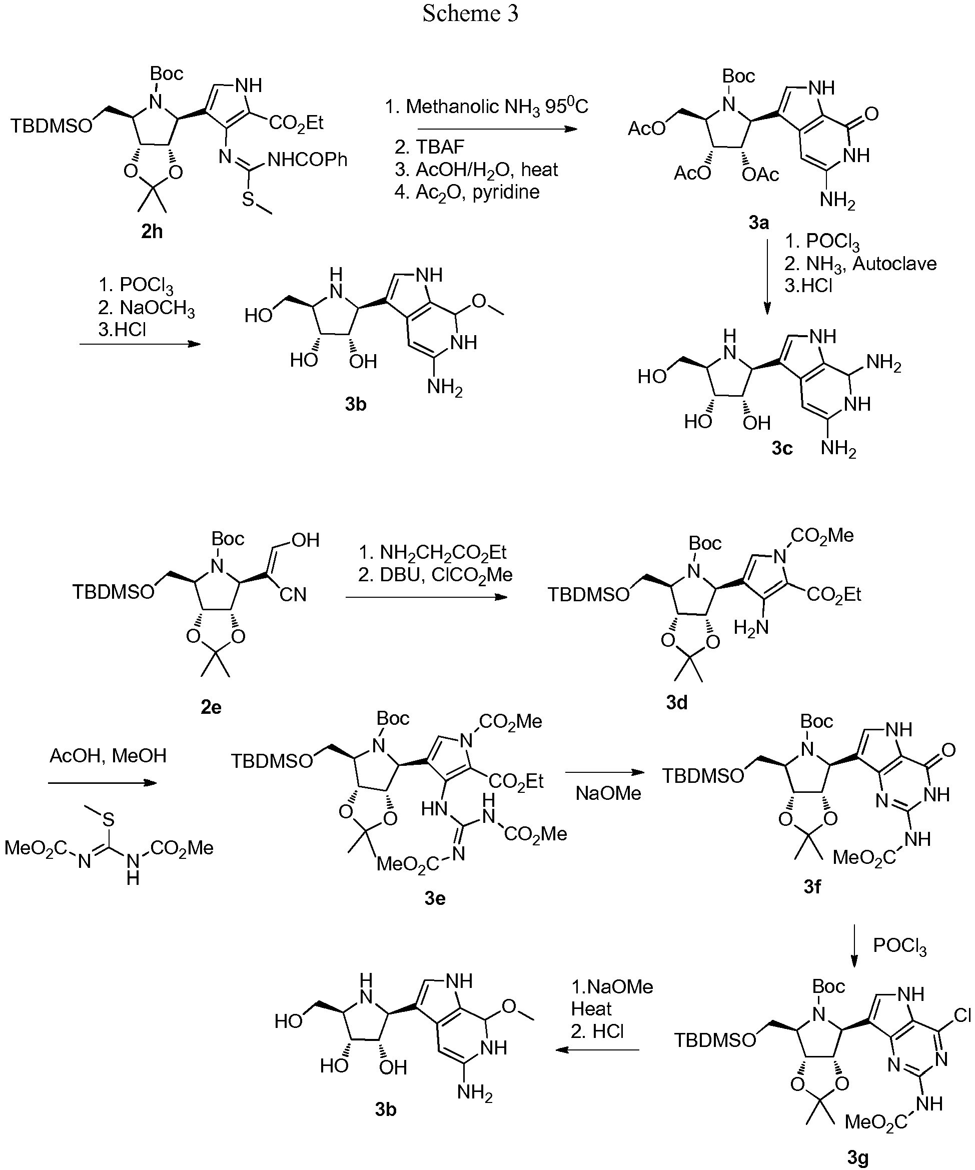

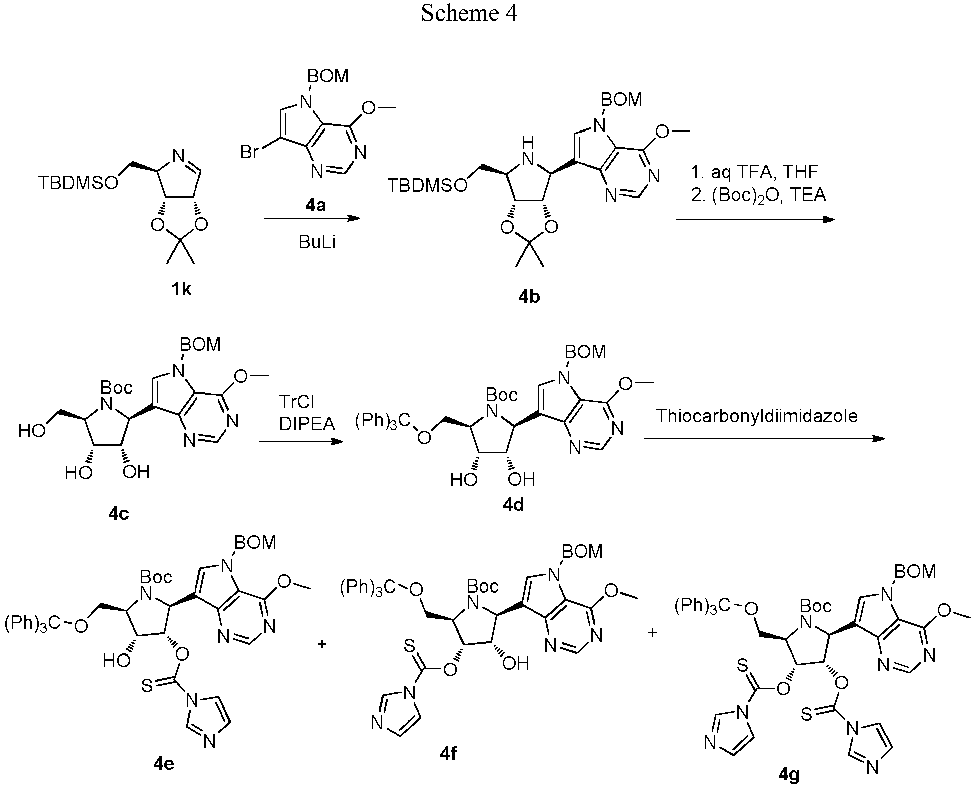

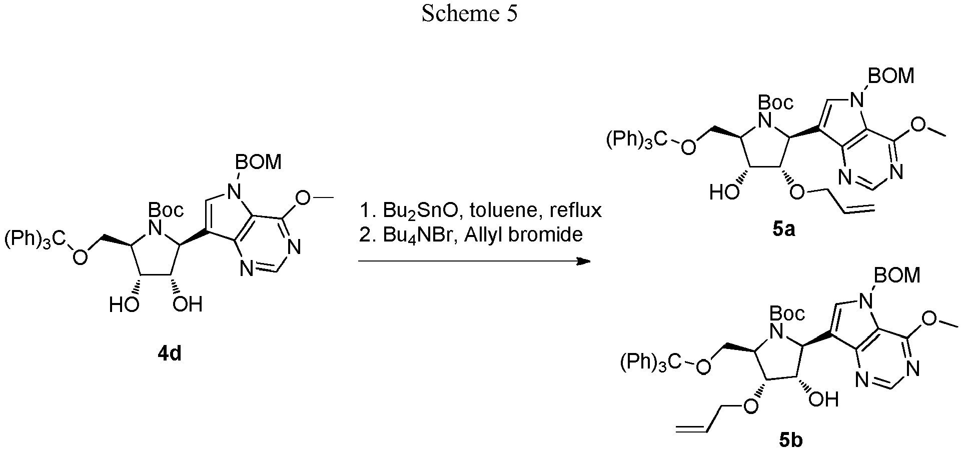

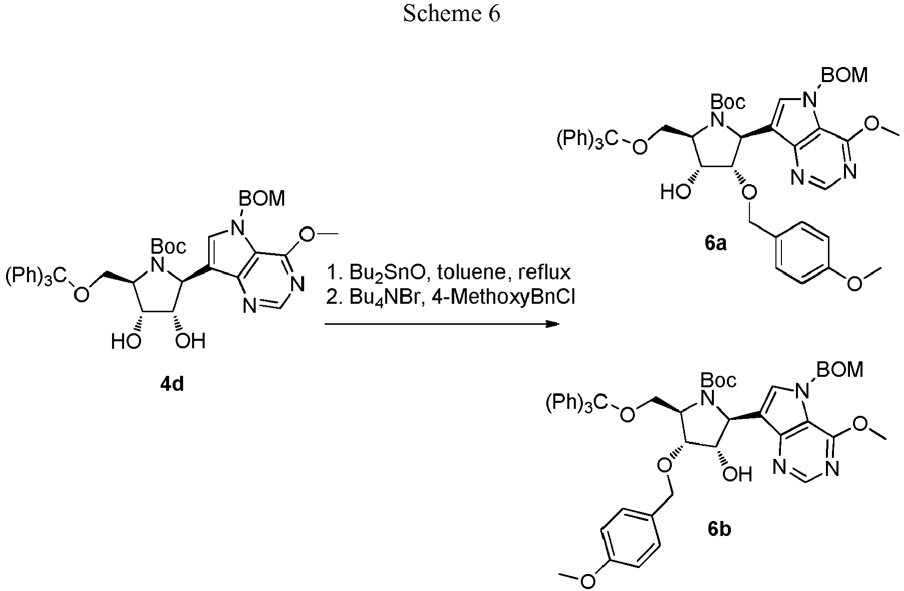

Reference for preparation of **5a, 6a, 5b** and **6b:** Exploring Structure-Activity Relationships of Transition State Analogues of Human Purine Nucleoside Phosphorylase; Gary B. Evans, Richard H. Furneaux, Andrzej Lewandowicz, Vern L. Schramm, and Peter C. Tyler; J. Med. Chem. 2003, 46, 3412-3423.

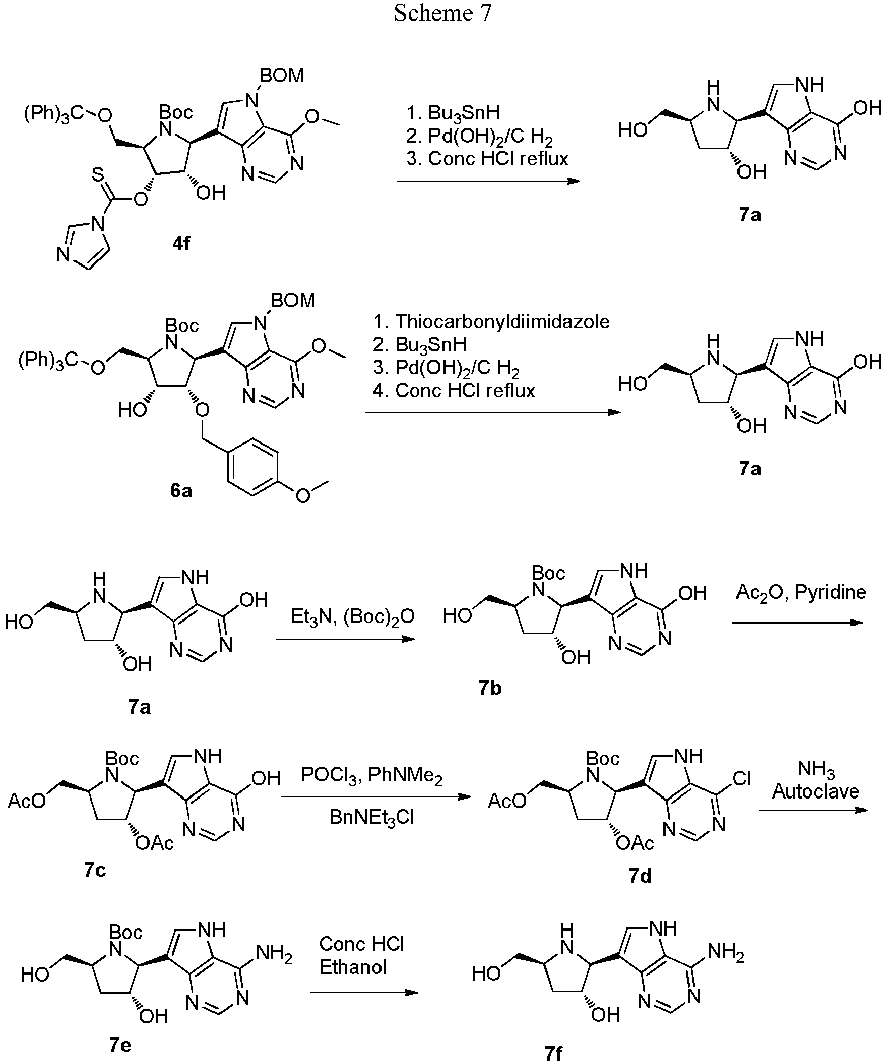

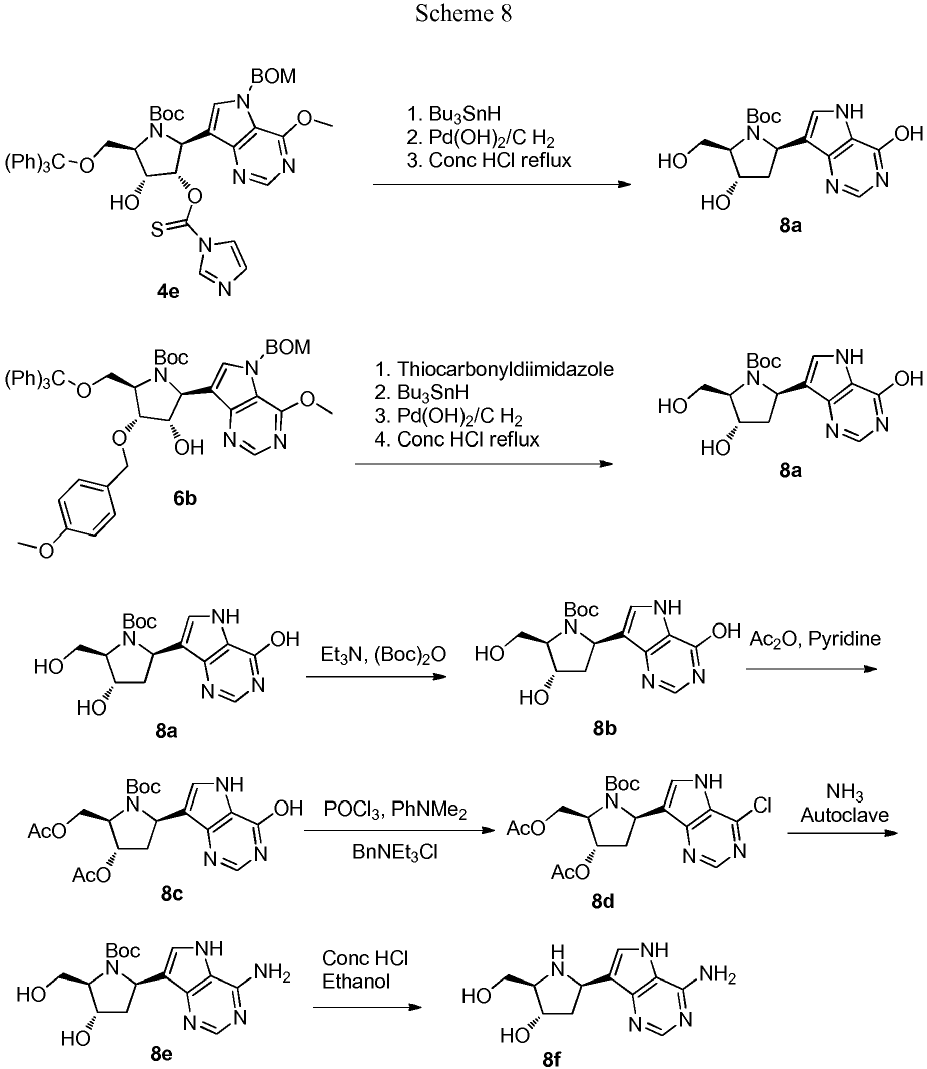

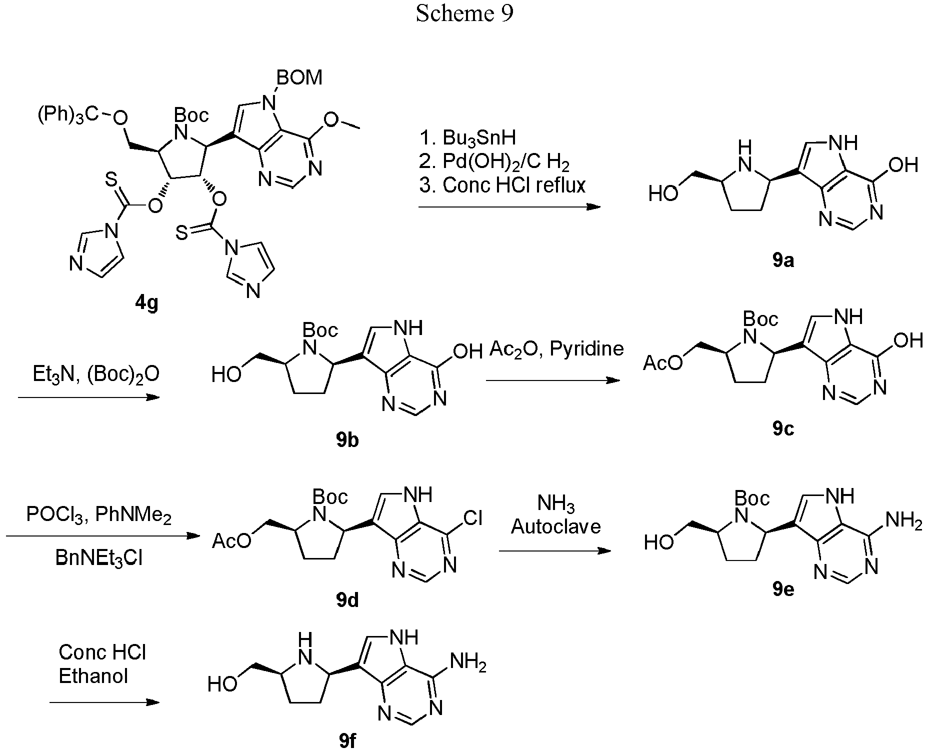

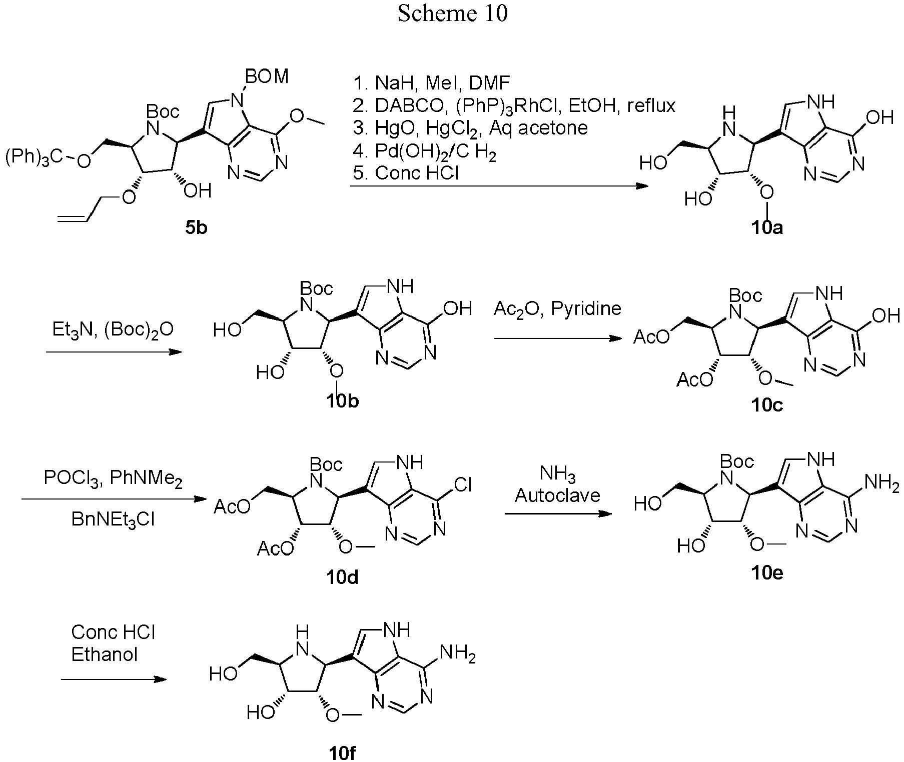

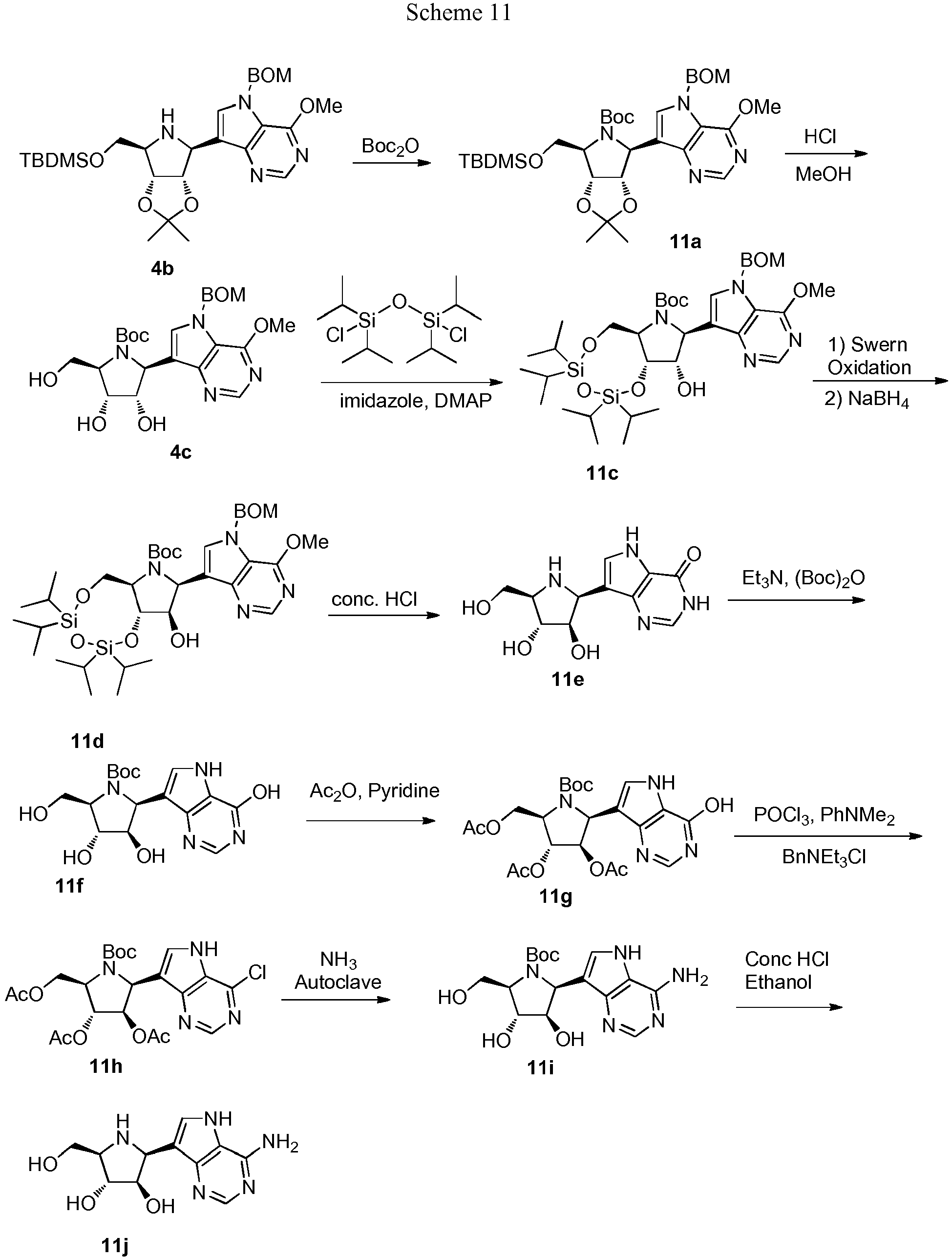

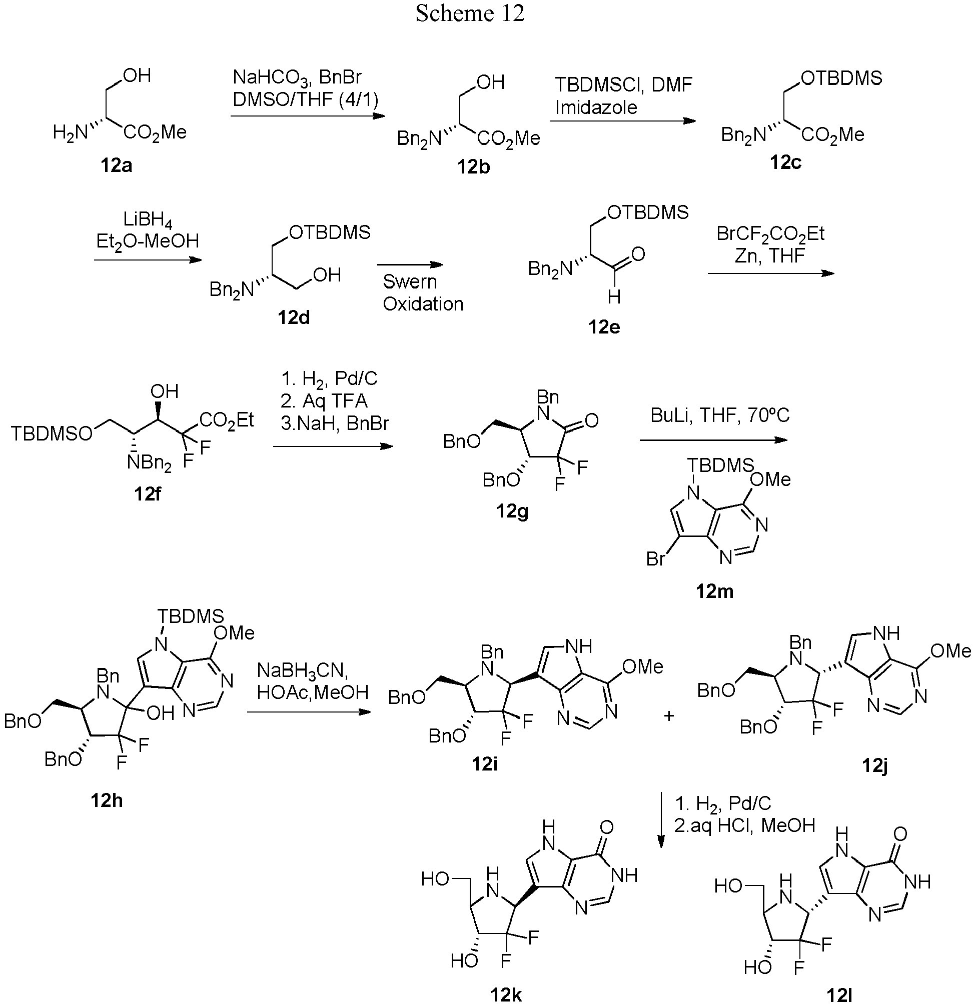

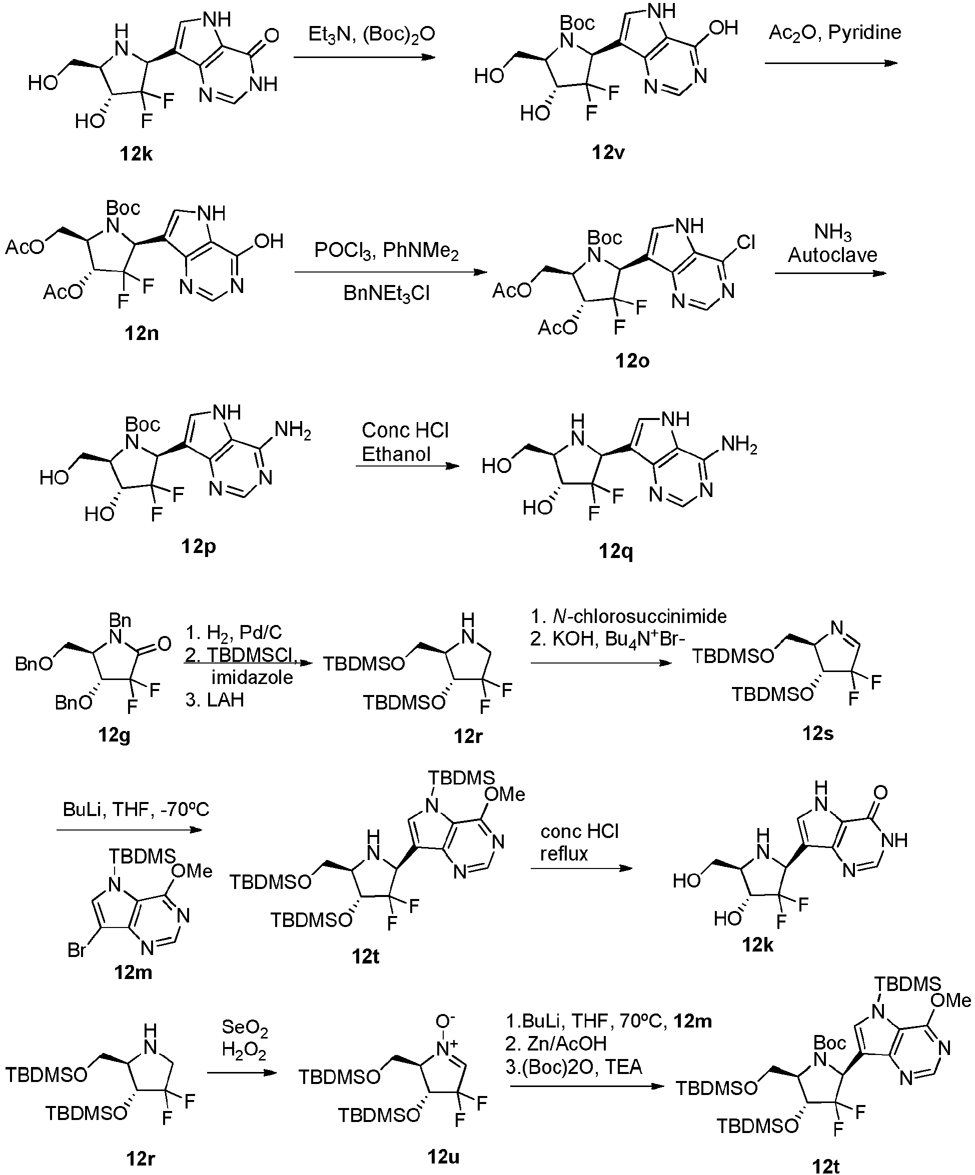

Reference for preparation of **12m:** Addition of lithiated 9-deazapurine derivatives to a carbohydrate cyclicimine: Convergent synthesis of the aza-C-nucleoside immucillins; Evans, G. B.; Furneaux, R. H.; Hutchinson, T. L.; Kezar, H. S.; Morris, P. E. Jr.; Schramm, V. L.; Tyler, P. C; J. Org. Chem. 2001, 66, 5723-5730.

Reference for preparation of **12k:** Exploring Structure-Activity Relationships of Transition State Analogues of Human Purine Nucleoside Phosphorylase; Gary B. Evans, Richard H. Furneaux, Andrzej Lewandowicz, Vern L. Schramm, and Peter C. Tyler; J. Med. Chem. 2003, 46, 3412-3423.

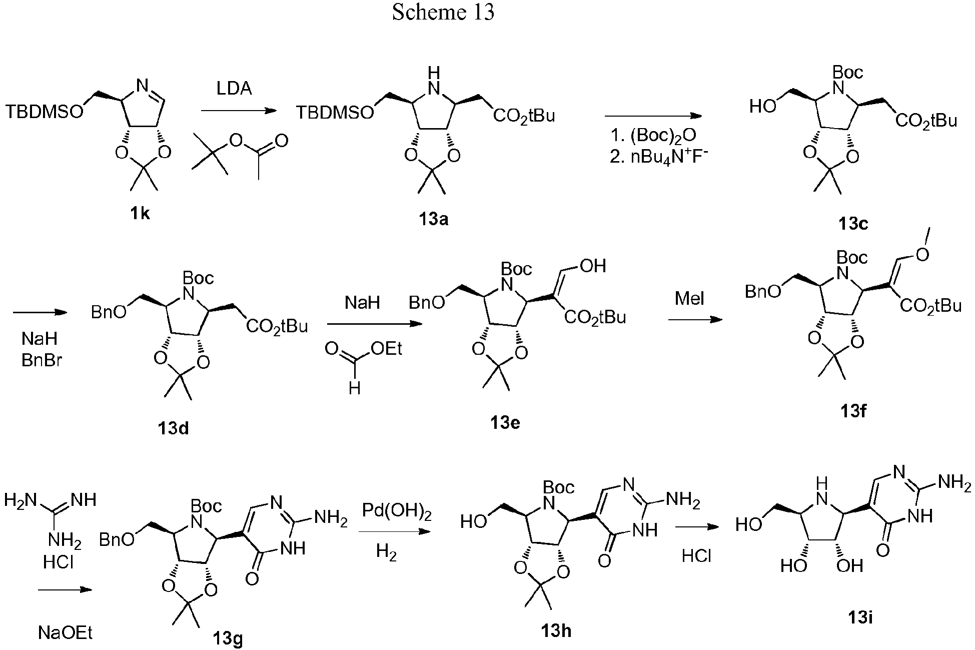

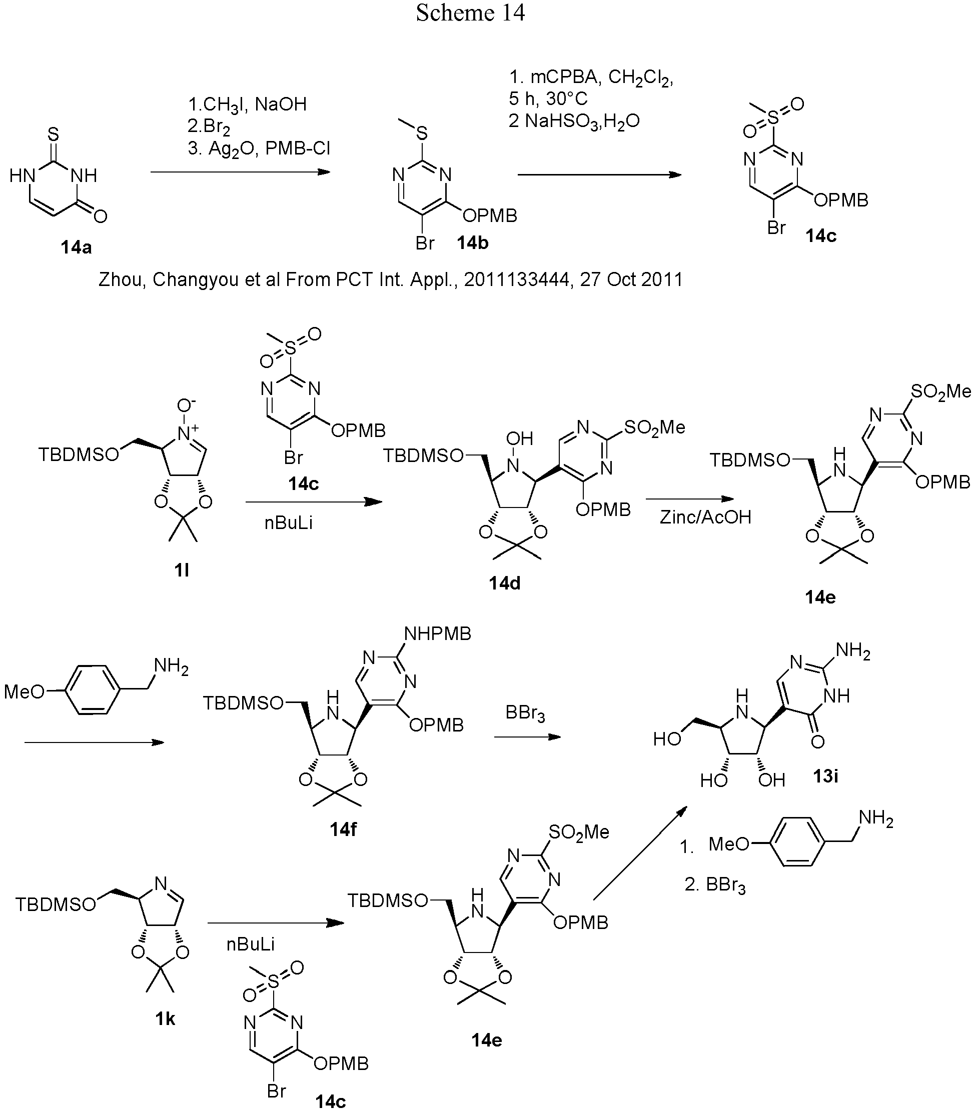

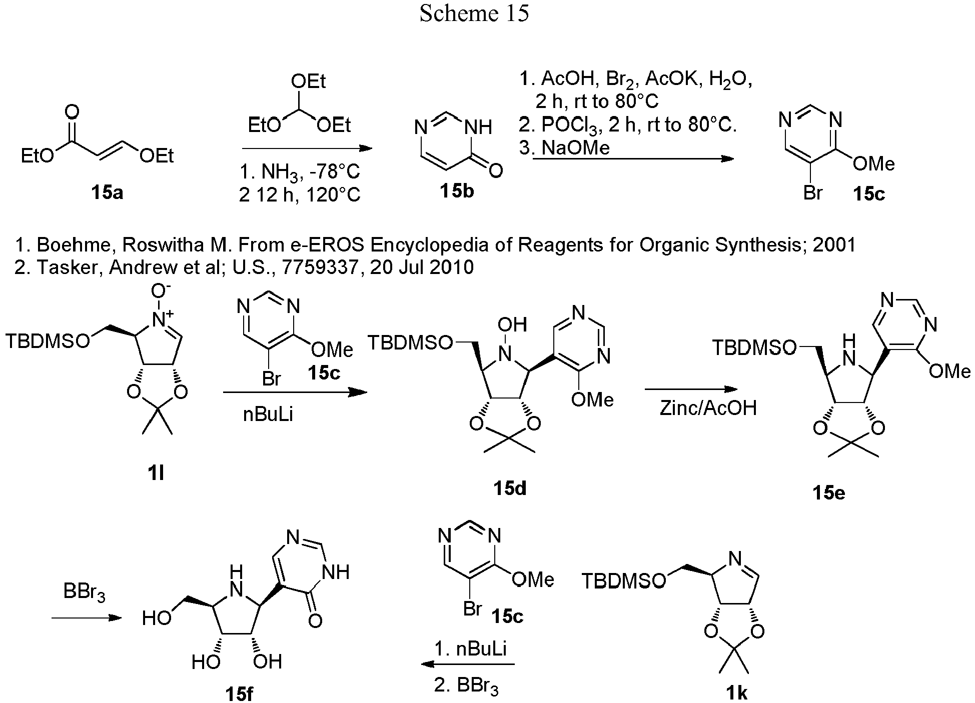

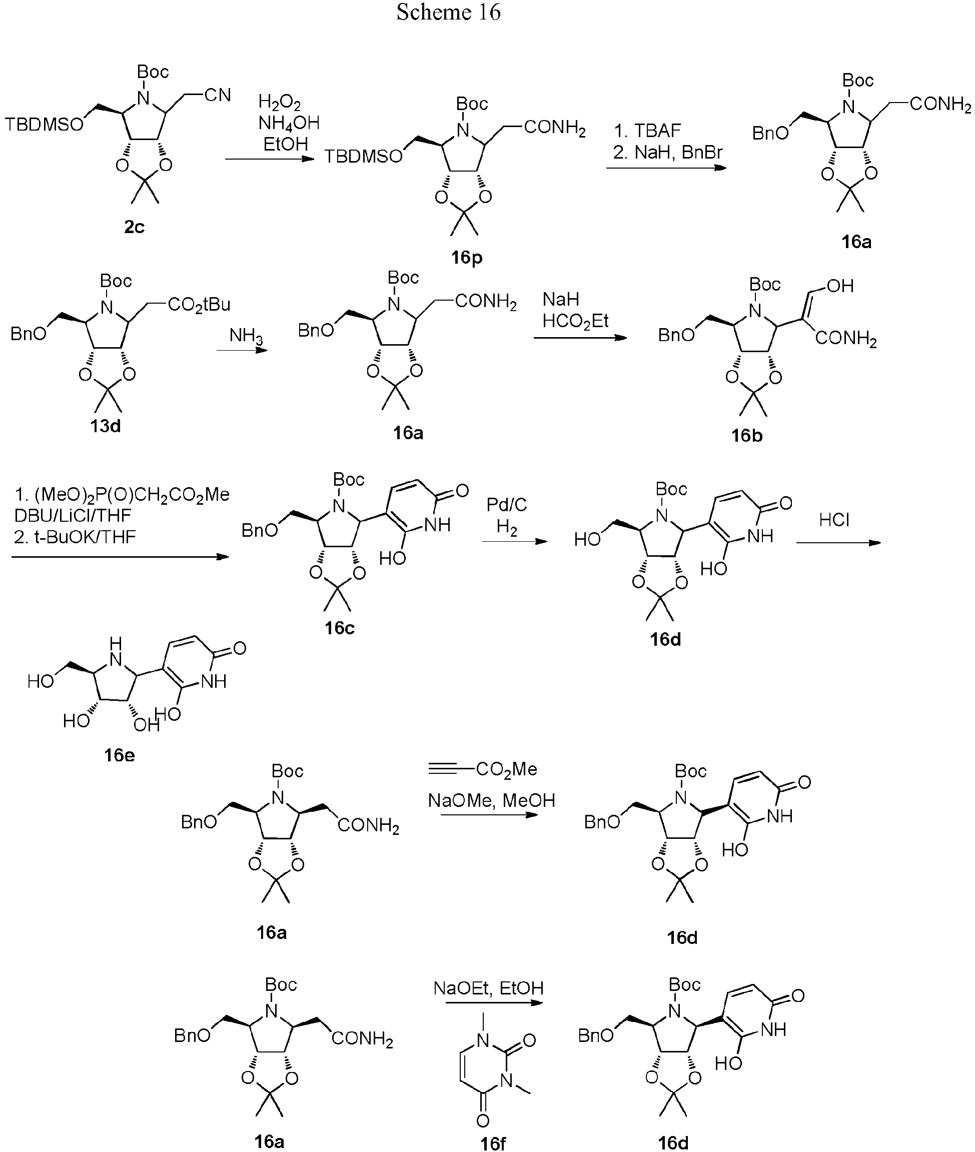

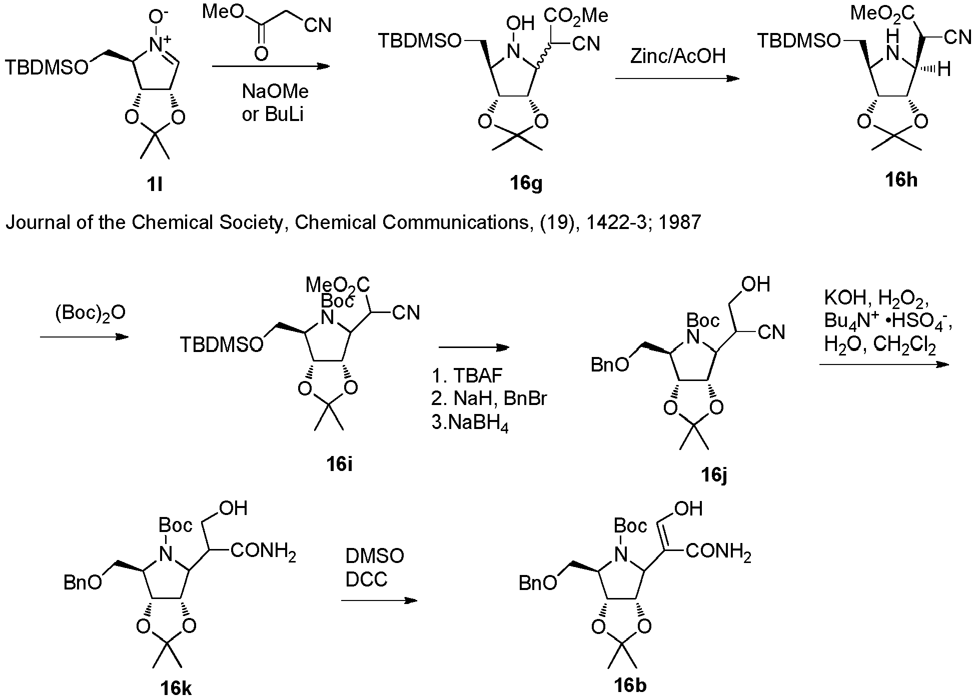

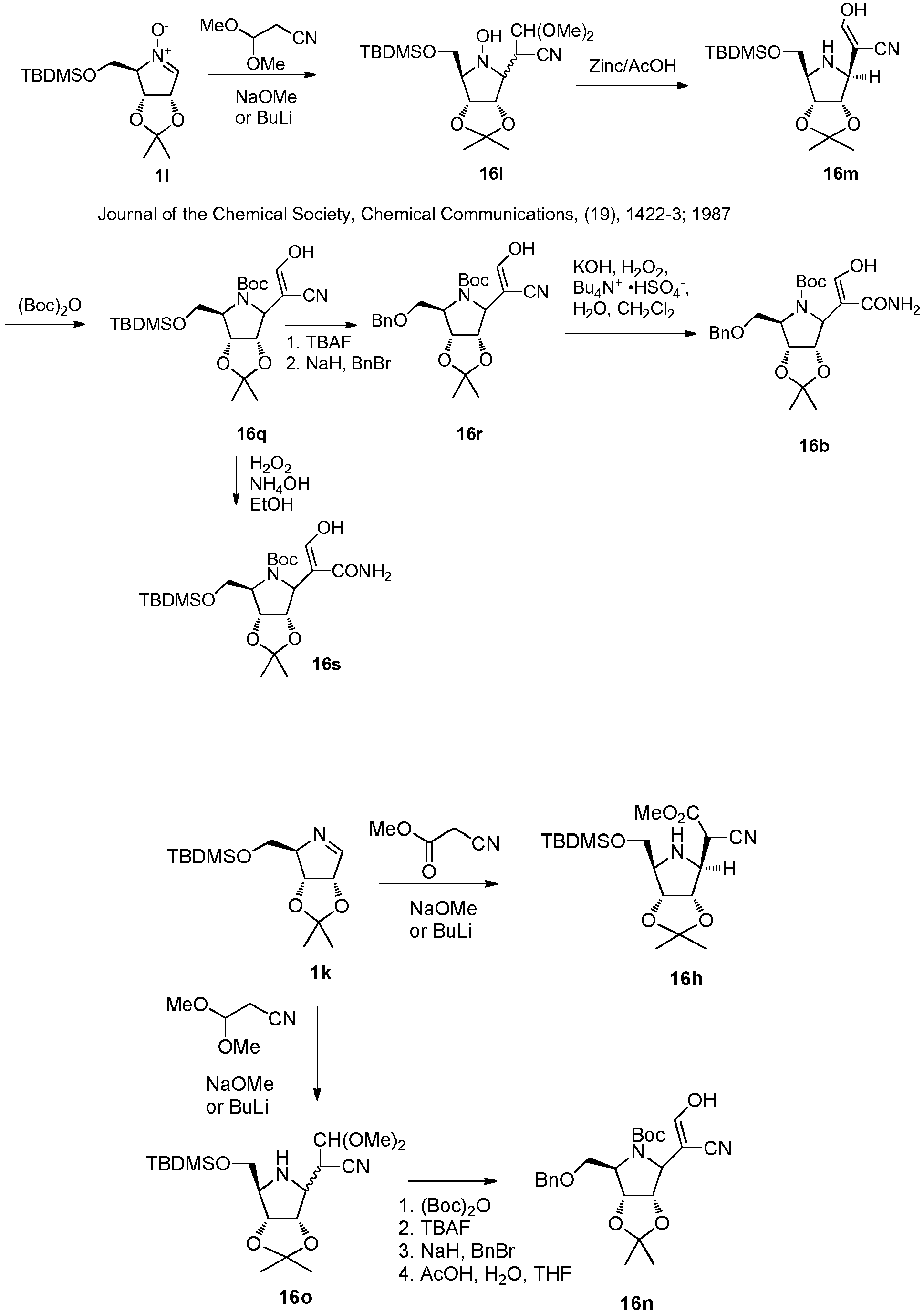

Reference for transformation similar to **16b to 16c:** Synthesis and Cross-Coupling Reactions of 7-Azaindoles via a New Donor-Acceptor Cyclopropane; Zheng, Xiaomei; Kerr, Michael A.; Organic Letters (2006), 8(17), 3777-3779.

### References for transformation similar to 16a to 16d:

1. A novel pyrimidine to pyridine ring transformation reaction. A facile synthesis of 2,6-dihydroxypyridines; Hirota, Kosaku et al.; Journal of the American Chemical Society, (1979), 101(15), 4423-5.
2. Preparation of pyridines and pyridine N-oxides as modulators of thrombin for treatment of disease related to thrombin activity; Player, Mark R.; Lu, Tianbao; Hu, Huaping; Zhu, Xizhen; Teleha, Christopher; Kreutter, Kevin; PCT Int. Appl. (2007), 89 pp, WO 2007109459 (incorporated by reference).
3. Synthesis of 5-fluorouracil derivatives containing an inhibitor of 5-fluorouracil degradation; Hirohashi, Mitsuru; Kido, Masaru; Yamamoto, Yoshihito; Kojima, Yutaka; Jitsukawa, Koichiro; Fujii, Setsuro; Chemical & Pharmaceutical Bulletin (1993), 41(9), 1498-506.
4. Boron-containing small molecules; Jarnagin, Kurt and Akama, Tsutomu; PCT Int. Appl., 2011094450 (incorporated by reference), 04 Aug 2011.

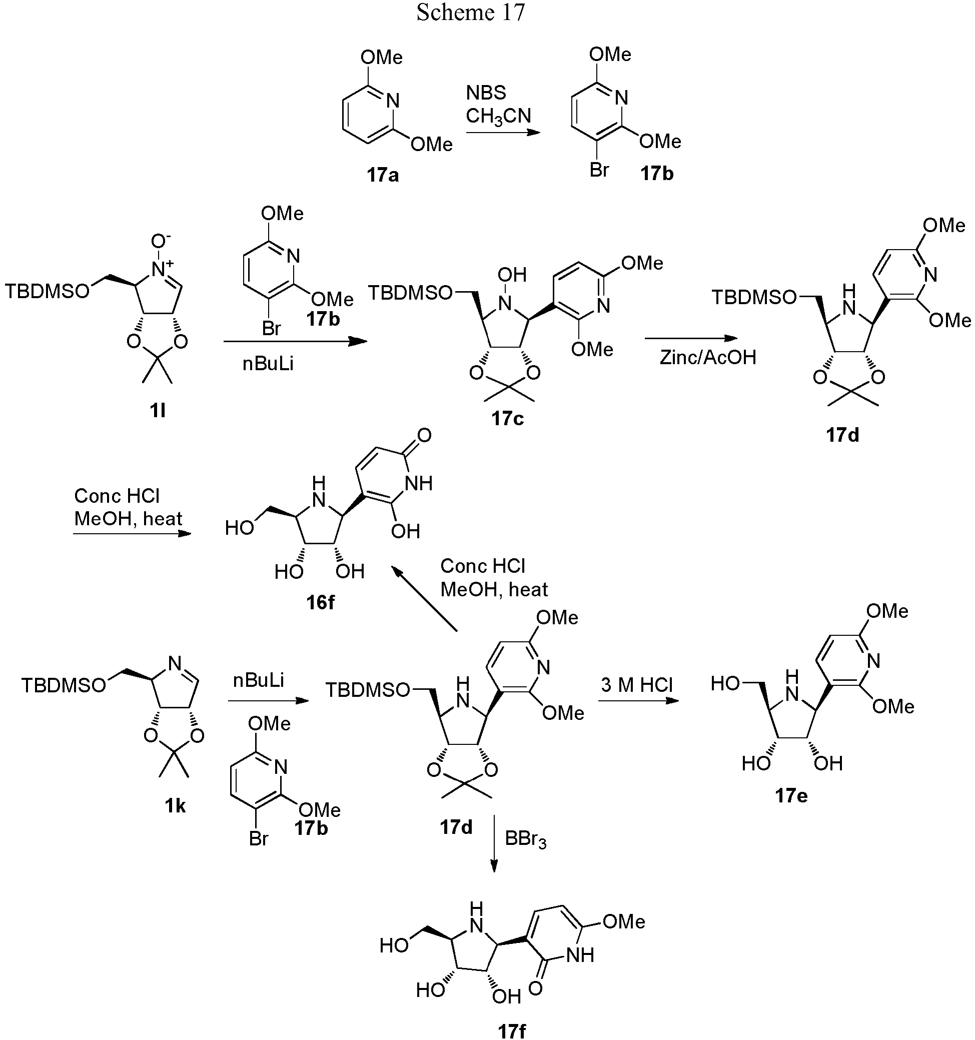

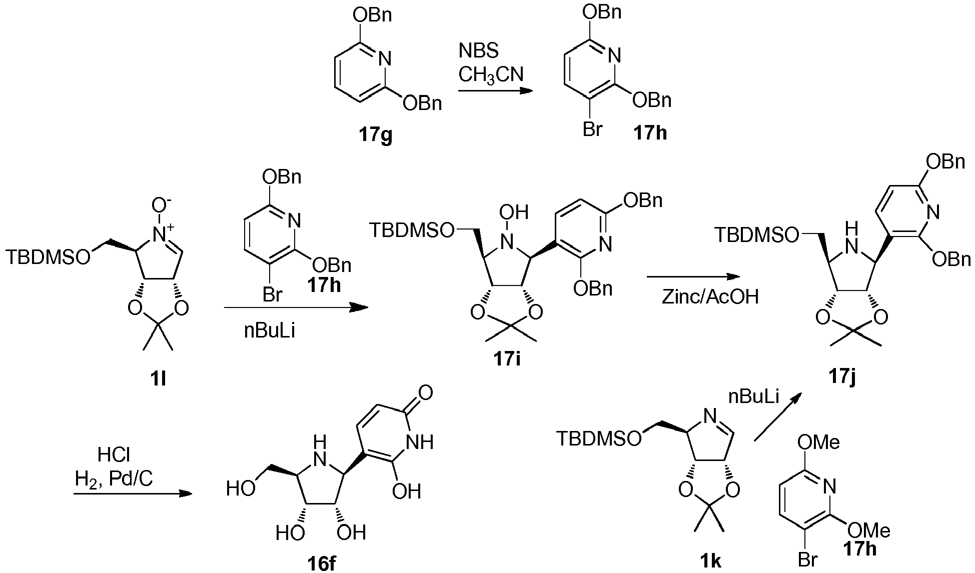

Reference for preparation of 3-bromo-2,6-dimethoxypyridine (**17b**): Noncryogenic synthesis of functionalized 2-methoxypyridines by halogen-magnesium exchange using lithium dibutyl(isopropyl)magnesate(1-) and lithium chloride; Struk, Lukasz and Sosnicki, Jacek G; Synthesis, 44(5), 735-746; 2012.

Reference for preparation of 2,6-bis(benzyloxy)-3-bromopyridine (**17h**): Approaches to the synthesis of 1-deazauridine and 2'-deoxy-1-deazauridine; Mertes, Mathias P.; Zielinski, James; Pillar, Conrad; Journal of Medicinal Chemistry (1967), 10(2), 320-5.

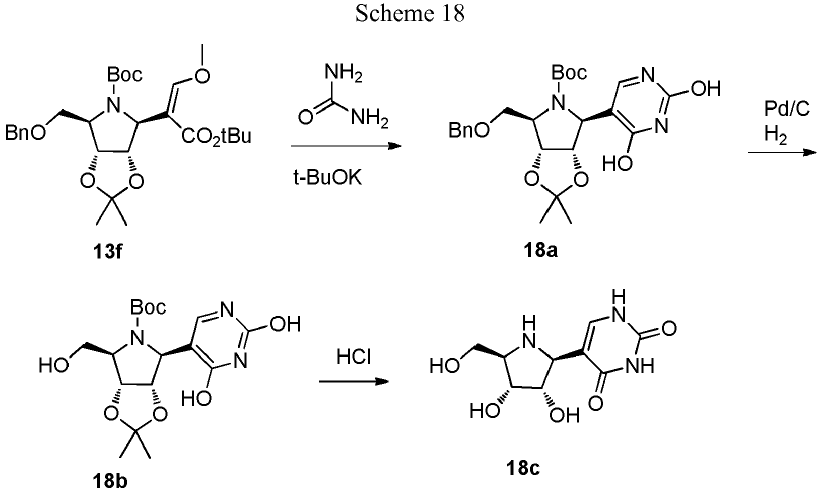

Reference for transformation similar to **13d** to **18a**: Synthesis of 2'-Fluoro and 2',4'-Dimethyl Pyrimidine C-Nucleoside Analogs as Potential Anti-HCV Agents; Liu, Lian Jin; Hong, Joon Hee.; Nucleosides, Nucleotides & Nucleic Acids (2010), 29(3), 216-227.

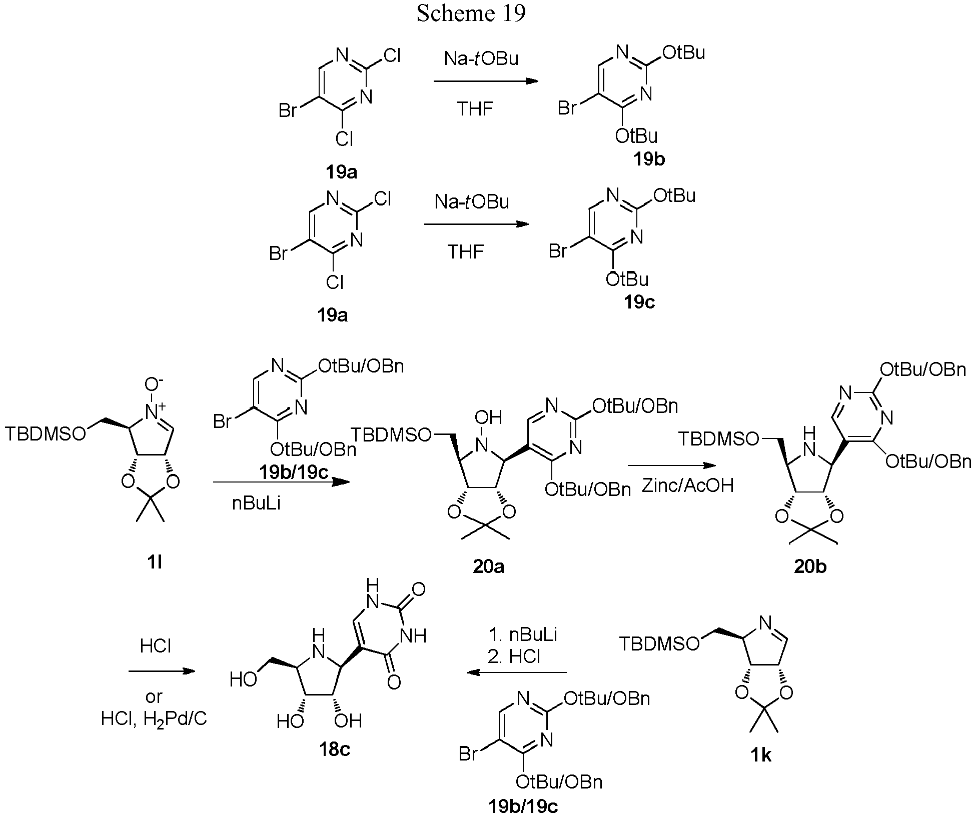

Reference for synthesis of 5-bromo-2,4-di-tert-butoxypyrimidine **(19b):** First synthesis of 1-deazacytidine, the C-nucleoside analogue of Cytidine Matthieu Sollogoub, Keith R. Fox, Vicki E. C. Powersa and Tom Brown; Tetrahedron Letters, 43 (2002) 3121-3123.

### References for synthesis of 5-bromo-2,4-di-tert-butoxypyrimidine (19c):

1. Synthesis and coordination chemistry of pyrimidine-substituted phosphine ligands; Nixon, Tracy D. et al.; Inorganica Chimica Acta, 380, 252-260; 2012.
2. Spiro compounds, processes for preparing them, pharmaceutical compositions containing them, and their use as as modulators of dopamine D3 receptors; Bertani, Barbara et al.; PCT Int. Appl., 2007125061 (incorporated by reference), 08 Nov 2007.
3. "Molecular Chameleons." Design and Synthesis of a Second Series of Flexible Nucleosides; Seley, Katherine L. et al.; Journal of Organic Chemistry, 70(5), 1612-1619; 2005.
4. Synthesis of 5-(dihydroxyboryl)-2'-deoxyuridine and related boron-containing pyrimidines; Schinazi, Raymond F. and Prusoff, William H; Journal of Organic Chemistry, 50(6), 841-7; 1985.

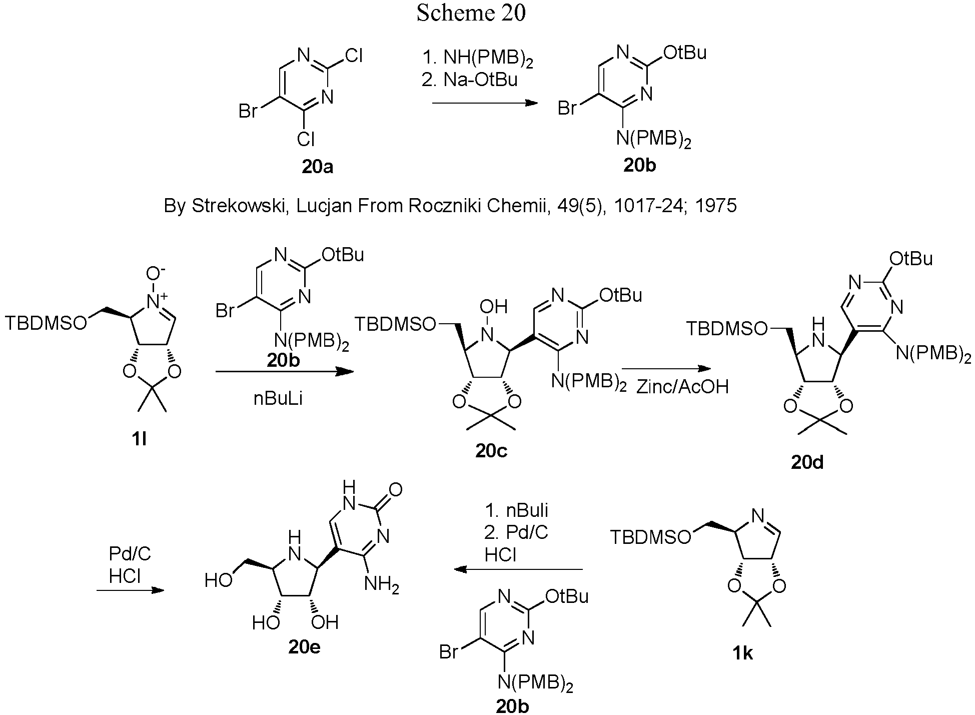

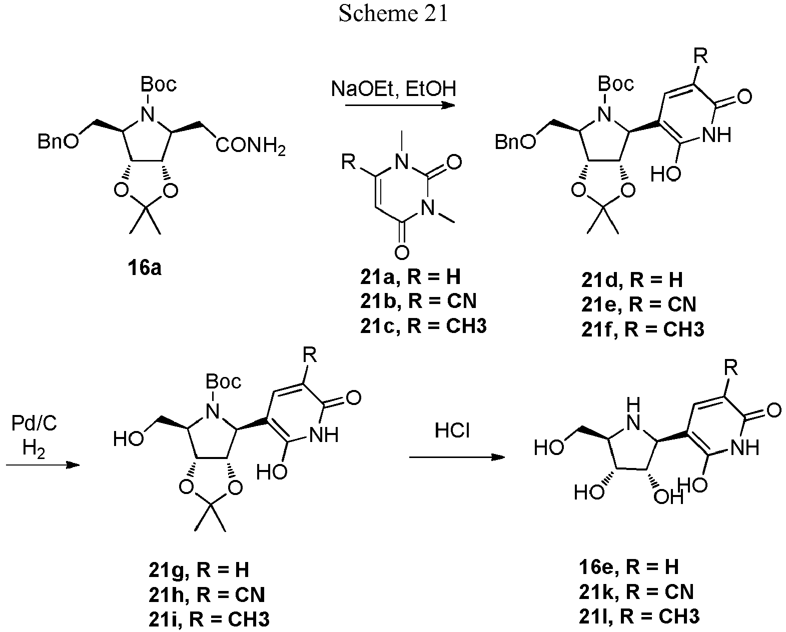

Reference for transformation similar to compound **16 a** to compounds **21d, 21e and 21f**: A novel Pyrimidine to pyridine ring transformation reaction. A facile synthesis of 2,6-dihydroxypyridines; Hirota, Kosaku; Kitade, Yukio; Senda, Shigeo; Halat, Michael J.; Watanabe, Kyoichi A.; Fox, Jack J; Journal of the American Chemical Society (1979), 101(15), 4423-5.

### References for synthesis of 1,3-Dimethyl-6-cyanouracil (21b):

1. Hydrocyanation of conjugated carbonyl compounds, Nagata, Wataru and Yoshioka, Mitsuru; From Organic Reactions (Hoboken, NJ, United States), 25, No pp. given; 1977
2. 6-Cyanouracils from 5-diazouracils; Kloetzer, Wilhelm and Romani, Sigrun; From Liebigs Annalen der Chemie, (8), 1429-32; 1981.
3. Photoinduced reactions. 122. Intermolecular photoaddition involving 1,4-transfer of cyano group. 2. Photoaddition of 6-cyano-1,3-dimethyluracil with acetylenic compounds; By Saito, Isao et al.; Tetrahedron Letters, 21(24), 2317-20; 1980.
4. Pyrimidine derivatives and related compounds. XXVII. Reaction of 6-cyano-1,3-dimethyluracil with nucleophiles; By Senda, Shigeo et al.; Chemical & Pharmaceutical Bulletin, 23(8), 1708-13; 1975.

### References for synthesis of 1,3,6-Trimethylpyrimidine-2,4(1H,3H)-dione (21c):

1. Preparation of pyridopyrimidinylacetamide derivatives for use as TRPA1 modulators; By Kumar, Sukkeerthi et al.; From PCT Int. Appl., 2011132017 (incorporated by reference), 27 Oct 2011.
2. Nanomolar Potency Pyrimido-pyrrolo-quinoxalinedione CFTR Inhibitor Reduces Cyst Size in a Polycystic Kidney Disease Model; Tradtrantip, Lukmanee et al.; Journal of Medicinal Chemistry, 52(20), 6447-6455; 2009.

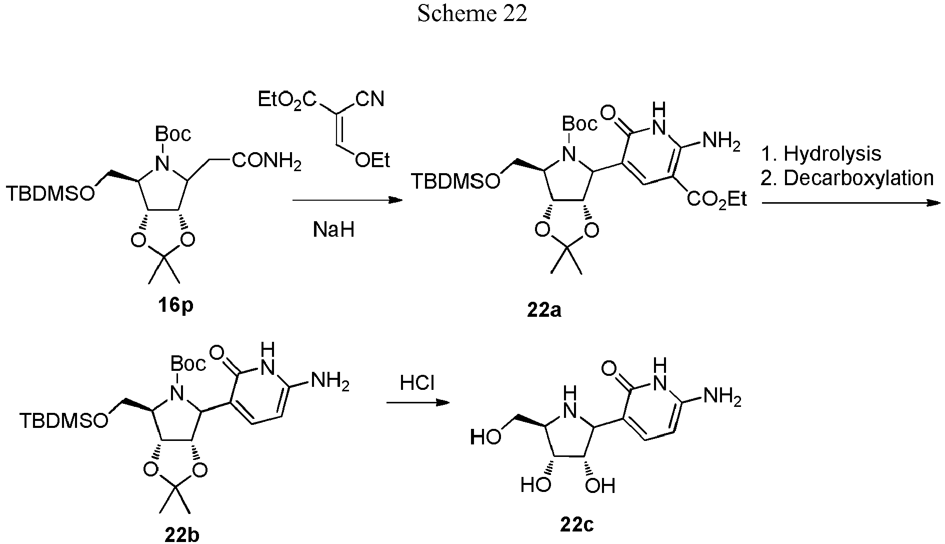

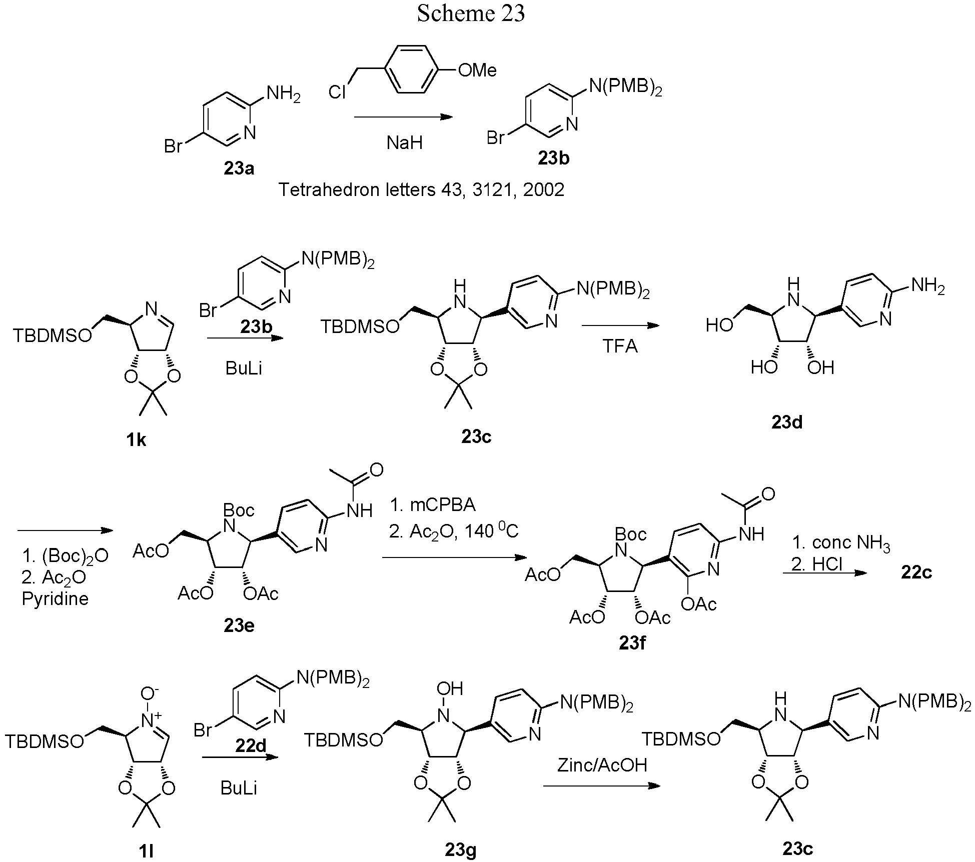

Reference for transformation similar to compound **23e** to compounds **23f:** First synthesis of 1-deazacytidine, the C-nucleoside analogue of Cytidine Matthieu Sollogoub, Keith R. Fox, Vicki E. C. Powersa and Tom Brown; Tetrahedron Letters, 43 (2002) 3121-3123.

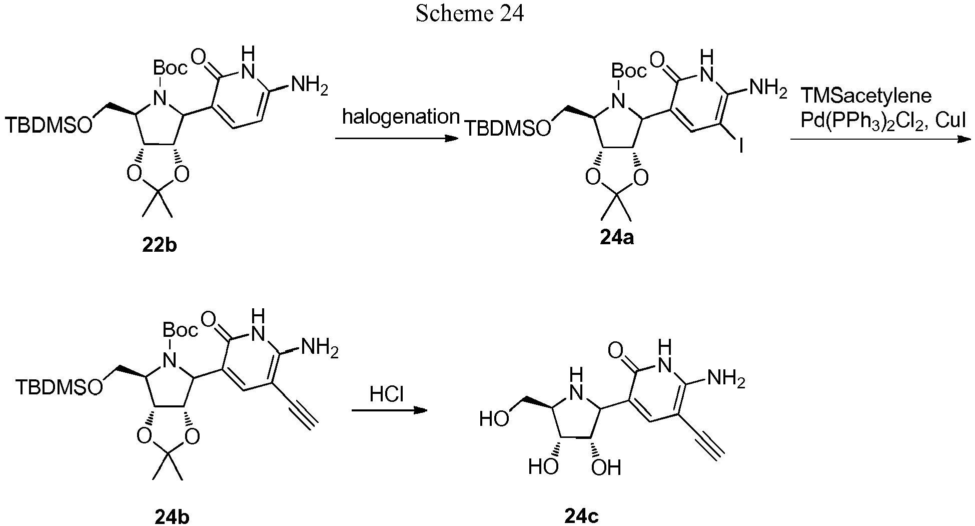

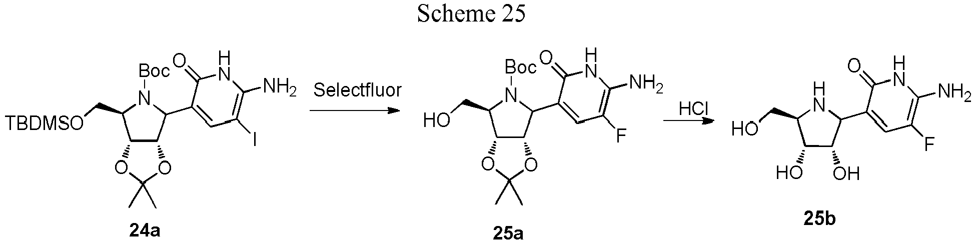

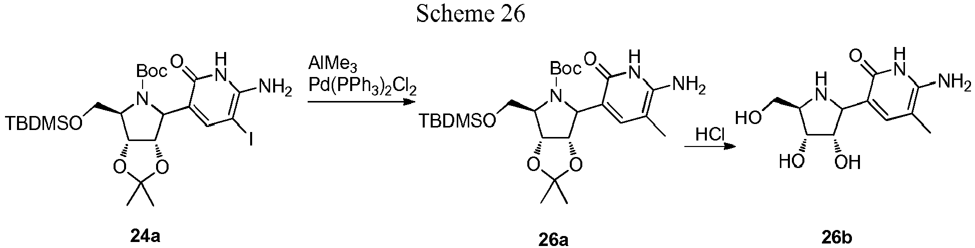

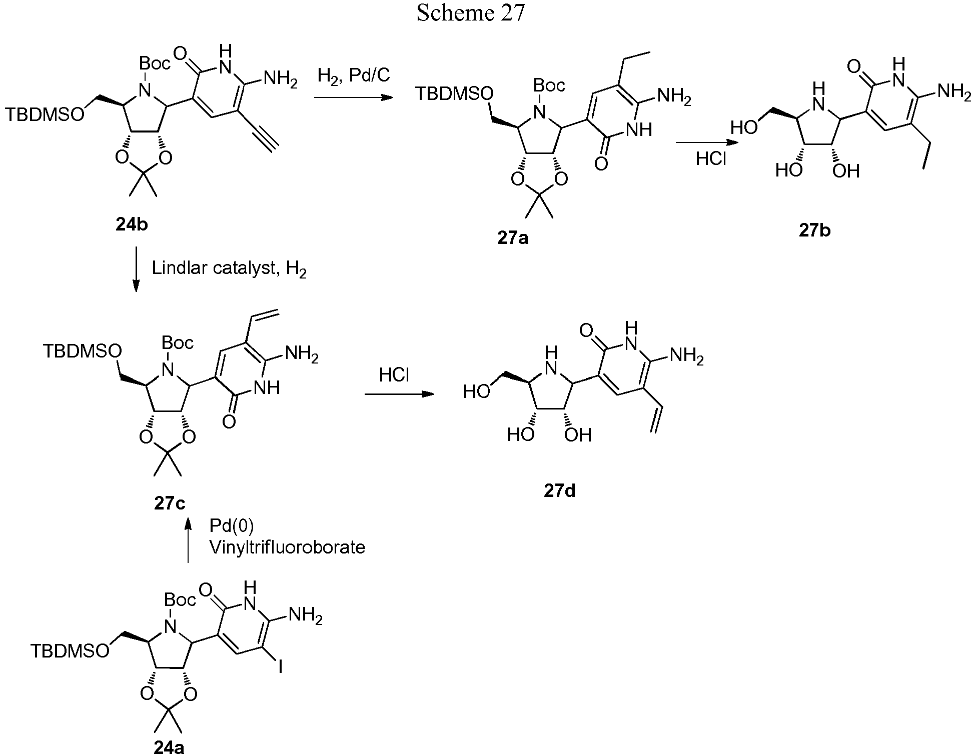

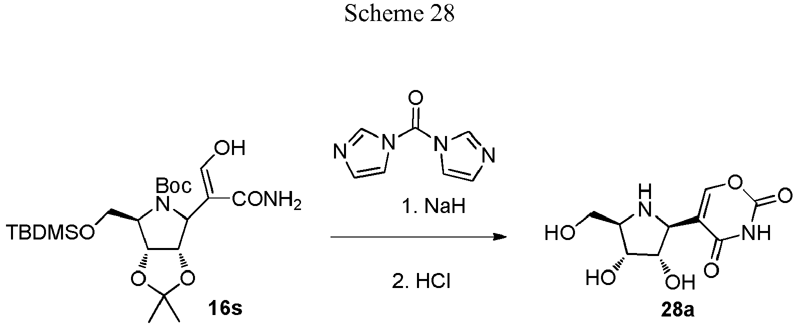

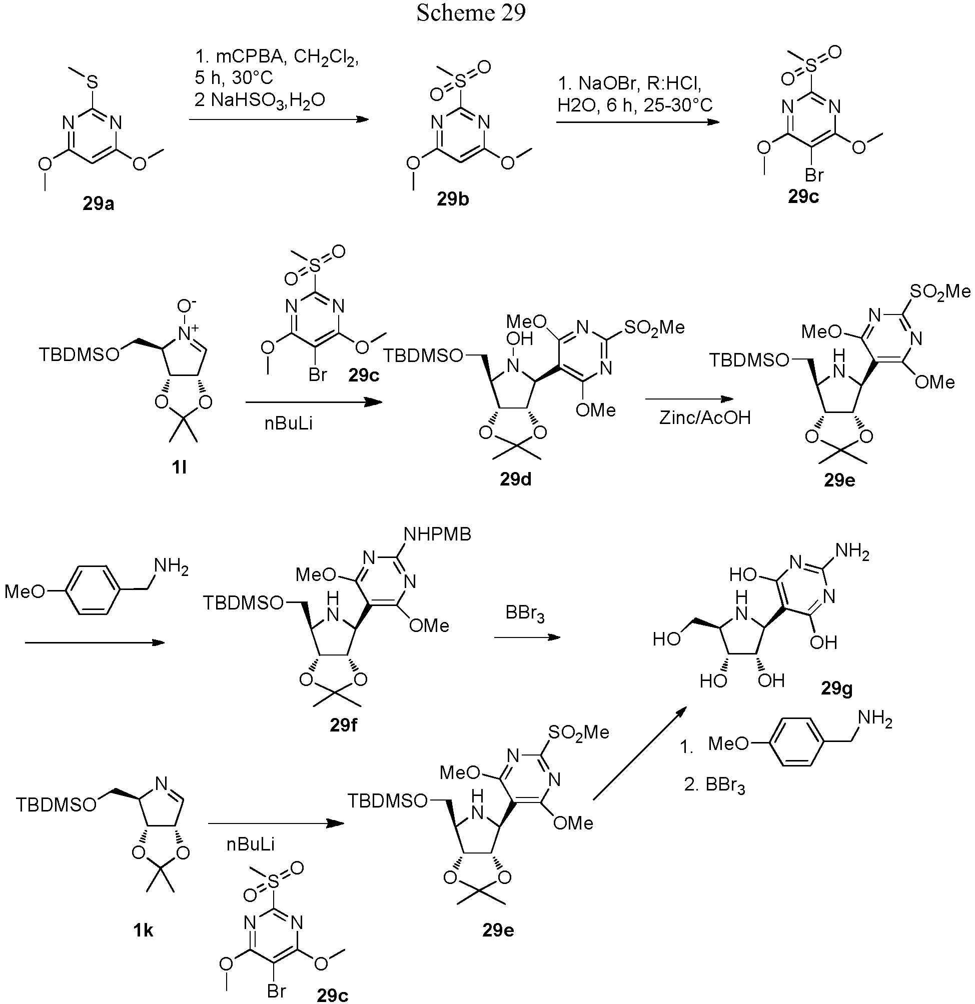

### References for preparation of 29b:

1. A novel Pyrimidine-based stable-isotope labeling reagent and its application to quantitative analysis using matrix-assisted laser desorption/ionization mass spectrometry; Zhang, Jing et al.; Journal of Mass Spectrometry, 42(11), 1514-1521; 2007.
2. A facile synthesis of [14C]pyrithiobac-sodium; Ravi, S. et al., Journal of Labelled Compounds and Radiopharmaceuticals, 49(4), 339-343; 2006.
3. Novel Benzo[1,4]diazepin-2-one Derivatives as Endothelin Receptor Antagonists Bolli, Martin H. et al.; From Journal of Medicinal Chemistry, 47(11), 2776-2795; 2004.

Reference for preparation of **29c**: Synthesis, characterization and biological activity of some new 5-halo-4,6-dimethoxy-2-(alkoxy or aryloxy)pyrimidines; Paniraj, A. S. et al. Pharma Chemica, 3(3), 63-72; 2011.

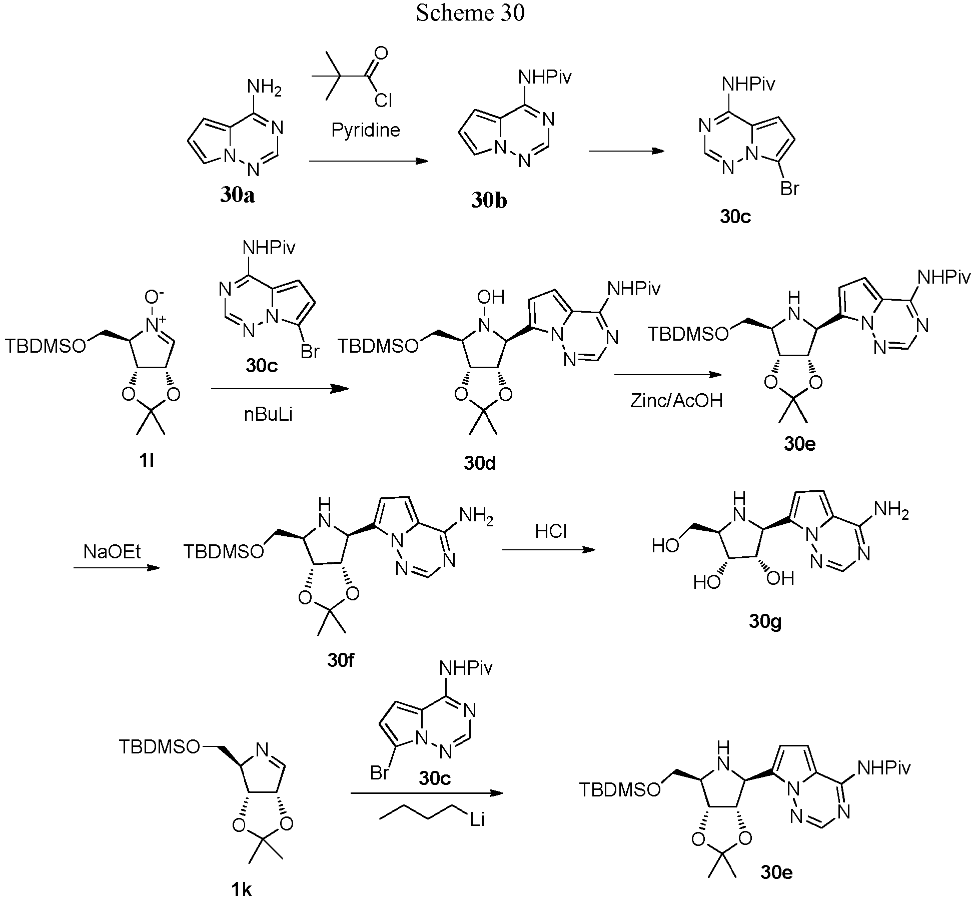

### References for preparation of compound 30a:

1. Preparation of substituted 4-amino-pyrrolotriazine derivatives useful for treating hyper-proliferative disorders and diseases associated with angiogenesis; By Dixon, Julie et al., PCT Int. Appl., 2007064931 (incorporated by reference), 07 Jun 2007.
2. Synthesis of pyrrolo[2,1-f][1,2,4]triazine congeners of nucleic acid purines via the N-amination of 2-substituted pyrroles; Patil, Shirish A. et al., From Journal of Heterocyclic Chemistry, 31(4), 781-6; 1994.

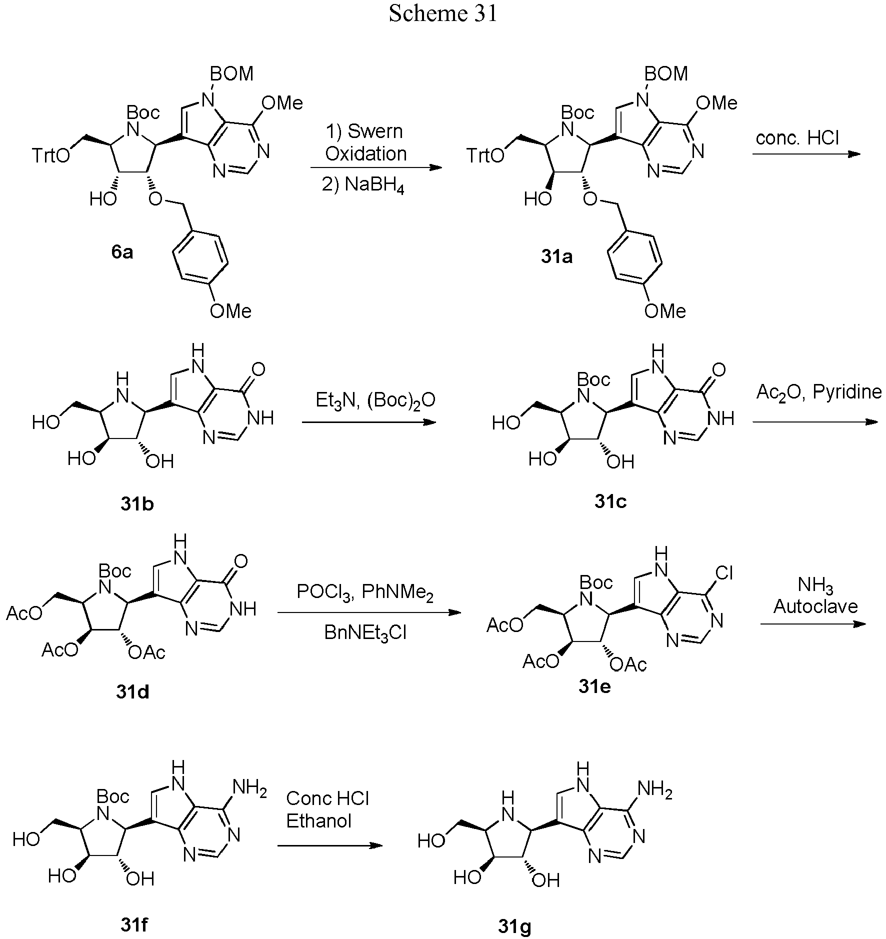

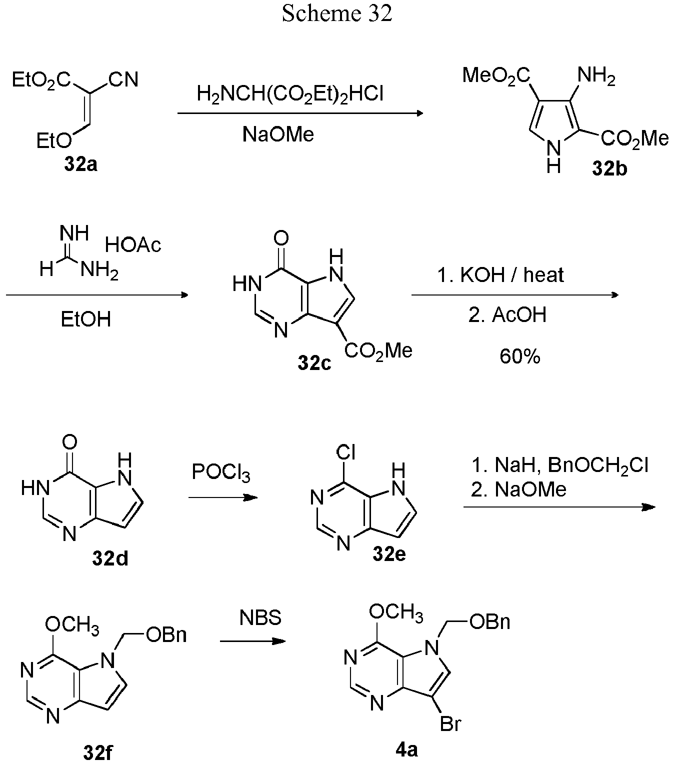

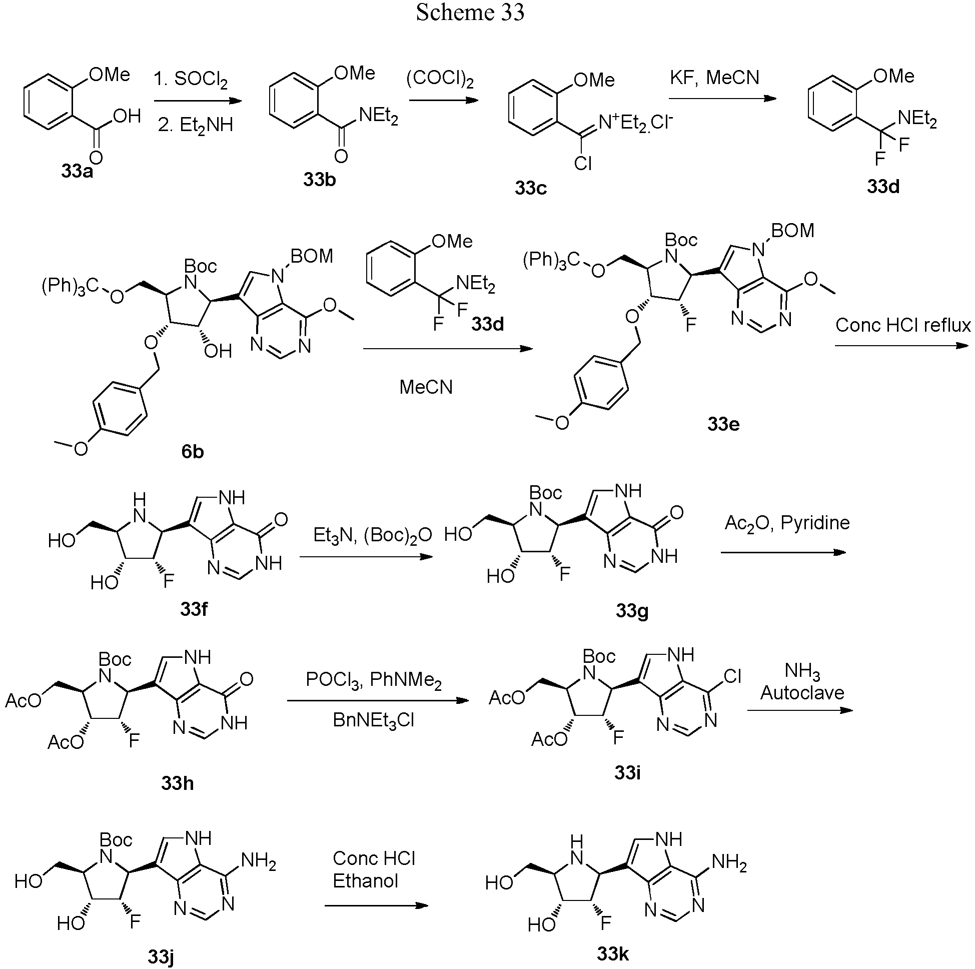

Reference for transformation similar to **6b** to **33e** using **33d:** Method of fluorination using N,N-diethyl-α,α -difluorobenzylamines; Hara, Shoji; Fukuhara, Tsuyoshi; PCT Int. Appl. (2004), 50 pp. WO 2004050676 A1 (incorporated by reference) 20040617. Application: WO 2003-JP15336 (20031201).

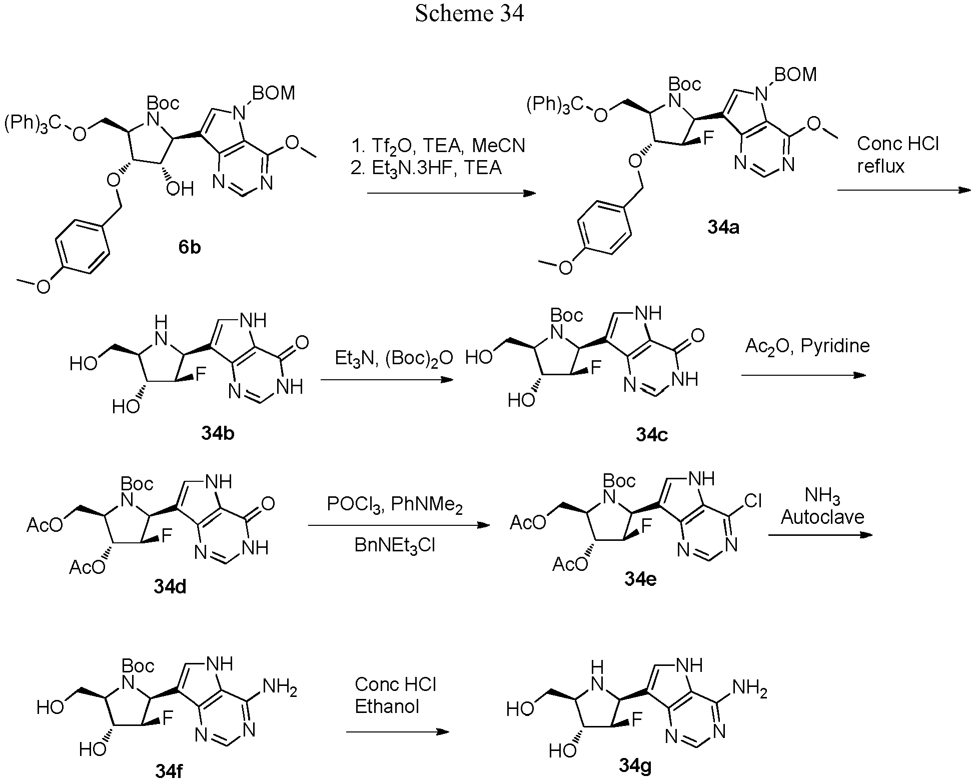

Reference for transformation similar to **6b** to **34a:** Direct and convenient conversion of alcohols to fluorides; Yin, Jingjun; Zarkowsky, Devin S.; Thomas, David W.; Zhao, Matthew M.; Huffman, Mark A; Organic Letters (2004), 6(9), 1465-1468.

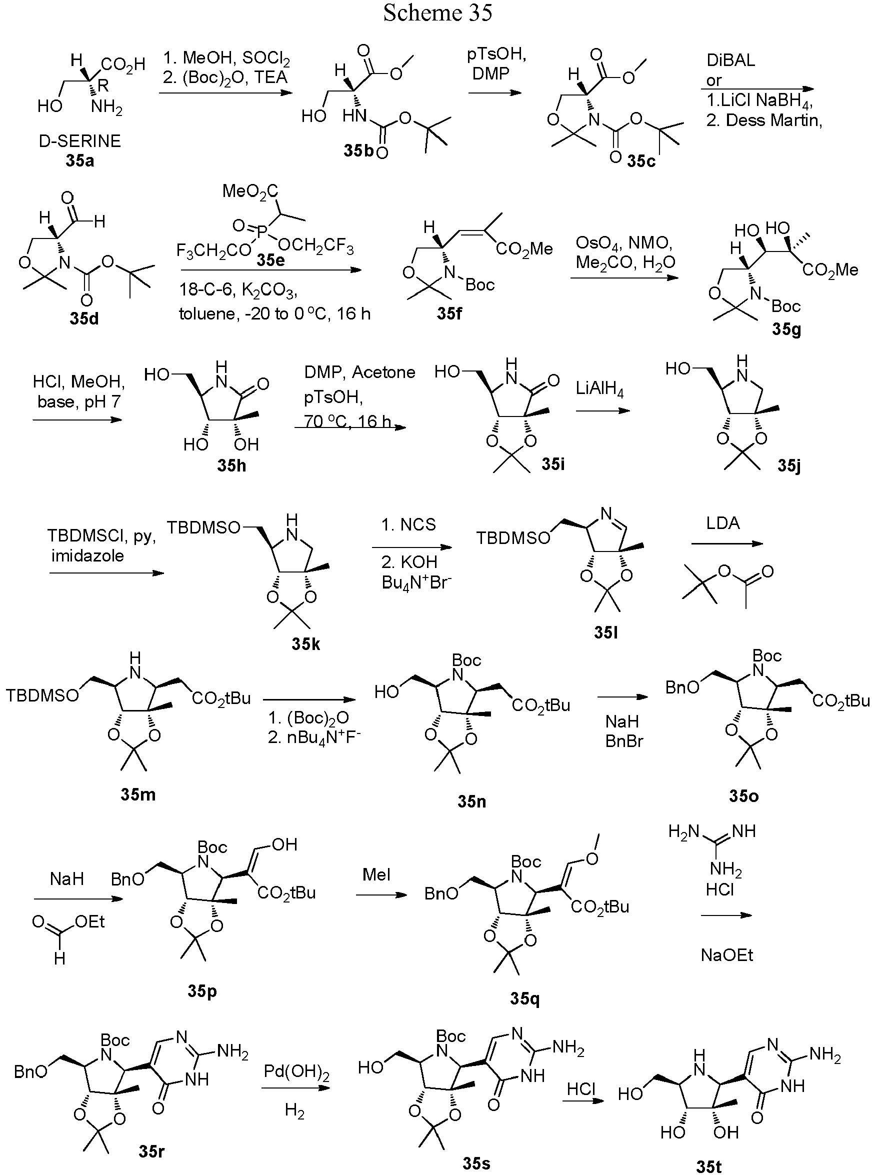

### Preparation of compound 35k is reported in:

1. Synthetic approaches to imino sugar (2R,3R,4S)-2-(hydroxymethyl)-4-methylpyrrolidine-3,4-diol from naturally occurring sugar and amino acid; Kotian, Pravin L. et al.; Tetrahedron Letters, 52(3), 365-368; 2011.
2. Preparation of aza nucleoside analogs and their use as antiviral agents and inhibiting viral RNA polymerases; Babu, Yarlagadda S. et al.; PCT Int. Appl., 2006002231 (incorporated by reference), 05 Jan 2006.

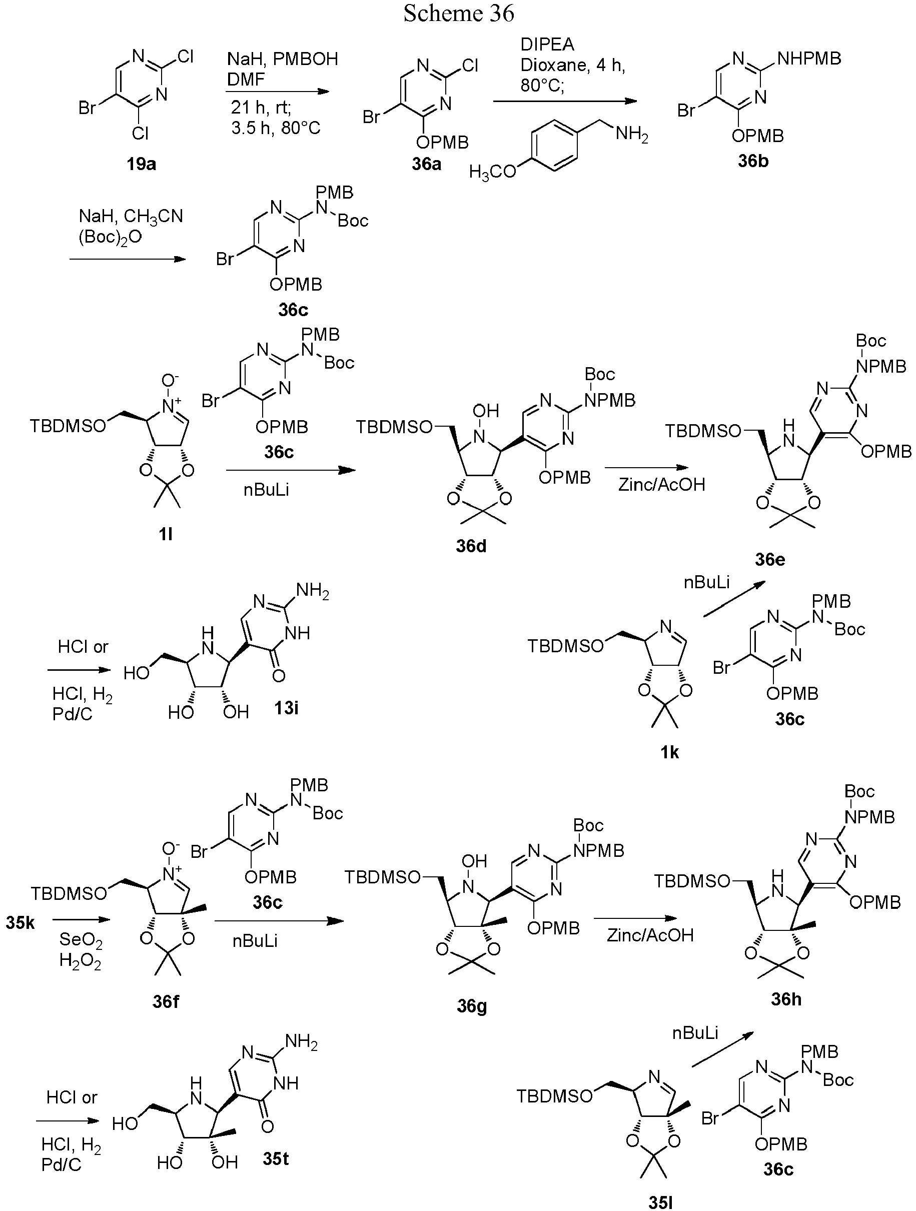

Reference for making compound similar to compound **36c**: Tetrahydronaphthyridine derivatives as cholesteryl ester transferase protein inhibitors and a process for preparing them; Kubota, Hitoshi et al.; U.S. Pat. Appl. Publ., 20070032485 (incorporated by reference), 08 Feb 2007.

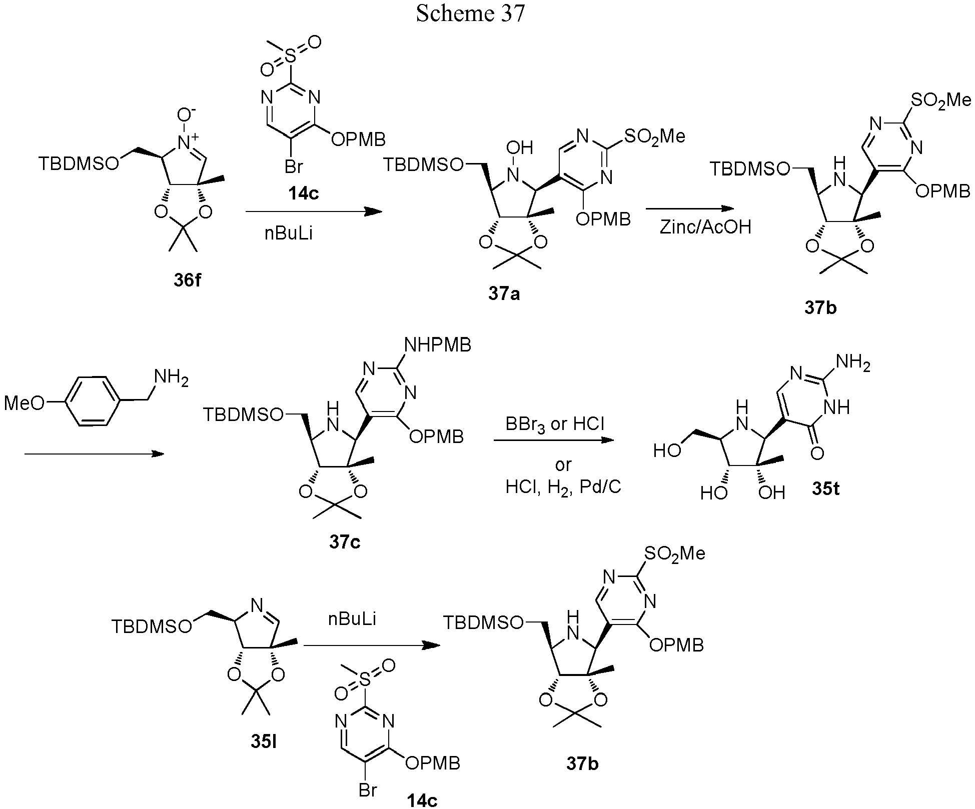

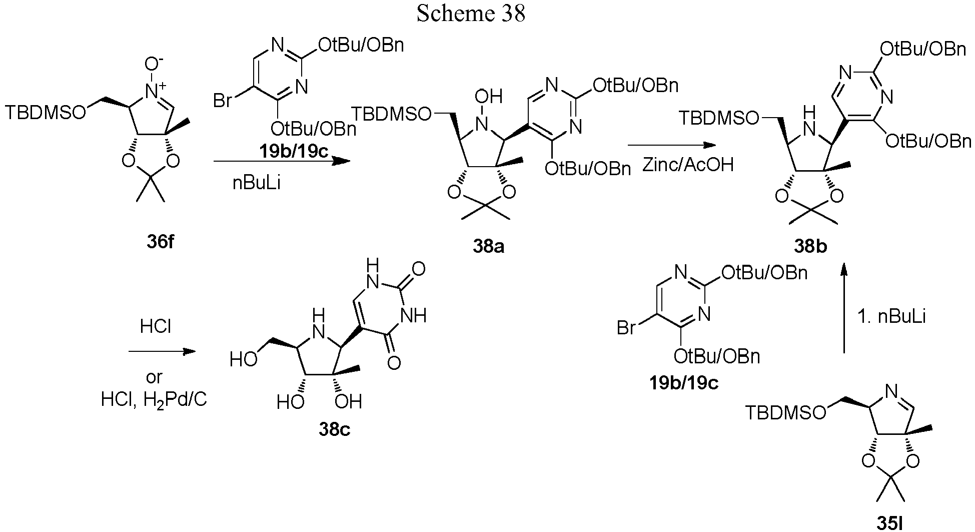

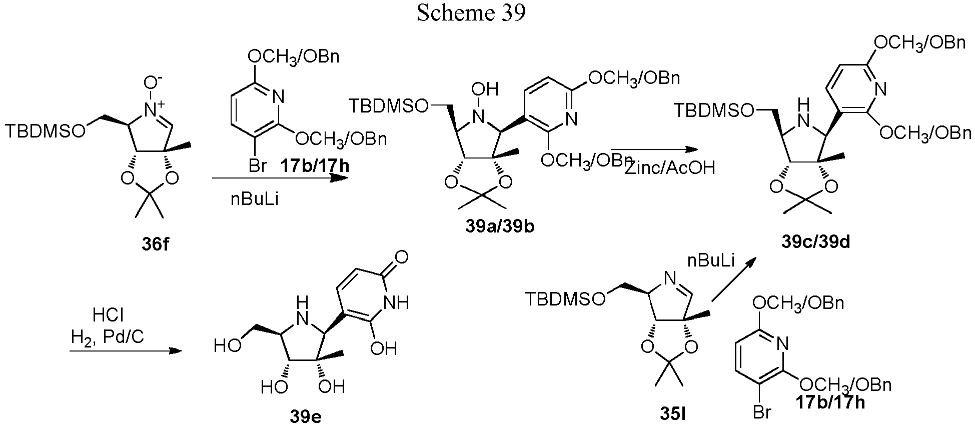

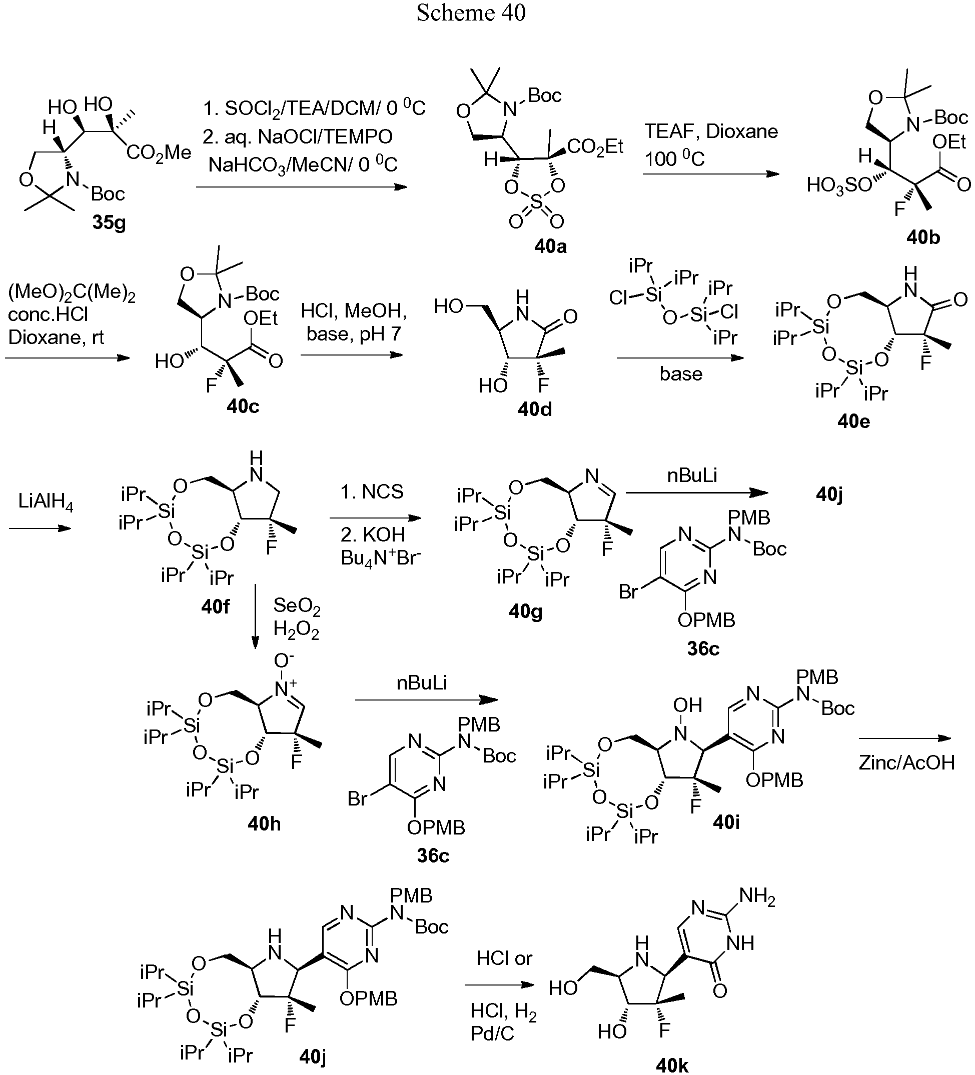

Reference for approach similar to conversion of compound **35g** to compound **40d:** An Efficient and Diastereoselective Synthesis of PSI-6130: A Clinically Efficacious Inhibitor of HCV NS5B Polymerase; Wang, Peiyuan et al.; Journal of Organic Chemistry, 74(17), 6819-6824; 2009.

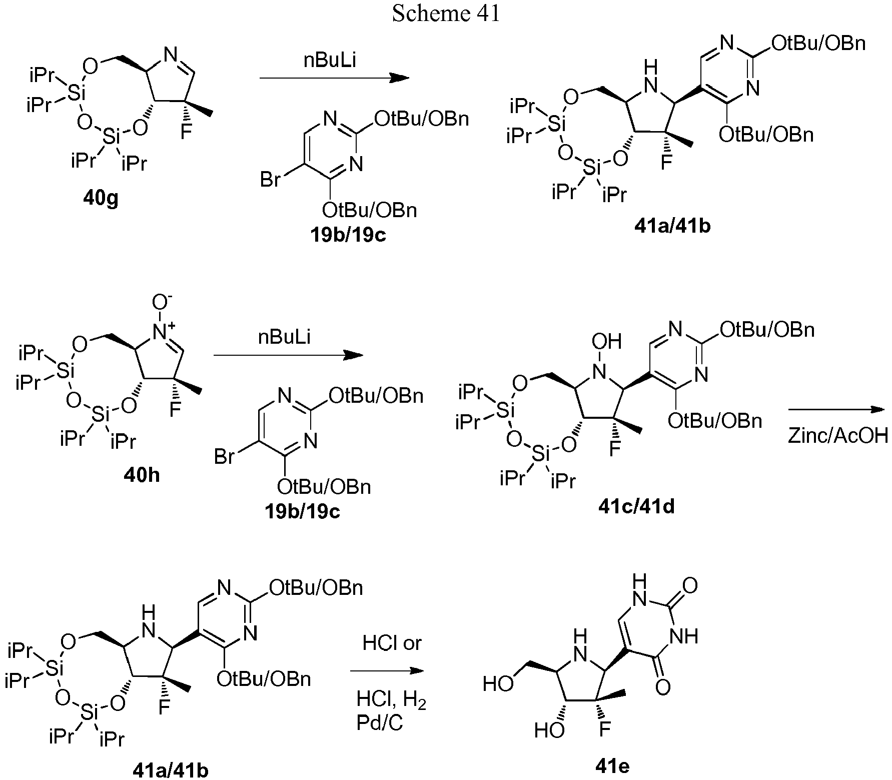

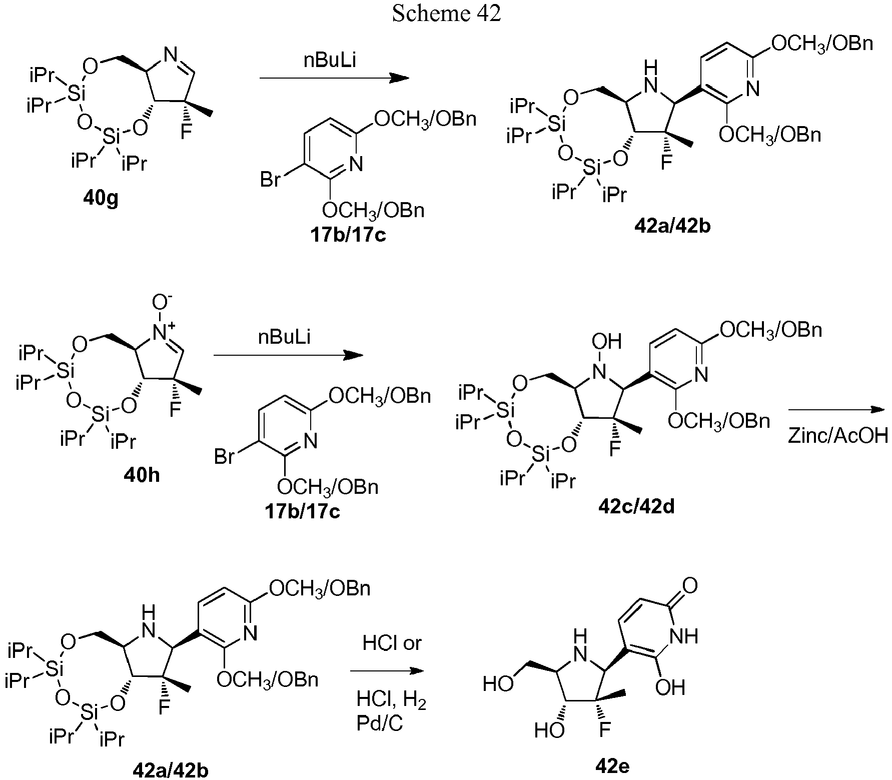

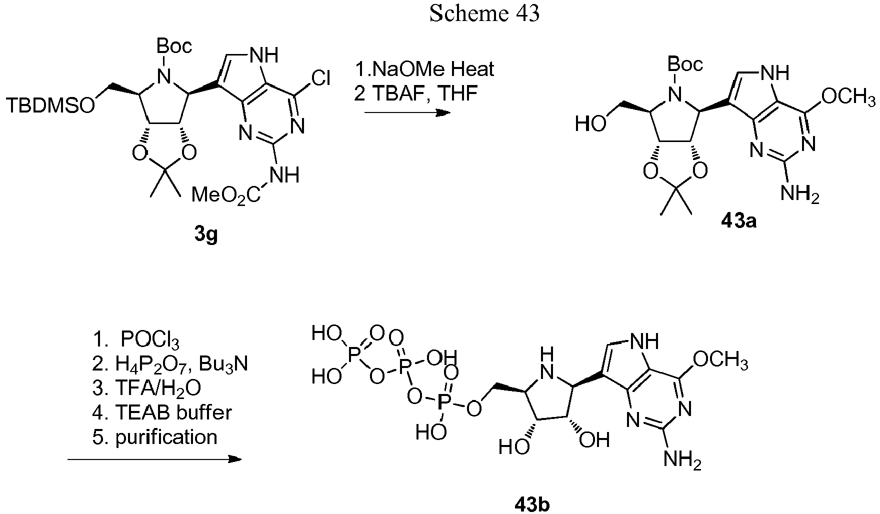

The compounds of the invention can be formulated as pharmaceutical compositions and administered to a mammalian host, such as a human patient, in a variety of forms adapted to the chosen route of administration, e.g., orally or parenterally, by intravenous, intraperitoneal, intramuscular, topical or subcutaneous routes.

Thus, the present compounds may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1 % of active compound. The percentage of the compositions and preparations may, of course, be varied and may conveniently be between about 2% to about 60% of the weight of a given unit dosage form. The amount of active compound in such therapeutically useful compositions is such that an effective dosage level will be obtained.

The tablets, troches, pills, capsules, and the like may also contain the following diluents and carriers: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The active compound may also be administered intravenously or intraperitoneally by infusion or injection. Solutions of the active compound or its salts can be prepared in water or physiologically acceptable aqueous solution, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, methods of preparation can include vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compounds of Formula I to the skin are known to the art; for example, see Jacquet et al. (U.S. Pat. No. 4,608,392; incorporated by reference), Geria (U.S. Pat. No. 4,992,478; incorporated by reference), Smith et al. (U.S. Pat. No. 4,559,157; incorporated by reference), and Wortzman (U.S. Pat. No. 4,820,508; incorporated by reference).

Useful dosages of the compounds of the invention can be determined by comparing their *in vitro* activity and *in vivo* activity in animal models. Methods for the extrapolation of effective dosages in mice, and other animals, to humans are known to the art; for example, see U.S. Pat. No. 4,938,949 (incorporated by reference).

The amount of the compound, or an active salt or derivative thereof, required for use in treatment will vary not only with the particular compound or salt selected but also with the route of administration, the nature of the condition being treated, and the age and condition of the patient and will be ultimately at the discretion of the attendant physician or clinician.

In general, however, a suitable dose will be in the range of from about 0.5 to about 100 mg/kg body weight of the recipient per day, e.g., from about 3 to about 90 mg/kg of body weight per day, from about 6 to about 75 mg per kilogram body weight per day, from about of 10 to about 60 mg/kg/day, or from about 15 to about 50 mg/kg/day.

Compounds of the invention can be conveniently formulated in unit dosage form; for example, containing 5 to 1000 mg, 10 to 750 mg, or 50 to 500 mg of active ingredient per unit dosage form. In one embodiment, the invention provides a composition comprising a compound of the invention formulated in such a unit dosage form. The desired dose may conveniently be presented in a single dose or as divided doses to be administered at appropriate intervals, for example, as two, three, four or more sub-doses per day. The sub-dose itself may be further divided, e.g., into a number of discrete loosely spaced administrations, such as multiple inhalations from an insufflator or by application of a plurality of drops into the eye.

Compounds of the invention can also be administered in combination with other therapeutic agents, for example, other agents that are useful for treating a viral infection.

The invention also provides a kit comprising a compound of the invention, or a pharmaceutically acceptable salt thereof:, at least one other therapeutic agent, packaging material, and instructions for administering the compound of the invention or the pharmaceutically acceptable salt thereof and the other therapeutic agent or agents to a mammal to treat a viral infection in the mammal. In one embodiment, the mammal is a human.

### EXAMPLES

The invention now being generally described, it will be more readily understood by reference to the following examples, which are included merely for purposes of illustration of certain aspects of the present invention, and are not intended to limit the invention.

### Example 1: (2S,3R,4R,5R)-2-(4-amino-5H-pyrrolo[3,2-d]pyrimidin-7-yl)-5-(hydroxymethyl)pyrrolidine-3,4-diol (11j)

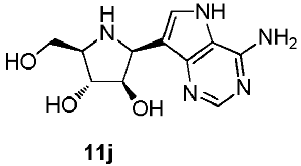

To a solution of (2S,3R,4R,5R)-tert-butyl 2-(4-amino-5H-pyrrolo[3,2-d]pyrimidin-7-yl)-3,4-dihydroxy-5-(hydroxymethyl)pyrrolidine-1-carboxylate **11i** (0.018 g, 0.049 mmol) in ethanol (5 mL) was added 6 N aqueous HCl (0.107 mL, 0.640 mmol). The reaction mixture was stirred at room temperature for 30 min and concentrated to dryness in vacuum. The residue obtained was recrystallized from ethanol to afford (2S,3R,4R,5R)-2-(4-amino-5H-pyrrolo[3,2-d]pyrimidin-7-yl)-5-(hydroxymethyl)pyrrolidine-3,4-diol **11j** (0.002 g, 15.30 % yield) as an off-white solid; ¹H NMR (300 MHz, DMSO-*d*₆) δ 8.59 (s, 1H), 8.09 (s, 1H), 5.05 (s, 1H), 4.08 - 4.05 (m, 1H), 4.01 - 3.98 (m, 1H), 3.83 - 3.70 (m, 3H); MS (ES+) 266.1 (M+1), (ES-) 263.8 (M-1).

### Preparation of (2S,3R,4R,5R)-tert-butyl 2-(4-amino-5H-pyrrolo[3,2-d]pyrimidin-7-yl)-3,4-dihydroxy-5-(hydroxymethyl)pyrrolidine-1-carboxylate (11i)

### Step 1: Preparation of D-Ribono lactone (1b)

A 22-L three-neck flask fitted with a mechanical stirrer, a 1 L pressure-equalizing addition funnel, and an efficient condenser was charged with D-ribose (**1a**) (2.0 kg, 13.33 mol) solid sodium bicarbonate (2.24 kg, 26.66 mole) and water (12 L). The reaction mixture was stirred at room temperature for 1 h at which time most of the solid disappeared. The reaction vessel was placed in an ice-bath with the internal temperature maintained at 5 ± 1 °C. The addition funnel was filled with bromine (710 mL, 13.86 mol) and the bromine was added to the vigorously stirred aqueous solution at a rate of about 5 mL/min such that the temperature was maintained between 5-10 °C. When the addition was completed (about 2.5 h) the resulting orange solution was stirred for an additional 3 h. To the reaction mixture was added solid sodium hydrogen sulfite (∼ 75 g) in small lots until the orange color was completely discharged. The clear aqueous solution was transferred to a 20-L evaporating flask, and evaporated to dryness on a rotary evaporator (80 °C, 10 mm Hg) over a period of 4 h, to leave a semi-solid residue. To the residue was added ethyl alcohol (∼ 4 L) and stirred at 40 °C for 1 h. The mixture was cooled down and filtered over a funnel to remove most of the insoluble inorganic salts. The solid residue was washed with ethyl alcohol (1 L). The filtrate was transferred to a 20-L evaporating flask and concentrated to dryness on a rotary evaporator (50 °C, 10 mm Hg) to furnish a solid residue. To this residue was added ethyl alcohol (∼ 3 L) and the slurry was stirred at room temperature for 12 h. The solid was collected by filtration and washed with ethyl alcohol (750 mL). The product D-Ribono lactone **(1b)** was dried in a vacuum oven at 40 °C (0.1 mm Hg). Yield 1.28 kg (65%); M.P. 77 - 80 °C; ¹H NMR (D₂O) δ 4.72 (d, 1 H), 4.57 (t, 1 H), 4.42 (d, 1 H), 3.80 (m, 2 H).

### Step 2: Preparation of 2,3-O-isopropylidene D-Ribono-1,4-lactone (1c)

A 50-L jacketed reaction vessel was charged with D-ribono-1,4-lactone **(1b)** (3.0 kg, 20.27 mol), and 30 L of ACS grade acetone. The reaction mixture was stirred at room temperature for 1 h. The internal temperature of the reaction vessel was lowered to 10 °C and conc. sulfuric acid (49 mL) was added slowly to the reaction mixture. Upon addition of the sulfuric acid the internal reaction temperature was allowed to warm up slowly. The reaction mixture was stirred at this temperature for 2.5 - 3 h. The reaction was monitored by TLC (TLC; 9:1, methylenechloride: methyl alcohol, R*_{f}* = 0.75). The reaction mixture was neutralized by addition of solid sodium bicarbonate (∼500 g) till the pH was neutral. The reaction mixture was filtered over a funnel. The solid residue containing inorganic salts was washed with acetone (3 L). The filtrate was transferred to a 20-L evaporation flask and evaporated to dryness (50 °C, 10 mm Hg) to give a semi-solid compound. The residue was taken in ethyl acetate (3 L) and stirred at room temperature for 4 h on rotary evaporator. The solid 2,3-O-isopropylidene D-Ribono-1,4-lactone **(1c)**, was collected by filtration and dried in a vacuum oven for 16 h at 40 °C (0.1 mm Hg). Yield: 1.819 kg (48%); MP 136 -140 °C; ¹H NMR (CDCl₃) δ 4.8 (dd, 2 H), 4.6 (s, 1 H), 3.85 (dd, 2 H), 1.5 (s, 3 H), 1.4 (s, 3 H).

### Step 3: Preparation of 2,3-O-isopropylidene 5-O-methanesulfonyl D Ribono-1,4-lactone (1d)

A solution of 2,3-O-isopropylidene D-Ribono-1,4-lactone **(1c)** (4.3 kg, 22.96 mol) in ACS grade pyridine (20 L) was stirred in a 50 L reaction vessel at room temperature for 15 min till complete dissolution. The internal temperature of the reaction vessel was lowered to -15 °C followed by slow addition of methane sulfonylchloride (1.96 L, 25.26 mol) over a period of 2 h. The internal temperature was maintained at 0 - 5 °C. The reaction was stirred at 0 °C for ∼ 2 h under inert atmosphere till the reaction TLC showed no SM (TLC; 7:3 ethyl acetate:hexane, R*_{f}* = 0.85). Upon completion of the reaction DCM (10 L) was added and extracted with 3 N HCl (4 times, pH = 3), [Back extract the aqueous layer with DCM (5 L) each time] followed by quick saturated NaHCO₃ wash. The organic fraction was dried over sodium sulfate, filtered and evaporated to syrup. Yield: 4.89 kg (80 %). The product 2,3-O-isopropylidene 5-O-methanesulfonyl D Ribono-1,4-lactone **(1d)** was taken to the next step without any further purification; ¹H NMR (CDCl₃) δ 4.8 (m, 3H), 4.5 (m, 2H), 3.08 (s, 3H), 1.5 (s, 3H), 1.4 (s, 3H).

### Step 4: Preparation of 2,3-O-isopropylidene L Lyxono-1,4-lactone (1e)

To 2,3-O-isopropylidene 5-O-methanesulfonyl D Ribono-1,4-lactone **(1d)** (3.04 kg, 11.37 mol) was added water (10 L), followed by slow addition of solid KOH (1.83 kg, 32.77 mol). [Caution: The compound goes into solution upon addition of solid KOH. The reaction is exothermic while adding KOH so the reaction vessel has to be placed in an ice-bath]. By the time the addition of KOH was complete the reaction temperature had reached 45 °C. The reaction mixture was stirred at ∼ RT for 3 h. The solution was again cooled down in ice-bath and then acidified to pH =3 (exact) using conc. HCl solution. The reaction mixture was evaporated to give a solid brown residue. The residue was stirred twice with boiling acetone (∼ 5 L) for 1 h. and the organics was decanted. The remaining salts were then dissolved in minimum amount of water and pH adjusted to 3 using conc. HCl (∼ 200 mL). The aqueous solution was concentrated and the solid residue was extracted with acetone (∼5 L). The organic layer was dried, filtered, and evaporated to give white needles of 2,3-O-isopropylidene L Lyxono-1,4-lactone **(1e).** Crystallization can be carried in hot acetone. Yield: 1.6 kg (75%); ¹H NMR (D₂O) δ 5.00 (m, 2H), 3.8 (m, 3H), 1.5 (s, 3H), 1.4 (s, 3H).

### Step 5: Preparation of 2,3-O-isopropylidene 5-O-tertbutyldimethylsilyl L Lyxono-1,4-lactone (1f)

A 22-L 3-neck flask fitted with mechanical stirrer was added 2,3-O-isopropylidene L Lyxono-1,4-lactone **(1e)** (2.0 kg, 10.63 mol), DMAP (∼25 g), Imidazole (1.60 kg, 23.40 mol, 2.2 equiv.) and stirred in ACS grade DMF (8 L) for 1 h. The reaction temperature was lowered to 0 °C using ice-bath. To the reaction mixture was added TBDMSCI (2.08 kg, 13.81 mol, 1.3 equiv.) slowly over a period of 2 h. The reaction mixture was stirred at room temperature under inert atmosphere for 14 h. Upon completion of the reaction as indicated by TLC (7:3, EtOAc: hexane, R*_{f}* = 0.80), the reaction mixture was poured in ice water and extracted with EtOAc (x 2). The organic layer was separated, dried and filtered to give an oily residue. The reaction vessel, which contains the product, was placed in an ice-bath followed by addition of hexanes (∼3 L). The compound does crystallize in hexane. Filter the crystals and wash the crystals with minimal amount of hexanes and place the product in vacuum oven at 40 °C overnight to furnish 2,3-O-isopropylidene 5-O-tertbutyldimethylsilyl L Lyxono-1,4-lactone **(1f)** 3.01 kg (93%); ¹H NMR (CDCl₃) δ 4.8 (s, 2H), 4.5 (m, 1H), 3.9 (m, 2H), 1.5 (s, 3H), 1.4 (s, 3H), 0.9 (s, 9H), 0.0 (s, 6H).

### Step 6: Preparation of 2-(tert-Butyldimethylsilanoxy)-1-(5-hydroxymethyl-2,2-dimethyl-[1,3]dioxolano-4-yl)-ethanol (1g)

A solution of 2,3-O-isopropylidene 5-O-tertbutyldimethylsilyl L Lyxono-1,4-lactone **(1f)** (3.00 kg, 9.93 mol) in THF:MeOH (9:1 v/v mixture, 15 L) was stirred at RT for 0.5 h till complete dissolution was observed. The internal temperature of the reaction vessel was lowered to -5 °C. Sodium borohydride (751 g, 19.86 mol) was added in small portions such that the temperature did not exceed 15-17 °C. Addition of the reagent was completed over a period of 1 h. Allow the reaction to attain room temperature over a period of 3 h and then continue stirring at this temperature for 18 h. The reaction mixture was monitored by TLC (3:7, ethylacetate: hexane, R*_{f}* = 0.15). Upon completion of the reaction the solution was diluted with EtOAc (5 L), and washed with 1 N HCl solution (2 times). The organic layer was washed with water, dried and evaporated to give an oily residue. To this add ∼ 3 L of hexanes and cool the evaporating flask in ice-bath. The crystals will crash out of the solution. Filter the crystals and wash with -250 mL of hexanes. Dry in vacuum oven at 40 °C for 24 h, to furnish 2-(tert-Butyldimethylsilanoxy)-1-(5-hydroxymethyl-2,2-dimethyl-[1,3]dioxolano-4-yl)-ethanol **(1g)** Yield: 2.32 kg (77%); ¹H NMR (CDCl₃) δ 4.2 (m, 2H), 3.7 (m, 5H), 1.5 (s, 3H), 1.4 (s, 3H), 0.9 (s, 9H), 0.0 (s, 6H).

### Step 7: Preparation of Methanesulfonic acid 2-(tertbutyldimethylsilayloxy)-1-1(5-methanesulfonyloxymethyl-2,2-dimethyl-[1,3]dioxolan-4-yl)-ethyl ester (1h)

A 500 mL 3-neck flask was charged with dry pyridine (20 mL), catalytic amount of DMAP followed by addition of methane sulfonyl chloride (4.98 mL, 64.4 mmol) at 0 °C. 2-(tert-Butyldimethylsilanoxy)-1-(5-hydroxymethyl-2,2-dimethyl-[1,3]dioxolano-4-yl)-ethanol **(1g)** (5.0 g, 16.3 mmol) dissolved in dry pyridine (20 mL) was added slowly to the reaction vessel. The reaction was stirred under inert atmosphere for 4 h at this temperature. (TLC; 1:9 ethylacetate:hexane, R*_{f}* = 0.85). Upon completion of the reaction, add 1ml of water and 100 mL EtOAc and stir. Extract the organic layer with water, dry and evaporate to give syrup of methanesulfonic acid 2-(tertbutyldimethylsilayloxy)-1-1(5-methanesulfonyloxymethyl-2,2-dimethyl-[1,3]dioxolan-4-yl)-ethyl ester **(1h).** Yield: 8.7 g (90%). The crude was taken to the next step without any further purification.

### Step 8: Preparation of 5-O-tertbutyldimethylsilyl-1,4-N-benzylimino-2,3-O-ispropylidene-D-ribitol (1i)

To methanesulfonic acid 2-(tertbutyldimethylsilayloxy)-1-1(5-methanesulfonyloxymethyl-2,2-dimethyl-[1,3]dioxolan-4-yl)-ethyl ester **(1h)** (8.6 g) was added neat benzylamine (10 mL) and the reaction was heated to 70 °C for 48 h. TLC (4:1 hexane: EtOAc, R*_{f}* = 0.68) showed that the reaction was complete. The reaction mixture was cooled down and brine was added to the reaction mixture. Extract the reaction mixture with dichloromethane, wash with water, dry and evaporate to furnish syrup which contained a lot of the amine reagent. The residue was taken up in toluene and to that dry ice chips were added so as to precipitate out the salts. Filter the solid and evaporate the filtrate to furnish the desired product 5-O-tertbutyldimethylsilyl-1,4-N-benzylimino-2,3-O-ispropylidene-D-ribitol **(1i)** (5.6 g, 92%). This was taken directly to the next step without any further purification, ¹H NMR (CDCl₃) δ 7.2 - 7.4 (m, 5H), 4.65 (m, 1H), 4.55 (dd, 1H), 4.0 (d, 1H), 3.6 - 3.8 (m, 3H), 3.1 (dd, 1H), 3.0 (m, 1H), 2.75 (dd, 1H), 1.5 (s, 3H), 1.34 (s, 3H), 0.9 (s, 9H), 0.0 (s, 6H).

### Step 9: Preparation of 5-O-tertbutyldiemthylsilyl-1,4-imino-2,3-O-ispropylidene-D-ribitol (1j)

To 5-O-tertbutyldimethylsilyl-1,4-N-benzylimino-2,3-O-ispropylidene-D-ribitol **(1i)** (5.93 g, 15.74 mmol) in ethanol (15 mL) was added Pd/C (50 mg) and the reaction was hydrogenated at 80 psi for 5 h, or until TLC (3:2, hexane: EtOAc, R*_{f}* = 0.18) showed the reaction to be complete. The reaction mixture was filtered over Celite pad and the Celite pad was washed with ethanol (25 mL). The filtrate was passed through a Millipore filter (0.25 µ) to remove traces of the catalyst and evaporated to furnish 5-O-tertbutyldiemthylsilyl-1,4-imino-2,3-O-ispropylidene-D-ribitol **(1j)** as a syrup. Yield: 3.5 g (75% - steps); ¹H NMR (CDCl₃) δ 4.65 (m, 2H), 3.60 (dd, 2H), 3.24 (t, 1H), 3.00 (d, 2H), 1.5 (s, 3H), 1.34 (s, 3H), 0.9 (s, 9H), 0.0 (s, 6H).

### Step 10: Preparation of (3aR,4R,6aS)-4-(((tert-butyldimethylsilyl)oxy)methyl)-2,2-dimethyl-4,6a-dihydro-3aH-[1,3]dioxolo[4,5-c]pyrrole (1k)

A solution of 5-O-tertbutyldiemthylsilyl-1,4-imino-2,3-O-ispropylidene-D-ribitol **(1j)** (94 g, 327 mmol) in toluene (470 mL) is added to a suspension of N-Chlorosuccinimide (54.6 g, 408.8 mmol) in toluene (470 mL) at 17 to 23°C over a period of 60 to 90 minutes. The reaction mixture was stirred at 17 to 23°C for 1 hour, chilled to -3 to 3°C and stirred for additional hour. The succinimide by-product is removed by filtration and the filtered solution charged directly to a 60% potassium hydroxide solution (458 g, 8175 mmol in 305 mL of water) containing tetrabutylammonium bromide (10.53 g, 32.7 mmol). The reaction mixture is stirred at -5 to 5°C for 17 h. Water (700 mL) is then added to the two-phase mixture to dissolve inorganic precipitates and the toluene product solution is washed with an ammonium acetate buffer (pH ∼4.5), buffered brine solution (700 mL) and stabilized with triethylamine prior to drying by circulation through magnesium sulphate and then by charging magnesium sulphate to the reactor. The dried solution containing (3aR,4R,6aS)-4-(((tert-butyldimethylsilyl)oxy)methyl)-2,2-dimethyl-4,6a-dihydro-3aH-[1,3]dioxolo[4,5-c]pyrrole **(1k)** in toluene is used as such immediately for next step.

### Step 11: Preparation of 1S-5-O-tert-butyldimethylsilyl-1,4-dideoxy-1-C-[(4-methyoxypyrrolo[3,2-d]pyrimidin-9-N-(benzyloxomethyl)-7-yl)]-1,4-imino-2,3-O-isopropylidine-D-ribitol (4b)

6-Methoxy-*N*-(benzyloxymethyl)-9-deazahypoxanthine **(4a)** (271.0 g, 0.775 mole) was added to a 22 L 3-neck round-bottom flask containing anhydrous anisole (1.7 L) under a N₂ atmosphere. This mixture was heated gently until the mixture became homogenous (≈ 45 °C). The mixture was cooled to ambient temperature and anhydrous ether (2.9 L) was added. The reaction flask was placed into a cooling bath and cooled to -70 °C using dry ice/acetone. At ≈-20 °C, the bromide started precipitating as a fine white solid. To the suspension was added nBuLi (1.6 *N,* 486 mL, 0.778 mol) over a 1.2 h period via a dropping funnel such that the internal temperature was maintained < -50 °C. After the last addition, TLC (30% EtOAc/hexane) analysis indicated < 2% of the bromide remained. (3aR,4R,6aS)-4-(((tertbutyldimethylsilyl)oxy)methyl)-2,2-dimethyl-4,6a-dihydro-3aH-[1,3]dioxolo[4,5-c]pyrrole **(1k)** (183 g, 0.642 mole) in toluene was added over a 15 minute period via an addition funnel maintaining the internal temperature below -50 °C. The reaction mixture was a pale-amber color. The reaction flask was removed from the cooling bath and allowed to warm. The reaction mixture was allowed to warm to -2 °C and TLC (40% EtOAc/hexane, visualized with Ehrlichs reagent) showed no remaining (3aR,4R,6aS)-4-(((tertbutyldimethylsilyl)oxy)methyl)-2,2-dimethyl-4,6a-dihydro-3aH-[1,3]dioxolo[4,5-c]pyrrole **(1k).** The reaction was quenched with H₂O (2 L) and extracted with ether (2 x 2 L). The combined organic layers were dried (MgSO₄) and concentrated *in vacuum* (high vacuum at 60 °C was used to remove anisole) to give a crude dark oil of 1S-5-*O*-tert-butyldimethylsilyl-1,4-didcoxy-1-*C*-[(4-methyoxypyrrolo[3,2-*d*]pyrimidin-9-*N*-(benzyloxomethyl)-7-yl)]-1,4-imino-2,3-*O*-isopropylidine-D-ribitol **(4b)** which was suitable for use in the next step: yield 284 g (79%). A small amount (5 g) of the crude mixture was purified by flash column chromatography (silica gel, eluting with 0-40% ethyl acetate in hexane) to furnish 1S-5-*O-*tert-butyldimethylsilyl-1,4-dideoxy-1-*C*-[(4-methyoxypyrrolo[3,2-*d*]pyrimidin-9-*N-*(benzyloxomethyl)-7-yl)]-1,4-imino-2,3-*O*-isopropylidine-D-ribitol **(4b)** as an orange syrup (3.4 g); ¹H NMR (DMSO-*d₆*) δ 0.02 (s, 3 H), 0.03 (s, 3 H), 0.8 (s, 9 H), 1.25 (s, 3 H), 1.48 (s, 3 H), 3.11-3.20 (m, 1 H), 3.60-3.71 (m, 2 H), 4.05 (s, 3 H), 4.26 (d, 1 H, J = 4.7 Hz), 4.49 (s, 2 H), 4.52-4.56 (m, 1 H), 4.81-4.85 (m, 1 H), 5.71 (s, 2 H), 7.21-7.32 (m, 5 H), 7.80 (s, 1 H), 8.40 (s, 1 H); ¹³C NMR (CDCl₃) δ -5.46, -5.43, 18.30, 25.53, 25.88, 27.63, 53.43, 61.59, 62.54, 66.14, 70.14, 76.93, 82.32, 86.40, 114.43, 116.22, 116.56, 127.67, 127.93, 128.43, 130.55, 136.93, 149.61, 149.82, 156.16; IR 3420, 1610 cm⁻¹; MS (ES+) m/z 555.3; Analysis Calculated for C₂₉H₄₂N₄O₅Si: C, 62.79; H, 7.63; N, 10.10; Found :C, 62.95; H, 7.59; N, 9.95.

### Step 12: Preparation of 1S-N-tert-butoxycarbonyl-5-O-tert-butyldimethylsilyl-1,4-dideoxy-1-C-[(4-methyoxypyrrolo[3,2-d]pyrimidin-9-N-(benzyloxomethyl)-7-yl)]-1,4-imino-2,3-O-isopropylidine-D-ribitol (11a)

Crude 1S-5-*O*-tert-butyldimethylsilyl-1,4-dideoxy-1-*C*-[(4-methyoxypyrrolo[3,2-*d*]pyrimidin-9-*N*-(benzyloxomethyl)-7-yl)]-1,4-imino-2,3-*O*-isopropylidine-D-ribitol **(4b)** (275 g, 0.496 mole) was taken up in CH₂Cl₂ (1.4 L) and cooled to 5 °C in an ice/water bath. To this cooled mixture was added Boc₂O (168.5 g, 0.772 mole) in four portions such that the reaction mixture temperature was maintained < 10 °C. After 30 min, TLC (40% ethyl acetate/hexane) showed no starting material remained. The crude mixture was absorbed on SiO₂ (700 g) and purified by flash chromatography (silica gel 1.5 Kg, eluting with 10% ethyl acetate in hexane). The appropriate fractions were pooled and concentrated in vacuum to give 1S-N-tert-butoxycarbonyl-5-O-tert-butyldimethylsilyl-1,4-dideoxy-1-C-[(4-methyoxypyrrolo[3,2-d]pyrimidin-9-N-(benzyloxomethyl)-7-yl)]-1,4-imino-2,3-O-isopropylidine-D-ribitol **(11a)** (272 g, 84%) as a yellow syrup; ¹H NMR (CDCl₃) δ 0.02 (s, 3 H), 0.03 (s, 3 H), 0.82 (s, 9 H), 1.31-1.58 (m, 15 H) 2.05-2.09 (m, 1 H); 3.58-3.80 (m, 2 H), 4.08 (s, 3 H), 4.17-4.32 (m, 1 H), 4.44 (s, 2 H), 4.84-5.71 (m, 4 H), 7.19-7.33 (m, 5 H), 7.46 (s, 1 H), 8.51 (s, 1 H); ¹³C NMR (CDCl₃) δ -5.31, -5.20, 14.10, 14.20, 18.32, 21.01, 22.64, 25.56, 25.93, 27.46, 28.46, 31.58, 53.44, 60.34, 62.48, 70.08, 76.96, 79.84, 111.69, 115.89, 127.67, 127.93, 128.43, 136.90, 148.62, 149.90, 154.38, 156.19; IR 1692, 1608 cm⁻¹; MS (ES+) m/z 655.3; Analysis Calculated for C₃₄H₅₀N₄O₇Si: C, 62.43; H, 7.65; N, 8.56;Found: C, 62.79; H, 7.89; N, 8.47.

### Step 13: Preparation of (2S,3S,4R,5R)-tert-butyl 2-(5-(benzyloxymethyl)-4-methoxy-5H-pyrrolo[3,2-d]pyrimidin-7-yl)-3,4-dihydroxy-5-(hydroxymethyl)pyrrolidine-1-carboxylate (4c)

To a solution of 1S-N-tert-butoxycarbonyl-5-O-tert-butyldimethylsilyl-1,4-dideoxy-1-C-[(4-methyoxypyrrolo[3,2-d]pyrimidin-9-N-(benzyloxomethyl)-7-yl)]-1,4-imino-2,3-O-isopropylidine-D-ribitol **(11a)** (65.6 g, 100 mmol) in methanol (260 mL) at 35 °C was added 1 N aqueous HCl (145 mL) slowly with stirring. After stirring for 1 h, the heating was suspended and the reaction was stirred for 24h at room temperature. After cooling with ice, ammonium hydroxide was used to adjust pH to about 9. The reaction mixture was concentrated in vacuum and the residue obtained was purified by flash column chromatography eluting with ethyl acetate to afford (2*S,*3*S,*4*R,*5*R*)-*tert*-butyl 2-(5-(benzyloxymethyl)-4-methoxy-5*H*-pyrrolo[3,2-*d*]pyrimidin-7-yl)-3,4-dihydroxy-5-(hydroxymethyl)pyrrolidine-1-carboxylate **(4c)** (47.5 g, 95 % yield); ¹H NMR: (DMSO-*d*₆) δ 8.43 (s, 1H), 7.80 (s, 1H), 7.23-7.32 (m, 5H), 5.70 (m, 3H), 5.06 (m, 1H), 4.91 (m, 1H), 4.68 (m, 1H), 4.39 (s, 2H), 4.37 (m, 1H), 4.05 (s, 3H), 3.98 (m, 2H), 3.58 (m, 2H), 1.02-1.41 (m, 9H); MS (ES+) 501.5 (M+1).

### Step 14: (6aR,8S,9S,9aR)-tert-Butyl 8-(5-(benzyloxymethyl)-4-methoxy-5H-pyrrolo[3,2-d]pyrimidin-7-yl)-9-hydroxy-2,2,4,4-tetraisopropyltetrahydro-[1,3,5,2,4]trioxadisilocino[7,6-b]pyrrole-7(8H)-carboxylate (11c)

To the solution of (2*S*,3*S*,4*R*,5*R*)-*tert*-butyl 2-(5-(benzyloxymethyl)-4-methoxy-5*H-*pyrrolo [3,2-*d*]pyrimidin-7-yl)-3,4-dihydroxy-5-(hydroxymethyl)pyrrolidine-1-carboxylate **(4c)** (10 g, 20 mmol) and imidazole (5.45 g, 80 mmol) in DMF (50 mL) at 0 °C was added dropwise 1,3-Dichloro-1,1,3,3-tetraisopropyl-disiloxane (7.04 mL, 22 mmol) and stirred at room temperature overnight. The reaction mixture was diluted with ethyl acetate and then washed with water, dried, and concentrated to drynesss. The residue was purified by flash column chromatography eluting with hexanes in ethyl acetate (2:1) to afford (6*aR*,8*S*,9*S*,9*aR*)-*tert*-Butyl 8-(5-(benzyloxymethyl)-4-methoxy-5*H*-pyrrolo[3,2-*d*]pyrimidin-7-yl)-9-hydroxy-2,2,4,4-tetraisopropyltetrahydro-[1,3,5,2,4]trioxadisilocino[7,6-*b*]pyrrole-7(8*H*)-carboxylate **(11c)** (8.3 g, 56%) as a foam; ¹H NMR: (DMSO-*d*₆) δ 8.41 (s, 1H), 7.56 (s, 1H), 7.16-7.29 (m, 5H), 5.76 (br, 1H), 5.65 (d, *J =* 9.0Hz, 1H), 5.30 (d, *J =* 3.0Hz, 1H), 4.96 (s, 1H), 4.55 (dd, J= 12.0, 3.0Hz, 1H), 4.43 (s, 2H), 4.30 (br, 1H), 4.04 (s, 3H), 3.64 (m, 1H), 1.20-1.43 (m, 9H), 0.85-1.02 (m, 30H). MS (ES+) 744 (M+1).

### Step 15: (6aR,8S,9R,9aR)-tert-Butyl 8-(5-(benzyloxymethyl)-4-methoxy-5H-pyrrolo[3,2-d]pyrimidin-7-yl)-9-hydroxy-2,2,4,4-tetraisopropyltetrahydro-[1,3,5,2,4]trioxadisilocino[7,6-b]pyrrole-7(8H)-carboxylate (11d)

A solution of DMSO (2.5 mL, 35 mmol) in DCM (60 mL) was cooled down to -70 °C, and trifluoroacetic anhydride (2.5 mL, 18 mmol) was added to the stirred solution. After stirring for 10 min, a solution of (6*aR*,8*S*,9*S*,9*aR*)-*tert*-Butyl 8-(5-(benzyloxymethyl)-4-methoxy-5*H* pyrrolo[3,2-*d*]pyrimidin-7-yl)-9-hydroxy-2,2,4,4-tetraisopropyltetrahydro-[1,3,5,2,4]trioxadisilocino[7,6-*b*]pyrrole-7(8*H*)-carboxylate **(11c)** (2.3 g, 3.1 mmol) in DCM (50 mL) was added followed by triethylamine (3.7 mL, 50 mmol). After stirring for 15 min, the solution was allowed to warm up room temperature and washed with water, dried, and then concentrated in vacuum to give the intermediate ketone, which was directly applied to the next step without purification. The ketone obtained was dissolved in methanol (100 mL) and cooled to 0 °C. Sodium borohydride (1.2 g, 30 mmol) was added to this solution and the reaction mixture was stirred for 30 min. The reaction mixture was concentrated and diluted with ethyl acetate and then washed with water, dried, and concentrated to drynesss. The residue was purified by chromatography eluting with hexanes in ethyl acetate (2:1) to afford (6*aR*,8*S*,9*R*,9*aR*)-*tert*-Butyl 8-(5-(benzyloxymethyl)-4-methoxy-5*H*-pyrrolo[3,2-*d*]pyrimidin-7-yl)-9-hydroxy-2,2,4,4-tetraisopropyltetrahydro-[1,3,5,2,4]trioxadisilocino[7,6-*b*]pyrrole-7(8*H*)-carboxylate **(11d)** as a syrup (2.2 g, 96%); ¹H NMR: (DMSO-*d*₆) δ 8.35 (s, 1H), 7.62 (s, 1H), 7.19-7.29 (m, 5H), 5.75 (d, *J =* 12.0Hz, 1H), 5.71 (d, *J =* 12.0Hz, 1H), 5.27 (d, *J =* 6.0Hz, 1H), 5.15 (d, *J =* 6.0Hz, 1H), 4.96 (m, 1H), 4.48 (d, *J =* 12.0Hz, 1H), 4.40 (d, *J =* 12.0Hz, 1H), 4.24 (m, 2H), 4.12 (m, 1H), 4.04 (s, 3H), 3.51 (m, 2H), 3.37 (dd, *J =* 15.0, 6.0Hz, 1H), 1.31 (s, 9H), 0.83-1.02 (m, 27H). MS (ES+) 744.0 (M+1).

### Step 16: 7-((2S,3R,4R,5R)-3,4-Dihydroxy-5-(hydroxymethyl)pyrrolidin-2-yl)-3H-pyrrolo[3,2-d]pyrimidin-4(5H)-one hydrochloride (11e).

A solution of (6*aR*,8*S*,9*R*,9*aR*)-*tert*-Butyl 8-(5-(benzyloxymethyl)-4-methoxy-5*H-*pyrrolo[3,2-*d*]pyrimidin-7-yl)-9-hydroxy-2,2,4,4-tetraisopropyltetrahydro-[1,3,5,2,4]trioxadisilocino[7,6-*b*]pyrrole-7(8*H*)-carboxylate **(11d)** (2.4 g, 3.22 mmol) in conc. HCl (20 mL) was heated at reflux with stirring for 1 hour. The solution was then concentrated in vacuum to dryness, redissolved in water, and decolorized with activated carbon. After filtering over diatomaceous earth, the product was crystallized by the addition of ethanol to give 7-((2*S*,3*R*,4*R*,5*R*)-3,4-Dihydroxy-5-(hydroxymethyl)pyrrolidin-2-yl)-3*H*-pyrrolo[3,2-*d*]pyrimidin-4(5*H*)-one hydrochloride **(11e)** as white solid (0.42 g, 43%); ¹H NMR (D₂O) δ 7.95 (s, 1H), 7.73 (s, 1H), 5.11 (d, *J =* 3.0Hz, 1H), 4.35 (m, 1H), 4.17 (s, 1H), 3.97 (dd, *J =* 12.0, 6.0Hz, 1H), 3.85 (dd, *J* = 12.0, 9.0Hz, 1H), 3.67 (m, 1H). ¹³C NMR: δ 155.12, 143.39, 142.49, 130.21, 117.36, 105.34, 76.37, 75.91, 67.12, 59.60, 57.51; HRMS: Calculated for C₁₁H₁₅N₄O₄ (M+H⁺): 267.1088; Found: 267.1081.

### Step 17: (2R,3R,4R,5S)-tert-butyl 3,4-dihydroxy-2-(hydroxymethyl)-5-(4-oxo-4,5-dihydro-3H-pyrrolo[3,2-d]pyrimidin-7-yl)pyrrolidine-1-carboxylate (11f)

To a suspension of 7-((2S,3R,4R,5R)-3,4-dihydroxy-5-(hydroxymethyl)pyrrolidin-2-yl)-3H-pyrrolo[3,2-d]pyrimidin-4(5H)-one dihydrochloride **(11e)** (0.37 g, 1.091 mmol) in a methanol (5 mL), water (5.00 mL) was added triethylamine (0.456 mL, 3.27 mmol) at room temperature followed by di-tert-butyl dicarbonate (0.731 g, 3.35 mmol). The reaction mixture was stirred at room temperature overnight. The reaction mixture was concentrated in vacuum and the residue obtained was purified by flash column chromatography (silica gel 12 g, eluting with 0-100% (9:1) ethyl acetate/methanol in hexane) to furnish (2R,3R,4R,5S)-tert-butyl 3,4-dihydroxy-2-(hydroxymethyl)-5-(4-oxo-4,5-dihydro-3H-pyrrolo[3,2-d]pyrimidin-7-yl)pyrrolidine-1-carboxylate **(11f)** (0.289 g, 0.789 mmol, 72.3 % yield) as a white solid; ¹H NMR (300 MHz, DMSO, 300K) δ 11.83 (s, 1H), 11.77 (d, *J* = 3.5 Hz, 1H), 7.76 (d, *J* = 3.5 Hz, 1H), 7.15 (d, *J* = 2.9 Hz, 1H), 5.36 - 5.26 (m, 1H), 5.23 - 5.16 (m, 2H), 5.08 - 4.90 (m, 1H), 4.08 (s, 1H), 3.85 (dd, *J* = 11.0, 5.4 Hz, 2H), 3.70 - 3.60 (m, 1H), 3.58 - 3.52 (m, 1H), 1.03 (bs, 9H); ¹H NMR (300 MHz, DMSO, 370K) δ 11.50 (s, 1H), 11.41 (s, 1H), 7.70 (s, 1H), 7.13 (d, *J* = 3.0 Hz, 1H), 5.20 (d, *J* = 6.0 Hz, 1H), 5.08 - 4.75 (m, 2H), 4.74 - 4.50 (m, 1H), 4.08 (d, *J =* 2.7 Hz, 1H), 3.95 - 3.80 (m, 2H), 3.69 (dd, *J =* 10.6, 3.7 Hz, 1H), 3.63 - 3.54 (m, 1H), 1.16 (s, 9H); MS (ES+) 389.0 (M+Na); (ES-) 365.0 (M-1).

### Step 18: (2R,3R,4R,5S)-2-(acetoxymethyl)-1-(tert-butoxycarbonyl)-5-(4-oxo-4,5-dihydro-3H-pyrrolo[3,2-d]pyrimidin-7-yl)pyrrolidine-3,4-diyl diacetate (11g)

To a suspension of (2R,3R,4R,5S)-tert-butyl 3,4-dihydroxy-2-(hydroxymethyl)-5-(4-oxo-4,5-dihydro-3H-pyrrolo[3,2-d]pyrimidin-7-yl)pyrrolidine-1-carboxylate **(11f)** (0.275 g, 0.751 mmol) in pyridine (2 mL, 24.73 mmol) and dichloromethane (2.5 mL) was added DMAP (0.030 g, 0.247 mmol) and acetic anhydride (0.758 mL, 8.04 mmol) at room temperature. The reaction mixture was concentrated in vacuum and the residue obtained was purified by flash column chromatography [silica gel, 0-100% (9:1) ethyl acetate/methanol in hexane] to furnish (2R,3R,4R,5S)-2-(acetoxymethyl)-1-(tert-butoxycarbonyl)-5-(4-oxo-4,5-dihydro-3H-pyrrolo[3,2-d]pyrimidin-7-yl)pyrrolidine-3,4-diyl diacetate **(11g)** (0.225 g, 0.457 mmol, 60.8 % yield) as a white solid; ¹H NMR (300 MHz, DMSO, 300K) δ 12.05 (s, 1H), 11.88 (s, 1H), 7.78 (s, 1H), 7.24 (s, 1H), 5.37 (d, *J =* 5.5 Hz, 1H), 5.31 (s, 2H), 4.47 (d, *J =* 6.8 Hz, 2H), 4.00 (t, J= 6.4 Hz, 1H), 2.10 (s, 3H), 2.06 (s, 3H), 1.74 (s, 3H), 1.15 (s, 9H); MS (ES+) 492.9 (M+1), 514.9 (M+Na), (ES-) 981.8 (2M-1); MS (ES+) 492.9 (M+1), 514.9 (M+Na), (ES-) 981.8 (2M-1).

### Step 19: (2R,3R,4R,5S)-2-(acetoxymethyl)-1-(tert-butoxycarbonyl)-5-(4-chloro-5H-pyrrolo[3,2-d]pyrimidin-7-yl)pyrrolidine-3,4-diyl diacetate (11h)

To a solution of (2R,3R,4R,5S)-2-(acetoxymethyl)-1-(tert-butoxycarbonyl)-5-(4-oxo-4,5-dihydro-3H-pyrrolo[3,2-d]pyrimidin-7-yl)pyrrolidine-3,4-diyl diacetate **(11g)** (0.22 g, 0.45 mmol) in acetonitrile (5 mL) was added benzyltriethylammonium chloride (205 mg, 0.9 mmol, 2 equiv.), dimethylaniline (0.09 mL, 0.68 mmol, 1.5 equiv.), followed by POCl₃ (0.25 mL, 2.7 mmol, 6 equiv.) at room temperature. A clear light yellow colored solution was obtained. The reaction mixture was slowly heated up to 80 °C and held at this temperature for 10 minutes. TLC in 9:1 chloroform: methanol shows that the reaction is >98% completed. The reaction mixture was allowed to slowly cool down to 50.0 °C and added silica gel (1 gm). The reaction mixture was concentrated under vacuum to dryness and slurry obtained was purified by flash column chromatography (silica gel 12 g, eluting with 0-100% (9:1) ethyl acetate/methanol in hexane) to furnish (2R,3R,4R,5S)-2-(acetoxymethyl)-1-(tert-butoxycarbonyl)-5-(4-chloro-5H-pyrrolo[3,2-d]pyrimidin-7-yl)pyrrolidine-3,4-diyl diacetate **(11h)** (0.1 gm, 44%) as a white solid; 1H NMR (300 MHz, DMSO) ä 12.45 (s, 1H), 8.63 (s, 1H), 7.86 (s, 1H), 5.52 (d, J = 5.6 Hz, 1H), 5.41 - 5.32 (m, 2H), 4.53 (d, J = 6.7 Hz, 2H), 4.08 - 4.03 (m, 1H), 2.12 (s, 3H), 2.07 (s, 3H), 1.68 (s, 3H), 1.11 (s, 9H); MS (ES+) 511.961, (ES-) 508.758.

### Step 20: Preparation of (2S,3R,4R,5R)-tert-butyl 2-(4-amino-5H-pyrrolo[3,2-d]pyrimidin-7-yl)-3,4-dihydroxy-5-(hydroxymethyl)pyrrolidine-1-carboxylate (11i)

Ammonia (30 mL, 1386 mmol) was condensed into Ethanol (30 mL) at -50°C and added to a solution of (2R,3R,4R,5S)-2-(acetoxymethyl)-1-(tert-butoxycarbonyl)-5-(4-chloro-5H-pyrrolo[3,2-d]pyrimidin-7-yl)pyrrolidine-3,4-diyl diacetate **(11h)** (0.1 g, 0.196 mmol) in ethanol (10.0 mL) in a autoclave and heated at 80°C for 24 h. The pressure rises to 200 psi. The reaction was cooled to -50°C and concentrated in vacuum to dryness. The residue obtained was purified twice by flash column chromatography (silica gel 4 g, eluting with 0-100% methanol in chloroform) to furnish (2S,3R,4R,5R)-tert-butyl 2-(4-amino-5H-pyrrolo[3,2-d]pyrimidin-7-yl)-3,4-dihydroxy-5-(hydroxymethyl)pyrrolidine-1-carboxylate **(11i)** (0.018 g, 25.2 %) as a white solid; MS (ES+) 366.0 (M+1); (ES-) 364.1 (M-1).

### Preparation of Methoxy-N-(benzyloxymethyl)-9-bromo-9-deazahypoxanthine (4a)

### Step 1: Preparation of Dimethyl 3-amino-1H-pyrrole-2,4-dicarboxylate (32b)

To a solution of diethyl aminomalonate (370.4 g, 1.75 mol) in methanol (3.6 L) at room temperature was added a 5.4 M solution of NaOMe (975 mL, 5.25 mole) in one portion (the reaction mixture was light brown in color). To the reaction mixture was added ethyl (ethoxymethylene)cyanoacetate **(32a)** (296 g, 1.75 mol) in three portions (not much temperature change was observed during the addition ∼1 °C change, the reaction color changes from light brown to dark brown). The reaction mixture was heated at refluxed for 48 hours (TLC analysis 50 % ethyl acetate in hexane was done to check disappearance of starting material). The reaction mixture was neutralized by the addition of AcOH (210 mL, 3.5 mole) to pH 6. The reaction mixture was concentrated in vacuum to furnish brown residue. Residue was triturated with water (3 L), filtered, washed with water (500 mL) and hexanes. It was air-dried for 48 h and in vacuum oven at 60 °C to furnish dimethyl 3-amino-1*H*-pyrrole-2,4-dicarboxylate **(32b)** 287 g (83%) as a brown solid. It was used as such for next step.

### Step 2: Preparation of 3H,5H-Pyrrolo[3,2-d]pyrimidin-4-one (32c)

A mixture of dimethyl 3-amino-1*H*-pyrrole-2,4-dicarboxylate **(32b)** (286 g, 1.44 mole) and formamidine acetate (451g, 4.33 mole) in ethanol (2.8 L, 2 mL/mmole) was heated at reflux overnight. The reaction mixture was not homogenous initially but after couple of hours of reflux seems homogenous and dark brown in color (the stirring becomes difficult as solid starts falling out of solution). TLC analysis of an aliquot (50% ethyl acetate in hexane) indicates still some unreacted starting material was present. The reaction mixture was continued to heat at reflux for additional 24 hours and cooled to room temperature. The solid obtained was collected by filtration washed with water and hexane and dried in vacuum to furnish 3H,5H-Pyrrolo[3,2-d]pyrimidin-4-one **(32c)** (223 g, 80%) as a light brown solid. The material was used as such without purification.

### Step 3: Preparation of 3H,5H-Pyrrolo[3,2-d]pyrimidin-4-one (32d)

A mixture of 3H,5H-Pyrrolo[3,2-d]pyrimidin-4-one **(32c)** (130.4 g, 0.675 mole) in 2 N KOH (1.35 L, 2.7 mole) was heated at gentle reflux for 40 h. The reaction mixture was cooled to 60 °C and cautiously neutralized with glacial acetic acid (162 mL, 2.7 mole) to pH 6 (foaming due to decarboxylation was observed and the color of the reaction mixture was black). The reaction mixture was cooled to room temperature and the solid obtained was collected by filtration washed with water (2 x 250 mL) air dried and the dried in high vacuum over P₂O₅ to furnish product as a blackish gray solid (145 g, 159%). NMR of the product indicates lot of acetic acid or its salt so the yield is higher TLC shows clean product plus some product in the baseline using CMA-80 as solvent system). The product was triturated with water (400 mL) and neutralized with saturated aqueous NaHCO₃ till no effervescence and pH is around 7-8). The blackish gray solid was collected by filtration and washed with water to furnish on air drying for 48 hour, 67.62 g (74%) of product. The product was further dried in vacuum at ethanol reflux temperature to give 3H,5H-Pyrrolo[3,2-d]pyrimidin-4-one **(32d)** as a blackish gray powder; MP of an analytically pure sample > 250°C; ¹HNMR (360 MHz, DMSO-*d₆*) δ 12.05 (s, D₂O exchangeable, 1H), 11.82 (s, D₂O exchangeable, 1H), 7.77 (s, 1H), 7.36 (s, 1H), 6.35 (s, 1H). ¹³C-NMR (DMSO-*d*₆) 153.88, 144.80, 141.66, 127.51, 117.92, 103.10; IR (KBr) 3107, 3030 and 1674 cm⁻¹; MS (ES+) 136.2 (M+1); Analysis Calculated for C₆H₅N₃O: C, 53.33; H, 3.73; N, 31.10; Found: C, 53.38; H, 3.77; N, 31.11.

### Step 4: Preparation of 4-Chloropyrrolo[3,2-d]Pyrimidine (32e)

To a sample of 3H,5H-Pyrrolo[3,2-d]pyrimidin-4-one **(32d)** (31.08 g, 230 mmol) under N₂ was added phosphorus oxychloride (60 mL, 644 mol). The mixture was heated at reflux for 1 h during which time the reaction became black homogenous. The reaction was cooled in an ice-water bath and then poured into chipped ice (775 mL) with stirring. The pH of the aqueous solution was slowly adjusted to ∼ pH 8 with concentrated NH₄OH (225 mL) with continued cooling of the mixture. The resulting precipitate was collected by vacuum filtration and washed with water. The solid was transferred to a drying tray and dried in vacuum at 110 °C to furnish 4-Chloropyrrolo[3,2-*d*]Pyrimidine (32e) (31.48 g, 89%) as a dark gray solid. An analytical sample was obtained by column chromatography (Silica gel, EtOAc-hexanes, 35:65) followed by evaporation of the relevant fractions. Trituration of the solid with EtOAc-MeOH afforded 4-Chloropyrrolo[3,2-d]Pyrimidine **(32e)** as an off-white solid, MP >150 °C (dec); ¹H NMR (DMSO-*d₆*) δ 12.43 (s, D₂O exchangeable, 1H), 8.61 (s, 1H), 7.97 (dd, *J =* 2.8, 2.8 Hz; D₂O exchange collapse to d, 1H), 6.72 (dd, *J =* 1.7, 3.5 Hz; D₂O exchange collapse to d, 1H). ¹³C-NMR (DMSO-*d*₆) 151.30, 149.58, 142.12, 134.83, 124.32, 102.70; IR (neat) 3128, 3078, 2979, 1621 cm⁻¹; MS (ES+) 154.01 (100%, M+1) and 156.01 (33%); Analysis calculated for C₆H₄N₃Cl: C, 46.93; H, 2.63; N, 27.36; Cl, 23.09; Found: C, 47.10; H, 2.79; N, 27.15; Cl, 22.93.

### Step 5: Preparation of 6-Methoxy-N-(benzyloxymethyl)-9-deazahypoxanthine (32f)

To the suspension of pre-washed NaH (20 g, 500 mmol, 60% oil dispersion, washed with hexanes 2 times) in anhydrous THF (1.0 L) cooled to 4 °C was added portion wise solid 4-Chloropyrrolo[3,2-d]Pyrimidine **(32e)** (61.4 g, 400 mmol) cautiously with stirring under N₂ in portions over 10-15 min such that H₂ gas evolution was controlled. After about an hour gas evolution ceased and benzyl chloromethyl ether (61 mL, 440 mmol) was added drop wise over 45 min at 4°C (additional gas evolution was observed). The resulting mixture was allowed to warm to ambient temperature and stir for 1 h. The reaction mixture was cooled to 4°C and quenched carefully with sodium meth oxide (93 mL, 5.4 M solution in methanol, 500 mmol). The mixture was allowed to warm to ambient temperature overnight and neutralized with glacial acetic acid (30 mL, 500 mmol) to pH 6. The mixture was concentrated and the residue triturated with water (2 x 400 mL). The aqueous layer was decanted and the residue dried in vacuum. The residue was taken in ethyl acetate (250 mL) and boiled to reflux and filtered through a fluted filter paper. The residue was boiled with ethyl acetate (2 x 100 mL) and filtered (the residue left behind is unwanted compound and doesn't move in TLC analysis 50% ethyl acetate in hexane). The filtrates were combined concentrated in vacuum to 250 mL and kept in refrigerator overnight. The brown crystals obtained was collected by filtration washed with ice cold ethyl acetate/hexane (2 x 100 mL) and dried in vacuum to furnish 6-Methoxy-*N*-(benzyloxymethyl)-9-deazahypoxanthine **(32f)** (46.64 g, 43%) as a orange brown solid, an analytical sample was prepared by recrystallization from ethyl acetate; MP 123 - 127 °C; ¹H NMR (DMSO-*d₆*) δ 8.44 (s, 1H), 7.86 (d, J= 3.1 Hz, 1 H), 7.31 - 7.22 (m, 5 H), 6.62 (d, J= 3.6 Hz, 1 H), 5.75 (s, 2 H), 4.49 (s, 2 H), 4.05 (s, 3 H); ¹³C-NMR (DMSO-*d₆*) 156.11, 151.59, 150.09, 137.82, 134.80, 128.53, 127.87, 127.77, 114.99, 103.08, 77.55, 69.95, 53.73; IR (KBr) 1602 cm⁻¹; MS (ES+) 269.97 (M+1); Analysis calculated for C₁₅H₁₅N₃O₂: C, 66.90; H, 5.61; N, 15.60; Found: C, 67.09; H, 5.60; N, 15.60.

### Step 6: Preparation of 6-Methoxy-N-(benzyloxymethyl)-9-bromo-9-deazahypoxanthine (4a)

To a solution of 6-Methoxy-*N*- (benzyloxymethyl)-9-deazahypoxanthine **(32f)** (59.81 g, 222 mmol) in dichloromethane (225 mL) under N₂ cooled to 4°C (homogenous reaction mixture) was added NBS (40.3 g, 224 mol) in portions over 30 min such that the reaction temperature remained below 15°C. The mixture was stirred at 0 °C for 15 mins and allowed to warm to room temperature over 15 mins (TLC analysis 50% ethyl acetate in hexane). The reaction mixture was vacuum filtered to remove insoluble succinimide. The filtrate was washed with water (2 x 250 mL) and brine (200 mL), dried (Na₂SO₄), filtered and concentrated in vacuum to furnish product as a light brown solid. The solid was dissolved by boiling in ethyl acetate (200 mL) and diluted with hexane (200 mL). The solution was boiled to reflux and filtered hot very quickly (to avoid solid crystallizing out). The filtrate was then boiled and added hexane in increments of 200 mL (total volume of hexane 1600 mL). The hot solution was decanted if needed to remove insoluble residues (The product is soluble in hot 10 % ethyl acetate in hexane). The hot filtrate was allowed to cool to room temperature and then kept in freezer overnight. The solid obtained was collected by filtration and washed with hexane and dried in vacuum at room temperature to furnish 6-methoxy-N-(benzyloxymethyl)-9-bromo-9-deazahypoxanthine **(4a)** (59.6 g, 77%), as a light yellow solid: MP 103 - 108° C; ¹H NMR (DMSO-*d₆*) δ 8.51 (s, 1H), 8.12 (s, 1H), 7.31 - 7.22 (m, 5H), 5.74 (s, 2H), 4.52 (s, 2H), 4.07 (s, 3H). ¹³C-NMR (DMSO-*d*₆) 156.19, 150.66, 148.14, 137.59, 133.45, 128.38, 127.80, 127.67, 115.02, 90.90, 77.79, 70.25, 54.07; IR (KBr) 3078, 1602, 1542 cm⁻¹; MS (ES+) 348.27 (100%), 350.28 (98%); Analysis Calculated for C₁₅H₁₄N₃O₂Br: C, 51.74; H, 4.05; N, 12.07; Found: C, 51.72; H, 4.04; N, 12.06.

### Example 2: (2S,3S,4S,5R)-2-(4-amino-5H-pyrrolo[3,2-d]pyrimidin-7-yl)-5-(hydroxymethyl)pyrrolidine-3,4-diol (31g)

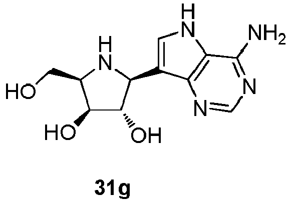

To a solution of (2S,3S,4S,5R)-tert-butyl 2-(4-amino-5H-pyrrolo[3,2-d]pyrimidin-7-yl)-3,4-dihydroxy-5-(hydroxymethyl)pyrrolidine-1-carboxylate **(31f)** (0.010 g, 0.027 mmol) dissolved in ethanol (4 mL) was added 6 N aqueous hydrogen chloride (0.059 mL, 0.356 mmol). The reaction mixture was stirred at room temperature for 30 min and concentrated in vacuum to dryness. The residue obtained was recrystallized from ethanol to afford (2S,3S,4S,5R)-2-(4-amino-5H-pyrrolo[3,2-d]pyrimidin-7-yl)-5-(hydroxymethyl)pyrrolidine-3,4-diol (31 g) (0.004 g, 55 %) as a off-white solid; ¹H NMR (300 MHz, DMSO-*d₆*) δ 14.43 (s, 1H, D₂O exchangeable), 13.02 (s, 1H, D₂O exchangeable), 10.11 (s, 1H, D₂O exchangeable), 9.03 (s, 3H, D₂O exchangeable), 8.63 (s, 1H), 7.99 (d, J = 3.2 Hz, 1H), 6.03 (s, 1H, D₂O exchangeable), 4.74 (s, 1H), 4.41 (dd, J = 3.9, 2.0 Hz, 1H), 4.16 (d, J = 2.8 Hz, 1H), 3.88 - 3.62 (m, 3H); ¹H NMR (300 MHz, DMSO-*d₆* D₂O) δ 8.62 (s, 1H), 7.98 (s, 1H), 4.74 (d, J = 3.8 Hz, 1H,), 4.39 (dd, J = 3.8, 2.1 Hz, 1H), 4.16 (t, J = 2.7 Hz, 1H), 3.88 - 3.67 (m, 3H).MS (ES+): MS (ES+) 266.07 (M+1), (ES-) 263.94 (M-1).

### Preparation of (2S,3S,4S,5R)-tert-butyl 2-(4-amino-5H-pyrrolo[3,2-d]pyrimidin-7-yl)-3,4-dihydroxy-5-(hydroxymethyl)pyrrolidine-1-carboxylate (31f)

### Step 1: Preparation of (2S,3S,4R,5R)-tert-butyl 2-(5-((benzyloxy)methyl)-4-methoxy-5H-pyrrolo[3,2-d]pyrimidin-7-yl)-3,4-dihydroxy-5-((trityloxy)methyl)pyrrolidine-1-carboxylate (4d)

To a solution of (2*S*,3*S*,4*R*,5*R*)-*tert*-Butyl 2-(5-(benzyloxymethyl)-4-methoxy-5*H-*pyrrolo[3,2-*d*]pyrimidin-7-yl)-3,4-dihydroxy-5-(hydroxymethyl)pyrrolidine-1-carboxylate **(4c)** (5 g, 10 mmol) in dichloromethane (85 mL) was added at room temperature trityl chloride (3.36 g, 12 mmol), DMAP (0.4 g, 3 mmol) and triethylamine (7 mL, 40 mmol). The reaction mixture was stirred for 16h and additional trityl chloride (2.87 g, 10.3 mmol) was added and continued stirring for 6h. The reaction mixture was washed with water (2 x 100 mL). The water layer was extracted with chloroform (100 mL) and the organic layers were combined. The organic layer was washed with saturated aqueous NaHCO3 (2 x 100 mL), dried, filtered and concentrated in vacuum to dryness. The residue obtained was purified by flash column chromatography (silica gel 200gm, eluting with 20 to 30% ethyl acetate in hexane) to furnish (2S,3S,4R,5R)-tert-butyl 2-(5-((benzyloxy)methyl)-4-methoxy-5H-pyrrolo[3,2-d]pyrimidin-7-yl)-3,4-dihydroxy-5-((trityloxy)methyl)pyrrolidine-1-carboxylate (4d) (5.0 g, 67%) as a white solid which was used as such for next step. Analytical data matches as reported in literature for compound **4d** (J. Med. Chem. 2003, 46, 3412-3423).

### Step 2: preparation of (2S,3S,4R,5R)-tert-butyl 2-(5-((benzyloxy)methyl)-4-methoxy-5H-pyrrolo[3,2-d]pyrimidin-7-yl)-4-hydroxy-3-((4-methoxybenzyl)oxy)-5-((trityloxy)methyl)pyrrolidine-1-carboxylate (6a)

To a solution of (2S,3S,4R,5R)-tert-butyl 2-(5-((benzyloxy)methyl)-4-methoxy-5H-pyrrolo[3,2-d]pyrimidin-7-yl)-3,4-dihydroxy-5-((trityloxy)methyl)pyrrolidine-1-carboxylate **(4d)** (5 g, 6.73 mmol) in benzene (100 mL) was added dibutyltin oxide (1.68 g, 6.75 mmol) and heated in a Dean-Stark apparatus for 1 h. To the reaction was added tetrabutylammonium bromide (2.16 g, 6.7 mmol), 4-methoxybenzyl chloride (2.08 g, 13.3 mmol) and continued heating for 18 h. The reaction mixture was concentrated in vacuum to dryness and the residue obtained was suspended in ethyl acetate. The suspension was diluted with hexanes and filtered through Celite and filter cake was washed with ethyl acetate. The filtrate was concentrated in vacuum to dryness to furnish crude residue which was purified by flash column chromatography (silica gel 100 gm, eluting with 0 to 50% ethyl acetate in hexane) to furnish (2S,3S,4R,SR)-tert-butyl 2-(5-((benzyloxy)methyl)-4-methoxy-5H-pyrrolo[3,2-d]pyrimidin-7-yl)-4-hydroxy-3-((4-methoxybenzyl)oxy)-5-((trityloxy)methyl)pyrrolidine-1-carboxylate **(6a)** (3 g, 52%), followed by (2S,3S,4R,5R)-tert-butyl 2-(5-((benzyloxy)methyl)-4-methoxy-5H-pyrrolo[3,2-d]pyrimidin-7-yl)-3-hydroxy-4-((4-methoxybenzyl)oxy)-5-((trityloxy)methyl)pyrrolidine-1-carboxylate **(6b)** (0.793 g, 14%); MS (ES+) 864 (M+1). Analytical data matches as reported in literature for compound **6a** and **6d** (J. Med. Chem. 2003, 46, 3412-3423.

### Step 3: Preparation of (2S,3S,4S,5R)-tert-butyl 2-(5-((benzyloxy)methyl)-4-methoxy-5H-pyrrolo[3,2-d]pyrimidin-7-yl)-4-hydroxy-3-((4-methoxybenzyl)oxy)-5-((trityloxy)methyl)pyrrolidine-1-carboxylate (31a)

A solution of DMSO (2.5 mL, 35 mmol) in DCM (60 mL) was cooled down to -70 °C, and trifluoroacetic anhydride (2.5 mL, 18 mmol) was added to the stirred solution. After stirring for 10 min, a solution of (2S,3S,4R,5R)-tert-butyl 2-(5-((benzyloxy)methyl)-4-methoxy-5H-pyrrolo[3,2-d]pyrimidin-7-yl)-4-hydroxy-3-((4-methoxybenzyl)oxy)-5-((trityloxy)methyl)pyrrolidine-1-carboxylate **(6a)** (3.1 g, 2.7 mmol) in DCM (50 mL) was added followed by triethylamine (3.7 mL, 50 mmol). T reaction mixture was stirred for 15 min, allowed to warm up to room temperature and washed with water, dried, and then concentrated to give the intermediate ketone, which was directly applied to the next step without purification. The above ketone was dissolved in methanol (100 mL) and cooled to 0 °C. Sodium borohydride (1.2 g, 30 mmol) was added to this solution and the reaction mixture was stirred for 30 min. The reaction mixture was concentrated and diluted with ethyl acetate and then washed with water, dried, and concentrated to drynesss. The residue was purified by chromatography using hexanes and ethyl acetate (2:1) to afford (2S,3S,4S,5R)-tert-butyl 2-(5-((benzyloxy)methyl)-4-methoxy-5H-pyrrolo[3,2-d]pyrimidin-7-yl)-4-hydroxy-3-((4-methoxybenzyl)oxy)-5-((trityloxy)methyl)pyrrolidine-1-carboxylate **(31a)** (2.45 g, 79%) as a syrup; ¹H NMR: (DMSO-*d*₆) δ 8.32 (s, 1H), 7.06-7.44 (m, 23H), 6.73 (d, *J* = 9.0Hz, 2H), 5.60 (d, *J =* 6.0Hz, 1H), 5.58 (s, 1H), 5.25 (br, 1H), 4.93 (d, *J* = 6.0Hz, 1H), 4.45 (m, 2H), 4.31 (m, 2H), 4.17 (m, 1H), 4.03 (s, 3H), 3.67 (s, 3H), 3.56 (dd, 1H), 3.39 (m, 1H), 1.15-1.20 (m, 9H). MS (ES+) 864 (M+1).

### Step 4: Preparation of 7-((2S,3S,4S,5R)-3,4-Dihydroxy-5-(hydroxymethyl)pyrrolidin-2-yl)-3H-pyrrolo[3,2-d]pyrimidin-4(5H)-one hydrochloride (31b)

(2S,3S,4S,5R)-tert-butyl 2-(5-((benzyloxy)methyl)-4-methoxy-5H-pyrrolo[3,2-d]pyrimidin-7-yl)-4-hydroxy-3-((4-methoxybenzyl)oxy)-5-((trityloxy)methyl)pyrrolidine-1-carboxylate (**31a**) (2.3 g, 2.67 mmol) was suspended in conc. HCl (20 mL). The mixture was heated to reflux and stirred for 1 hour. The solution was then concentrated to dryness, redissolved in water, and decolorized with activated carbon. After filtering over diatomaceous earth, the product was crystallized by the addition of ethanol to give 7-((2*S*,3*S*,4*S*,5*R*)-3,4-Dihydroxy-5-(hydroxymethyl)pyrrolidin-2-yl)-3*H*-pyrrolo[3,2-*d*]pyrimidin-4(5*H*)-one hydrochloride **(31b)** (0.62 g, 76%) as white solid; ¹H NMR: (D₂O) δ 7.97 (s, 1H), 7.64 (s, 1H), 4.74 (d, *J* = 6.0Hz, 1H), 4.62 (dd, *J* = 6.0, 3.0Hz, 1H), 4.34 (dd, *J* = 6.0, 3.0Hz, 1H), 3.85-3.95 (m, 3H). ¹³C NMR: δ 155.03, 143.11, 142.68, 129.38, 117.76, 108.27, 79.44, 74.58, 62.79, 58.94, 57.06; MS (ES+) 267.12 (M+1).

### Step 5: Preparation of (2R,3S,4S,5S)-tert-butyl 3,4-dihydroxy-2-(hydroxymethyl)-5-(4-oxo-4,5-dihydro-3H-pyrrolo[3,2-d]pyrimidin-7-yl)pyrrolidine-1-carboxylate (31c)

To a suspension of 7-((2*S*,3*S*,4*S*,5*R*)-3,4-Dihydroxy-5-(hydroxymethyl)pyrrolidin-2-yl)-3*H-*pyrrolo[3,2-*d*]pyrimidin-4(5*H*)-one hydrochloride (**31b**) (0.56 g, 1.651 mmol) in a Methanol (5 mL), Water (5.00 mL) was added triethylamine (0.690 mL, 4.95 mmol) at room temperature followed by di-tert-butyl dicarbonate (1.106 g, 5.07 mmol). The reaction mixture was stirred at room temperature overnight. The reaction mixture was concentrated in vacuum and the residue obtained was purified by flash column chromatography (silica gel 12 g, eluting with 0-100% (9:1) ethyl acetate/methanol in hexane) to furnish (2R,3S,4S,5S)-tert-butyl 3,4-dihydroxy-2-(hydroxymethyl)-5-(4-oxo-4,5-dihydro-3H-pyrrolo[3,2-d]pyrimidin-7-yl)pyrrolidine-1-carboxylate (**31c**) (0.411 g, 1.122 mmol, 67.9 % yield) as a white solid; ¹H NMR (300 MHz, DMSO-*d₆*, 300K) δ 11.50 (s, 2H), 7.73 (s, 1H), 7.16 (s, 1H), 4.86 (s, 3H), 4.54 (d, *J* = 5.8 Hz, 1H), 4.23 (t, *J* = 6.2 Hz, 1H), 4.04 (t, *J* = 6.7 Hz, 1H), 3.93 - 3.82 (m, 2H), 3.74 (d, *J* = 7.2 Hz, 1H), 1.18 (s, 9H); ¹H NMR (300 MHz, DMSO-*d₆*, 370K) δ 11.50 (s, 2H), 7.73 (s, 1H), 7.16 (s, 1H), 4.86 (s, 3H), 4.54 (d, *J* = 5.8 Hz, 1H), 4.23 (t, *J* = 6.2 Hz, 1H), 4.04 (t, *J* = 6.7 Hz, 1H), 3.93 - 3.82 (m, 2H), 3.74 (d, *J =* 7.2 Hz, 1H), 1.18 (s, 9H); MS (ES+) 754.9 (2M+Na); (ES-) 365.3 (M-1).

### Step 6: Preparation of (2R,3S,4S,5S)-2-(acetoxymethyl)-1-(tert-butoxycarbonyl)-5-(4-oxo-4,5-dihydro-3H-pyrrolo[3,2-d]pyrimidin-7-yl)pyrrolidine-3,4-diyl diacetate (31d)

To a suspension of (2R,3S,4S,5S)-tert-butyl 3,4-dihydroxy-2-(hydroxymethyl)-5-(4-oxo-4,5-dihydro-3H-pyrrolo[3,2-d]pyrimidin-7-yl)pyrrolidine-1-carboxylate (**31c**) (0.4 g, 1.092 mmol) in pyridine (2 mL, 24.73 mmol) and dichloromethane (2.5 mL) was added N,N-dimethylpyridin-4-amine (0.030 g, 0.247 mmol) and acetic anhydride (0.758 mL, 8.04 mmol) at room temperature. The reaction mixture was concentrated in vacuum and the residue obtained was purified by flash column chromatography [silica gel, 0-100% (9:1)ethyl acetate/methanol in hexane] to furnish (2R,3S,4S,5S)-2-(acetoxymethyl)-1-(tert-butoxycarbonyl)-5-(4-oxo-4,5-dihydro-3H-pyrrolo[3,2-d]pyrimidin-7-yl)pyrrolidine-3,4-diyl diacetate (**31d**) (0.462 g, 0.938 mmol, 86 % yield) as a white solid; ¹H NMR (300 MHz, DMSO-*d₆*) δ 12.02 (s, 1H), 11.91 (s, 1H), 7.81 (s, 1H), 7.22 (s, 1H), 5.93 - 5.81 (m, 1H), 5.37 (t, *J* = 6.7 Hz, 1H), 4.88 (d, *J* = 5.7 Hz, 1H), 4.46 - 4.31 (m, 3H), 1.99 (s, 3H), 1.95 (s, 3H), 1.91 (s, 3H), 1.24 (s, 9H); MS (ES+) 493 (M+1), 514.9 (M+Na); (ES-) 983 (2M-1).

### Step 7: Preparation of (2R,3S,4S,5S)-2-(acetoxymethyl)-1-(tert-butoxycarbonyl)-5-(4-chloro-5H-pyrrolo[3,2-d]pyrimidin-7-yl)pyrrolidine-3,4-diyl diacetate (31e)

To a solution of (2R,3S,4S,5S)-2-(acetoxymethyl)-1-(tert-butoxycarbonyl)-5-(4-oxo-4,5-dihydro-3H-pyrrolo[3,2-d]pyrimidin-7-yl)pyrrolidine-3,4-diyl diacetate (**31d**) (460 mg, 0.93 mmol, 1.0 equiv.) in acetonitrile (10 mL) was added benzyltriethylammonium chloride (424 mg, 1.86 mmol, 2.0 equiv.), dimethylaniline (0.18 mL, 1.4 mmol, 1.5 equiv.), followed by POCl₃ (0.51 mL, 5.6 mmol, 6 equiv.) at room temperature. A clear light yellow colored solution was obtained. The reaction mixture was slowly heated up to 80 °C and held at this temperature for 10 minutes. TLC in 9:1 chloroform: methanol shows that the reaction is >98% completed. The reaction mixture was allowed to slowly cool down to 50.0 °C and added silica gel (1 gm). The reaction mixture was concentrated under vacuum to dryness and slurry obtained was purified by flash column chromatography (silica gel 12 g, eluting with 0-100% (9:1) ethyl acetate/methanol in hexane) to furnish (2R,3S,4S,5S)-2-(acetoxymethyl)-1-(tert-butoxycarbonyl)-5-(4-chloro-5H-pyrrolo[3,2-d]pyrimidin-7-yl)pyrrolidine-3,4-diyl diacetate **(31e)** (0.2 g, 42%) as a white solid; ¹H NMR (300 MHz, DMSO-*d₆*) δ 12.42 (s, 1H), 8.64 (s, 1H), 7.84 (s, 1H), 5.93 (t, *J* = 6.6 Hz, 1H), 5.44 (t, *J* = 7.1 Hz, 1H), 5.01 (d, *J* = 6.0 Hz, 1H), 4.48 - 4.35 (m, 3H), 1.99 (s, 3H), 1.95 (s, 3H), 1.92 (s, 3H), 1.22 (s, 9H); MS (ES+) 511.96, (ES-) 508.773.

### Step 8: Preparation of (2S,3S,4S,5R)-tert-butyl 2-(4-amino-5H-pyrrolo[3,2-d]pyrimidin-7-yl)-3,4-dihydroxy-5-(hydroxymethyl)pyrrolidine-1-carboxylate (31f)

Ammonia (30 mL, 1386 mmol) was condensed into Ethanol (30 mL) at -50°C and added to a solution of (2R,3S,4S,5S)-2-(acetoxymethyl)-1-(tert-butoxycarbonyl)-5-(4-chloro-5H-pyrrolo[3,2-d]pyrimidin-7-yl)pyrrolidine-3,4-diyl diacetate (**31e**) (0.185 g, 0.362 mmol) in Ethanol (10.0 mL) in a autoclave and heated at 80°C for 24 h. The pressure rises to 200 psi. The reaction was cooled to -50°C and concentrated in vacuum to dryness. The residue obtained was purified twice by flash column chromatography (silica gel 4g, eluting with 0-100% methanol in chloroform) to furnish (2S,3S,4S,5R)-tert-butyl 2-(4-amino-5H-pyrrolo[3,2-d]pyrimidin-7-yl)-3,4-dihydroxy-5-(hydroxymethyl)pyrrolidine-1-carboxylate (**31f**) (0.01 g, 0.027 mmol, 7.56 % yield) as a white solid; ¹H NMR (300 MHz, DMSO-*d₆*) δ 10.79 (s, 1H), 8.00 (d, J = 17.7 Hz, 1H), 7.39 (s, 1H), 6.85 (s, 2H), 5.25 (d, J = 4.4 Hz, 1H), 5.21 - 5.12 (m, 1H), 4.37 - 4.23 (m, 2H), 4.03 - 3.86 (m, 2H), 3.75 (dd, J = 9.6, 7.4 Hz, 1H), 3.65 (d, J = 10.6 Hz, 1H), 0.95 (bs, 9H); MS (ES+) 366.0 (M+1); (ES-) 363.8 (M-1).

### Example 3: 2-amino-5-((2S,3S,4R,5R)-3,4-dihydroxy-5-(hydroxymethyl)pyrrolidin-2-yl)pyrimidin-4(3H)-one dihydrochloride (13i)

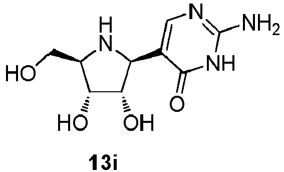

(3aS,4S,6R,6aR)-tert-butyl 4-(2-amino-6-oxo-1,6-dihydropyrimidin-5-yl)-6-(hydroxymethyl)-2,2-dimethyldihydro-3aH-[1,3 ]dioxolo[4,5-c]pyrrole-5(4H)-carboxylate (**13h**) (0.120 g, 0.314 mmol) was suspended in the 3.5 M aqueous HCl (0.897 mL, 3.14 mmol). The reaction mixture was stirred at room temperature for 3h and then concentrated in vacuum to dryness. The residue was crystallized from ethanol/water to furnish 2-amino-5-((2S,3S,4R,5R)-3,4-dihydroxy-5-(hydroxymethyl)pyrrolidin-2-yl)pyrimidin-4(3H)-one (**13i**) (0.05 g, 0.159 mmol, 50.6 % yield) as a white solid; ¹H NMR (300 MHz, DMSO-*d₆*) δ 9.97 (s, 1H), 8.22 (s, 1H), 7.95 (s, 2H), 7.81 (s, 1H), 5.30 (bs, 3H), 4.29 (dd, J = 8.4, 4.6 Hz, 1H), 4.16 (dd, J = 15.2, 7.6 Hz, 1H), 4.10 - 4.04 (m, 1H), 3.65 (d, J = 5.3 Hz, 2H), 3.48 - 3.40 (m, 1H); ¹H NMR (300 MHz, DMSO-*d₆*/D₂O) δ 7.80 (s, 1H), 4.30 (dd, J = 8.4, 4.7 Hz, 1H), 4.18 (d, J = 8.5 Hz, 1H), 4.11 - 4.04 (t, J = 4 Hz, 1H), 3.46 (d, J = 3.3 Hz, 1H), 3.49 - 3.44 (m, 1H); MS (ES+) 243.1; (ES-) 240.9 (M-1); Analysis, Calculated for: C₉H₁₄N₄O₄.2HCl.0.5H₂O: C, 33.35; H, 5.29; Cl, 21.87; N, 17.28; Found: C, 33.37; H, 5.32; Cl, 21.88; N, 17.22.

### Preparation of (3aS,4S,6R,6aR)-tert-butyl 4-(2-amino-6-oxo-1,6-dihydropyrimidin-5-yl)-6-(hydroxymethyl)-2,2-dimethyldihydro-3aH-[1,3]dioxolo[4,5-c]pyrrole-5(4H)-carboxylate (13h)

### Step 1: Preparation of tert-butyl 2-((3aS,4S,6R,6aR)-6-(((tertbutyldimethylsilyl)oxy)methyl)-2,2-dimethyltetrahydro-3aH-[1,3]dioxolo[4,5-c]pyrrol-4-yl)acetate (13a)

Tert-butyl acetate (54.0 mL, 400 mmol) was added dropwise to a stirred solution of LDA (200 mL, 400 mmol) in THF such that the reaction temperature was maintained below -65 °C. The resulting solution was left to stir for 20 min after which time a solution of freshly prepared (3aR,4R,6aS)-4-(((tert-butyldimethylsilyl)oxy)methyl)-2,2-dimethyl-4,6a-dihydro-3aH-[1,3]dioxolo[4,5-c]pyrrole (**1k**) (22.84 g, 80 mmol) in toluene was added at such a rate that the reaction temperature was maintained below -65 °C, and the resulting solution was then allowed to warm to room temperature over a period of 2 h. The reaction was quenched by the addition of saturated aqueous ammonium chloride (200 mL), and the organic layer was separated washed with brine (100 mL), dried filtered and concentrated in vacuum. This gave 58 gm of crude product. Crude was purified by flash column chromatography eluting with 0-50% ethyl acetate in hexane to furnish tert-butyl 2-((3aS,4S,6R,6aR)-6-(((tertbutyldimethylsilyl)oxy)methyl)-2,2-dimethyltetrahydro-3aH-[1,3]dioxolo[4,5-c]pyrrol-4-yl)acetate (**13a**) (22.916 g, 57.1 mmol, 71.3 % yield) as a yellow oil; ¹H NMR (300 MHz, CDCl₃) δ 4.32 (dd, J = 7.0, 4.6 Hz, 1H), 4.18 (dd, J = 7.0, 5.2 Hz, 1H), 3.71 (dd, J = 10.2, 4.1 Hz, 1H), 3.57 (dd, J = 10.2, 5.8 Hz, 1H), 3.32 (dt, J = 8.3, 5.0 Hz, 1H), 3.15 (dd, J = 10.0, 4.4 Hz, 1H), 2.55 (dd, J =15.9, 4.8 Hz, 1H), 2.35 (dd, J = 16.0, 8.3 Hz, 1H), 1.45 (s, 3H), 1.38 (s, 9H), 1.25 (s, 3H), 0.83 (s, 9H), -0.00 (s, 6H); ¹H NMR (300 MHz, DMSO) δ 4.26 (dd, *J =* 6.7, 3.8 Hz, 1H), 4.16 (dd, *J =* 6.7, 4.4 Hz, 1H), 3.53 - 3.44 (m, 2H), 3.20 (dt, *J =* 8.0, 5.5 Hz, 1H), 3.02 (dd, *J =* 9.5, 5.6 Hz, 1H), 2.64 (s, 1H), 2.38 (dd, *J =* 15.1, 5.7 Hz, 1H), 2.20 (dd, *J =* 15.1, 8.1 Hz, 1H), 1.35 (s, 12H), 1.18 (s, 3H), 0.83 (s, 9H), -0.00 (s, 6H) MS (ES+) 402.0 (M+1); 803.0 (2M+1).

### Step 2: Preparation of (3aS,6R,6aR)-tert-butyl 4-(2-(tert-butoxy)-2-oxoethyl)-6-(hydroxymethyl)-2,2-dimethyldihydro-3aH-[1,3] dioxolo [4,5-c]pyrrole-5(4H)-carboxylate (13c)

Di-tertbutyl dicarbonate (19.42 mL, 84 mmol) was added, portionwise, to a stirred solution of-butyl 2-((3aS,6R,6aR)-6-((tert-butyldimethylsilyloxy)methyl)-2,2-dimethyltetrahydro-3aH-[1,3]dioxolo[4,5-c]pyrrol-4-yl)acetate (BCX-6491, 22.4 g, 55.8 mmol) in methanol (100 mL) at room temperature overnight. The reaction was monitored by TLC and on completion n-tetrabutylammonium fluoride (55.8 mL, 55.8 mmol) 1 M in THF (1.0 M) was added dropwise and the resulting solution left to stir for 14 h.TLC shows reaction was only 50% completed additional tetrabutylammonium fluoride trihydrate (17.60 g, 55.8 mmol) was added and stirred for additional 5 h. TLC shows not much change and new lower spot being formed. The reaction was concentrated in vacuum to remove methanol and THF. The residue obtained was taken in water (200 mL) and extracted twice with ethyl acetate (100 mL). The organic layers were combined washed with water, dried, filtered and concentrated in vacuum. The residue was purified by flash column chromatography (silica gel 600 gm, eluting with 0-100% ethyl acetate in hexane) to furnish (3aS,4S,6R,6aR)-tert-butyl 4-(2-(tert-butoxy)-2-oxoethyl)-6-(((tert-butyldimethylsilyl)oxy)methyl)-2,2-dimethyldihydro-3aH-[1,3]dioxolo[4,5-c]pyrrole-5(4H)-carboxylate (**13b**) (8.73, 17.40 mmol, 31.2 % yield) as a colorless oil. Further elution gave (3aS,6R,6aR)-tert-Butyl 4-(2-tert-butoxy-2-oxoethyl)-6-(hydroxymethyl)-2,2-dimethyldihydro-3aH-[1,3]dioxolo [4,5-c]pyrrole-5(4H)-carboxylate (**13c**) (9.356 g, 24.15 mmol, 43.3 % yield) as colorless oil; ¹H NMR (300 MHz, DMSO-*d₆*) δ 5.04 (dd, *J =* 9.4, 4.6 Hz, 1H), 4.65 (d, *J =* 5.8 Hz, 1H), 4.44 (t, *J =* 6.1 Hz, 1H), 4.06 - 3.96 (m, 1H), 3.87 - 3.71 (m, 1H), 3.54 - 3.44 (m, 1H), 3.34 - 3.28 (m, 1H), 2.59 - 2.52 (m, 1H), 2.48 - 2.41 (m, 1H), 1.40 (s, 9H), 1.39 (s, 9H), 1.34 (d, *J* = 4.0 Hz, 3H), 1.24 (s, 3H); MS (ES+) 388.095 (M+1), 410.05 (M+Na); (ES-) 386.065 (M-1).

### Step 3: Preparation of (3aR,4R,6S,6aS)-tert-butyl 4-((benzyloxy)methyl)-6-(2-(tert-butoxy)-2-oxoethyl)-2,2-dimethyldihydro-3aH-[1,3]dioxolo[4,5-c]pyrrole-5(4H)-carboxylate (13d)

To a solution of (3aS,6R,6aR)-*tert*-Butyl 4-(2-tert-butoxy-2-oxoethyl)-6-(hydroxymethyl)-2,2-dimethyldihydro-3aH-[1,3]dioxolo[4,5-c]pyrrole-5(4H)-carboxylate (**13c**) (9.3 g, 24.00 mmol) in DMF (25 mL) and benzyl bromide (11.48 mL, 96 mmol) at 0 °C was added sodium hydride 60% in mineral oil (1.248 g, 31.2 mmol) portionwise at 0 °C. The reaction mixture was allowed to warm to room temperature and stirred for 2 h. TLC (20% E/H) showed reaction complete. The mixture was diluted with toluene (300 mL), washed with water, brine (150 mL), dried over MgSO₄ and concentrated to dryness to give crude mixture (25 g) as light yellow oil, which contained all the left over BnBr. The mixture was purified by 120g flash column chromatography (silica gel 120 g, eluting with 0-50% ethyl acetate in hexane) to afford (3aR,4R,6S,6aS)-tert-butyl 4-((benzyloxy)methyl)-6-(2-(tert-butoxy)-2-oxoethyl)-2,2-dimethyldihydro-3aH-[1,3]dioxolo[4,5-c]pyrrole-5(4H)-carboxylate (**13d**) (11.296 g, 23.65 mmol, 99 % yield) as colorless oil; ¹H NMR (300 MHz, DMSO-*d₆*) δ 7.42 - 7.23 (m, 5H), 4.70 (m, 1H), 4.50 (m, 3H), 4.08 - 3.90 (m, 2H), 3.56 - 3.40 (m, 2H), 2.57 - 2.38 (m, 2H), 1.49 -1.29 (m, 21H), 1.25 (s, 3H); MS (ES+) 478.0 (M+1), 500.0 (M+Na).

### Step 4: preparation of (3aR,4R,6S,6aS)-tert-butyl 4-((benzyloxy)methyl)-6-((Z)-3-(tert-butoxy)-1-hydroxy-3-oxoprop-1-en-2-yl)-2,2-dimethyldihydro-3aH-[1,3]dioxolo[4,5-c]pyrrole-5(4H)-carboxylate (13e)

To a solution of (3aR,4R,6S,6aS)-tert-butyl 4-((benzyloxy)methyl)-6-(2-(tert-butoxy)-2-oxoethyl)-2,2-dimethyldihydro-3aH-[1,3]dioxolo[4,5-c]pyrrole-5(4H)-carboxylate (**13d**) (3.79 g, 7.94 mmol) in DMF (10 mL) cooled in ice-water bath was added sodium hydride (60% in mineral oil, 0.984 g, 24.6 mmol) portion wise over a period of 20 min. The reaction mixture was stirred at room temperature for 1 h and then again cooled in ice-water bath. To the cold solution was treated with ethyl formate (1.923 mL, 23.81 mmol) slowly to control evolution of gases. The reaction was stirred at room temperature overnight. TLC (E/H, 25%) showed no SM. The product (3aR,4R,6S,6aS)-tert-butyl 4-((benzyloxy)methyl)-6-((Z)-3-(tert-butoxy)-1-hydroxy-3-oxoprop-1-en-2-yl)-2,2-dimethyldihydro-3aH-[1,3]dioxolo[4,5-c]pyrrole-5(4H)-carboxylate (**13e**) was used as such for the next step.

### Step 5: Preparation of (3aR,4R,6S,6aS)-tert-butyl 4-((benzyloxy)methyl)-6-((Z)-3-(tert-butoxy)-1-methoxy-3-oxoprop-1-en-2-yl)-2,2-dimethyldihydro-3aH-[1,3]dioxolo[4,5-c]pyrrole-5(4H)-carboxylate (13f)

The crude product (3aR,4R,6S,6aS)-tert-butyl 4-((benzyloxy)methyl)-6-((Z)-3-(tert-butoxy)-1-hydroxy-3-oxoprop-1-en-2-yl)-2,2-dimethyldihydro-3aH-[1,3]dioxolo[4,5-c]pyrrole-5(4H)-carboxylate (**13e**) from above step-4 was cooled in ice-water bath and iodo methane (1.48 mL, 23.73 mmol) was added over a period of 10 min and the resulting mixture was stirred at room temperature for 4 h. The reaction mixture was quenched carefully with water (50 mL) and extracted with ethyl acetate (3 x 50 mL). The combined organic were washed with water, brine, dried over MgSO₄ and concentrated to dryness to give crude (3aR,4R,6S,6aS)-tert-butyl 4-((benzyloxy)methyl)-6-((Z)-3-(tert-butoxy)-1-methoxy-3-oxoprop-1-en-2-yl)-2,2-dimethyldihydro-3aH-[1,3]dioxolo[4,5-c]pyrrole-5(4H)-carboxylate (**13f**) (3.76 g, 7.24 mmol, 91 % yield) as dark black oil, which was used as such for the next step.

### Step 6 Preparation of (3aS,4S,6R,6aR)-tert-butyl 4-(2-amino-6-oxo-1,6-dihydropyrimidin-5-yl)-6-((benzyloxy)methyl)-2,2-dimethyldihydro-3aH-[1,3]dioxolo[4,5-c]pyrrole-5(4H)-carboxylate (13g)

Guanidine hydrochloride (0.594 g, 6.16 mmol) was added to freshly prepared NaOEt solution in Ethanol (prepared by dissolving 0.151 g of Na in 30 mL of absolute Ethanol) and stirred for 10 min at room temperature. Then reaction mixture was filtered through Celite and the filtrate was added to (3aR,4R,6S,6aS)-tert-butyl 4-((benzyloxy)methyl)-6-((Z)-3-(tert-butoxy)-1-methoxy-3-oxoprop-1-en-2-yl)-2,2-dimethyldihydro-3aH-[1,3]dioxolo[4,5-c]pyrrole-5(4H)-carboxylate (**13f**) (1.6 g, 3.08 mmol). The reaction mixture was heated at reflux overnight and concentrated in vacuum to dryness. The residue obtained was purified by flash column chromatography [silica gel 25 g, eluting with (ethyl acetate/methanol 9:1) in hexane, 0-100%] to furnish (3aS,4S,6R,6aR)-tert-butyl 4-(2-amino-6-oxo-1,6-dihydropyrimidin-5-yl)-6-((benzyloxy)methyl)-2,2-dimethyldihydro-3aH-[1,3]dioxolo[4,5-c]pyrrole-5(4H)-carboxylate (**13g**) (0.426 g, 29.3 % yield) as a oil; ¹H NMR (300 MHz, DMSO-*d₆*) δ 10.93 (bs, 1H, exchangeable), 7.32 (m, 6H), 6.41 (bs, 2H, exchangeable), 4.69 (dd, *J =* 12.0, 6.5 Hz, 2H), 4.48 (m,3H), 4.05 - 3.95 (m, 1H), 3.65 - 3.44 (m, 2H), 1.39 (s, 3H), 1.32 (s, 9H), 1.24 (s, 3H); MS (ES+) 473.0 (M+1), 495.0 (M+Na); (ES-) 470.9 (M-1).

### Step 7: Preparation of (3aS,4S,6R,6aR)-tert-butyl 4-(2-amino-6-oxo-1,6-dihydropyrimidin-5-yl)-6-(hydroxymethyl)-2,2-dimethyldihydro-3aH-[1,3]dioxolo[4,5-c]pyrrole-5(4H)-carboxylate (13h)

To a solution of (3aS,4S,6R,6aR)-tert-butyl 4-(2-amino-6-oxo-1,6-dihydropyrimidin-5-yl)-6-((benzyloxy)methyl)-2,2-dimethyldihydro-3aH-[1,3]dioxolo[4,5-c]pyrrole-5(4H)-carboxylate (**13g**) (0.45 g, 0.952 mmol) dissolved in MeOH (20 mL) was added Pd(OH)₂ (20% on Carbon, 0.223 g). The slurry was hydrogenated at 50 psi for 36 hr. (TLC CMA80, showed reaction was not complete). Additional Pd(OH)₂ (200 mg) was added and hydrogenated at 50 psi overnight. The catalyst was removed by filtration through a Celite pad. The filtrate was concentrated in vacuum to dryness and the residue obtained was purified by flash column chromatography (silica gel 4 g, eluting with CMA80 in CHCl₃, 0-100%) to give (3aS,6R,6aR)-tert-butyl (3aS,4S,6R,6aR)-tert-butyl 4-(2-amino-6-oxo-1,6-dihydropyrimidin-5-yl)-6-(hydroxymethyl)-2,2-dimethyldihydro-3aH-[1,3]dioxolo[4,5-c]pyrrole-5(4H)-carboxylate (**13h**) (156 mg, 42.8 % yield) as a foam; ¹H NMR (300 MHz, DMSO-*d₆*) δ 10.98 (s, 1H), 7.42 (s, 1H), 6.51 (s, 2H), 4.96 (t, *J =* 5.5 Hz, 1H), 4.76 - 4.58 (m, 2H), 4.56 - 4.40 (m, 1H), 3.83 (s, 1H), 3.62 - 3.47 (m, 2H), 1.40 (s, 3H), 1.32 (s, 9H), 1.24 (s, 3H);MS (ES+) 383.1 (M+1); (ES-) 380.9 (M-1).

### Example 4: 5-amino-3-((2S,3S,4R,5R)-3,4-dihydroxy-5-(hydroxymethyl)pyrrolidin-2-yl)-1H-pyrrolo[2,3-c]pyridin-7(6H)-one dihydrochloride (2j)

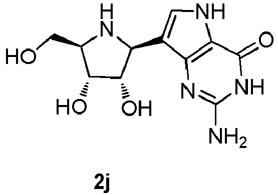

To a solution of (3aS,4S,6R,6aR)-tert-butyl 4-(2-amino-4-oxo-4,5-dihydro-3H-pyrrolo[3,2-d]pyrimidin-7-yl)-6-(((tert-butyldimethylsilyl)oxy)methyl)-2,2-dimethyldihydro-3aH-[1,3]dioxolo[4,5-c]pyrrole-5(4H)-carboxylate (**2i**) (0.1 g, 0.187 mmol) in methanol (2 mL) was added concentrated HCl (0.8 mL, 9.6 mmol) and heated at 50 °C for 1 h. The reaction mixture was concentrated in vacuum to dryness and the solid obtained was triturated with ethanol and collected by filtration to afford on drying in vacuum 5-amino-3-((2S,3S,4R,5R)-3,4-dihydroxy-5-(hydroxymethyl)pyrrolidin-2-yl)-1H-pyrrolo[2,3-c]pyridin-7(6H)-one (**2j**) (0.04 g, 76%) as a white solid; ¹H NMR (300 MHz, D₂O) δ 7.40 (s, 1H), 4.35 (dd, *J=* 9.2, 5.0 Hz, 1H), 4.10 (dd, *J =* 4.9, 3.2 Hz, 1H), 3.64 (d, *J =* 4.6 Hz, 2H), 3.56 (q, *J =* 4.2 Hz, 1H); MS (ES+) 282.47 (M+1); (ES-) 280.45 (M-1)

### Preparation of (3aS,4S,6R,6aR)-tert-butyl 4-(2-amino-4-oxo-4,5-dihydro-3H-pyrrolo[3,2-d]pyrimidin-7-yl)-6-(((tert-butyldimethylsilyl)oxy)methyl)-2,2-dimethyldihydro-3aH-[1,3]dioxolo[4,5-c]pyrrole-5(4H)-carboxylate (2i)

### Step 1: Preparation of (3aR,4S,6aS)-4-((tert-butyldimethylsilyl)methyl)-2,2-dimethyl-4,6a-dihydro-3aH-[1,3]dioxolo[4,5-c]pyrrole 5-oxide (11)

To a stirred solution of (3aR,4S,6aS)-4-((tert-butyldimethylsilyl)methyl)-2,2-dimethyltetrahydro-3aH-[1,3]dioxolo[4,5-c]pyrrole (**1j**) (33.0 g, 122 mmol) and Selenium Dioxide (0.634 g, 5.71 mmol) in acetone (100 mL) at 0° C was added slowly hydrogen peroxide (20 mL, 228 mmol) and stirred at 0° C for 5 h. The reaction mixture was diluted with chloroform (250 mL) and washed with water (3 x 50 mL). The organic layers were combined dried, filtered and concentrated in vacuum. The compound solidified on standing at room temperature overnight.
Add hexane (100 mL) and filter solid this gave on filtering and drying (3aS,4S,6R,6aR)-tert-butyl (3aR,4S,6aS)-4-((tert-butyldimethylsilyl)methyl)-2,2-dimethyl-4,6a-dihydro-3aH-[1,3]dioxolo[4,5-c]pyrrole 5-oxide (**11**) (11.361 gm, 33%). The filtrate was concentrated in vacuum to dryness and triturated with hexane and solid was collected by filtration to furnish a second crop (1.772 gm, 5%) of (3aR,4S,6aS)-4-((tert-butyldimethylsilyl)methyl)-2,2-dimethyl-4,6a-dihydro-3aH-[1,3]dioxolo[4,5-c]pyrrole 5-oxide (**11**). The filtrate was further dried and triturated in hexane and solid was collected by filtration to furnish (3aR,4S,6aS)-4-((tert-butyldimethylsilyl)methyl)-2,2-dimethyl-4,6a-dihydro-3aH-[1,3]dioxolo[4,5-c]pyrrole 5-oxide (**11**) as a third crop (1.709 gm, 5%). The filtrate was purified on silica gel column eluting with 0-50% ethyl acetate in hexane to furnish (3aR,4S,6aS)-4-((tertbutyldimethylsilyl)methyl)-2,2-dimethyl-4,6a-dihydro-3aH-[1,3]dioxolo[4,5-c]pyrrole 5-oxide (**11**) (6.71 g, 23.51 mmol, 20.58 % yield) as a light yellow solid, overall yield (66 %); ¹H NMR (300 MHz, DMSO-*d₆*) δ 7.06 (d, *J* = 1.4 Hz, 1H), 5.03 (dt, *J* = 6.2, 1.5 Hz, 1H), 4.75 (d, *J* = 6.2 Hz, 1H), 4.03 - 3.79 (m, 3H), 1.32 (s, 3H), 1.27 (s, 3H), 0.79 (s, 9H), -0.00 (s, 3H), -0.03 (s, 3H); MS (ES+) 302.322 (M+1).

### Step 2 Preparation of 2-((3aS,6R,6aR)-6-(((tert-butyldimethylsilyl)oxy)methyl)-5-hydroxy-2,2-dimethyltetrahydro-3aH-[1,3]dioxolo[4,5-c]pyrrol-4-yl)acetonitrile (2a)

To a solution of n-BuLi (10.3 mL, 16.5 mmol) in THF (30 mL) at -78 °C was added dropwise acetonitrile (0.9 mL, 17.33 mmol) and stirred for 30 mins at -78°C. To the precipitate of lithiated acetonitrile was added dropwise a solution of (3aR,4R,6aS)-4-((tert-butyldimethylsilyloxy)methyl)-2,2-dimethyl-4,6a-dihydro-3aH-[1,3]dioxolo[4,5-c]pyrrole 5-oxide (**11**) (1 g, 3.3 mmol) in THF (5 mL) at -78°C and stirred for 1 h at -78°C. The reaction mixture was quenched with water (10 mL), allowed to warm to room temperature and diluted with hexanes (50 mL). The organic layer was separated dried, filtered and concentrated in vacuum to furnish 2-((3aS,6R,6aR)-6-(((tert-butyldimethylsilyl)oxy)methyl)-5-hydroxy-2,2-dimethyltetrahydro-3aH-[1,3]dioxolo[4,5-c]pyrrol-4-yl)acetonitrile (**2a**) (1.25 g, 110%) as a brown oil which was pure enough to use as such for next step, ¹H NMR (300 MHz, Chloroform-*d*) δ 5.42 (s, 1H), 4.32 (q, *J* = 7.5, 5.2 Hz, 2H), 3.93 - 3.71 (m, 3H), 3.65 (t, *J* = 6.7 Hz, 1H), 2.76 (qd, *J* = 17.1, 4.6 Hz, 2H), 1.52 (s, 3H), 1.31 (s, 3H), 0.91 (s, 9H), 0.10 (s, 6H).

### Step 3: Preparation of 2-((3aS,4S,6R,6aR)-6-(((tert-butyldimethylsilyl)oxy)methyl)-2,2-dimethyltetrahydro-3aH-[1,3]dioxolo[4,5-c]pyrrol-4-yl)acetonitrile (2b)

To 2-((3aS,6R,6aR)-6-(((tert-butyldimethylsilyl)oxy)methyl)-5-hydroxy-2,2-dimethyltetrahydro-3aH-[1,3]dioxolo[4,5-c]pyrrol-4-yl)acetonitrile (**2a**) (1.25 gm, 3.3 mmol) obtained from above step was added glacial acetic acid (7 mL) and zinc dust (2 g). The reaction mixture was stirred at room temperature for 6 h and filtered through a pad of Celite. The filter cake wash with ethyl acetate and filtrate was concentrated in vacuum to dryness. Residue obtained was dissolved in ethyl acetate (25 mL), washed with saturated NaHCO₃ (25 mL), dried, filtered and concentrated in vacuum to dryness to furnish 2-((3aS,4S,6R,6aR)-6-(((tert-butyldimethylsilyl)oxy)methyl)-2,2-dimethyltetrahydro-3aH-[1,3]dioxolo [4,5-c]pyrrol-4-yl)acetonitrile (**2b**) (1.1 gm, 100%) as a brown oil, which was pure enough to be used as such in next step. ¹H NMR (300 MHz, Chloroform-*d*) δ 4.48 (dd, *J* = 6.5, 3.7 Hz, 1H), 4.31 (dd, *J* = 6.1 Hz, 1H), 3.71 (qd, *J* = 10.5, 9.8, 4.4 Hz, 2H), 3.40 (q, *J* = 5.7, 5.1 Hz, 1H), 3.32 (p, *J* = 4.3 Hz, 1H), 2.77 - 2.54 (m, 2H), 2.12 (s, 1H), 1.53 (d, *J* = 7.8 Hz, 3H), 1.33 (s, 3H), 0.91 (s, 9H), 0.08 (s, 6H).

### Step 4: Preparation of (3aR,4R,6S,6aS)-tert-butyl 4-(((tert-butyldimethylsilyl)oxy)methyl)-6-(cyanomethyl)-2,2-dimethyldihydro-3aH-[1,3]dioxolo[4,5-c]pyrrole-5(4H)-carboxylate (2c)

To a solution of 2-((3aS,4S,6R,6aR)-6-(((tert-butyldimethylsilyl)oxy)methyl)-2,2-dimethyltetrahydro-3aH-[1,3]dioxolo[4,5-c]pyrrol-4-yl)acetonitrile (**2b**) obtained from above step (1.1 g, 3.3 mmol) in chloroform was added Boc anhydride (0.75 g, 3.44 mmol) and stirred at room temperature for 3 h. The reaction mixture was concentrated in vacuum to dryness and the residue obtained was purified by flash column chromatography eluting with (0-50%) ethyl acetate in hexane to furnish (3aR,4R,6S,6aS)-tert-butyl 4-(((tertbutyldimethylsilyl)oxy)methyl)-6-(cyanomethyl)-2,2-dimethyldihydro-3aH-[1,3]dioxolo[4,5-c]pyrrole-5(4H)-carboxylate (**2c**) (1.0 g, 71%) as an oil; ¹H NMR (300 MHz, Chloroform-*d*); ¹H NMR (300 MHz, Chloroform-*d*) δ 4.62 - 4.55 (m, 1H), 4.53 - 4.40 (m, 1H), 4.07 (d, *J* = 2.6 Hz, 1H), 3.62 (q, *J* = 3.0 Hz, 2H), 2.77 - 2.54 (m, 2H), 1.44 (s, 3H), 1.39 (d, *J=* 2.9 Hz, 9H), 1.25 (s, 3H), 0.82 (s, 9H), 0.00 (s, 6H).

### Step 5: preparation of (3aR,4R,6S,6aS)-tert-butyl 4-(((tert-butyldimethylsilyl)oxy)methyl)-6-((E/Z)-1-cyano-2-(dimethylamino)vinyl)-2,2-dimethyldihydro-3aH-[1,3]dioxolo[4,5-c]pyrrole-5(4H)-carboxylate (2d)

To a solution of furnish (3aR,4R,6S,6aS)-tert-butyl 4-(((tert-butyldimethylsilyl)oxy)methyl)-6-(cyanomethyl)-2,2-dimethyldihydro-3aH-[1,3]dioxolo[4,5-c]pyrrole-5(4H)-carboxylate (**2c**) (0.88 g, 2.06 mmol) in DMF (12 mL) was added 1-tert-butoxy-N,N,N',N'-tetramethylmethanediamine (1.5 mL, 7.24 mmol) and heated at 70 °C for 3 h. The reaction mixture was cooled to room temperature diluted with toluene (25 mL), washed with water (2 x 25 mL), dried filtered and concentrated in vacuum to furnish product containing a mixture of (3aR,4R,6S,6aS)-tert-butyl 4-(((tert-butyldimethylsilyl)oxy)methyl)-6-((E/Z)-1-cyano-2-(dimethylamino)vinyl)-2,2-dimethyldihydro-3aH-[1,3]dioxolo[4,5-c]pyrrole-5(4H)-carboxylate (**2d**) and (3aR,4R,6S,6aS)-tert-butyl 4-(((tert-butyldimethylsilyl)oxy)methyl)-6-((E/Z)-1-cyano-2-hydroxyvinyl)-2,2-dimethyldihydro-3aH-[1,3]dioxolo[4,5-c]pyrrole-5(4H)-carboxylate (**2e**) as a brown oil.

### Step 6: Preparation of (3aR,4R,6S,6aS)-tert-butyl 4-(((tert-butyldimethylsilyl)oxy)methyl)-6-((E/Z)-1-cyano-2-hydroxyvinyl)-2,2-dimethyldihydro-3aH-[1,3]dioxolo[4,5-c]pyrrole-5(4H)-carboxylate (2e)

To a mixture containing (3aR,4R,6S,6aS)-tert-butyl 4-(((tert-butyldimethylsilyl)oxy)methyl)-6-((E/Z)-1-cyano-2-(dimethylamino)vinyl)-2,2-dimethyldihydro-3aH-[1,3]dioxolo[4,5-c]pyrrole-5(4H)-carboxylate (**2d**) and (3aR,4R,6S,6aS)-tert-butyl 4-(((tertbutyldimethylsilyl)oxy)methyl)-6-((Z)-1-cyano-2-hydroxyvinyl)-2,2-dimethyldihydro-3aH-[1,3]dioxolo[4,5-c]pyrrole-5(4H)-carboxylate (**2e**) from above (2.06 mmol) was added THF, acetic acid and water (1:1:1, 20 mL) and stirred at room temperature for 4 h. The reaction mixture was extracted with chloroform (2 x 25 mL). The organic layers were combined washed with saturated aqueous NaHCO₃, dried, filtered and concentrated in vacuum to furnish (3aR,4R,6S,6aS)-tert-butyl 4-(((tert-butyldimethylsilyl)oxy)methyl)-6-((E/Z)-1-cyano-2-hydroxyvinyl)-2,2-dimethyldihydro-3aH-[1,3]dioxolo[4,5-c]pyrrole-5(4H)-carboxylate (**2e**) (0.9 g, 96%) as an oil. ¹H NMR (300 MHz, Chloroform-*d*) δ 11.50 (s, 1H), 7.13 (d, *J* = 9.1 Hz, 1H), 4.92 (d, *J* = 5.7 Hz, 1H), 4.86 - 4.82 (m, 1H), 4.77 (s, 1H), 4.00 (ddd, *J* = 9.4, 5.3, 1.5 Hz, 1H), 3.66 (dd, *J* = 10.1, 5.2 Hz, 1H), 3.54 (t, *J* = 9.7 Hz, 1H), 1.49 (s, 9H), 1.47 (s, 3H), 1.34 (s, 3H), 0.91 (d, *J* = 1.9 Hz, 9H), 0.09 (d, *J* = 2.8 Hz, 6H); MS (ES-) 453.39 (M-1).

### Step 7: Preparation of 1-benzyl 2-ethyl 3-amino-4-((3aS,4S,6R,6aR)-5-(tert-butoxycarbonyl)-6-(((tert-butyldimethylsilyl)oxy)methyl)-2,2-dimethyltetrahydro-3aH-[1,3]dioxolo [4,5-c]pyrrol-4-yl)-1H-pyrrole-1,2-dicarboxylate (2f)

To a solution of (3aR,4R,6S,6aS)-tert-butyl 4-(((tert-butyldimethylsilyl)oxy)methyl)-6-((E/Z)-1-cyano-2-hydroxyvinyl)-2,2-dimethyldihydro-3aH-[1,3]dioxolo[4,5-c]pyrrole-5(4H)-carboxylate (**2e**) (2.5 g, 5.48 mmol) in methanol (50 mL) was added ethyl glycinate hydrochloride (3.3 g, 23.6 mmol), sodium acetate (3.9 g, 47.6 mmol) and stirred at room temperature for 16 h. The reaction mixture was concentrated in vacuum and the residue obtained was dissolved in dichloromethane (50 mL) and washed with water (25 mL). The organic layer was dried filtered and concentrated in vacuum. The residue obtained was purified by flash column chromatography
to afford N-tert-butoxycarbonyl-7-O-tert-butyldimethylsilyl-2-cyano-1,2,3,6-tetradeoxy-1-N-(ethoxycarbonylmethylamino)-3,6-imino-4,5-O-isopropylidene-d-allo-hept-1-enitol as a mixture of isomers. To a solution of this mixture of isomers in dichloromethane (50 mL) was added 1,8-diazabicyclo[5.4.0]undec-7-ene (11.74 mL,75.6 mmol) and benzyl chloroformate (5.74 mL, 28 mmol) and heated at reflux for 4 h. The reaction mixture was cooled to room temperature and washed with dilute aqueous HCl (25 mL), aqueous sodium bicarbonate (25 mL), dried, filtered and concentrated in vacuum. The residue obtained was purified by flash column chromatography to furnish 1-benzyl 2-ethyl 3-amino-4-((3aS,4S,6R,6aR)-5-(tert-butoxycarbonyl)-6-(((tert-butyldimethylsilyl)oxy)methyl)-2,2-dimethyltetrahydro-3aH-[1,3]dioxolo[4,5-c]pyrrol-4-yl)-1H-pyrrole-1,2-dicarboxylate (**2f**) (2.4 g, 65 %) as a colorless oil.

### Step 8: Preparation of (3aS,4S,6R,6aR)-tert-butyl 4-(4-amino-5-(ethoxycarbonyl)-1H-pyrrol-3-yl)-6-(((tert-butyldimethylsilyl)oxy)methyl)-2,2-dimethyldihydro-3aH-[1,3]dioxolo[4,5-c]pyrrole-5(4H)-carboxylate (2g)

To a solution of 1-benzyl 2-ethyl 3-amino-4-((3aS,4S,6R,6aR)-5-(tert-butoxycarbonyl)-6-(((tert-butyldimethylsilyl)oxy)methyl)-2,2-dimethyltetrahydro-3aH-[1,3]dioxolo[4,5-c]pyrrol-4-yl)-1H-pyrrole-1,2-dicarboxylate (**2f**) (2.4 g, 3.56 mmol) in ethanol (100 mL) was added 10% Pd/C
(0.3 g) and hydrogenated at 45 psi for 4h. The reaction mixture was filtered through Celite and concentrated in vacuum to afford of (3aS,4S,6R,6aR)-tert-butyl 4-(4-amino-5-(ethoxycarbonyl)-1H-pyrrol-3-yl)-6-(((tert-butyldimethylsilyl)oxy)methyl)-2,2-dimethyldihydro-3aH-[1,3]dioxolo[4,5-c]pyrrole-5(4H)-carboxylate (**2g**) (1.7 g, 70which was pure enough to be taken for next step.

### Step 9: Preparation of (3aS,4S,6R,6aR)-tert-butyl 4-(4-((E)-(benzamido(methylthio)methylene)amino)-5-(ethoxycarbonyl)-1H-pyrrol-3-yl)-6-(((tertbutyldimethylsilyl)oxy)methyl)-2,2-dimethyldihydro-3aH-[1,3]dioxolo[4,5-c]pyrrole-5(4H)-carboxylate (2h)

To a solution of A solution of (3aS,4S,6R,6aR)-tert-butyl 4-(4-amino-5-(ethoxycarbonyl)-1H-pyrrol-3-yl)-6-(((tert-butyldimethylsilyl)oxy)methyl)-2,2-dimethyldihydro-3aH-[1,3]dioxolo[4,5-c]pyrrole-5(4H)-carboxylate (**2g**) (1.7 g, 3.15 mmol) in dichloromethane (100 mL) at 0 °C was added benzoyl isothiocyanate (0.57 g, 3.46 mmol) and stirred for 30 min. The reaction mixture was warmed to room temperature and added 1,8-diazabicyclo[5.4.0]undec-7-ene (1.13 mL, 7.58 mmol), methyl iodide (1.4 mL, 22.5 mmol) were added. The reaction mixture was stirred at room temperature overnight and concentrated in vacuum. The residue obtained was purified by flash column chromatography (silica gel, eluting with 0-50% ethyl acetate in hexane) to furnish (3aS,4S,6R,6aR)-tert-butyl 4-(4-((E)-(benzamido(methylthio)methylene)amino)-5-(ethoxycarbonyl)-1H-pyrrol-3-yl)-6-(((tertbutyldimethylsilyl)oxy)methyl)-2,2-dimethyldihydro-3aH-[1,3]dioxolo[4,5-c]pyrrole-5(4H)-carboxylate (**2h**) (1.328 g, 58%) as an oil, MS (ES+) 717.42 (M+1), (ES-) 715.45 (M-1.)

### Step 10: Preparation of (3aS,4S,6R,6aR)-tert-butyl 4-(2-amino-4-oxo-4,5-dihydro-3H-pyrrolo[3,2-d]pyrimidin-7-yl)-6-(((tert-butyldimethylsilyl)oxy)methyl)-2,2-dimethyldihydro-3aH-[1,3]dioxolo[4,5-c]pyrrole-5(4H)-carboxylate (2i)

Ammonia was bubbled at room temperature to a solution of (3aS,4S,6R,6aR)-tert-butyl 4-(4-((E)-(benzamido(methylthio)methylene)amino)-5-(ethoxycarbonyl)-1H-pyrrol-3-yl)-6-(((tertbutyldimethylsilyl)oxy)methyl)-2,2-dimethyldihydro-3aH-[1,3]dioxolo[4,5-c]pyrrole-5(4H)-carboxylate (**2h**) (1.3 g, 1.81 mmol) in methanol (100 mL) at room temperature in an autoclave for 20 mins. The vessel was sealed and heated at 95 °C for 18 h. The reaction mixture was concentrated in vacuum and the residue obtained was purified by flash column chromatography (silica gel, eluting with 0-25% methanol in chloroform) to furnish (3aS,4S,6R,6aR)-tert-butyl 4-(2-amino-4-oxo-4,5-dihydro-3H-pyrrolo[3,2-d]pyrimidin-7-yl)-6-(((tert-butyldimethylsilyl)oxy)methyl)-2,2-dimethyldihydro-3aH-[1,3]dioxolo[4,5-c]pyrrole-5(4H)-carboxylate (**2i**) (0.56 g, 58%); MS (ES+) 536.68 (M+1), (ES-) 534.51 (M-1)

### Example 5: (2S,3S,4R,5R)-2-(4-aminopyrrolo[2,1-f][1,2,4]triazin-7-yl)-5-(hydroxymethyl)pyrrolidine-3,4-diol dihydrochloride (30g)

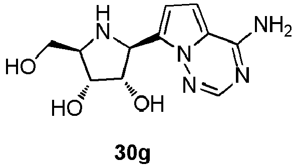

To a solution of 7-((3aS,6R,6aR)-6-((tert-butyldimethylsilyloxy)methyl)-2,2-dimethyltetrahydro-3aH-[1,3]dioxolo[4,5-c]pyrrol-4-yl)pyrrolo[1,2-f][1,2,4]triazin-4-amine (**30f**) (51 mg, 0.122 mmol) in acetonitrile (6 mL) was added 2 N hydrogen chloride (0.260 mL, 0.519 mmol) and stirred at room temperature for 15 h. The solid obtained was collected by filtration washed with acetonitrile and dried under vacuum to furnish (2S,3S,4R,5R)-2-(4-aminopyrrolo[2,1-f][1,2,4]triazin-7-yl)-5-(hydroxymethyl)pyrrolidine-3,4-diol (**30g**) (24 mg, 54%) as an off white solid; ¹H NMR (300 MHz, DMSO-*d₆*) δ 10.38 (s, 1H), 9.13 (s, 1H), 8.67 (s, 2H), 8.15 (s, 1H), 7.25 (d, J = 4.7 Hz, 1H), 6.98 (d, J = 4.6 Hz, 1H), 4.97 - 4.83 (m, 1H), 4.57 (dd, J = 7.8, 4.4 Hz, 1H), 4.15 (t, J = 4.1 Hz, 1H), 3.72 - 3.60 (m, 2H), 3.60 - 3.50 (m, 1H); ¹H NMR (300 MHz, DMSO-*d₆*/D₂O) d 8.12 (s, 1H), 7.20 (d, J = 4.6 Hz, 1H), 6.97 (d, J = 4.7 Hz, 1H), 4.91 (d, J = 8.0 Hz, 1H), 4.60 (dd, J = 8.0, 4.4 Hz, 1H), 4.16 (t, J = 4.0 Hz, 1H), 3.80-3.54 (m,3H); MS (ES+): 266.3 (M+1).

### Preparation of 7-((3aS,4S,6R,6aR)-6-(((tert-butyldimethylsilyl)oxy)methyl)-2,2-dimethyltetrahydro-3aH-[1,3]dioxolo[4,5-c]pyrrol-4-yl)pyrrolo[2,1-f][1,2,4]triazin-4-amine (30f)

### Step 1: Preparation of N-(pyrrolo[2,1-f][1,2,4]triazin-4-yl)pivalamide (30b)

To a solution of pyrrolo[2,1-f][1,2,4]triazin-4-amine (**30a**) (23.9 gm, 178.2 mmol) in pyridine (120 mL, 5 mL/gm) at room temperature was added pivaloyl chloride (32.88 mL, 267.3 mmol) and stirred at room temperature until reaction was complete (2.5 h). The reaction mixture was concentrated in vacuum to remove pyridine and residue was purified by flash column chromatography (silica gel, eluting 0-100% ethylacetate in hexane) to furnish N-(pyrrolo[2,1-f][1,2,4]triazin-4-yl)pivalamide (**30b**) (23.2 gm, 59.6%) as a light yellow solid. ¹H NMR (300 MHz, DMSO-d6) δ 10.28 (s, 1H), 8.29 (s, 1H), 8.01 (dd, *J* = 2.6, 1.5 Hz, 1H), 6.96 (dd, *J* = 4.6, 1.5 Hz, 1H), 6.90 (dd, *J* = 4.7, 2.6 Hz, 1H), 1.29 (s, 9H); MS (ES+) 241.2 (M+Na), (ES-) 217.3 (M-1).

### Step 2: Preparation of N-(7-Bromopyrrolo[2,1-f][1,2,4]triazin-4-yl)pivalamide (30c)

To a solution of furnish N-(7-bromopyrrolo[2,1-f][1,2,4]triazin-4-yl)pivalamide (**30b**) (16.8 gm, 77 mmol) in chloroform (840 mL) at 0-5 °C was added NBS (13.7 gm, 77 mmol) in portions of 1 gm every 10 mins. The reaction mixture was stirred for 1 h after the addition of NBS was completed. The reaction mixture was filtered and the filtrate was concentrated in vacuum and purified by flash column chromatography eluting with 0-100% ethyl acetate in hexane to furnish N-(7-Bromopyrrolo[2,1-f][1,2,4]triazin-4-yl)pivalamide (**30c**) (13.95 gm, 61%) as a yellow solid.; ¹H NMR (300 MHz, DMSO-*d₆*) δ 10.43 (s, 1H), 8.43 (s, 1H), 7.10 (m, 2H), 1.29 (s, 9H); MS (ES+) 321.1 (M+Na), (ES-) 295.1, 297.1 (M-1); Analysis Calculated for C₁₁H₁₃BrN₄O: C, 44.46; H, 4.41; Br, 26.89; N, 18.85; Found: C, 44.47; H, 4.41; Br, 26.72; N, 18.71.

### Step 3: Preparation of N-(7-((3aS,4S,6R,6aR)-6-(((tert-butyldimethylsilyl)oxy)methyl)-2,2-dimethyltetrahydro-3aH-[1,3]dioxolo[4,5-c]pyrrol-4-yl)pyrrolo[2,1-f][1,2,4]triazin-4-yl)pivalamide (30e)

To a solution of N-(7-bromopyrrolo[1,2-f][1,2,4]triazin-4-yl)pivalamide (**30c**) (1.783 g, 6.00 mmol) in THF (30 mL) cooled to -78 °C was added dropwise n-butyl lithium (1.6 M in hexanes, 12.40 mL, 19.84 mmol) and stirred at -78 °C for 1 h. To the anion formed was added a freshly prepared solution of N-(7-((3aS,6R,6aR)-6-((tert-butyldimethylsilyloxy)methyl)-2,2-dimethyltetrahydro-3aH-[1,3]dioxolo[4,5-c]pyrrol-4-yl)pyrrolo[1,2-f][1,2,4]triazin-4-yl)pivalamide (**1k**) (6 mmol) in toluene (25 mL) dropwise at -78 °C and warmed to room temperature over a period of 1 h. The reaction mixture was quenched with saturated aqueous NH₄Cl (120 mL), extracted with ethyl acetate (200 mL, 100 mL). The combined organic extracts were washed with brine (100 mL), dried over MgSO₄, filtrated and concentrated in vacuum to furnish crude residue. The residue was purified by flash column chromatography (silica gel 48 gm, eluting with 0-35% ethyl acetate in hexane) to furnish N-(7-((3aS,4S,6R,6aR)-6-(((tert-butyldimethylsilyl)oxy)methyl)-2,2-dimethyltetrahydro-3aH-[1,3]dioxolo[4,5-c]pyrrol-4-yl)pyrrolo[2,1-f][[1,2,4]triazin-4-yl)pivalamide (**30e**) (0.243 g, 8%) as a brown solid; ¹H NMR (300 MHz, DMSO-*d₆*) δ 10.27 (s, 1H), 8.30 (s, 1H), 6.93 (d, *J* = 4.6 Hz, 2H), 4.82 (dd, *J* = 6.6, 4.4 Hz, 1H), 4.62 (s, 1H), 4.51 (dd, *J =* 6.8, 3.2 Hz, 1H), 3.57 (d, *J* = 5.4 Hz, 2H), 3.29 - 3.19 (m, 2H), 1.48 (s, 3H), 1.28 (s, 9H), 1.27 (s, 3H), 0.86 (s, 9H), 0.03 (s, 6H); MS (ES+) 504.4 (M+1).

### Step 4: Preparation of 7-((3aS,4S,6R,6aR)-6-(((tert-butyldimethylsilyl)oxy)methyl)-2,2-dimethyltetrahydro-3aH-[1,3]dioxolo[4,5-c]pyrrol-4-yl)pyrrolo[2,1-f][[1,2,4]triazin-4-amine (30f)

To a solution of N-(7-((3aS,6R,6aR)-6-((tert-butyldimethylsilyloxy)methyl)-2,2-dimethyltetrahydro-3aH-[1,3]dioxolo[4,5-c]pyrrol-4-yl)pyrrolo[1,2-f][1,2,4]triazin-4-yl)pivalamide (**30e**) (103 mg, 0.204 mmol) in MeOH (8 mL) was added with sodium ethanolate (0.170 mL, 0.456 mmol) and stirred at 50 °C for 4 h. The reaction mixture was neutralized with aqueous 1 N HCl and concentrated in vacuum to dryness. The residue was purified by flash column chromatography [silica gel 4 g, eluting with chloroform/methanol (1:0 to 19:1)] to afford 7-((3aS,4S,6R,6aR)-6-(((tert-butyldimethylsilyl)oxy)methyl)-2,2-dimethyltetrahydro-3aH-[1,3]dioxolo[4,5-c]pyrrol-4-yl)pyrrolo[2,1-f][[1,2,4]triazin-4-amine (**30f**) (60 mg, 70%) as an off-white solid; ¹H NMR (300 MHz, DMSO-*d₆*) δ 7.78 (s, 1H), 7.67 (s, 2H), 6.79 (d, *J =* 4.4 Hz, 1H), 6.58 (d, *J* = 4.4 Hz, 1H), 4.78 (dd, *J =* 6.8, 4.8 Hz, 1H), 4.51 - 4.41 (m, 2H), 3.57 (s, 2H), 3.14 (s, 2H), 1.44 (s, 3H), 1.24 (s, 3H), 0.84 (s, 9H), -0.00 (s, 6H); MS (ES+) 420.3 (M+1).

### Example 6: (2S,3S,4R,5R)-2-(2,6-dimethoxypyridin-3-yl)-5-(hydroxymethyl)pyrrolidine-3,4-diol (17e)

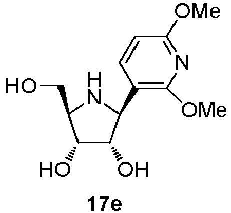

To a stirred solution of (3aR,4R,6S,6aS)-4-(((tert-butyldimethylsilyl)oxy)methyl)-6-(2,6-dimethoxypyridin-3-yl)-2,2-dimethyltetrahydro-3aH-[1,3]dioxolo[4,5-c] (**17d**) (1.2g, 2.83 mmol) in acetonitrile (30 mL) at room temperature was added 3 M hydrochloric acid (3.77 mL, 11.30 mmol) and stirred for 24 h. The reaction mixture was concentrated in vacuum and the residue obtained was azeotroped with ethanol (4 x 50 mL). The residue obtained was purified by flash column chromatography [silica gel, 25 g, eluting with CMA 80 in chloroform 0 to 100%] to afford (3S,4R,5R)-2-(2,6-dimethoxypyridin-3-yl)-5-(hydroxymethyl)pyrrolidine-3,4-diol (**17e**) (590 mg, 77 %) as a light brown syrup; ¹H NMR (300 MHz, DMSO-*d₆*) δ 7.73 (d, *J* = 8.1 Hz, 1H), 6.34 (d, *J* = 8.0 Hz, 1H), 4.52 (dd, *J* = 5.0, 2.1 Hz, 3H, D₂O exchangeable), 4.08 (d, *J* = 5.4 Hz, 1H), 3.86 (s, 3H), 3.83 (s, 3H), 3.69 - 3.59 (m, 2H), 3.52 - 3.37 (m, 2H), 3.32 (s, 1H, D₂O exchangeable), 2.98 (q, *J* = 4.8 Hz, 1H). MS (ES+) 271.3 (M+1), (ES-) 269.5 (M-1).

### Preparation of (3aR,4R,6S,6aS)-4-(((tert-butyldimethylsilyl)oxy)methyl)-6-(2,6-dimethoxypyridin-3-yl)-2,2-dimethyltetrahydro-3aH-[1,3]dioxolo[4,5-c] (17d)

### Step 1: Preparation of 3-bromo-2,6-dimethoxypyridine (17b)

2,6-Dimethoxypyridine (**17a**) (7.38 g, 0.053 mol), N-bromosuccinimide (9.43 g, 0.053 mol) and 133 mL of dry acetonitrile were placed in a one-neck flask (250 mL) and stirred at reflux for 10 h. After this time acetonitrile was distilled off under reduced pressure using a rotary evaporator (60 °C 160/mbar) and 100 mL of saturated aqueous solution of sodium metabisulfite was added and the total content was shaken. The suspension was extracted with ethyl acetate (2 x 100 mL) and the combined organic extracts were washed with 100 mL of 1 M NaOH, dried over anhydrous MgSO₄ filtered and concentrated in vacuum to dryness yielding 3-bromo-2,6-dimethoxypyridine (**17b**) (11 g, 50.4 mmol, 95 % yield) as a colorless oil; which was used as such for next step.

¹H NMR (300 MHz, DMSO-*d₆*) δ 7.86 (d, J = 8.3 Hz, 1H), 6.37 (d, J = 8.3 Hz, 1H), 3.93 (s, 3H), 3.87 (s, 3H); 1H NMR (300 MHz, Chloroform-d) δ 7.64 (d, J = 8.3 Hz, 1H), 6.24 (d, J = 8.3 Hz, 1H), 4.00 (s, 3H), 3.91 (s, 3H).

### Step 2: Preparation of (3aR,4R,6S,6aS)-4-(((tert-butyldimethylsilyl)oxy)methyl)-6-(2,6-dimethoxypyridin-3-yl)-2,2-dimethyltetrahydro-3aH-[1,3]dioxolo[4,5-c] (17d)

To a stirred solution of 3-bromo-2,6-dimethoxypyridine (**17b**) (2.73 g, 12.50 mmol) in Tetrahydrofuran (30 mL) was added n-Butyl lithium (1.6 M solution in hexanes, 7.81 mL, 12.50 mmol) at -78 °C and stirred at the same temperature for 1 hr. To the anion formed at - 78 °C was added a freshly prepared solution of (3aR,4R,6aS)-4-((tert-butyldimethylsilyloxy)methyl)-2,2-dimethyl-4,6a-dihydro-3aH-[1,3]dioxolo[4,5-c]pyrrole (**1k**) (2.85 g, 10 mmol) in toluene (1.2 molar solution) over a period of 15 minutes. The reaction stirred for 30 minutes at -78 °C, quenched with water (50 mL) and extracted with ethyl acetate (3 x 100 mL). The combined organic layers were washed with water (50 mL), brine (50 mL) dried, filtered and concentrated in vacuum. The crude residue was purified by flash column chromatography (silica gel 40 g, eluting with 0-100% ethyl acetate in hexanes) to afford (3aR,4R,6aS)-4-((tert-butyldimethylsilyloxy)methyl)-6-(2,6-dimethoxypyridin-3-yl)-2,2-dimethyltetrahydro-3aH-[1,3]dioxolo[4,5-c]pyrrole (**17d**) (1.35 g, 31.8 % yield) as a light brown syrup.

¹H NMR (300 MHz, DMSO-*d₆*) δ 7.64 (d, *J* = 8.1 Hz, 1H), 6.29 (d, *J* = 8.0 Hz, 1H), 4.42 - 4.28 (m, 2H), 4.09 (d, *J* = 4.3 Hz, 1H), 3.82 (s, 3H), 3.79 (s, 3H), 3.57 (d, *J* = 5.0 Hz, 2H), 3.05 (d, *J* = 4.8 Hz, 1H), 2.85 (s, 1H, D₂O exchangeable), 1.40 (s, 3H), 1.17 (s, 3H), 0.82 (s, 9H), -0.00 (d, *J=* 1.3 Hz, 6H). Mass spec (ES+) 425.1 (M+1).

### Example 7: 3-((2S,3S,4R,5R)-3,4-dihydroxy-5-(hydroxymethyl)pyrrolidin-2-yl)-6-methoxypyridin-2(1H)-one (17f)

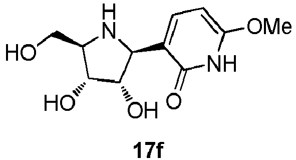

To a stirred solution of afford (3aR,4R,6aS)-4-((tert-butyldimethylsilyloxy)methyl)-6-(2,6-dimethoxypyridin-3-yl)-2,2-dimethyltetrahydro-3aH-[1,3]dioxolo[4,5-c]pyrrole (**17d**) (0.45 g, 1.665 mmol) in dichloromethane (20 mL) was added boron tribromide (1M solution in dichloromethane) (4.16 mL, 4.16 mmol) at -78 °C over a period of 30 minutes. The reaction was allowed to warm to room temperature overnight. The reaction was cooled to -78 °C and added additional boron tribromide (1 M solution in dichloromethane) (4.16 mL, 4.16 mmol) and warmed to room temperature. The reaction mixture was stirred for additional 16 h cooled to 0 °C and quenched with methanol (30 mL) and water (2 mL). The reaction mixture was refluxed for 2 h and concentrated in vacuum to dryness. To the residue was added methanol (50 mL) and concentrated in vacuum to dryness, this operation was repeated 3 times. The residue obtained was purified by flash column chromatography (silica gel 12 g, eluting with 0 to 100% CMA 80 in chloroform) to afford 3-((2S,3S,4R,5R)-3,4-dihydroxy-5-(hydroxymethyl)pyrrolidin-2-yl)-6-methoxypyridin-2(1H)-one (**17f**) (70 mg, 16.41 % yield) as a light brown solid.

¹H NMR (300 MHz, DMSO-*d₆*) δ 7.33 (d, *J* = 8.0 Hz, 1H), 6.10 (d, *J* = 7.9 Hz, 1H), 4.90 (s, 3H, D₂O exchangeable), 4.03 (d, *J=* 7.9 Hz, 1H), 3.79 - 3.74 (m, 2H), 3.73 (s, 3H), 3.67 (d, *J* = 5.7 Hz, 1H), 3.43 (d, *J=* 5.7 Hz, 2H), 3.12 (dt, *J=* 8.4, 4.0 Hz, 1H, D₂O exchangeable). Mass spec (ES+) 257.2 (M+1), 255.1 (M-1).

### Example 8: 5-((2S,3S,4R,5R)-3,4-dihydroxy-5-(hydroxymethyl)pyrrolidin-2-yl)-6-hydroxypyridin-2(1H)-one (16f)

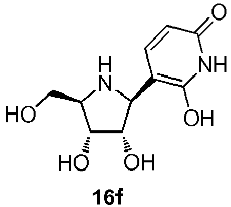

To a stirred solution of afford (3aR,4R,6aS)-4-((tert-butyldimethylsilyloxy)methyl)-6-(2,6-dimethoxypyridin-3-yl)-2,2-dimethyltetrahydro-3aH-[1,3]dioxolo[4,5-c]pyrrole (**17d**) (0.77 g, 0.285 mmol) in methanol (5 mL) was added conc HCl (4.25 mL, 140 mmol) and heated at reflux for 5 h. The reaction mixture weas cooled to room temperature and concentrated in vacuum to dryness. The residue obtained was triturated with hot ethanol and solid obtained was collected removed by filtration. The filtrate was concentrated in vacuum and the residue obtained was purified by flash column chromatography (silica gel 4 g, eluting with 0-100% methanol in chloroform) to furnish 5-((2S,3S,4R,5R)-3,4-dihydroxy-5-(hydroxymethyl)pyrrolidin-2-yl)-6-hydroxypyridin-2(1H)-one (**16f**); MS (ES+) 243.1(M+1), 241.1 (M-1).

### Example 9: General Procedures for Antiviral and Pharmacokinetics Testing

Healthy 8-to-10-week-old male Sprague-Dawley rats will be randomly assigned to control and experimental groups, N = 4 per group. All animals will be housed and fed in standard manner. On the day of the experiment, all animals will be isolated and fasted approximately 15 hours prior to dose in metabolic cages. Food will be returned to animals two hours post dose with control or experimental agent. Water will be delivered *ad libitum.* After each animal is weighed, all control animals will be administered 10 mg/kg body weight of carrier or placebo by oral gavage at time 0, while all experimental animals will be administered 10 mg/kg body weight compound by oral gavage at time 0. Serial blood samples will be obtained at time 0, 15 min, 30 min, 1 hr, 2 hr, 4 hr, 8 hr, 12 hr, and 24 hr. All samples will be transferred to microcentrifuge tubes and centrifuged at 14,000 rpm for 3 min. Plasma from each tube will be removed and transferred to a pre-labeled microcentrifuge tube and put on dry ice until samples are transferred to -80 °C freezer for storage until analysis. Individual samples will then be analyzed for compound. Plasma concentration-versus-time data will be analyzed by non-compartmental approaches using the WinNonlin software program. Pharmacokinetic parameter Tₘₐₓ, Cₘₐₓ, T_{½}, AUC₍₀₋ₗₐₛₜ₎, AUC_{(0-inf)}, MRT_{(0-inf)} and graphs of plasma and liver concentrations versus time profile were obtained.

### Example 10: Effects of Viral RNA Polymerase Inhibitors on Replication of Measles Virus in African Green Monkey Kidney Cells

### Materials and Methods

Vero-76 cells (African green monkey kidney cells) will be obtained from the American Type Culture Collection (ATCC, Manassas, VA). The cells will be routinely passed in minimal essential medium (MEM with 0.15% NaHCO₃; Hyclone Laboratories, Logan, UT, USA) supplemented with 5% fetal bovine serum (FBS, Hyclone). When evaluating compounds, the serum will be reduced to a final concentration of 2.5 %, and gentamicin added to the test medium to a final concentration of 50 µg/mL. Measles virus (MV), strain Chicago, will be obtained from the Centers for Disease Control (Atlanta, GA).

### Antiviral Testing Procedures

### Cytopathic Effect Inhibition Assay (Visual Assay)

Cells will be seeded to 96-well flat-bottomed tissue culture plates (Corning Glass Works, Corning, NY), 0.2 mL/well, at the proper cell concentration, and incubated overnight at 37 °C in order to establish a cell monolayer. When the monolayer has been established, the growth medium will be decanted and the various dilutions of test compound added to each well (3 wells/dilution, 0.1 mL/well). Compound diluent medium will be added to cell and virus control wells (0.1 mL/well). Virus, diluted in test medium, will be added to compound test wells (3 wells/dilution of compound) and to virus control wells (6 wells) at 0.1 mL/well. Virus (viral MOI = 0.001) will be added approximately 5 min after compound. Test medium without virus will be added to all toxicity control wells (2 wells/dilution of each test compound) and to cell control wells (6 wells) at 0.1 mL/well. The plates will be incubated at 37 °C in a humidified incubator with 5% CO₂, 95% air atmosphere until virus control wells have adequate cytopathic effect (CPE) readings (80-100% cell destruction). This is expected to be achieved from 4-11 days after virus exposure to cells, depending on the virus. Cells will be examined microscopically for CPE, this being scored from 0 (normal cells) to 4 (maximal, 100%, CPE). The cells in the toxicity control wells will be observed microscopically for morphologic changes attributed to cytotoxicity. This cytotoxicity (cell destruction and/or morphology change) will be also graded at 100% toxicity, 80% cytotoxicity), 60% cytotoxicity, 40% cytotoxicity, 20% cytotoxicity, and 0 (normal cells). The 50% effective dose (EC₅₀) and 50% cytotoxic dose (IC₅₀) will be calculated by regression analysis of the virus CPE data and the toxicity control data, respectively. The selective index (SI) for each compound tested will be calculated using the formula: SI = CC₅₀/EC₅₀.

### Neutral Red (NR) Uptake Assay of CPE Inhibition

NR uptake was chosen as the dye quantitation method for evaluating antiviral drugs based on the findings of Smee et al. (Virol. Methods 2002, 106: 71-79; herein incorporated by reference in its entirety). This assay will be done on the same CPE inhibition test plates described above to verify the inhibitory activity and the cytotoxicity observed by visual observation. The NR assay will be performed using a modified method of Cavenaugh et al. (Invest. New Drugs 1990, 8:347-354; herein incorporated by reference in its entirety) as described by Barnard et al. (Antiviral Chem. Chernother. 2001, 12:220-231; herein incorporated by reference in its entirety). Briefly, medium will be removed from each well of a plate scored for CPE from a CPE inhibition assay, 0.034% NR was added to each well of the plate and the plate incubated for 2 hr at 37 °C in the dark. The NR solution will then be removed from the wells. After rinsing (sometimes cells slough from the plate causing erroneous low up of neutral red) and aspirating to dryness, the remaining dye will be extracted for 30 min at room temperature in the dark from the cells using absolute ethanol buffered with Sorenson citrate buffer. Absorbances at 540 nm/405 nm will then be read with a microplate reader (Opsys MR™, Dynex Technologies, Chantilly, VA, USA). Absorbance values are expressed as percents of untreated controls and EC₅₀, CC₅₀ and SI values were calculated as described above.

### Virus Yield Reduction Assay:

Virus yield reduction assays will be performed using the cell culture 50% infectious dose (CCID₅₀) assay essentially as described previously (Antimicrob. Agents Chemother. 1992, 3:1837-1842; herein incorporated by reference in its entirety). Briefly, supernatants from each will be serially diluted in triplicate wells of 96-well plates containing Vero-76 cells. Plates will be incubated for 6 days and then checked for virus- induced CPE. Quantitation of virus yield titers will be by the endpoint method of Reed and Muench (Am. J. Hyg. 1938, 27:493-498; herein incorporated by reference in its entirety). The EC₉₀ value will be calculated using linear regression to estimate the concentration necessary to inhibit virus yield by 90% or a one log₁₀ decrease in virus titer.

### Example 11: Effects of Viral RNA Polymerase Inhibitor (Compound 12i) on Replication of Various RNA Viruses

### Materials and Methods

African green monkey kidney cells (MA-104) may be obtained from Whitaker MA Bioproducts, Walkersville, MD, USA). All Vero cells (African green monkey kidney cells, human carcinoma of the larynx cells (A-549), and Madin-Darby canine kidney cells may be obtained from the American Type Culture Collection (ATCC, Manassas, VA). A-549 cells will be cultured in Dulbecco's minimal essential medium (DMEM) supplemented with 0.15 % NaHCO₃ (Hyclone Laboratories, Logan, UT, USA) and with 10% fetal bovine serum (FBS, Hyclone). The remaining cells will be routinely passed in minimal essential medium (MEM with 0.15% NaHCO₃; Hyclone Laboratories, Logan, UT, USA) supplemented with 5% fetal bovine serum (FBS, Hyclone).

When evaluating compounds, the serum will be reduced to a final concentration of 2.5%, and gentamicin added to the test medium to a final concentration of 50 µg/mL. Test medium for influenza assays will consist of MEM without serum, 0.18% NaHCO₃, 20 µg trypsin/mL, 2.0 µg EDTA/mL, and 50 µg gentamicin/mL.

For evaluation of toxicity in actively growing cells, cytotoxicity will be evaluated by determining the total number of cells as reflected by a NR uptake assay after a 3-day exposure to several concentrations of compound. To quantitate cell growth at 72 h in the presence or absence of drug, plates will be seeded with 1 x 10³ MDCK cells, and after 4 h (allowed all cells to attach plate wells) exposed to selected concentrations of drug in MEM or MEM. After 72 h the plates will be treated as described above for the NR assay. Absorbance values will be evaluated as percent of untreated controls and CC₅₀ values were calculated by regression analysis.

Dengue virus 2 (DV-2), strain New Guinea C, Respiratory syncytial virus (RSV) A2, Rhinovirus 2 (RV-2), strain HOP, Tacaribe virus (TCV), strain TRVL 11573, Venezuelan equine encephalitis virus (VEE), and Yellow fever virus (YFV), strain 17D, may be purchased from American Type Culture Collection (ATCC; Manassas, VA). All influenza viruses, Measles virus (MV), strain Chicago, SARS corona virus (SARS-CoV), strain Urbani, and West Nile virus (WNV), prototypic New York 1999 isolate designated strain 996625, may be obtained from the Centers for Disease Control (Atlanta, GA). Punta Toro virus (PTV), Adames strain, may be obtained from Dr. Dominique Pifat of the U. S. Army Medical Research Institute for Infectious Diseases, Ft. Detrick (Frederick, MD). Rift Valley fever virus (RVFV) vaccine strain, MP-12, and Junin virus (JUNV) vaccine strain, Candid 1, have been kindly provided by Dr. Robert Tesh (World Reference Center for Emerging and Viruses and Arboviruses, University of Texas Medical Branch, Galveston, TX). Pichinde virus (PICV), strain An 4763, have been provided by Dr. David Gangemi (Clemson University, Clemson, South Carolina). Parainfluenza virus type 3 (PIV-3), strain 14702/5/95, may be obtained from Jacquelin Boivin (Hospitale St. Justin, Montreal, Canada). Adenovirus (AV-1) type 1, strain Chicago/95, was isolated from the tracheal washings of a pediatric patient and was provided by M.F. Smaron (Department of Medicine, University of Chicago, Chicago IL).

### Antiviral Testing Procedure

Cytopathic Effect inhibition Assay (Visual Assay): Cells will be seeded to 96-well flat-bottomed tissue culture plates (Corning Glass Works, Corning, NY), 0.2 mL/well, at the proper cell concentration, and incubated overnight at 37 °C in order to establish a cell monolayer. When the monolayer has been established, the growth medium is decanted and the various dilutions of test compound added to each well (3 wells/dilution, 0.1 mL/well). Compound diluent medium will be added to cell and virus control wells (0.1 mL/well). Virus, diluted in test medium, will be added to compound test wells (3 wells/dilution of compound) and to virus control wells (6 wells) at 0.1 mL/well. Virus (viral MOT = 0.001) will be added approximately 5 min after compound. Test medium without virus will be added to all toxicity control wells (2 wells/dilution of each test compound) and to cell control wells (6 wells) at 0.1 mL/well. The plates will be incubated at 37 °C in a humidified incubator with 5% CO₂, 95% air atmosphere until virus control wells have adequate cytopathic effect (CPE) readings (80-100% cell destruction). This is expected to be achieved from 4-11 days after virus exposure to cells, depending on the virus. Cells will then be examined microscopically for CPE, this being scored from 0 (normal cells) to 4 (maximal, 100 %) CPE. The cells in the toxicity control wells will be observed microscopically for morphologic changes attributed to cytotoxicity. This cytotoxicity (cell destruction and/or morphology change) will also be graded at 100% toxicity, 80% cytotoxicity), 60% cytotoxicity, 40% cytotoxicity, 20% cytotoxicity, and 0 (normal cells). The 50% effective dose (EC₅₀) and 50% cytotoxic dose (IC₅₀) were calculated by regression analysis of the virus CPE data and the toxicity control data, respectively. The selective index (SI) for each compound tested will be calculated using the formula: SI = CC₅₀/EC₅₀.

### Neutral Red (NR) Uptake Assay of CPE Inhibition and Compound Cytotoxicity

NR uptake was chosen as the dye quantitation method for evaluating antiviral drugs based on the findings of Smee *et al.* (*supra*). This assay was done on the same CPE inhibition test plates described above to verify the inhibitory activity and the cytotoxicity observed by visual observation. The NR assay will be performed using a modified method of Cavenaugh *et al.* (*supra*) as described by Barnard *et al.* (*supra*). In these procedures, medium was removed from each well of a plate scored for CPE from a CPE inhibition assay, 0.034% NR was added to each well of the plate and the plate incubated for 2 hr at 37 °C in the dark. The NR solution was then removed from the wells. After rinsing (sometimes cells slough from the plate causing erroneous low up of neutral red) and aspirating to dryness, the remaining dye was extracted for 30 min at room temperature in the dark from the cells using absolute ethanol buffered with Sorenson citrate buffer. Absorbances at 540 nm/405 nm are read with a microplate reader (Opsys MR™^{,} Dynex Technologies, Chantilly, VA, USA). Absorbance values were expressed as percents of untreated controls and EC₅₀, CC₅₀ and SI values were calculated as described above.

### Example 12: Ebola Virus Mouse Prophylaxis Study

A testing compound will be administered i.p., i.m., and orally (300 mg/kg/day, BID) to 8-12 week old C57BL/6 mice (N = 10 per group, 4 groups - one saline- and three drug-treated groups). Eight days of treatment starting 4 h prior to infection. Mouse-adapted Ebola virus (Zaire) challenge will be administered intraperitoneally. Mortality and weight were monitored for 14 days post-infection.

Saline-treated mice infected with Ebola virus all die by day 8. The survival of mice treated with test compounds will be evaluated by comparison.

Saline-treated mice infected with Ebola virus exhibit overall weight loss until day 8 (all control mice are dead by day 8). Mice treated intraperitoneally or intramuscularly with test compounds will be compared with this control.

### Example 13: Yellow Fever Virus (YFV) Time Window Golden Hamster Study

Yellow fever virus (Jimenez strain) will be injected i.p. into female Syrian golden hamsters (99 g) at 20 CCID₅₀ per hamster (∼ 6.25 x LD50). Groups will be divided as follows: 1) test compound administered beginning -4 h (N = 15); 2) test compound administered beginning 1 dpi (days post-infection) (N = 10); 3) test compound administered beginning 2 dpi (N = 10); 4) test compound administered 3 dpi (N = 10); 5) test compound administered 4 dpi (N = 10); 6) ribavirin administered beginning -4h (N = 10); 7) saline vehicle beginning -4 h (N = 16); 8) uninfected hamsters administered test compound beginning -4 h (N = 3); 9) uninfected hamsters administered saline vehicle beginning -4 h (N = 3); and 10) uninfected, untreated normal controls (N = 3). Treatment dose will be 100 mg/kg i.p., BID for 7 days. Study endpoints are morality at 21 days, weight measured on days 0, 3, 5, and 6; serum and liver virus titers (day 4, compound **12i** at -4 h, and vehicle at -4 h), and ALT and AST on day 6.

### Example 14: Pharmaceutical Dosage Forms

The following illustrate representative pharmaceutical dosage forms, containing a compound of the invention ('Compound X'), for therapeutic or prophylactic use in humans.

| (i) Tablet 1 | mg/tablet |
|---|---|
| Compound X | 100.0 |
| Lactose | 77.5 |
| Povidone | 15.0 |
| Croscarmellose sodium | 12.0 |
| Microcrystalline cellulose | 92.5 |
| Magnesium stearate | 3.0 |
| | 300.0 |

| (ii) Tablet 2 | mg/tablet |
|---|---|
| Compound X | 20.0 |
| Microcrystalline cellulose | 410.0 |
| Starch | 50.0 |
| Sodium starch glycolate | 15.0 |
| Magnesium stearate | 5.0 |
| | 500.0 |

| (iii) Capsule | mg/capsule |
|---|---|
| Compound X | 10.0 |
| Colloidal silicon dioxide | 1.5 |
| Lactose | 465.5 |
| Pregelatinized starch | 120.0 |
| Magnesium stearate | 3.0 |
| | 600.0 |

| (iv) Injection 1 (1 mg/mL) | mg/mL |
|---|---|
| Compound X (free acid form) | 1.0 |
| Dibasic sodium phosphate | 12.0 |
| Monobasic sodium phosphate | 0.7 |
| Sodium chloride | 4.5 |
| 1.0 N Sodium hydroxide solution (pH adjustment to 7.0-7.5) | q.s. |
| Water for injection | q.s. ad 1 mL |

| (v) Injection 2 (10 mg/mL) | mg/mL |
|---|---|
| Compound X (free acid form) | 10.0 |
| Monobasic sodium phosphate | 0.3 |
| Dibasic sodium phosphate | 1.1 |
| Polyethylene glycol 400 | 200.0 |
| 1.0 N Sodium hydroxide solution (pH adjustment to 7.0-7.5) | q.s. |
| Water for injection | q.s. ad 1 mL |

| (vi) Aerosol | mg/can |
|---|---|
| Compound X | 20.0 |
| Oleic acid | 10.0 |
| Trichloromonofluoromethane | 5,000.0 |
| Dichlorodifluoromethane | 10,000.0 |
| Dichlorotetrafluoroethane | 5,000.0 |

The above formulations may be obtained by conventional procedures well known in the pharmaceutical art.

### INCORPORATION BY REFERENCE

All of the US and PCT published patent applications and US patents cited herein are incorporated by reference.

### EQUIVALENTS

The invention has been described with reference to various specific and preferred embodiments and techniques. However, it should be understood that many variations and modifications may be made while remaining within the spirit and scope of the invention.
The present application and invention further includes the subject matter of the following numbered clauses:
1. A compound represented by **R^{s}-B**, or a pharmaceutically acceptable salt thereof, wherein:
   **R^{s}-B** is selected from the group consisting of: 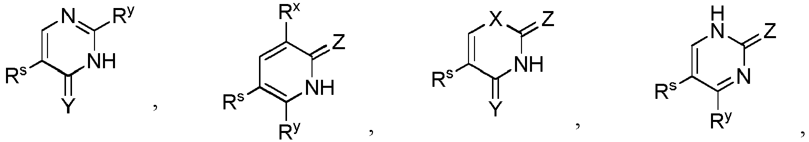 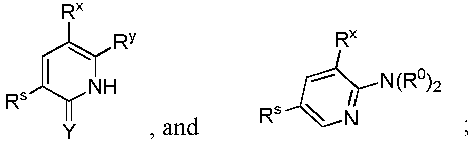
      X is selected from the group consisting of O, S, and NR¹⁰;
         R¹⁰, independently for each occurrence, is selected from the group consisting of H, C₁-C₆alkyl, aryl, heteroaryl, aralkyl, and heteroaralkyl;
      R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
         R⁰, independently for each occurrence, is H or C₁-C₆alkyl;
      R^{y} is selected from the group consisting of H, C₁-C₆alkyl, OR⁰, C₁-C₆alkenyl, C₁-C₆alkynyl, CN, N(R⁰)₂, and halide;
      Y is selected from the group consisting of O and S;
      Z is selected from the group consisting of O and S;
      R^{s} is represented by the formula: 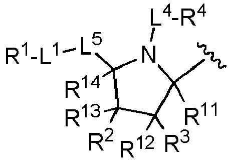
         L¹ and L⁴, each independently, are a bond or -C(R⁰)₂-O-;
         L⁵ is a bond, O, -C(R⁰)₂-O-, -C(R⁰)₂-S-, or -C(R⁰)₂-NH-;
         R¹ is selected from the group consisting of H, C₁-C₆alkyl, aminoacyl, aminothionyl, C₁-C₆acyl, R¹⁰OC(O)-, phosphoryl, and aminophosphoryl;
         R² and R³ are each independently H, halide, azide, C₁-C₆alkyl, C₁-C₆alkenyl, C₁-C₆alkynyl, CN, OR⁰, R¹⁰OC(O)-, or SR⁰;
            or R¹ and R², taken together, or R² and R³, taken together, may be selected from the group consisting of -OC(O)O-, -OC(S)O-, phosphoryl, and C₁-C₆alkylphosphoryl;
            or R² and R³, taken together, may form a bond between the carbon atoms to which they are attached;
         R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
         R¹¹ is selected from the group consisting of H, C₁-C₆alkyl, C₁-C₆alkenyl, C₁-C₆alkynyl, OR⁰, and CN;
         R¹² and R¹³ are each independently selected from the group consisting of H, C₁-C₆alkyl, OR⁰, C₁-C₆alkenyl, C₁-C₆alkynyl, CN, azide, and halide; and
         R¹⁴ is selected from the group consisting of H, C₁-C₆alkyl, OR⁰, C₁-C₆alkynyl, CN, azide, and halide.
2. A compound represented by **R^{s}-B**, or a pharmaceutically acceptable salt thereof, wherein:
   **R^{s}-B** is selected from the group consisting of: 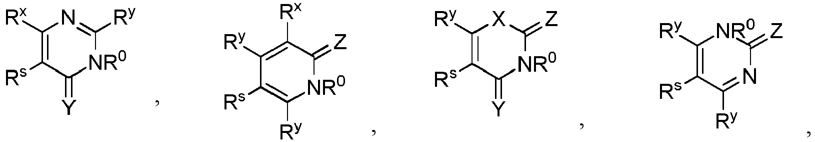 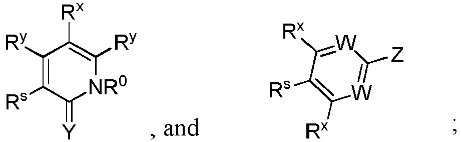
      W, independently for each occurrence, is CR^{x} or N;
      X is selected from the group consisting of O, S, and NR¹⁰;
         R¹⁰, independently for each occurrence, is selected from the group consisting of H, C₁-C₆alkyl, aryl, heteroaryl, aralkyl, and heteroaralkyl;
      R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
         R⁰, independently for each occurrence, is H or C₁-C₆alkyl;
      R^{y} is selected from the group consisting of H, C₁-C₆alkyl, OR⁰, C₁-C₆alkenyl, C₁-C₆alkynyl, CN, N(R⁰)₂, and halide;
      Y is selected from the group consisting of O and S;
      Z is selected from the group consisting of O and S;
      R^{s} is represented by the formula: 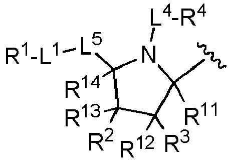
         L¹ and L⁴, each independently, are a bond or -C(R⁰)₂-O-;
         L⁵ is a bond, O, -C(R⁰)₂-O-, -C(R⁰)₂-S-, or -C(R⁰)₂-NH-;
         R¹ is selected from the group consisting of H, C₁-C₆alkyl, aminoacyl, aminothionyl, C₁-C₆acyl, R¹⁰OC(O)-, phosphoryl, and aminophosphoryl;
         R² and R³ are each independently H, halide, azide, C₁-C₆alkyl, C₁-C₆alkenyl, C₁-C₆alkynyl, CN, OR⁰, R¹⁰OC(O)-, or SR⁰;
            or R¹ and R², taken together, or R² and R³, taken together, may be selected from the group consisting of -OC(O)O-, -OC(S)O-, phosphoryl, and C₁-C₆alkylphosphoryl;
            or R² and R³, taken together, may form a bond between the carbon atoms to which they are attached;
         R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
         R¹¹ is selected from the group consisting of H, C₁-C₆alkyl, C₁-C₆alkenyl, C₁-C₆alkynyl, OR⁰, and CN;
         R¹² and R¹³ are each independently selected from the group consisting of H, C₁-C₆alkyl, C₁-C₆alkenyl, C₁-C₆alkynyl, OR⁰, CN, azide, and halide; and
         R¹⁴ is selected from the group consisting of H, C₁-C₆alkyl, OR⁰, C₁-C₆alkynyl, CN, azide, and halide.
3. A compound represented by **R^{s}-B**, or a pharmaceutically acceptable salt thereof, wherein:
   **R^{s}-B** is selected from the group consisting of: 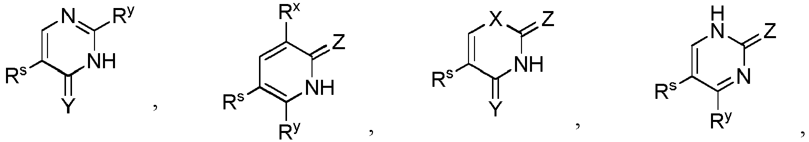 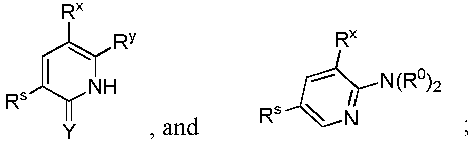
      X is selected from the group consisting of O, S, and NR¹⁰;
         R¹⁰, independently for each occurrence, is selected from the group consisting of H, C₁-C₆alkyl, aryl, heteroaryl, aralkyl, and heteroaralkyl;
      R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
         R⁰, independently for each occurrence, is H or C₁-C₆alkyl;
      R^{y} is selected from the group consisting of H, C₁-C₆alkyl, OR⁰, C₁-C₆alkenyl, C₁-C₆alkynyl, CN, N(R⁰)₂, and halide;
      Y is selected from the group consisting of O and S;
      Z is selected from the group consisting of O and S;
      R⁵ is selected from the group consisting of: 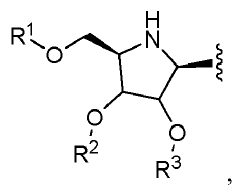 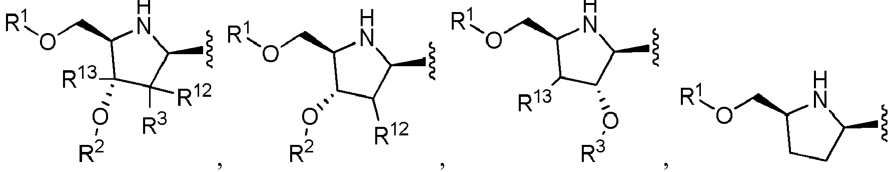 and 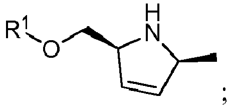
         R¹ is selected from the group consisting of H, C₁-C₆alkyl, aminoacyl, aminothionyl, C₁-C₆acyl, R¹⁰OC(O)-, phosphoryl, and aminophosphoryl;
         R² and R³ are each independently H, C₁-C₆alkyl, C₁-C₆alkenyl, or C₁-C₆alkynyl; and
         R¹² and R¹³ are each independently selected from the group consisting of H, C₁-C₆alkyl, C₁-C₆alkenyl, C₁-C₆alkynyl, OR⁰, CN, azide, and halide.
4. The compound of any one of clauses 1-3, wherein R^{s} is selected from the group consisting of: 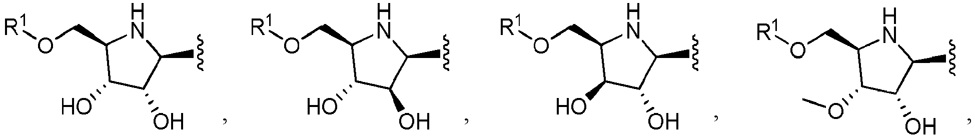 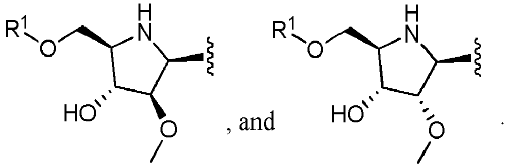
5. The compound of any one of clauses 1-3, wherein R^{s} is selected from the group consisting of: 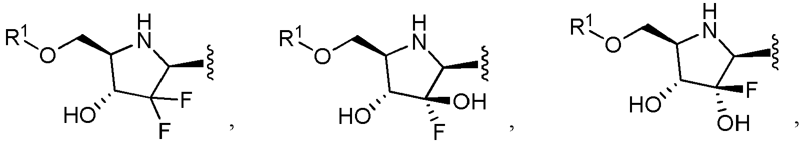 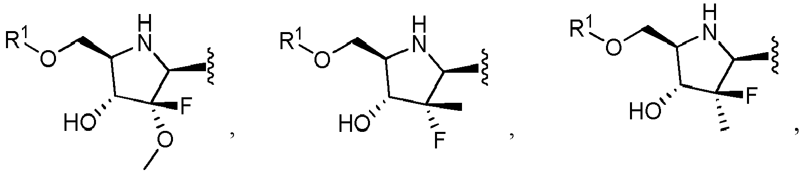 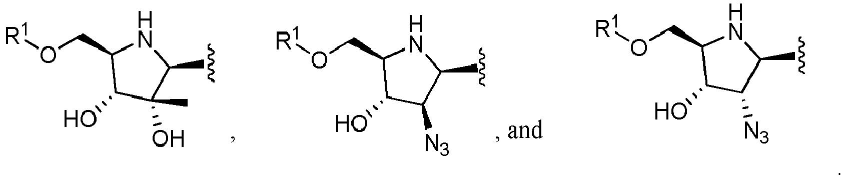
6. The compound of any one of clauses 1-3, wherein R^{s} is selected from the group consisting of: 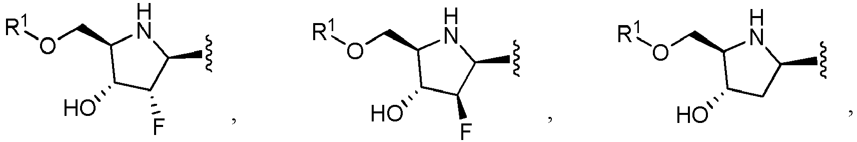 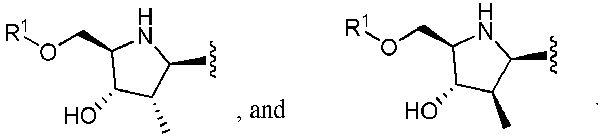
7. The compound of any one of clauses 1-3, wherein R^{s} is selected from the group consisting of: 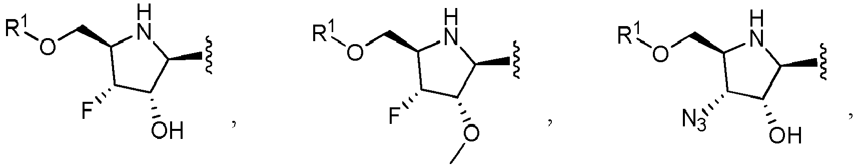 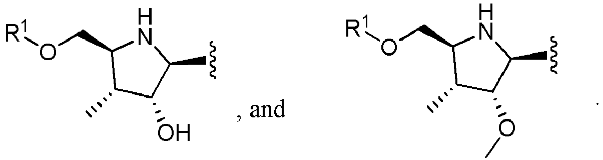
8. The compound of any one of clauses 1-3, wherein R^{s} is selected from the group consisting of: 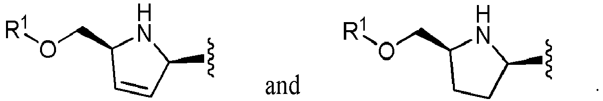
9. The compound of any one of clauses 1-8, wherein R¹ is H, methyl, or ethyl.
10. The compound of any one of clauses 1-8, wherein R¹ is phosphate, pyrophosphate, phosphoramidite, phosphite, or phosphonate.
11. The compound of any one of clauses 1-8, wherein R¹ is phosphoryl or aminophosphoryl.
12. The compound of clause 11, wherein R¹ is aminophosphoryl;
   aminophosphoryl is -P(=O)(OR⁵⁰)NR⁵¹R⁵²;
   R⁵⁰ is selected from the group consisting of H, (C₁-C₆)alkyl, aryl, arylalkyl, heteroaryl, heteroaralkyl, and -(CH₂)ₘSC(=O)C(CH₃)₂CH₂OH;
   m is 1 or 2;
   R⁵¹ is H or C₁-C₆alkyl;
   R⁵² is selected from the group consisting of H, C₁-C₆alkyl, aryl, aralkyl, heteroaryl, heteroaralkyl, and -CR⁶⁰R⁶¹C(=O)OR⁶²;
   R⁶⁰ and R⁶¹ each independently are H or C₁-C₆alkyl; and
   R⁶² is selected from the group consisting of H, C₁-C₆alkyl, aryl, aralkyl, heteroaryl, and heteroaralkyl.
13. The compound of any one of the preceding clauses, wherein **R^{s}-B** is selected from the group consisting of: 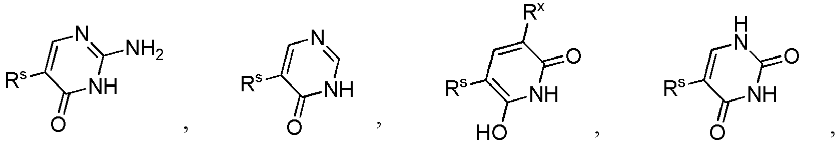 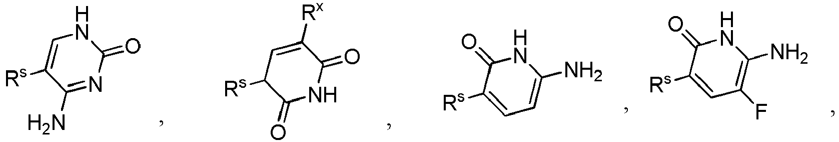 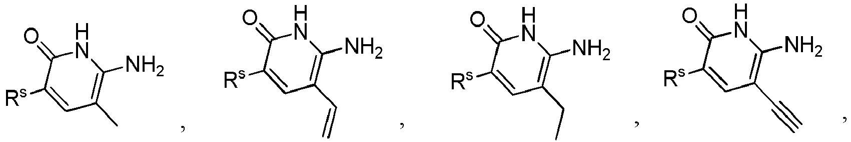 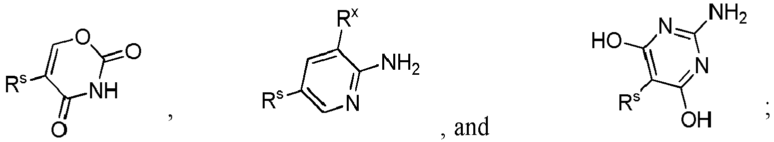 and R^{x} is selected from the group consisting of H, C₁-C₆alkyl, OR⁰, C₁-C₆alkenyl, C₁-C₆alkynyl, CN, NH₂, aminoalkyl, NO₂, azide, halide, aryl, and heteroaryl.
14. The compound of any one of clauses 1-13, wherein R^{x} is F, Cl, Br, or I.
15. The compound of any one of clauses 1-13, wherein R^{x} is H, methyl, ethyl, or propyl.
16. The compound of any one of clauses 1-13, wherein R^{x} is ethenyl or ethynyl.
17. The compound of any one of clauses 1-13, wherein R^{x} is CN, NH₂, or aminoalkyl.
18. A compound represented by **R^{s}-B**, or a pharmaceutically acceptable salt thereof, wherein:
   **R^{s}-B** is selected from the group consisting of: 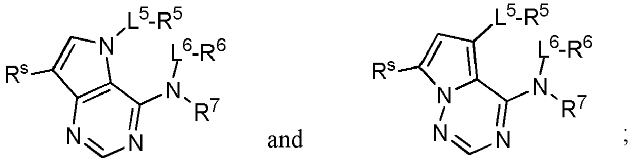
      R^{s} is represented by the formula: 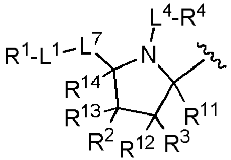
         L¹ is a bond;
         L⁴, L⁵, and L⁶, each independently, are a bond or -C(R⁰)₂-O-;
         L⁷ is O, -CH₂-, -CH(C₁-C₆alkyl)-, -C(R⁰)₂-S-, or -C(R⁰)₂-NH-;
            R⁰, independently for each occurrence, is H or C₁-C₆alkyl;
         R¹ is selected from the group consisting of H, C₁-C₆alkyl, aminoacyl, aminothionyl, C₁-C₆acyl, R¹⁰OC(O)-, phosphoryl, and aminophosphoryl;
         R² and R³ are each independently H, halide, C₁-C₆alkyl, C₁-C₆alkenyl, C₁-C₆alkynyl, CN, OR⁰, R¹⁰OC(O)-, or SR⁰;
            or R¹ and R², taken together, or R² and R³, taken together, may be selected from the group consisting of -OC(O)O-, -OC(S)O-, phosphoryl, and C₁-C₆alkylphosphoryl;
            or R² and R³, taken together, may form a bond between the carbon atoms to which they are attached;
         R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
            R¹⁰, independently for each occurrence, is selected from the group consisting of H, C₁-C₆alkyl, aryl, heteroaryl, aralkyl, and heteroaralkyl;
   R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
   R⁷ is H; or R⁶, R⁷, and the nitrogen to which they are bonded, taken together, represent -N=CR²⁰R²¹;
      R²⁰ and R²¹, each independently, are selected from the group consisting of H, C₁-C₆alkyl, amino, aryl, heteroaryl, aralkyl, and heteroaralkyl;
      R¹¹ is selected from the group consisting of H, C₁-C₆alkyl, C₁-C₆alkenyl, C₁-C₆alkynyl, OR⁰, CN, and halide;
      R¹² and R¹³ are each independently selected from the group consisting of H, C₁-C₆alkyl, C₁-C₆alkenyl, C₁-C₆alkynyl, OR⁰, CN, azide, and halide; and
      R¹⁴ is selected from the group consisting of H, C₁-C₆alkyl, OR⁰, C₁-C₆alkynyl, CN, azide, and halide.
19. A compound represented by **R^{s}-B**, or a pharmaceutically acceptable salt thereof, wherein:
   **R^{s}-B** is selected from the group consisting of: 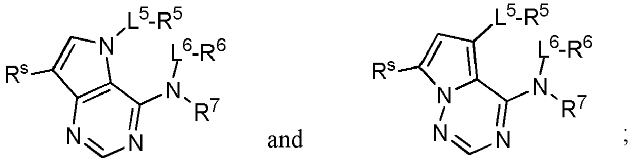
      R^{s} is represented by the formula: 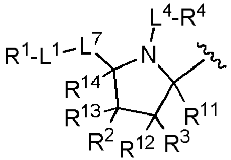
         L¹, L⁴, L⁵, and L⁶, each independently, are a bond or -C(R⁰)₂-O-;
         L⁷ is a bond, O, -C(R⁰)₂-O-, -C(R⁰)₂-S-, or -C(R⁰)₂-NH-;
            R⁰, independently for each occurrence, is H or C₁-C₆alkyl;
         R¹ is selected from the group consisting of H, C₁-C₆alkyl, aminoacyl, aminothionyl, C₁-C₆acyl, R¹⁰OC(O)-, phosphoryl, and aminophosphoryl;
         R² and R³ are each independently H, halide, C₁-C₆alkyl, C₁-C₆alkenyl, C₁-C₆alkynyl, CN, OR⁰, R¹⁰OC(O)-, or SR⁰;
            or R¹ and R², taken together, or R² and R³, taken together, may be selected from the group consisting of -OC(O)O-, -OC(S)O-, phosphoryl, and C₁-C₆alkylphosphoryl;
            or R² and R³, taken together, may form a bond between the carbon atoms to which they are attached;
         R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
            R¹⁰, independently for each occurrence, is selected from the group consisting of H, C₁-C₆alkyl, aryl, heteroaryl, aralkyl, and heteroaralkyl;
      R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
      R⁷ is H; or R⁶, R⁷, and the nitrogen to which they are bonded, taken together, represent -N=CR²⁰R²¹;
         R²⁰ and R²¹, each independently, are selected from the group consisting of H, C₁-C₆alkyl, amino, aryl, heteroaryl, aralkyl, and heteroaralkyl;
         R¹¹ is selected from the group consisting of C₁-C₆alkyl, C₁-C₆alkenyl, C₁-C₆alkynyl, OR⁰, and CN;
         R¹² and R¹³ are each independently selected from the group consisting of H, C₁-C₆alkyl, C₁-C₆alkenyl, C₁-C₆alkynyl, OR⁰, CN, azide, and halide; and
         R¹⁴ is selected from the group consisting of H, C₁-C₆alkyl, OR⁰, C₁-C₆alkynyl, CN, azide, and halide.
20. A compound represented by **R^{s}-B**, or a pharmaceutically acceptable salt thereof, wherein:
   **R^{s}-B** is selected from the group consisting of: 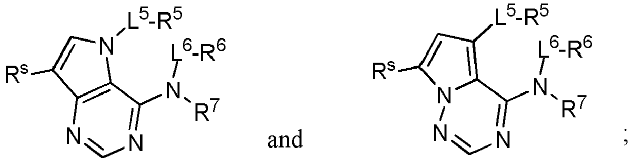
      R^{s} is represented by the formula: 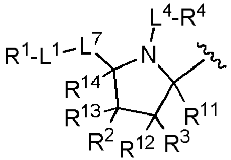
         L¹, L⁴, L⁵, and L⁶, each independently, are a bond or -C(R⁰)₂-O-;
         L⁷ is a bond, O, -C(R⁰)₂-O-, -C(R⁰)₂-S-, or -C(R⁰)₂-NH-;
            R⁰, independently for each occurrence, is H or C₁-C₆alkyl;
         R¹ is selected from the group consisting of H, C₁-C₆alkyl, aminoacyl, aminothionyl, C₁-C₆acyl, R¹⁰OC(O)-, phosphoryl, and aminophosphoryl;
         R² is H, halide, C₁-C₆alkyl, C₁-C₆alkenyl, C₁-C₆alkynyl, CN, OR⁰, R¹⁰OC(O)-, or SR⁰;
         R³ is H, halide, C₁-C₆alkyl, C₁-C₆alkenyl, C₁-C₆alkynyl, CN, R¹⁰OC(O)-, or SR⁰;
            or R¹ and R², taken together, or R² and R³, taken together, may be selected from the group consisting of -OC(O)O-, -OC(S)O-, phosphoryl, and C₁-C₆alkylphosphoryl;
            or R² and R³, taken together, may form a bond between the carbon atoms to which they are attached;
         R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
            R¹⁰, independently for each occurrence, is selected from the group consisting of H, C₁-C₆alkyl, aryl, heteroaryl, aralkyl, and heteroaralkyl;
      R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
      R⁷ is H; or R⁶, R⁷, and the nitrogen to which they are bonded, taken together, represent -N=CR²⁰R²¹;
         R²⁰ and R²¹, each independently, are selected from the group consisting of H, C₁-C₆alkyl, amino, aryl, heteroaryl, aralkyl, and heteroaralkyl;
         R¹¹ is selected from the group consisting of H, C₁-C₆alkyl, C₁-C₆alkenyl, C₁-C₆alkynyl, OR⁰, and CN;
         R¹² is selected from the group consisting of H, C₁-C₆alkyl, C₁-C₆alkenyl, C₁-C₆alkynyl, CN, azide, and halide;
         R¹³ is selected from the group consisting of H, C₁-C₆alkyl, C₁-C₆alkenyl, C₁-C₆alkynyl, OR⁰, CN, azide, and halide; and
         R¹⁴ is selected from the group consisting of H, C₁-C₆alkyl, OR⁰, C₁-C₆alkynyl, CN, azide, and halide.
21. A compound represented by **R^{s}-B**, or a pharmaceutically acceptable salt thereof, wherein:
   **R^{s}-B** is selected from the group consisting of: 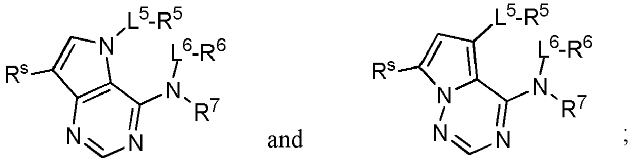
      R^{s} is represented by the formula: 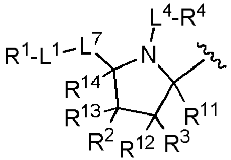
         L¹, L⁴, L⁵, and L⁶, each independently, are a bond or -C(R⁰)₂-O-;
         L⁷ is a bond, O, -C(R⁰)₂-O-, -C(R⁰)₂-S-, or -C(R⁰)₂-NH-;
            R⁰, independently for each occurrence, is H or C₁-C₆alkyl;
         R¹ is selected from the group consisting of H, C₁-C₆alkyl, aminoacyl, aminothionyl, C₁-C₆acyl, R¹⁰OC(O)-, phosphoryl, and aminophosphoryl;
         R² is H, halide, C₁-C₆alkyl, C₁-C₆alkenyl, C₁-C₆alkynyl, CN, R¹⁰OC(O)-, or SR⁰;
         R³ is H, halide, C₁-C₆alkyl, C₁-C₆alkenyl, C₁-C₆alkynyl, CN, OR⁰, R¹⁰OC(O)-, or SR⁰;
            or R¹ and R², taken together, or R² and R³, taken together, may be selected from the group consisting of -OC(O)O-, -OC(S)O-, phosphoryl, and C₁-C₆alkylphosphoryl;
            or R² and R³, taken together, may form a bond between the carbon atoms to which they are attached;
         R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
            R¹⁰, independently for each occurrence, is selected from the group consisting of H, C₁-C₆alkyl, aryl, heteroaryl, aralkyl, and heteroaralkyl;
      R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or R⁵ may be absent;
      R⁷ is H; or R⁶, R⁷, and the nitrogen to which they are bonded, taken together, represent -N=CR²⁰R²¹;
         R²⁰ and R²¹, each independently, are selected from the group consisting of H, C₁-C₆alkyl, amino, aryl, heteroaryl, aralkyl, and heteroaralkyl;
         R¹¹ is selected from the group consisting of H, C₁-C₆alkyl, C₁-C₆alkenyl, C₁-C₆alkynyl, OR⁰, and CN;
         R¹² is selected from the group consisting of C₁-C₆alkyl, OR⁰, alkynyl, CN, azide, and halide;
         R¹³ is selected from the group consisting of H, C₁-C₆alkyl, C₁-C₆alkenyl, C₁-C₆alkynyl, CN, azide, and halide; and
         R¹⁴ is selected from the group consisting of H, C₁-C₆alkyl, OR⁰, C₁-C₆alkynyl, CN, azide, and halide.
22. A compound represented by **R^{s}-B**, or a pharmaceutically acceptable salt thereof, wherein:
   **R^{s}-B** is selected from the group consisting of: 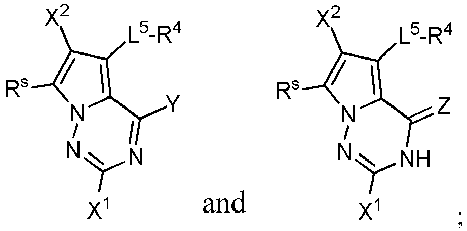
      R^{s} is selected from the group consisting of: 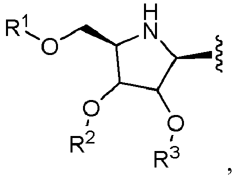 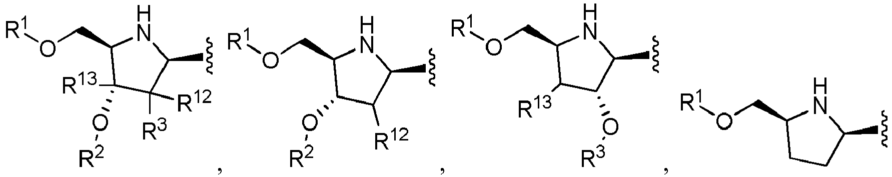 and 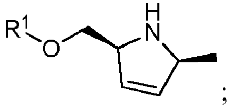
         R¹ is selected from the group consisting of H, C₁-C₆alkyl, aminoacyl, aminothionyl, C₁-C₆acyl, R¹⁰OC(O)-, phosphoryl, and aminophosphoryl;
         R² and R³ are each independently H, C₁-C₆alkyl, C₁-C₆alkenyl, or C₁-C₆alkynyl;
            R¹⁰, independently for each occurrence, is selected from the group consisting of H, C₁-C₆alkyl, aryl, heteroaryl, aralkyl, and heteroaralkyl;
         R¹² and R¹³ are each independently selected from the group consisting of H, C₁-C₆alkyl, C₁-C₆alkenyl, C₁-C₆alkynyl, OR⁰, CN, azide, and halide;
         L⁵ is a bond or -C(R⁰)₂-O-;
            R⁰, independently for each occurrence, is H or C₁-C₆alkyl;
         R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
      X¹ is selected from the group consisting of H, N(R⁰)₂, SH, thioalkyl, OR⁰, alkenyl, alkynyl, CN, and halide;
      X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
      Y is selected from the group consisting of H, OR⁰, N(R⁵)(R⁶), SH, thioalkyl, O-aryl, O-heteroaryl, and halide;
      Z is selected from the group consisting of O and S;
      R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
         R⁵, R⁶, and the nitrogen to which they are bonded, taken together,
         represent -N=CR²⁰R²¹; and
         R²⁰ and R²¹, each independently, are selected from the group consisting of H, C₁-C₆alkyl, amino, aryl, heteroaryl, aralkyl, and heteroaralkyl.
23. A compound represented by **R^{s}-B**, or a pharmaceutically acceptable salt thereof, wherein:
   **R^{s}-B** is selected from the group consisting of: 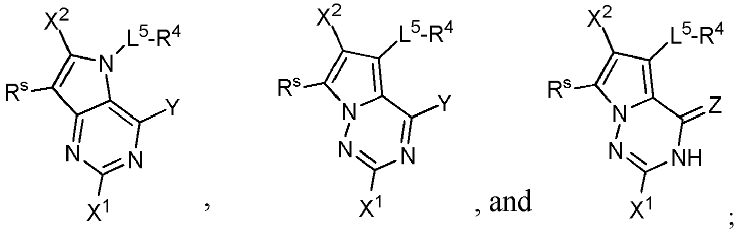
      R^{s} is selected from the group consisting of: 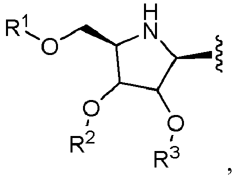 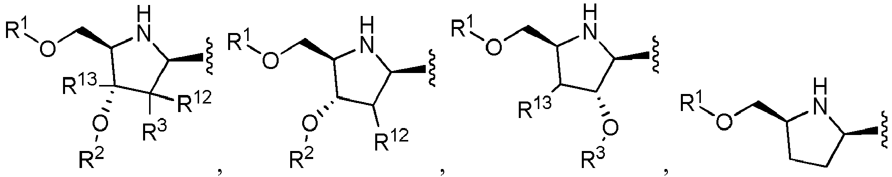 and 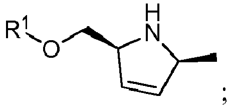
         R¹ is selected from the group consisting of H, C₁-C₆alkyl, aminoacyl, aminothionyl, C₁-C₆acyl, R¹⁰OC(O)-, phosphoryl, and aminophosphoryl;
         R² and R³ are each independently H, C₁-C₆alkyl, C₁-C₆alkenyl, or C₁-C₆alkynyl;
            R¹⁰, independently for each occurrence, is selected from the group consisting of H, C₁-C₆alkyl, aryl, heteroaryl, aralkyl, and heteroaralkyl;
         R¹² and R¹³ are each independently selected from the group consisting of H, C₁-C₆alkyl, C₁-C₆alkenyl, C₁-C₆alkynyl, OR⁰, CN, azide, and halide;
      L⁵ is a bond or -C(R⁰)₂-O-;
         R⁰, independently for each occurrence, is H or C₁-C₆alkyl;
      R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
      X¹ is selected from the group consisting of H, N(R⁰)₂, SH, thioalkyl, OR⁰, alkenyl, alkynyl, CN, and halide;
      X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
      Y is selected from the group consisting of H, OR⁰, N(R⁵)(R⁶), SH, thioalkyl, O-aryl, O-heteroaryl, and halide;
      Z is selected from the group consisting of O and S; and
         R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl, provided that both R⁵ and R⁶ are not H.
24. A compound represented by **R^{s}-B**, or a pharmaceutically acceptable salt thereof, wherein:
   **R^{s}-B** is selected from the group consisting of: 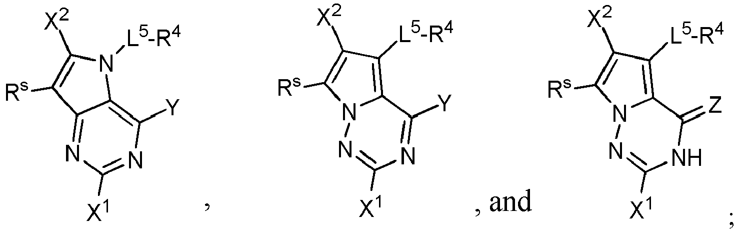
      R^{s} is selected from the group consisting of: 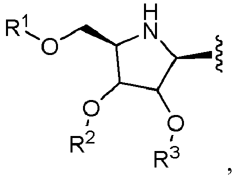 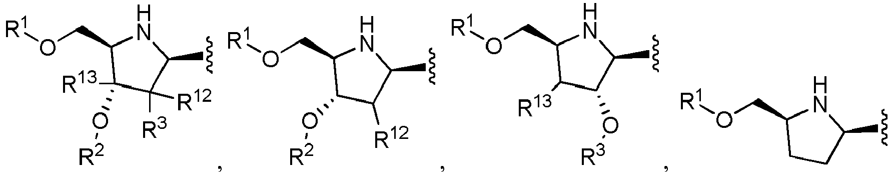 and 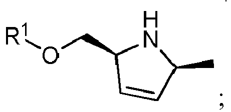
         R¹ is selected from the group consisting of H, C₁-C₆alkyl, aminoacyl, aminothionyl, C₁-C₆acyl, R¹⁰OC(O)-, phosphoryl, and aminophosphoryl;
            R¹⁰, independently for each occurrence, is selected from the group consisting of H, C₁-C₆alkyl, aryl, heteroaryl, aralkyl, and heteroaralkyl;
         R² and R³ are each independently H, C₁-C₆alkyl, C₁-C₆alkenyl, or C₁-C₆alkynyl;
         R¹² and R¹³ are each independently selected from the group consisting of H, C₁-C₆alkyl, C₁-C₆alkenyl, C₁-C₆alkynyl, OR⁰, CN, azide, and halide;
            R⁰, independently for each occurrence, is H or C₁-C₆alkyl;
      L⁵ is a bond or -C(R⁰)₂-O-;
      R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
      X¹ is selected from the group consisting of H, N(R⁰)₂, SH, thioalkyl, OR⁰, alkenyl, alkynyl, CN, and halide;
      X² is selected from the group consisting of N(R⁰)₂, SH, thioalkyl, OR⁰, O-aryl, O-heteroaryl, alkenyl, alkynyl, C₁-C₆acyl, carboxyl, CN, azide, and halide;
      Y is selected from the group consisting of H, OR⁰, N(R⁵)(R⁶), SH, thioalkyl, O-aryl, O-heteroaryl, and halide;
      Z is selected from the group consisting of O and S;
         R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
         R⁵, R⁶, and the nitrogen to which they are bonded, taken together,
         represent -N=CR²⁰R²¹; and
         R²⁰ and R²¹, each independently, are selected from the group consisting of H, C₁-C₆alkyl, amino, aryl, heteroaryl, aralkyl, and heteroaralkyl.
25. A compound represented by **R^{s}-B**, or a pharmaceutically acceptable salt thereof, wherein:
   **R^{s}-B** is selected from the group consisting of: 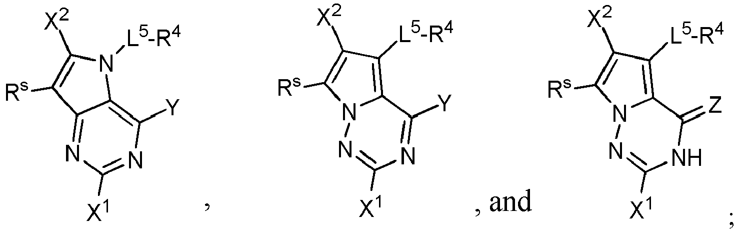
      R^{s} is selected from the group consisting of: 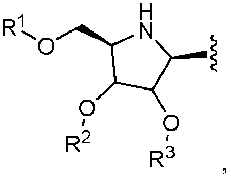 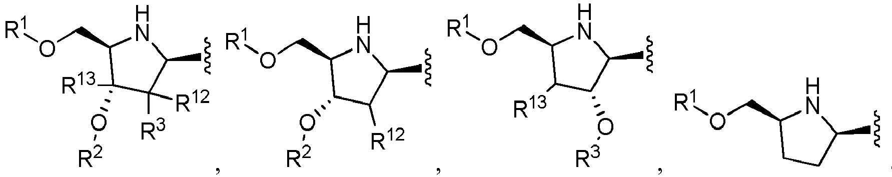 and 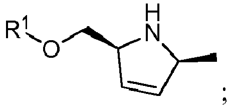
         R¹ is selected from the group consisting of H, C₁-C₆alkyl, aminoacyl, aminothionyl, C₁-C₆acyl, R¹⁰OC(O)-, phosphoryl, and aminophosphoryl;
         R² and R³ are each independently H, C₁-C₆alkyl, C₁-C₆alkenyl, or C₁-C₆alkynyl;
            R¹⁰, independently for each occurrence, is selected from the group consisting of H, C₁-C₆alkyl, aryl, heteroaryl, aralkyl, and heteroaralkyl;
         R¹² and R¹³ are each independently selected from the group consisting of H, C₁-C₆alkyl, C₁-C₆alkenyl, C₁-C₆alkynyl, OR⁰, CN, azide, and halide;
            R⁰, independently for each occurrence, is H or C₁-C₆alkyl;
      L⁵ is a bond or -C(R⁰)₂-O-;
      R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
      X¹ is selected from the group consisting of N(R⁰)₂, SH, thioalkyl, OR⁰, alkenyl, alkynyl, CN, and halide;
      X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
      Y is selected from the group consisting of H, OR⁰, N(R⁵)(R⁶), SH, thioalkyl, O-aryl, O-heteroaryl, and halide;
      Z is selected from the group consisting of O and S;
         R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
         R⁵, R⁶, and the nitrogen to which they are bonded, taken together,
         represent -N=CR²⁰R²¹; and
         R²⁰ and R²¹, each independently, are selected from the group consisting of H, C₁-C₆alkyl, amino, aryl, heteroaryl, aralkyl, and heteroaralkyl.
26. The compound of any one of clauses 18-25, wherein R^{s} is selected from the group consisting of: 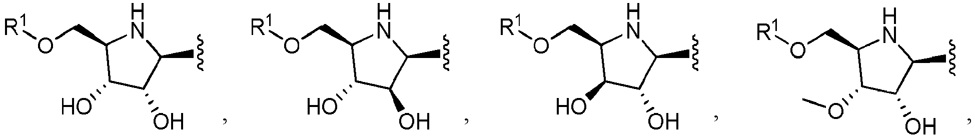 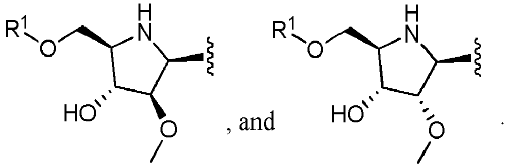
27. The compound of any one of clauses 18-25, wherein R^{s} is selected from the group consisting of: 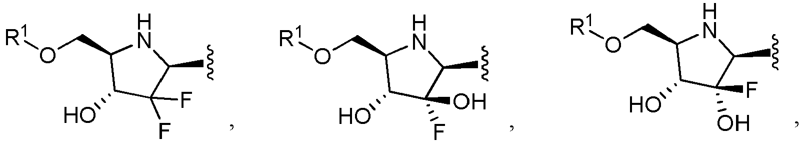 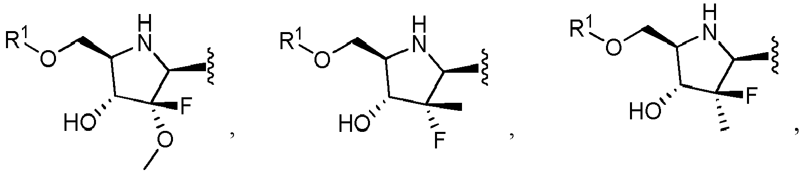 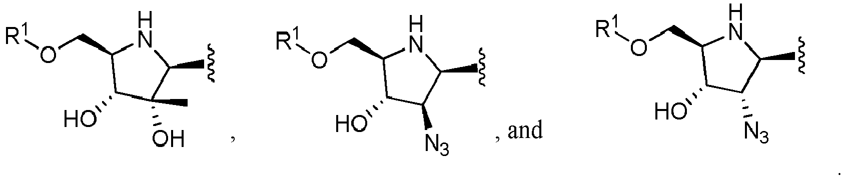
28. The compound of any one of clauses 18-25, wherein R^{s} is selected from the group consisting of: 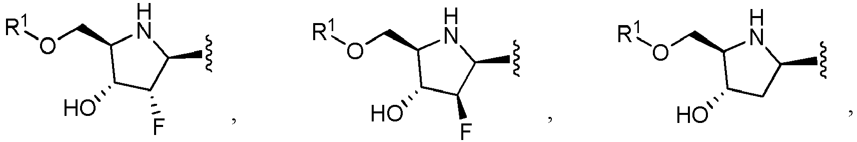 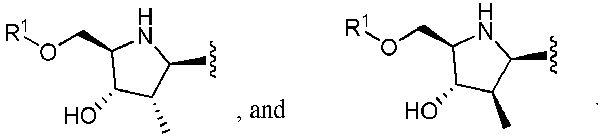
29. The compound of any one of clauses 18-25, wherein R^{s} is selected from the group consisting of: 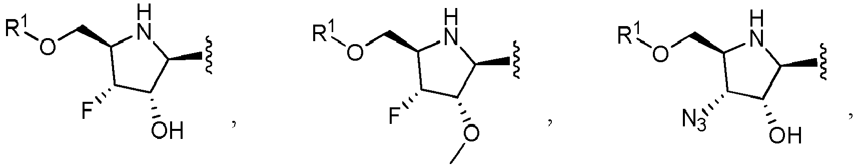 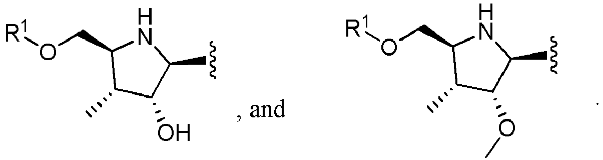
30. The compound of any one of clauses 18-25, wherein R^{s} is selected from the group consisting of: 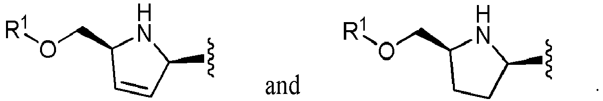
31. The compound of any one of clauses 18-21, wherein L⁵-R⁵ is H.
32. The compound of any one of clauses 18-21, wherein L⁴-R⁴ is H.
33. The compound of any one of clauses 18-21, wherein L⁷ is -CH(C₁-C₆alkyl)-.
34. The compound of any one of clauses 18, 20, or 21, wherein R¹¹, R¹², R¹³, and R¹⁴ are H.
35. The compound of clause 19, wherein R¹², R¹³, and R¹⁴ are H.
36. The compound of any one of clauses 18-21, wherein R¹ is selected from the group consisting of phosphate, pyrophosphate, phosphoramidite, phosphite, and phosphonate.
37. The compound of any one of clauses 18-21, wherein R¹ is phosphoryl or aminophosphoryl.
38. The compound of clause 36, wherein R¹ is aminophosphoryl;
   aminophosphoryl is -P(=O)(OR⁵⁰)NR⁵¹R⁵²;
   R⁵⁰ is selected from the group consisting of H, (C₁-C₆)alkyl, aryl, arylalkyl, heteroaryl, heteroaralkyl, and -(CH₂)ₘSC(=O)C(CH₃)₂CH₂OH;
   m is 1 or 2;
   R⁵¹ is H or C₁-C₆alkyl;
   R⁵² is selected from the group consisting of H, C₁-C₆alkyl, aryl, aralkyl, heteroaryl, heteroaralkyl, and -CR⁶⁰R⁶¹C(=O)OR⁶²;
   R⁶⁰ and R⁶¹ each independently are H or C₁-C₆alkyl; and
   R⁶² is selected from the group consisting of H, C₁-C₆alkyl, aryl, aralkyl, heteroaryl, and heteroaralkyl.
39. The compound of any one of the preceding clauses, wherein R⁵⁰ is H.
40. The compound of any one of the preceding clauses, wherein R⁵⁰ is aryl.
41. The compound of any one of the preceding clauses, wherein R⁵⁰ is - (CH₂)ₘSC(=O)C(CH₃)₂CH₂OH.
42. The compound of any one of the preceding clauses, wherein m is 2.
43. The compound of any one of the preceding clauses, wherein R⁵¹ is H.
44. The compound of any one of the preceding clauses, wherein R⁵² is aralkyl.
45. The compound of any one of the preceding clauses, wherein R⁵² is - CR⁶⁰R⁶¹C(=O)OR⁶².
46. The compound of any one of the preceding clauses, wherein R⁶⁰ is H; R⁶¹ is (C₁-C₆)alkyl; and R⁶² is (C₁-C₆)alkyl.
47. The compound of any one of the preceding clauses, wherein R¹ is selected from the group consisting of: 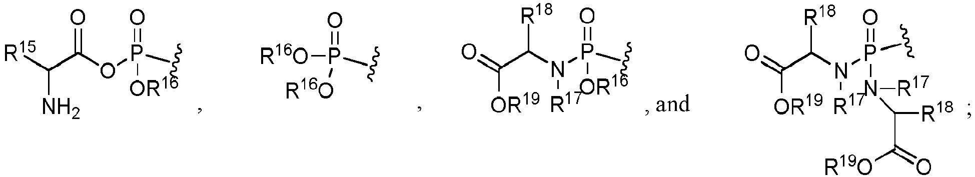 wherein:
   R¹⁵ is selected from the group consisting of H, C₁-C₆alkyl, C₁-C₆haloalkyl, aminoalkyl,
      thioalkyl, C₃-C₈cycloalkyl, C₂-C₇heterocyclyl, aryl, heteroaryl, aralkyl, heteroaralkyl, and naturally-occurring amino acid side chains;
   R¹⁶ is, independently for each occurrence, selected from the group consisting of H,
   C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl; or R¹⁶ taken together with the oxygen to which it is bonded may be a naturally-occurring amino acid;
   R¹⁷ is, independently for each occurrence, selected from the group consisting of H,
   C₁-C₆alkyl, C₁-C₆haloalkyl, C₃-C₈cycloalkyl, C₂-C₇heterocyclyl, aryl, heteroaryl, aralkyl, heteroaralkyl, and acyl;
   R¹⁸ is, independently for each occurrence, selected from the group consisting of H,
   C₁-C₆alkyl, C₁-C₆haloalkyl, aminoalkyl, thioalkyl, C₃-C₈cycloalkyl, C₂-C₇heterocyclyl, aryl, heteroaryl, aralkyl, heteroaralkyl, and naturally-occurring amino acid side chains; or R¹⁷ and R¹⁸, taken together, may form a 4-, 5-, or 6-membered ring; and
   R¹⁹ is, independently for each occurrence, selected from the group consisting of H,
   C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl.
48. A compound selected from the group consisting of: 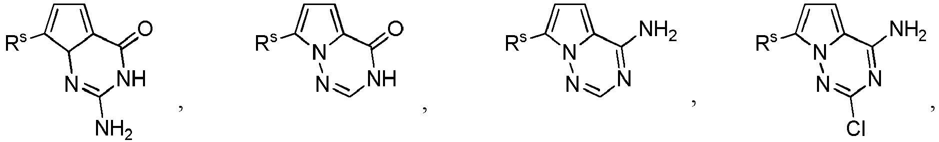 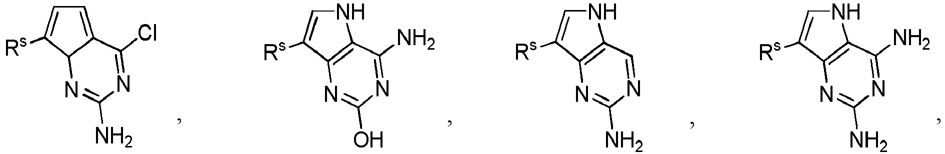 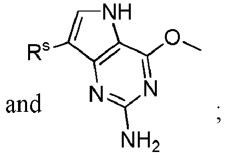 wherein R^{s} is selected from the group consisting of: 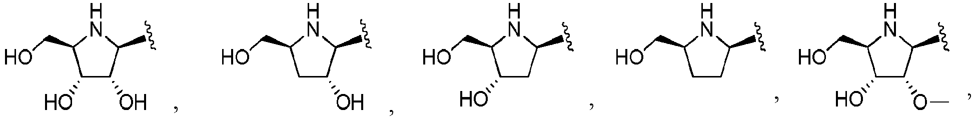 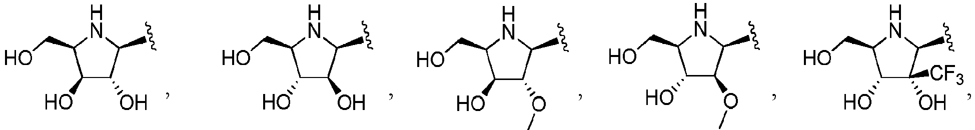 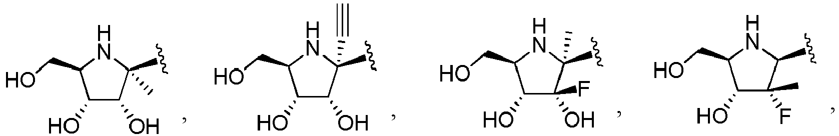 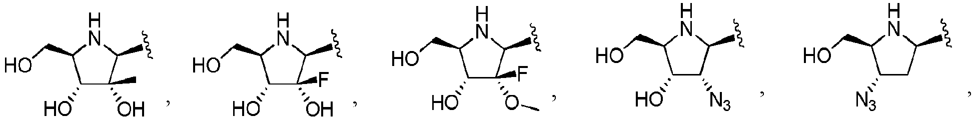 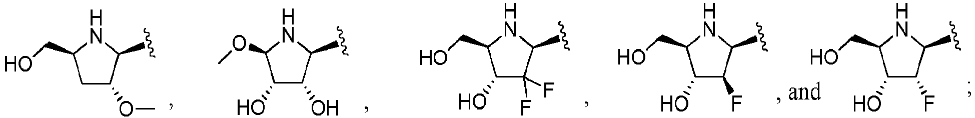 or a pharmaceutically acceptable salt thereof
49. A compound, or a pharmaceutically acceptable salt thereof, selected from the group consisting of: 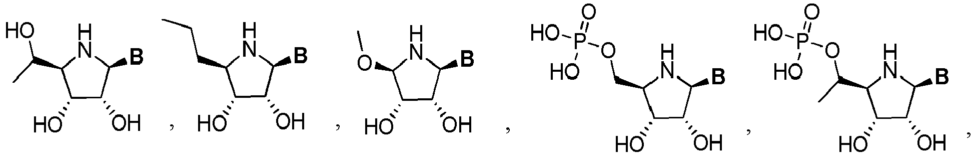 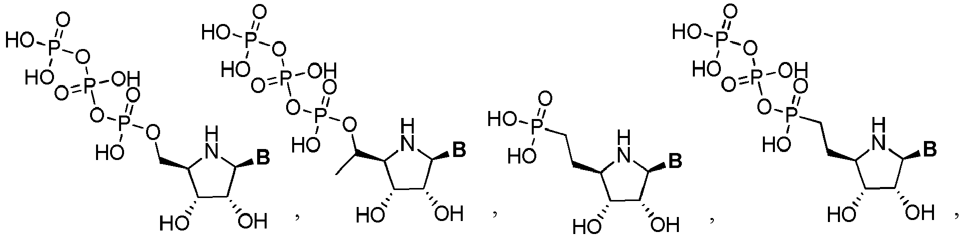 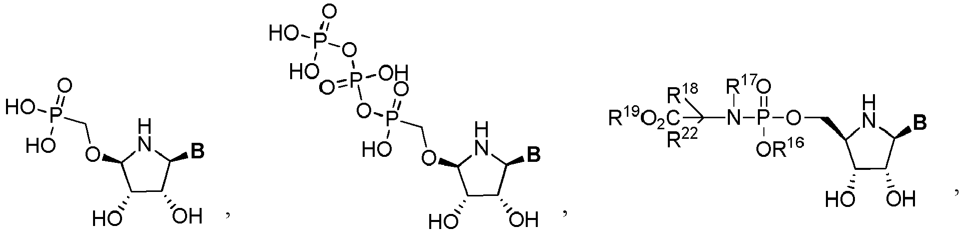 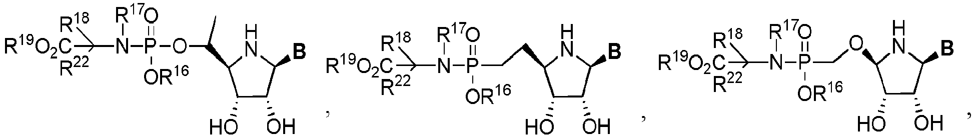 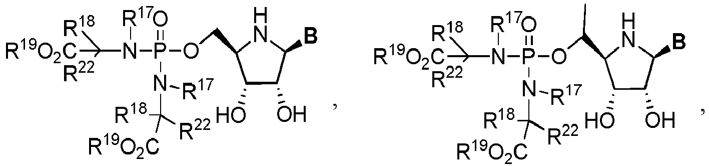 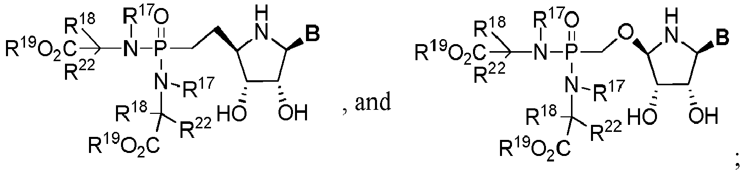 wherein:
   R¹⁶ is, independently for each occurrence, selected from the group consisting of H,
   C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl; or R¹⁶ taken together with the oxygen to which it is bonded may be a naturally-occurring amino acid;
   R¹⁸ is, independently for each occurrence, selected from the group consisting of H,
   C₁-C₆alkyl, C₁-C₆haloalkyl, aminoalkyl, thioalkyl, C₃-C₈cycloalkyl, C₂-C₇heterocyclyl, aryl, heteroaryl, aralkyl, heteroaralkyl, and naturally-occurring amino acid side chains;
   R¹⁹ is, independently for each occurrence, selected from the group consisting of H,
   C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl;
   R²² is, independently for each occurrence, selected from the group consisting of H,
   C₁-C₆alkyl, C₁-C₆haloalkyl, C₃-C₈cycloalkyl, C₂-C₇heterocyclyl, aryl, heteroaryl, aralkyl, and heteroaralkyl; or R¹⁷ and R²², taken together, may form a 4-, 5-, or 6-membered ring; and
   **B** is selected from the group consisting of: 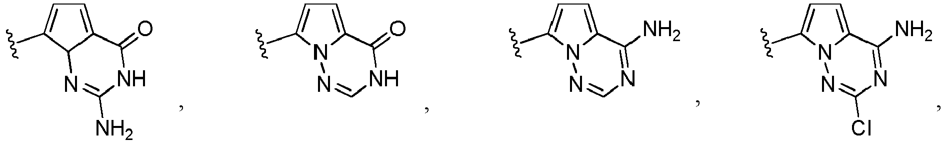 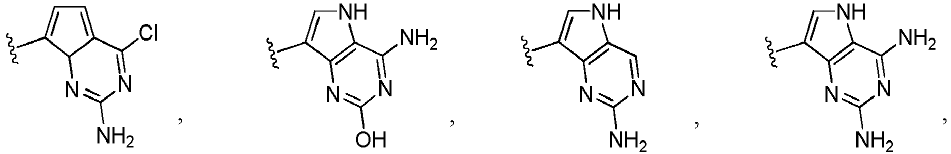 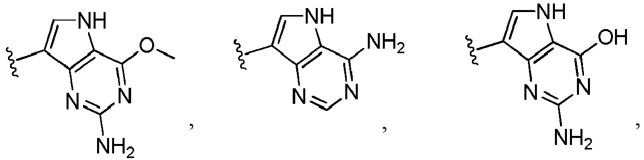 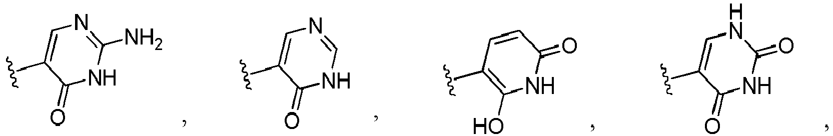 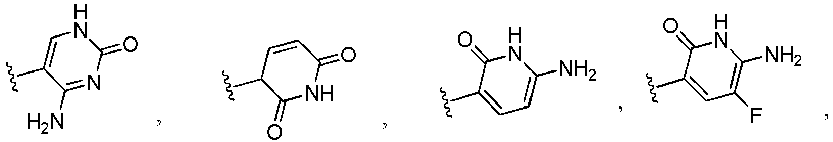 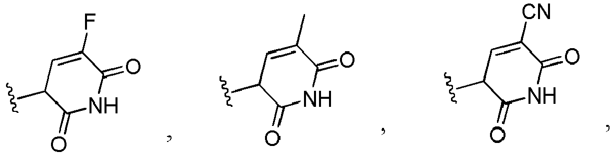 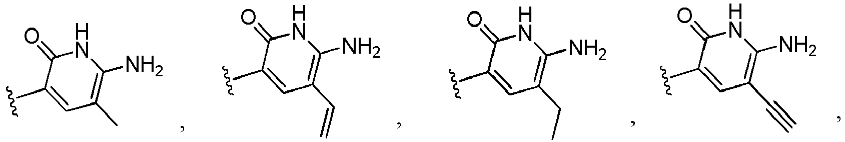 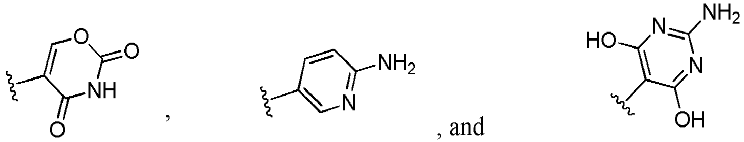
50. A compound selected from the group consisting of: 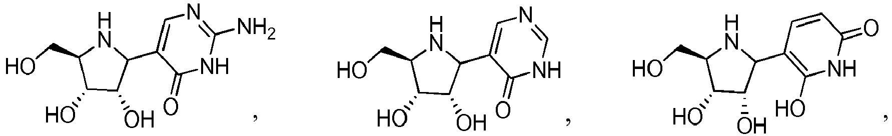 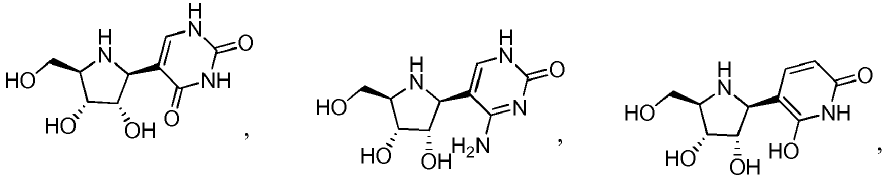 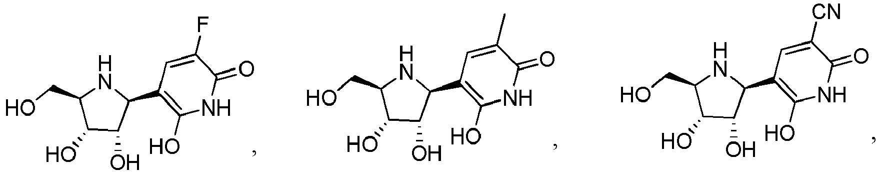 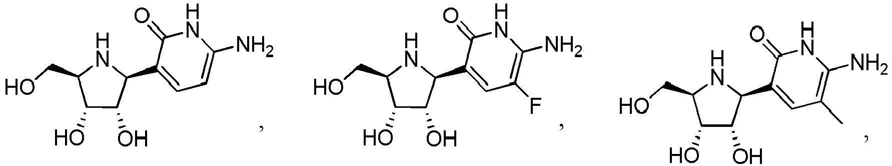 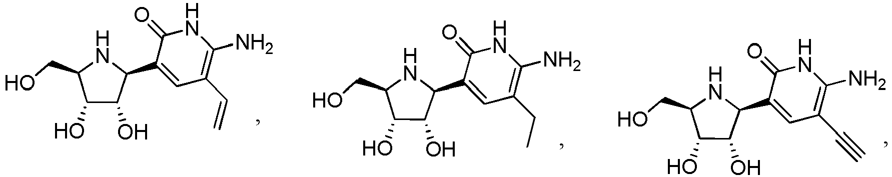 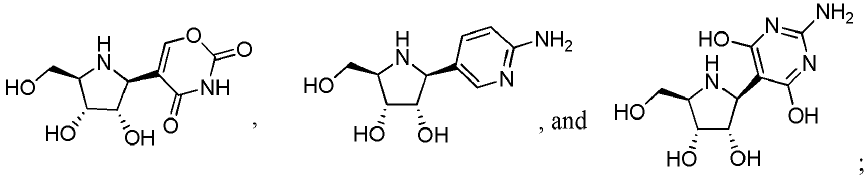 and pharmaceutically acceptable salts thereof.
51. A compound selected from the group consisting of: 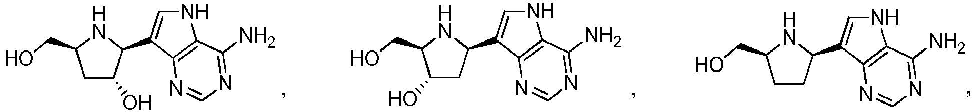 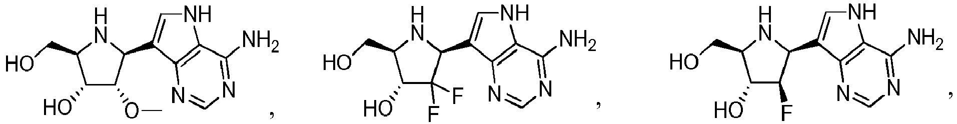 and 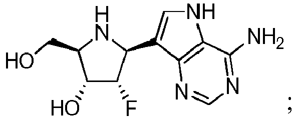 and pharmaceutically acceptable salts thereof.
52. A pharmaceutical composition, comprising a compound of any one of clauses 1-51, or a pharmaceutically acceptable salt thereof, and a pharmaceutically acceptable carrier.
53. A method of inhibiting replication of a virus, comprising
   contacting a virus with an effective amount of a compound of any one of clauses 1-51.
54. A method of treating a viral infection in a subject, comprising administering to a subject in need thereof an effective amount of a compound of any one of clauses 1-51.
55. The method of clause 53 or 54, wherein the virus is selected from the group consisting of RNA viruses.
56. The method of clause 55, wherein the virus is selected from the group consisting of orthomyxoviridae, paramyxoviridae, arenaviridae, bunyaviridae, flaviviridae, filoviridae, togaviridae, picornaviridae, and coronaviridae.
57. The method of clause 55, wherein the virus is selected from the group consisting of adenovirus, rhinovirus, hepatitis A virus, hepatitis C virus, polio virus, measles virus, Ebola virus, Coxsackie virus, West Nile virus, smallpox virus, yellow fever virus, Dengue Fever virus, influenza A virus, influenza B virus, lassa virus, lymphocytic choriomeningitis virus, Junin virus, machuppo virus, guanarito virus, hantavirus, Rift Valley Fever virus, La Crosse virus, California encephalitis virus, Crimean-Congo virus, Marburg virus, Japanese encephalitis virus, Kyasanur Forest virus, Venezuelan equine encephalitis virus, Eastern equine encephalitis virus, Western equine encephalitis virus, severe acute respiratory syndrome (SARS) virus, parainfluenza virus, respiratory syncytial virus, Punta Toro virus, Tacaribe virus, and Pichinde virus.
58. The method of clause 57, wherein the virus is selected from the group consisting of adenovirus, Dengue Fever virus, Marburg virus, influenza A virus, influenza B virus, Junin virus, measles virus, parainfluenza virus, Pichinde virus, Punta Toro virus, respiratory syncytial virus, rhinovirus, Rift Valley Fever virus, SARS virus, Tacaribe virus, Venezuelan equine encephalitis virus, West Nile virus, and yellow fever virus.
59. The method of clause 58, wherein the virus is selected from the group consisting of Ebola virus, yellow fever virus, Marburg virus, influenza A virus, and influenza B virus.

## Claims

1. A compound selected from the group consisting of: 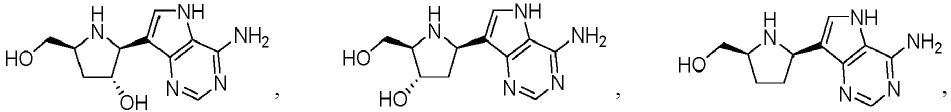 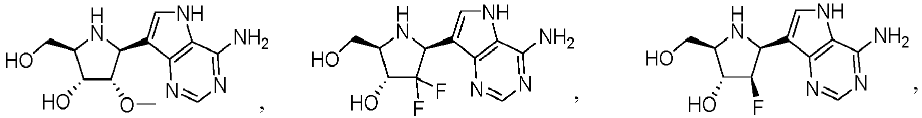 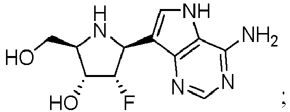 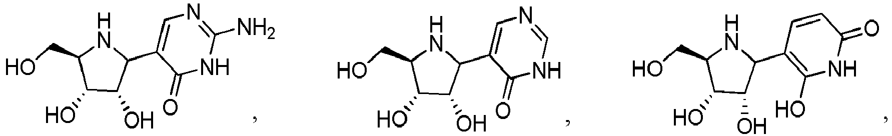 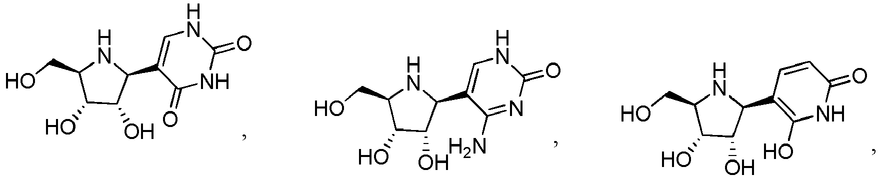 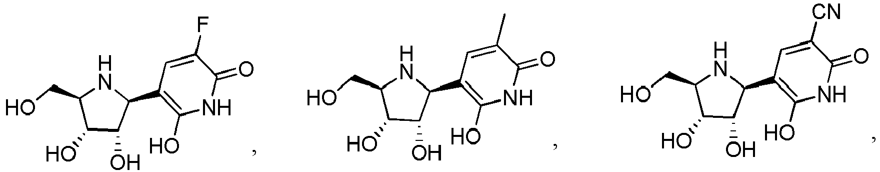 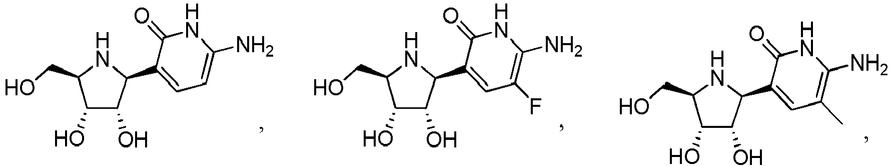 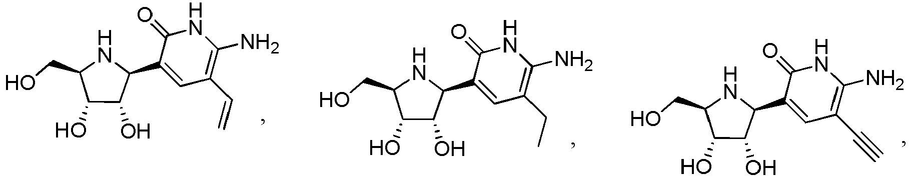 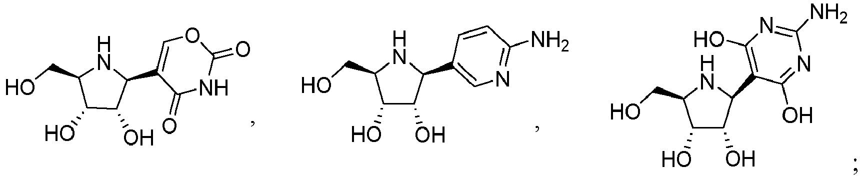 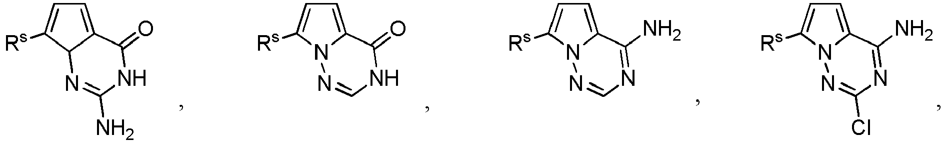 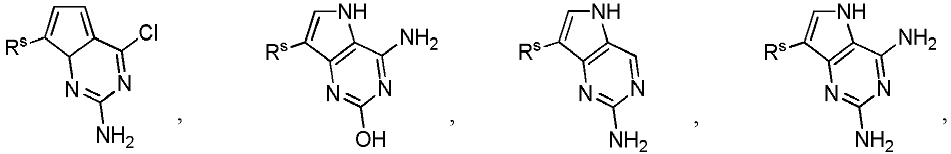 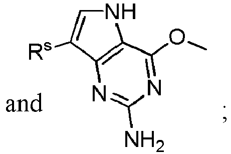 wherein R^{s} is selected from the group consisting of: 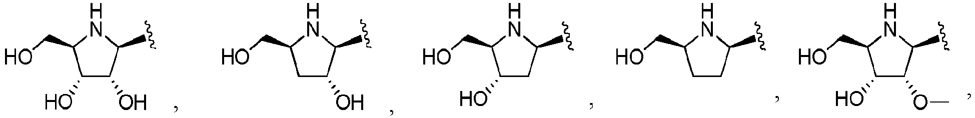 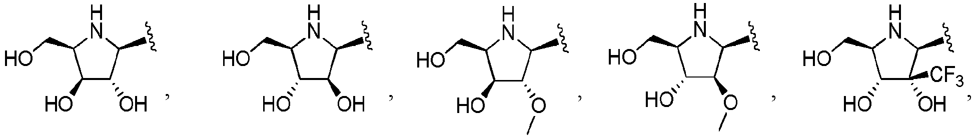 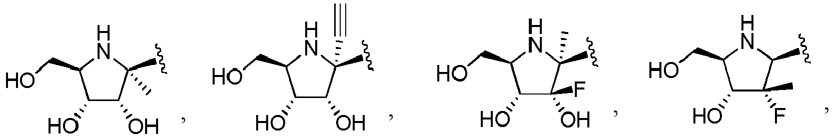 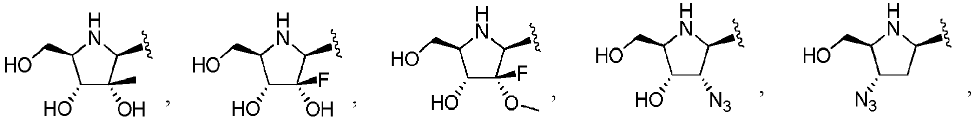 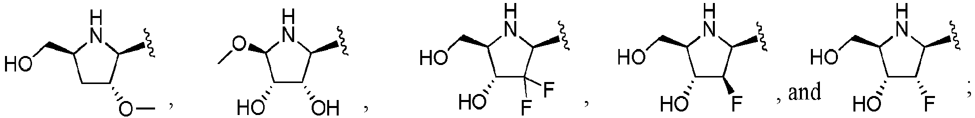 and pharmaceutically acceptable salts thereof.

2. A compound represented by:
(I) **R^{s}-B**, or a pharmaceutically acceptable salt thereof, wherein:
**R^{s}-B** is selected from the group consisting of: 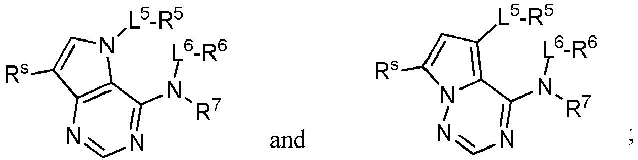
R^{s} is represented by the formula: 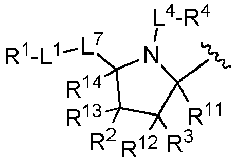
L¹ is a bond;
L⁴, L⁵, and L⁶, each independently, are a bond or -C(R⁰)₂-O-;
L⁷ is O, -CH₂-, -CH(C₁-C₆alkyl)-, -C(R⁰)₂-S-, or -C(R⁰)₂-NH-;
R⁰, independently for each occurrence, is H or C₁-C₆alkyl;
R¹ is selected from the group consisting of H, C₁-C₆alkyl, aminoacyl, aminothionyl, C₁-C₆acyl, R¹⁰OC(O)-, phosphoryl, and aminophosphoryl;
R² and R³ are each independently H, halide, C₁-C₆alkyl, C₁-C₆alkenyl, C₁-C₆alkynyl, CN, OR⁰, R¹⁰OC(O)-, or SR⁰;
or R¹ and R², taken together, or R² and R³, taken together, may be selected from the group consisting of -OC(O)O-, -OC(S)O-, phosphoryl, and C₁-C₆alkylphosphoryl;
or R² and R³, taken together, may form a bond between the carbon atoms to which they are attached;
R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
R¹⁰, independently for each occurrence, is selected from the group consisting of H, C₁-C₆alkyl, aryl, heteroaryl, aralkyl, and heteroaralkyl;
R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
R⁷ is H; or R⁶, R⁷, and the nitrogen to which they are bonded, taken together, represent -N=CR²⁰R²¹;
R²⁰ and R²¹, each independently, are selected from the group consisting of H, C₁-C₆alkyl, amino, aryl, heteroaryl, aralkyl, and heteroaralkyl;
R¹¹ is selected from the group consisting of H, C₁-C₆alkyl, C₁-C₆alkenyl, C₁-C₆alkynyl, OR⁰, CN, and halide;
R¹² and R¹³ are each independently selected from the group consisting of H, C₁-C₆alkyl, C₁-C₆alkenyl, C₁-C₆alkynyl, OR⁰, CN, azide, and halide; and
R¹⁴ is selected from the group consisting of H, C₁-C₆alkyl, OR⁰, C₁-C₆alkynyl, CN, azide, and halide;
(II) **R^{s}-B**, or a pharmaceutically acceptable salt thereof, wherein:
**R^{s}-B** is selected from the group consisting of: 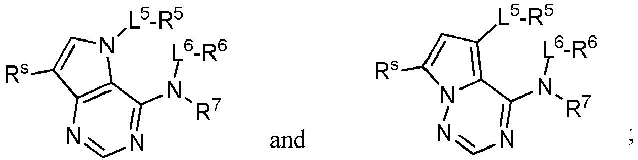
R^{s} is represented by the formula: 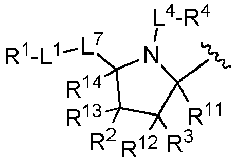
L¹, L⁴, L⁵, and L⁶, each independently, are a bond or -C(R⁰)₂-O-;
L⁷ is a bond, O, -C(R⁰)₂-O-, -C(R⁰)₂-S-, or -C(R⁰)₂-NH-;
R⁰, independently for each occurrence, is H or C₁-C₆alkyl;
R¹ is selected from the group consisting of H, C₁-C₆alkyl, aminoacyl, aminothionyl, C₁-C₆acyl, R¹⁰OC(O)-, phosphoryl, and aminophosphoryl;
R² and R³ are each independently H, halide, C₁-C₆alkyl, C₁-C₆alkenyl, C₁-C₆alkynyl, CN, OR⁰, R¹⁰OC(O)-, or SR⁰;
or R¹ and R², taken together, or R² and R³, taken together, may be selected from the group consisting of -OC(O)O-, -OC(S)O-, phosphoryl, and C₁-C₆alkylphosphoryl;
or R² and R³, taken together, may form a bond between the carbon atoms to which they are attached;
R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
R¹⁰, independently for each occurrence, is selected from the group consisting of H, C₁-C₆alkyl, aryl, heteroaryl, aralkyl, and heteroaralkyl;
R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
R⁷ is H; or R⁶, R⁷, and the nitrogen to which they are bonded, taken together, represent -N=CR²⁰R²¹;
R²⁰ and R²¹, each independently, are selected from the group consisting of H, C₁-C₆alkyl, amino, aryl, heteroaryl, aralkyl, and heteroaralkyl;
R¹¹ is selected from the group consisting of C₁-C₆alkyl, C₁-C₆alkenyl, C₁-C₆alkynyl, OR⁰, and CN;
R¹² and R¹³ are each independently selected from the group consisting of H, C₁-C₆alkyl, C₁-C₆alkenyl, C₁-C₆alkynyl, OR⁰, CN, azide, and halide; and
R¹⁴ is selected from the group consisting of H, C₁-C₆alkyl, OR⁰, C₁-C₆alkynyl, CN, azide, and halide;
(III) **R^{s}-B**, or a pharmaceutically acceptable salt thereof, wherein:
**R^{s}-B** is selected from the group consisting of: 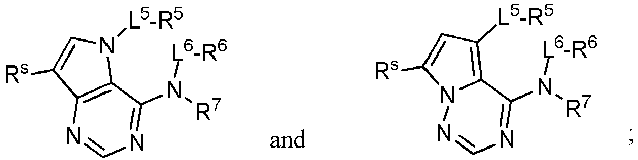
R^{s} is represented by the formula: 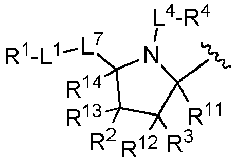
L¹, L⁴, L⁵, and L⁶, each independently, are a bond or -C(R⁰)₂-O-;
L⁷ is a bond, O, -C(R⁰)₂-O-, -C(R⁰)₂-S-, or -C(R⁰)₂-NH-;
R⁰, independently for each occurrence, is H or C₁-C₆alkyl;
R¹ is selected from the group consisting of H, C₁-C₆alkyl, aminoacyl, aminothionyl, C₁-C₆acyl, R¹⁰OC(O)-, phosphoryl, and aminophosphoryl;
R² is H, halide, C₁-C₆alkyl, C₁-C₆alkenyl, C₁-C₆alkynyl, CN, OR⁰, R¹⁰OC(O)-, or SR⁰;
R³ is H, halide, C₁-C₆alkyl, C₁-C₆alkenyl, C₁-C₆alkynyl, CN, R¹⁰OC(O)-, or SR⁰;
or R¹ and R², taken together, or R² and R³, taken together, may be selected from the group consisting of -OC(O)O-, -OC(S)O-, phosphoryl, and C₁-C₆alkylphosphoryl;
or R² and R³, taken together, may form a bond between the carbon atoms to which they are attached;
R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
R¹⁰, independently for each occurrence, is selected from the group consisting of H, C₁-C₆alkyl, aryl, heteroaryl, aralkyl, and heteroaralkyl;
R⁵ and R⁶ are, each independently, selected from the group consisting of H, C₁-C₆alkyl, C₃-C₈cycloalkyl, alkenyl, alkynyl, C₁-C₆acyl, alkylthio, aryl, aralkyl, heteroaryl, and heteroaralkyl; or R⁵ may be absent;
R⁷ is H; or R⁶, R⁷, and the nitrogen to which they are bonded, taken together, represent -N=CR²⁰R²¹;
R²⁰ and R²¹, each independently, are selected from the group consisting of H, C₁-C₆alkyl, amino, aryl, heteroaryl, aralkyl, and heteroaralkyl;
R¹¹ is selected from the group consisting of H, C₁-C₆alkyl, C₁-C₆alkenyl, C₁-C₆alkynyl, OR⁰, and CN;
R¹² is selected from the group consisting of H, C₁-C₆alkyl, C₁-C₆alkenyl, C₁-C₆alkynyl, CN, azide, and halide;
R¹³ is selected from the group consisting of H, C₁-C₆alkyl, C₁-C₆alkenyl, C₁-C₆alkynyl, OR⁰, CN, azide, and halide; and
R¹⁴ is selected from the group consisting of H, C₁-C₆alkyl, OR⁰, C₁-C₆alkynyl, CN, azide, and halide; or
(IV) **R^{s}-B**, or a pharmaceutically acceptable salt thereof, wherein:
**R^{s}-B** is selected from the group consisting of: 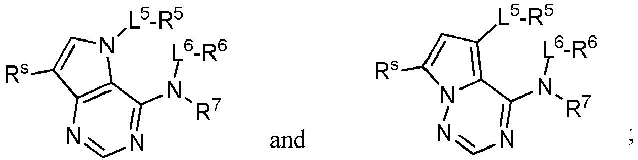
R^{s} is represented by the formula: 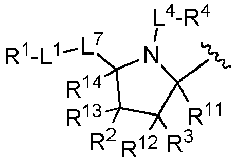
L¹, L⁴, L⁵, and L⁶, each independently, are a bond or -C(R⁰)₂-O-;
L⁷ is a bond, O, -C(R⁰)₂-O-, -C(R⁰)₂-S-, or -C(R⁰)₂-NH-;
R⁰, independently for each occurrence, is H or C₁-C₆alkyl;
R¹ is selected from the group consisting of H, C₁-C₆alkyl, aminoacyl, aminothionyl, C₁-C₆acyl, R¹⁰OC(O)-, phosphoryl, and aminophosphoryl;
R² is H, halide, C₁-C₆alkyl, C₁-C₆alkenyl, C₁-C₆alkynyl, CN, R¹⁰OC(O)-, or SR⁰;
R³ is H, halide, C₁-C₆alkyl, C₁-C₆alkenyl, C₁-C₆alkynyl, CN, OR⁰, R¹⁰OC(O)-, or SR⁰;
or R¹ and R², taken together, or R² and R³, taken together, may be selected from the group consisting of -OC(O)O-, -OC(S)O-, phosphoryl, and C₁-C₆alkylphosphoryl;
or R² and R³, taken together, may form a bond between the carbon atoms to which they are attached;
R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
R¹⁰, independently for each occurrence, is selected from the group consisting of H, C₁-C₆alkyl, aryl, heteroaryl, aralkyl, and heteroaralkyl;
R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or R⁵ may be absent;
R⁷ is H; or R⁶, R⁷, and the nitrogen to which they are bonded, taken together, represent -N=CR²⁰R²¹;
R²⁰ and R²¹, each independently, are selected from the group consisting of H, C₁-C₆alkyl, amino, aryl, heteroaryl, aralkyl, and heteroaralkyl;
R¹¹ is selected from the group consisting of H, C₁-C₆alkyl, C₁-C₆alkenyl, C₁-C₆alkynyl, OR⁰, and CN;
R¹² is selected from the group consisting of C₁-C₆alkyl, OR⁰, alkynyl, CN, azide, and halide;
R¹³ is selected from the group consisting of H, C₁-C₆alkyl, C₁-C₆alkenyl, C₁-C₆alkynyl, CN, azide, and halide; and
R¹⁴ is selected from the group consisting of H, C₁-C₆alkyl, OR⁰, C₁-C₆alkynyl, CN, azide, and halide.

3. A compound represented by:
(I) **R^{s}-B**, or a pharmaceutically acceptable salt thereof, wherein:
**R^{s}-B** is selected from the group consisting of: 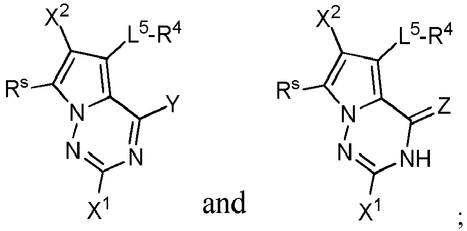
R^{s} is selected from the group consisting of: 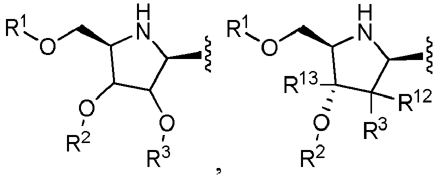 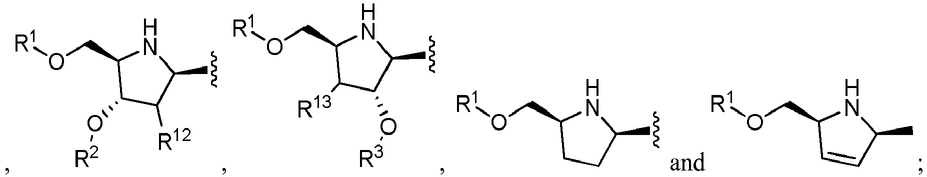
R¹ is selected from the group consisting of H, C₁-C₆alkyl, aminoacyl, aminothionyl, C₁-C₆acyl, R¹⁰OC(O)-, phosphoryl, and aminophosphoryl;
R² and R³ are each independently H, C₁-C₆alkyl, C₁-C₆alkenyl, or C₁-C₆alkynyl;
R¹⁰, independently for each occurrence, is selected from the group consisting of H, C₁-C₆alkyl, aryl, heteroaryl, aralkyl, and heteroaralkyl;
R¹² and R¹³ are each independently selected from the group consisting of H, C₁-C₆alkyl, C₁-C₆alkenyl, C₁-C₆alkynyl, OR⁰, CN, azide, and halide;
L⁵ is a bond or -C(R⁰)₂-O-;
R⁰, independently for each occurrence, is H or C₁-C₆alkyl;
R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
X¹ is selected from the group consisting of H, N(R⁰)₂, SH, thioalkyl, OR⁰, alkenyl, alkynyl, CN, and halide;
X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
Y is selected from the group consisting of H, OR⁰, N(R⁵)(R⁶), SH, thioalkyl, O-aryl, O-heteroaryl, and halide;
Z is selected from the group consisting of O and S;
R^{s} and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
R⁵, R⁶, and the nitrogen to which they are bonded, taken together,
represent -N=CR²⁰R²¹; and
R²⁰ and R²¹, each independently, are selected from the group consisting of H, C₁-C₆alkyl, amino, aryl, heteroaryl, aralkyl, and heteroaralkyl;
(II) **R^{s}-B,** or a pharmaceutically acceptable salt thereof, wherein:
**R^{s}-B** is selected from the group consisting of: 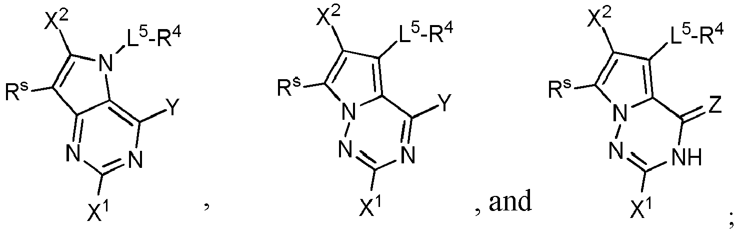
R^{s} is selected from the group consisting of: 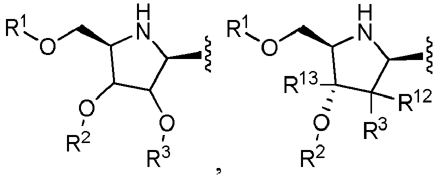 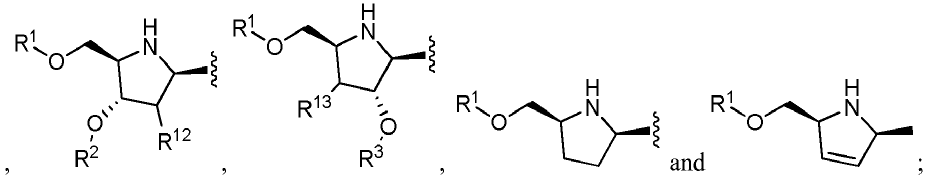
R¹ is selected from the group consisting of H, C₁-C₆alkyl, aminoacyl, aminothionyl, C₁-C₆acyl, R¹⁰OC(O)-, phosphoryl, and aminophosphoryl;
R² and R³ are each independently H, C₁-C₆alkyl, C₁-C₆alkenyl, or C₁-C₆alkynyl;
R¹⁰, independently for each occurrence, is selected from the group consisting of H, C₁-C₆alkyl, aryl, heteroaryl, aralkyl, and heteroaralkyl;
R¹² and R¹³ are each independently selected from the group consisting of H, C₁-C₆alkyl, C₁-C₆alkenyl, C₁-C₆alkynyl, OR⁰, CN, azide, and halide;
L⁵ is a bond or -C(R⁰)₂-O-;
R⁰, independently for each occurrence, is H or C₁-C₆alkyl;
R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
X¹ is selected from the group consisting of H, N(R⁰)₂, SH, thioalkyl, OR⁰, alkenyl, alkynyl, CN, and halide;
X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
Y is selected from the group consisting of H, OR⁰, N(R⁵)(R⁶), SH, thioalkyl, O-aryl, O-heteroaryl, and halide;
Z is selected from the group consisting of O and S; and
R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl, provided that both R⁵ and R⁶ are not H;
(III) **R^{s}-B,** or a pharmaceutically acceptable salt thereof, wherein:
**R^{s}-B** is selected from the group consisting of: 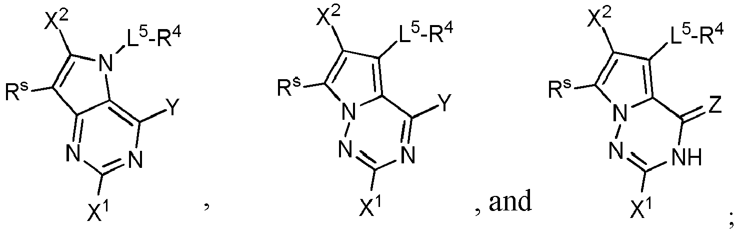
R^{s} is selected from the group consisting of: 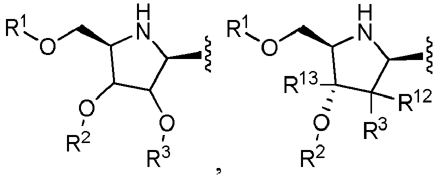 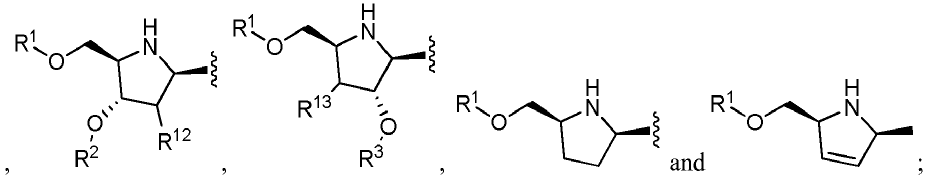
R¹ is selected from the group consisting of H, C₁-C₆alkyl, aminoacyl, aminothionyl, C₁-C₆acyl, R¹⁰OC(O)-, phosphoryl, and aminophosphoryl;
R¹⁰, independently for each occurrence, is selected from the group consisting of H, C₁-C₆alkyl, aryl, heteroaryl, aralkyl, and heteroaralkyl;
R² and R³ are each independently H, C₁-C₆alkyl, C₁-C₆alkenyl, or C₁-C₆alkynyl;
R¹² and R¹³ are each independently selected from the group consisting of H, C₁-C₆alkyl, C₁-C₆alkenyl, C₁-C₆alkynyl, OR⁰, CN, azide, and halide;
R⁰, independently for each occurrence, is H or C₁-C₆alkyl;
L⁵ is a bond or -C(R⁰)₂-O-;
R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
X¹ is selected from the group consisting of H, N(R⁰)₂, SH, thioalkyl, OR⁰, alkenyl, alkynyl, CN, and halide;
X² is selected from the group consisting of N(R⁰)₂, SH, thioalkyl, OR⁰, O-aryl, O-heteroaryl, alkenyl, alkynyl, C₁-C₆acyl, carboxyl, CN, azide, and halide;
Y is selected from the group consisting of H, OR⁰, N(R⁵)(R⁶), SH, thioalkyl, O-aryl, O-heteroaryl, and halide;
Z is selected from the group consisting of O and S;
R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
R⁵, R⁶, and the nitrogen to which they are bonded, taken together,
represent -N=CR²⁰R²¹; and
R²⁰ and R²¹, each independently, are selected from the group consisting of H, C₁-C₆alkyl, amino, aryl, heteroaryl, aralkyl, and heteroaralkyl; or
(IV) **R^{s}-B,** or a pharmaceutically acceptable salt thereof, wherein:
**R^{s}-B** is selected from the group consisting of: 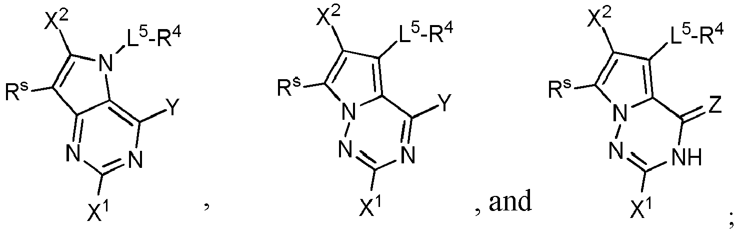
R^{s} is selected from the group consisting of: 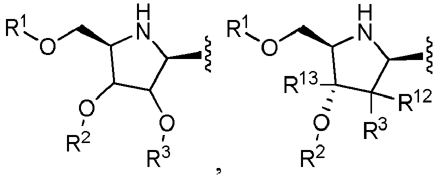 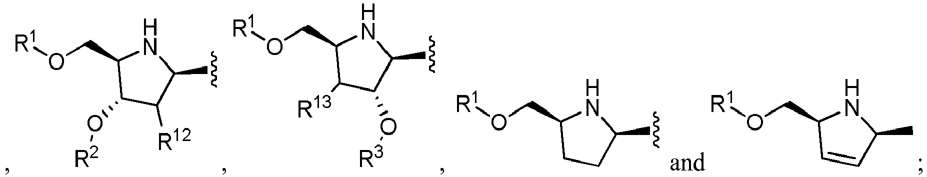
R¹ is selected from the group consisting of H, C₁-C₆alkyl, aminoacyl, aminothionyl, C₁-C₆acyl, R¹⁰OC(O)-, phosphoryl, and aminophosphoryl;
R² and R³ are each independently H, C₁-C₆alkyl, C₁-C₆alkenyl, or C₁-C₆alkynyl;
R¹⁰, independently for each occurrence, is selected from the group consisting of H, C₁-C₆alkyl, aryl, heteroaryl, aralkyl, and heteroaralkyl;
R¹² and R¹³ are each independently selected from the group consisting of H, C₁-C₆alkyl, C₁-C₆alkenyl, C₁-C₆alkynyl, OR⁰, CN, azide, and halide;
R⁰, independently for each occurrence, is H or C₁-C₆alkyl;
L⁵ is a bond or -C(R⁰)₂-O-;
R⁴ is selected from the group consisting of C₁-C₆alkyl, alkenyl, alkynyl, aryl, heteroaryl, aralkyl, heteroaralkyl, C₁-C₆acyl, or carboxyl; or R⁴ may be absent;
X¹ is selected from the group consisting of N(R⁰)₂, SH, thioalkyl, OR⁰, alkenyl, alkynyl, CN, and halide;
X² is selected from the group consisting of H, N(R⁰)₂, SH, thioalkyl, OR⁰, O-aryl, O-heteroaryl, alkenyl, alkynyl, C₁-C₆acyl, carboxyl, CN, azide, and halide;
Y is selected from the group consisting of H, OR⁰, N(R⁵)(R⁶), SH, thioalkyl, O-aryl, O-heteroaryl, and halide;
Z is selected from the group consisting of O and S;
R⁵ and R⁶ are, each independently, selected from the group consisting of H, C₁-C₆alkyl, C₃-C₈cycloalkyl, C₁-C₆alkenyl, C₁-C₆alkynyl, C₁-C₆acyl, alkylthio, aryl, aralkyl, heteroaryl, and heteroaralkyl; or
R⁵, R⁶, and the nitrogen to which they are bonded, taken together,
represent -N=CR²⁰R²¹; and
R²⁰ and R²¹, each independently, are selected from the group consisting of H, C₁-C₆alkyl, amino, aryl, heteroaryl, aralkyl, and heteroaralkyl.

4. The compound of claim 2 or 3, wherein R^{s} is selected from the group consisting of:

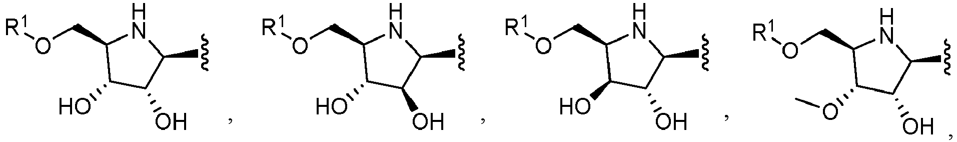

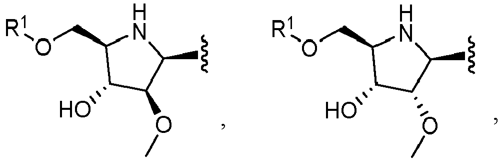

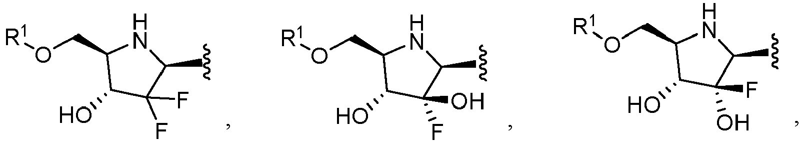

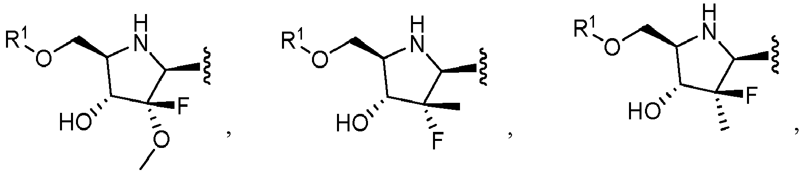

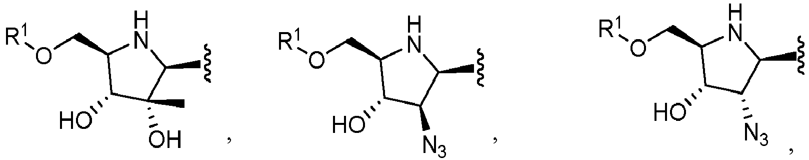

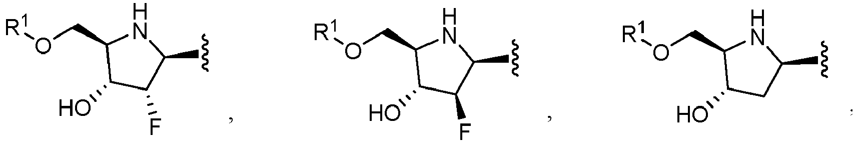

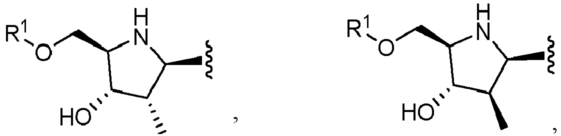

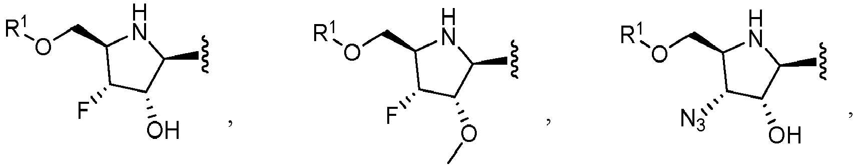

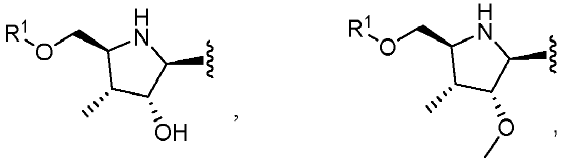

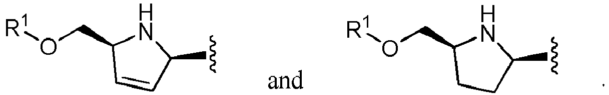

5. The compound of claim 2, wherein L⁵-R⁵ is H; and/or wherein L⁴-R⁴ is H; and/or
wherein L⁷ is -CH(C₁-C₆alkyl)-; and/or
wherein R¹ is selected from the group consisting of phosphate, pyrophosphate, phosphoramidite, phosphite, and phosphonate; optionally wherein R¹ is phosphoryl or aminophosphoryl; and preferably
wherein R¹ is aminophosphoryl;
aminophosphoryl is -P(=O)(OR⁵⁰)NR⁵¹R⁵²;
R⁵⁰ is selected from the group consisting of H, (C₁-C₆)alkyl, aryl, arylalkyl, heteroaryl, heteroaralkyl, and -(CH₂)ₘSC(=O)C(CH₃)₂CH₂OH;
m is 1 or 2;
R⁵¹ is H or C₁-C₆alkyl;
R⁵² is selected from the group consisting of H, C₁-C₆alkyl, aryl, aralkyl, heteroaryl, heteroaralkyl, and -CR⁶⁰R⁶¹C(=O)OR⁶²;
R⁶⁰ and R⁶¹ each independently are H or C₁-C₆alkyl; and
R⁶² is selected from the group consisting of H, C₁-C₆alkyl, aryl, aralkyl, heteroaryl, and heteroaralkyl.

6. The compound of claim 2, wherein R¹², R¹³, and R¹⁴ are H; optionally wherein R¹¹, R¹² R¹³, and R¹⁴ are H.

7. A compound represented by:
(I) **R^{s}-B,** or a pharmaceutically acceptable salt thereof, wherein:
**R^{s}-B** is selected from the group consisting of: 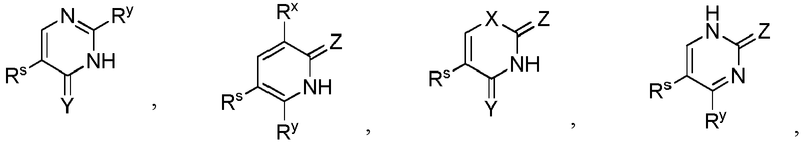 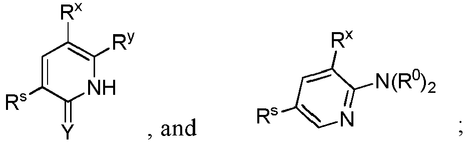
X is selected from the group consisting of O, S, and NR¹⁰;
R¹⁰, independently for each occurrence, is selected from the group consisting of H, C₁-C₆alkyl, aryl, heteroaryl, aralkyl, and heteroaralkyl;
R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
R⁰, independently for each occurrence, is H or C₁-C₆alkyl;
R^{y} is selected from the group consisting of H, C₁-C₆alkyl, OR⁰, C₁-C₆alkenyl, C₁-C₆alkynyl, CN, N(R⁰)₂, and halide;
Y is selected from the group consisting of O and S;
Z is selected from the group consisting of O and S;
R^{s} is represented by the formula: 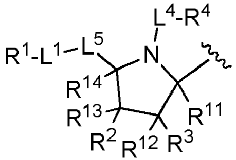
L¹ and L⁴, each independently, are a bond or -C(R⁰)₂-O-;
L⁵ is a bond, O, -C(R⁰)₂-O-, -C(R⁰)₂-S-, or -C(R⁰)₂-NH-;
R¹ is selected from the group consisting of H, C₁-C₆alkyl, aminoacyl, aminothionyl, C₁-C₆acyl, R¹⁰OC(O)-, phosphoryl, and aminophosphoryl;
R² and R³ are each independently H, halide, azide, C₁-C₆alkyl, C₁-C₆alkenyl, C₁-C₆alkynyl, CN, OR⁰, R¹⁰OC(O)-, or SR⁰;
or R¹ and R², taken together, or R² and R³, taken together, may be selected from the group consisting of -OC(O)O-, -OC(S)O-, phosphoryl, and C₁-C₆alkylphosphoryl;
or R² and R³, taken together, may form a bond between the carbon atoms to which they are attached;
R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
R¹¹ is selected from the group consisting of H, C₁-C₆alkyl, C₁-C₆alkenyl, C₁-C₆alkynyl, OR⁰, and CN;
R¹² and R¹³ are each independently selected from the group consisting of H, C₁-C₆alkyl, OR⁰, C₁-C₆alkenyl, C₁-C₆alkynyl, CN, azide, and halide; and
R¹⁴ is selected from the group consisting of H, C₁-C₆alkyl, OR⁰, C₁-C₆alkynyl, CN, azide, and halide;
(II) **R^{s}-B,** or a pharmaceutically acceptable salt thereof, wherein:
**R^{s}-B** is selected from the group consisting of: 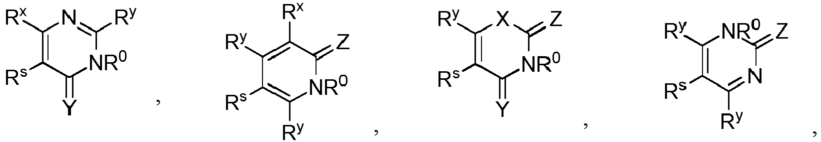 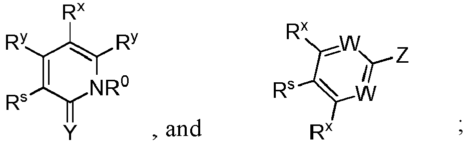
W, independently for each occurrence, is CR^{x} or N;
X is selected from the group consisting of O, S, and NR¹⁰;
R¹⁰, independently for each occurrence, is selected from the group consisting of H, C₁-C₆alkyl, aryl, heteroaryl, aralkyl, and heteroaralkyl;
R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
R⁰, independently for each occurrence, is H or C₁-C₆alkyl;
R^{y} is selected from the group consisting of H, C₁-C₆alkyl, OR⁰, C₁-C₆alkenyl, C₁-C₆alkynyl, CN, N(R⁰)₂, and halide;
Y is selected from the group consisting of O and S;
Z is selected from the group consisting of O and S;
R^{s} is represented by the formula: 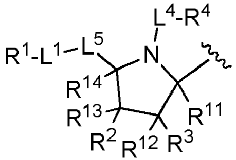
L¹ and L⁴, each independently, are a bond or -C(R⁰)₂-O-;
L⁵ is a bond, O, -C(R⁰)₂-O-, -C(R⁰)₂-S-, or -C(R⁰)₂-NH-;
R¹ is selected from the group consisting of H, C₁-C₆alkyl, aminoacyl, aminothionyl, C₁-C₆acyl, R¹⁰OC(O)-, phosphoryl, and aminophosphoryl;
R² and R³ are each independently H, halide, azide, C₁-C₆alkyl, C₁-C₆alkenyl, C₁-C₆alkynyl, CN, OR⁰, R¹⁰OC(O)-, or SR⁰;
or R¹ and R², taken together, or R² and R³, taken together, may be selected from the group consisting of -OC(O)O-, -OC(S)O-, phosphoryl, and C₁-C₆alkylphosphoryl;
or R² and R³, taken together, may form a bond between the carbon atoms to which they are attached;
R⁴ is, independently for each occurrence, selected from the group consisting of H, C₁-C₆alkyl, C₃-C₈cycloalkyl, C₁-C₆alkenyl, C₁-C₆alkynyl, C₁-C₆acyl, phosphoryl, aryl, aralkyl, heteroaryl, and heteroaralkyl;
R¹¹ is selected from the group consisting of H, C₁-C₆alkyl, C₁-C₆alkenyl, C₁-C₆alkynyl, OR⁰, and CN;
R¹² and R¹³ are each independently selected from the group consisting of H, C₁-C₆alkyl, C₁-C₆alkenyl, C₁-C₆alkynyl, OR⁰, CN, azide, and halide; and
R¹⁴ is selected from the group consisting of H, C₁-C₆alkyl, OR⁰, C₁-C₆alkynyl, CN, azide, and halide; or
(III) **R^{s}-B,** or a pharmaceutically acceptable salt thereof, wherein:
**R^{s}-B** is selected from the group consisting of: 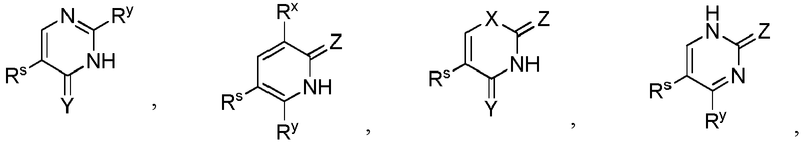 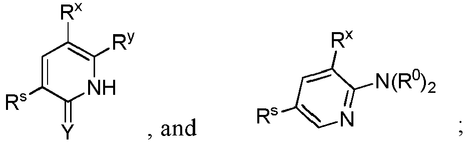
X is selected from the group consisting of O, S, and NR¹⁰;
R¹⁰, independently for each occurrence, is selected from the group consisting of H, C₁-C₆alkyl, aryl, heteroaryl, aralkyl, and heteroaralkyl;
R^{x} is selected from the group consisting of H, C₁-C₆alkyl, OR⁰, C₁-C₆alkenyl, C₁-C₆alkynyl, CN, N(R⁰)₂, NO₂, azide, halide, aryl, and heteroaryl;
R⁰, independently for each occurrence, is H or C₁-C₆alkyl;
R^{y} is selected from the group consisting of H, C₁-C₆alkyl, OR⁰, C₁-C₆alkenyl, C₁-C₆alkynyl, CN, N(R⁰)₂, and halide;
Y is selected from the group consisting of O and S;
Z is selected from the group consisting of O and S;
R^{s} is selected from the group consisting of: 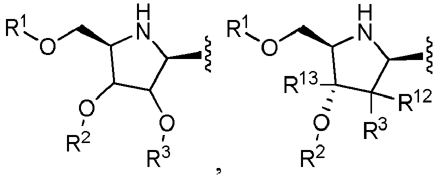 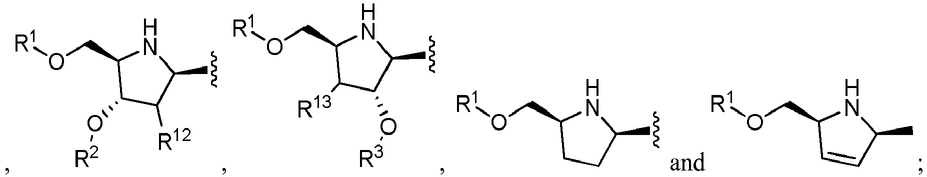
R¹ is selected from the group consisting of H, C₁-C₆alkyl, aminoacyl, aminothionyl, C₁-C₆acyl, R¹⁰OC(O)-, phosphoryl, and aminophosphoryl;
R² and R³ are each independently H, C₁-C₆alkyl, C₁-C₆alkenyl, or C₁-C₆alkynyl; and
R¹² and R¹³ are each independently selected from the group consisting of H, C₁-C₆alkyl, C₁-C₆alkenyl, C₁-C₆alkynyl, OR⁰, CN, azide, and halide.

8. The compound of claim 7, wherein R^{s} is selected from the group consisting of:

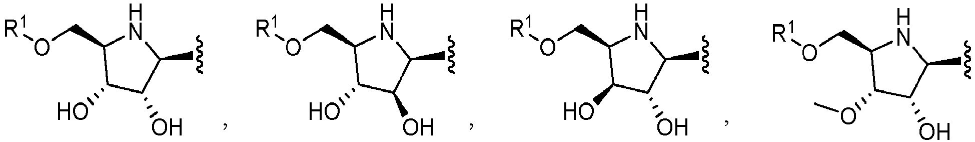

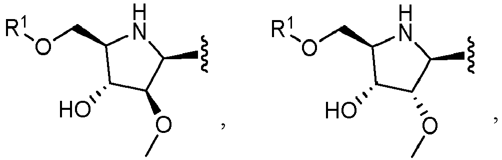

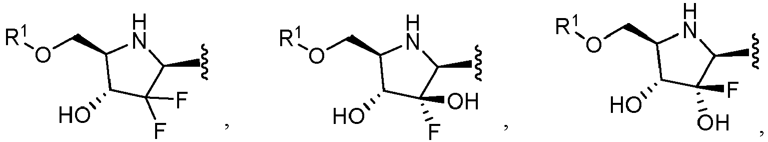

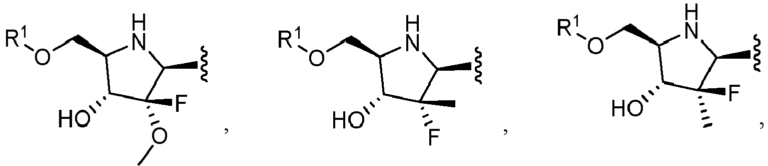

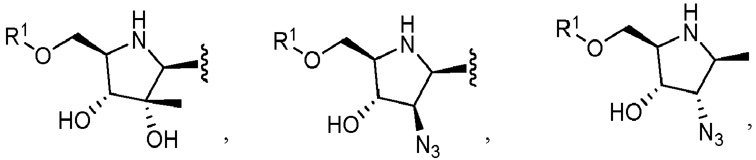

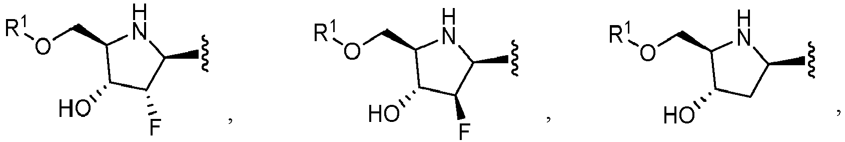

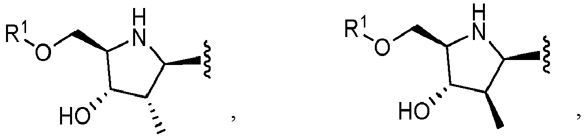

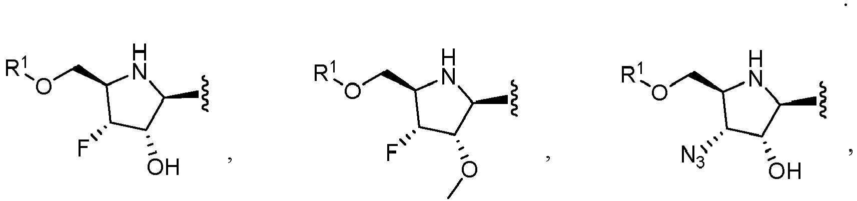

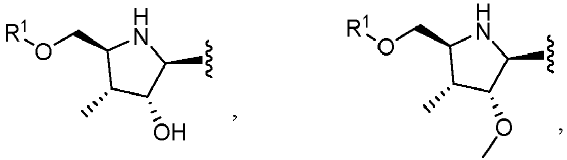

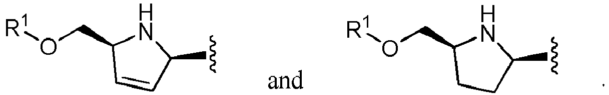

9. The compound of claim 7 or 8, wherein R¹ is H, methyl, or ethyl; or
R¹ is phosphate, pyrophosphate, phosphoramidite, phosphite, or phosphonate; or
R¹ is phosphoryl or aminophosphoryl.

10. The compound of claim 9, wherein R¹ is aminophosphoryl;
aminophosphoryl is -P(=O)(OR⁵⁰)NR⁵¹R⁵²;
R⁵⁰ is selected from the group consisting of H, (C₁-C₆)alkyl, aryl, arylalkyl, heteroaryl, heteroaralkyl, and -(CH₂)ₘSC(=O)C(CH₃)₂CH₂OH;
m is 1 or 2;
R⁵¹ is H or C₁-C₆alkyl;
R⁵² is selected from the group consisting of H, C₁-C₆alkyl, aryl, aralkyl, heteroaryl, heteroaralkyl, and -CR⁶⁰R⁶¹C(=O)OR⁶²;
R⁶⁰ and R⁶¹ each independently are H or C₁-C₆alkyl; and
R⁶² is selected from the group consisting of H, C₁-C₆alkyl, aryl, aralkyl, heteroaryl, and heteroaralkyl.

11. The compound of any one of claims 7-10, wherein **R^{s}-B** is selected from the group consisting of: 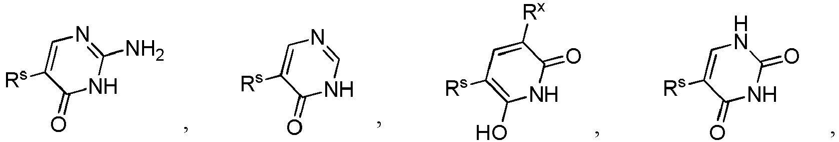 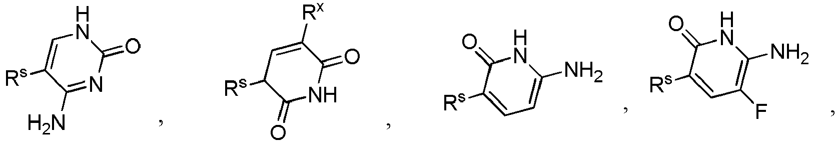 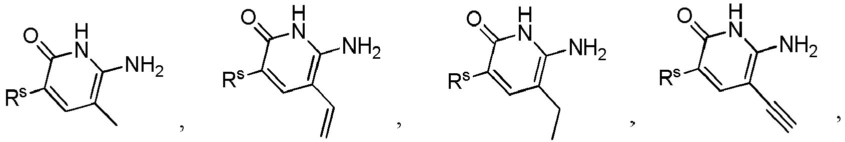 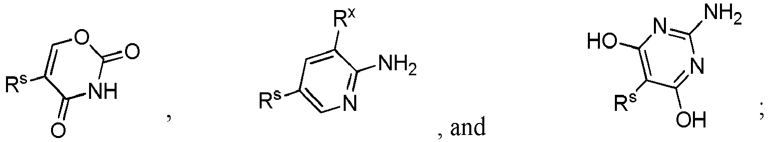 and
R^{x} is selected from the group consisting of H, C₁-C₆alkyl, OR⁰, C₁-C₆alkenyl, C₁-C₆alkynyl, CN, NH₂, aminoalkyl, NO₂, azide, halide, aryl, and heteroaryl.

12. The compound of any one of claims 7-11, wherein R^{x} is F, Cl, Br, or I; or
R^{x} is H, methyl, ethyl, or propyl; or
R^{x} is ethenyl or ethynyl; or
R^{x} is CN, NH₂, or aminoalkyl.

13. The compound of any one of claims 2-12, wherein R⁵⁰ is H; or
R⁵⁰ is aryl; or
R⁵⁰ is -(CH₂)ₘSC(=O)C(CH₃)₂CH₂OH; and or
wherein m is 2; and/or
wherein R⁵¹ is H; and/or
wherein R⁵² is aralkyl; or
R⁵² is -CR⁶⁰R⁶¹C(=O)OR⁶²; and/or
wherein R⁶⁰ is H; R⁶¹ is (C₁-C₆)alkyl; and R⁶² is (C₁-C₆)alkyl.

14. The compound of any one of claims 2-13, wherein R¹ is selected from the group consisting of: 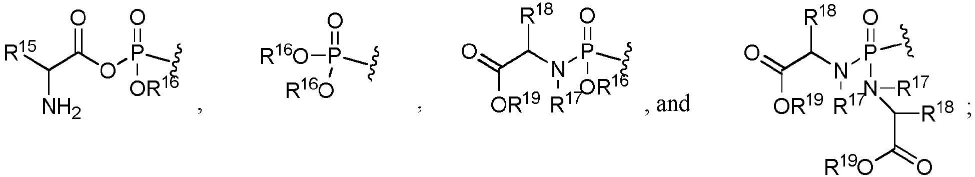 wherein:
R¹⁵ is selected from the group consisting of H, C₁-C₆alkyl, C₁-C₆haloalkyl, aminoalkyl,
thioalkyl, C₃-C₈cycloalkyl, C₂-C₇heterocyclyl, aryl, heteroaryl, aralkyl, heteroaralkyl, and naturally-occurring amino acid side chains;
R¹⁶ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl; or R¹⁶ taken together with the oxygen to which it is bonded may be a naturally-occurring amino acid;
R¹⁷ is, independently for each occurrence, selected from the group consisting of H,
C₁-C₆alkyl, C₁-C₆haloalkyl, C₃-C₈cycloalkyl, C₂-C₇heterocyclyl, aryl, heteroaryl, aralkyl, heteroaralkyl, and acyl;
R¹⁸ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, heteroaralkyl, and naturally-occurring amino acid side chains; or R¹⁷ and R¹⁸, taken together, may form a 4-, 5-, or 6-membered ring; and
R¹⁹ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl.

15. A compound, or a pharmaceutically acceptable salt thereof, selected from the group consisting of: 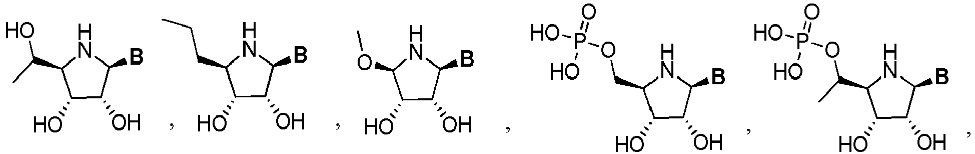 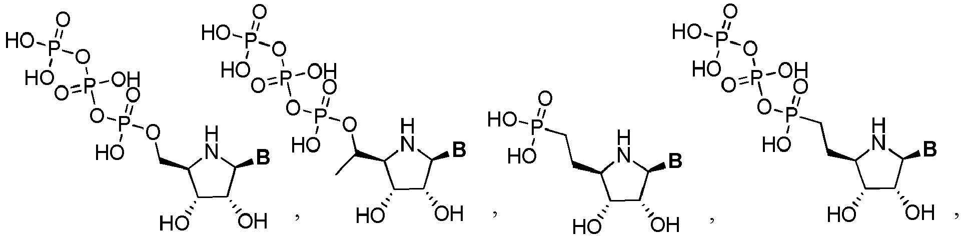 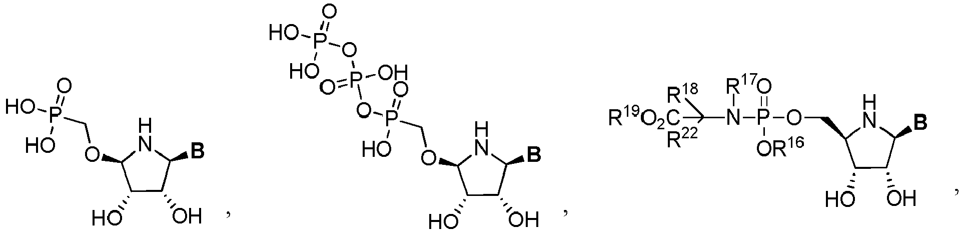 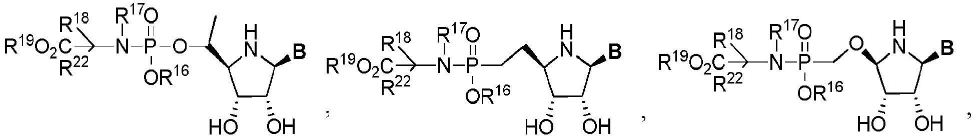 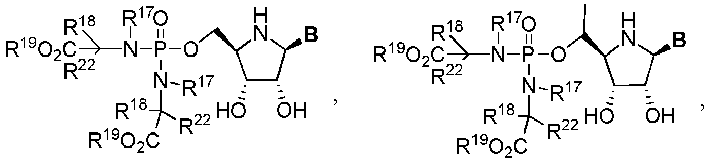 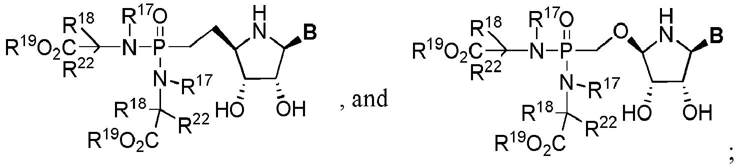 wherein:
R¹⁶ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl; or R¹⁶ taken together with the oxygen to which it is bonded may be a naturally-occurring amino acid;
R¹⁷ is, independently for each occurrence, selected from the group consisting of H,
C₁-C₆alkyl, C₁-C₆haloalkyl, C₃-C₈cycloalkyl, C₂-C₇heterocyclyl, aryl, heteroaryl, aralkyl, heteroaralkyl, and acyl;
R¹⁸ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, heteroaralkyl, and naturally-occurring amino acid side chains;
R¹⁹ is, independently for each occurrence, selected from the group consisting of H,
C₁-C₆alkyl, C₁-C₆haloalkyl, aminoalkyl, thioalkyl, C₃-C₈cycloalkyl, C₂-C₇heterocyclyl, aryl, heteroaryl, aralkyl, and heteroaralkyl;
R²² is, independently for each occurrence, selected from the group consisting of H,
C₁-C₆alkyl, C₁-C₆haloalkyl, C₃-C₈cycloalkyl, C₂-C₇heterocyclyl, aryl, heteroaryl, aralkyl, and heteroaralkyl; or R¹⁷ and R²², taken together, may form a 4-, 5-, or 6-membered ring; and
**B** is selected from the group consisting of:

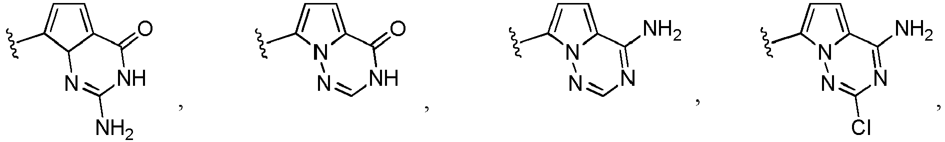

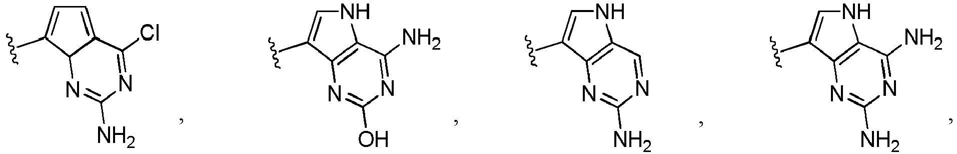

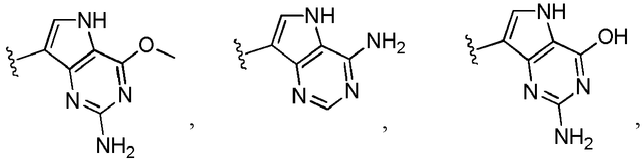

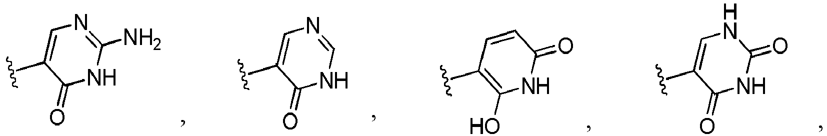

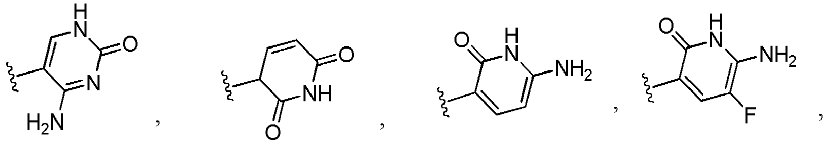

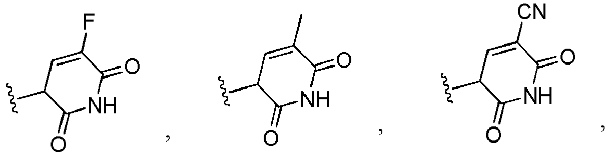

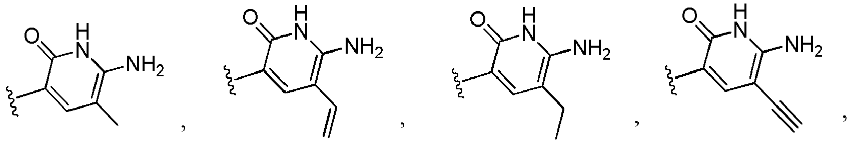

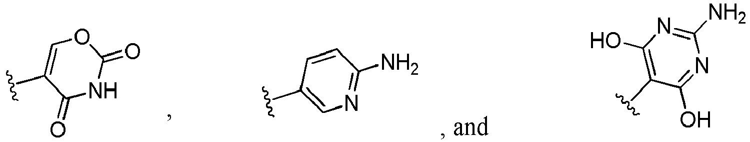

16. A pharmaceutical composition, comprising a compound of any one of claims 1-15 , or a pharmaceutically acceptable salt thereof, and a pharmaceutically acceptable carrier.

17. The compound of any one of claims 1-15 for use in treating a viral infection in a subject.

18. The compound for use according to claim 17, wherein the virus is selected from the group consisting of RNA viruses; or
wherein the virus is selected from the group consisting of orthomyxoviridae, paramyxoviridae, arenaviridae, bunyaviridae, flaviviridae, filoviridae, togaviridae, picornaviridae, and coronaviridae; or
wherein the virus is selected from the group consisting of adenovirus, rhinovirus, hepatitis A virus, hepatitis C virus, polio virus, measles virus, Ebola virus, Coxsackie virus, West Nile virus, smallpox virus, yellow fever virus, Dengue Fever virus, influenza A virus, influenza B virus, lassa virus, lymphocytic choriomeningitis virus, Junin virus, machuppo virus, guanarito virus, hantavirus, Rift Valley Fever virus, La Crosse virus, California encephalitis virus, Crimean-Congo virus, Marburg virus, Japanese encephalitis virus, Kyasanur Forest virus, Venezuelan equine encephalitis virus, Eastern equine encephalitis virus, Western equine encephalitis virus, severe acute respiratory syndrome (SARS) virus, parainfluenza virus, respiratory syncytial virus, Punta Toro virus, Tacaribe virus, and Pichinde virus; or
wherein the virus is selected from the group consisting of adenovirus, Dengue Fever virus, Marburg virus, influenza A virus, influenza B virus, Junin virus, measles virus, parainfluenza virus, Pichinde virus, Punta Toro virus, respiratory syncytial virus, rhinovirus, Rift Valley Fever virus, SARS virus, Tacaribe virus, Venezuelan equine encephalitis virus, West Nile virus, and yellow fever virus; or
wherein the virus is selected from the group consisting of Ebola virus, yellow fever virus, Marburg virus, influenza A virus, and influenza B virus.
